(19) **Europäisches Patentamt / European Patent Office / Office européen des brevets**

(11) **EP 4 197 545 A1**

(12) **EUROPEAN PATENT APPLICATION**
published in accordance with Art. 153(4) EPC

(43) Date of publication:
**21.06.2023 Bulletin 2023/25**

(21) Application number: **21850145.0**

(22) Date of filing: **30.07.2021**

(51) International Patent Classification (IPC):
**A61K 35/12** (2015.01)   **A61K 39/395** (2006.01)
**A61P 35/00** (2006.01)   **A61P 43/00** (2006.01)
**C07K 14/705** (2006.01)   **C07K 19/00** (2006.01)
**C12N 1/15** (2006.01)   **C12N 1/19** (2006.01)
**C12N 1/21** (2006.01)   **C12N 5/10** (2006.01)
**C12N 15/12** (2006.01)   **C12N 15/62** (2006.01)
**C12N 15/63** (2006.01)

(52) Cooperative Patent Classification (CPC):
**A61K 35/12; A61 39/395; A61P 35/00;
A61P 43/00; C07K 14/435; C07K 14/705;
C07K 19/00; C12N 5/10; C12N 15/62; C12N 15/63;
C12N 15/74; C12N 15/80; C12N 15/81**

(86) International application number:
**PCT/JP2021/028230**

(87) International publication number:
**WO 2022/025220 (03.02.2022 Gazette 2022/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.07.2020   JP 2020131116**

(71) Applicants:
• **CHUGAI SEIYAKU KABUSHIKI KAISHA**
  **Tokyo 115-8543 (JP)**
• **Yamaguchi University**
  **Yamaguchi 753-8511 (JP)**

(72) Inventors:
• **IGAWA, Tomoyuki**
  **Synapse, 138623 (SG)**
• **SAKURAI, Mika**
  **Tokyo 103-8324 (JP)**

• **SUZUKI, Takashi**
  **Kamakura-shi, Kanagawa 247-8530 (JP)**
• **TATSUMI, Kanako**
  **Gotemba-shi, Shizuoka 412-8513 (JP)**
• **SHIMIZU, Shun**
  **Gotemba-shi, Shizuoka 412-8513 (JP)**
• **TAMADA, Koji**
  **Ube-shi, Yamaguchi 755-8505 (JP)**
• **SAKODA, Yukimi**
  **Ube-shi, Yamaguchi 755-8505 (JP)**

(74) Representative: **Vossius & Partner
Patentanwälte Rechtsanwälte mbB
Siebertstraße 3
81675 München (DE)**

Remarks:
The complete document including Reference
Table(s) and the Sequence Listing(s) can be
downloaded from the EPO website

(54) **PHARMACEUTICAL COMPOSITION INCLUDING CELL EXPRESSING CHIMERIC RECEPTOR**

(57)   The present invention provides a pharmaceutical composition comprising cells expressing a chimeric receptor, for use in combination with administration of an antigen-binding molecule, wherein the chimeric receptor comprises an extracellular domain, the extracellular domain comprises an extracellular domain of an immunoreceptor, an extracellular domain variant of an immunoreceptor, or a portion thereof, and the antigen-binding molecule is a multispecific antigen-binding molecule having a target antigen recognition site and an immunoreceptor recognition site which recognizes the immunoreceptor.

EP 4 197 545 A1

**(Cont. next page)**

# Fig. 1

ADMINISTRATION OF CHIMERIC RECEPTOR IN
COMBINATION WITH BISPECIFIC ANTIBODY

DAMAGE TO TARGET CELL

CHIMERIC
RECEPTOR-
EXPRESSING CELL

BISPECIFIC ANTIBODY

+

CHIMERIC RECEPTOR

ENDOGENOUS
IMMUNORECEPTOR

TARGET
CELL

TARGET
CELL

CHIMERIC
RECEPTOR-
EXPRESSING CELL

ENDOGENOUS
IMMUNOCYTE

**Description**

TECHNICAL FIELD

The present invention relates to a chimeric receptor, a cell expressing a chimeric receptor, and a method for treating a disease using the cell, for example, adoptive cell immunotherapy, particularly, adoptive cell immunotherapy using T cells expressing chimeric receptor.

BACKGROUND ART

Chimeric antigen receptors (hereinafter, also referred to as "CARs") are chimeric proteins prepared by artificially fusing an antigen-binding molecule which recognizes an antigen on a cell surface with a signaling region that induces activation of immunocytes. A gene encoding CAR is introduced into immunocytes to prepare immunocytes expressing CAR. CAR-expressing cells exhibit cytotoxicity on cells expressing a target antigen without depending on interaction with major histocompatibility complex (MHC), and are used for treatment of diseases such as cancer by adoptive cell immunotherapy (Non Patent Literature 1).

Clinical trials of adoptive cell immunotherapy using CAR-T cells or CAR-NK cells obtained by introducing a CAR gene into T cells or natural killer cells are conducted all over the world. Results showing effectiveness against, in particular, hematopoietic malignancy such as leukemia and lymphoma have been obtained, and Kymriah (registered trademark) (Novartis, tisagenlecleucel) and Yescarta (registered trademark) (Gilead Sciences, axicabtagene ciloleucel) which are CAR-T having CD19 as an antigen have been approved as medicaments.

The establishment of techniques for preparing immunocytes capable of universally recognizing target cells expressing various antigens has a significant clinical relevance in making adoptive cell immunotherapy versatile. Heretofore, there have been some reports on such research (Patent Literatures 1 to 5 and Non Patent Literatures 2 to 5).

As another method for treatment of cancer, a method has been developed in which endogenous immunocytes are activated with an antigen bonding molecule to make the tumor immune active. Antibodies inhibiting CTLA-4, PD-1 and PD-L1 which are immune checkpoint molecules have been heretofore reported to have a therapeutic effect (Non Patent Literatures 6 and 7), and approved as medicaments. It has been demonstrated through a mouse model that an antitumor effect is developed by activating a costimulatory factor expressed on immunocytes with an agonist antibody (Non Patent Literature 8). Further, a treatment method has been devised in which a CD137 agonist antibody that activates immunocytes is administered in combination of CAR-T to enhance the drug efficacy (Patent Literature 6 and Non Patent Literature 9).

CITATION LIST

PATENT LITERATURE

[Patent Literature 1] WO2012/082841
[Patent Literature 2] WO2016/040441
[Patent Literature 3] WO2017/161333
[Patent Literature 4] WO2018/002358
[Patent Literature 5] WO2018/177966
[Patent Literature 6] WO2019/140425

NON PATENT LITERATURE

[Non Patent Literature 1] June CH and Sadelain M, N Engl J Med. (2018) 379, 64
[Non Patent Literature 2] Tamada et al, Clin Cancer Res. (2012) 18, 6436
[Non Patent Literature 3] Urbanska K et al, J Transl Med. (2014) 12, 347
[Non Patent Literature 4] Kudo K et al, Cancer Res. (2014) 74, 93
[Non Patent Literature 5] Karches CH et al, Clin Cancer Res. (2019) 25, 5890
[Non Patent Literature 6] Hodi FS et al, N Engl J Med. (2010) 363, 711
[Non Patent Literature 7] Robert C et al, N Engl J Med. (2015) 372, 320
[Non Patent Literature 8] Houot R et al, Blood. (2009) 114, 3431

[Non Patent Literature 9] Mardiana S et al, Cancer Res. (2017) 77, 1296

SUMMARY OF INVENTION

TECHNICAL PROBLEM

A problem of treatment of cancer by adoptive cell immunotherapy using CAR is a poor effect on solid tumors. This may be caused by an immunosuppressive mechanism in tumor microenvironments in addition to the fact that CAR-expressing cells which recognize only a single target antigen cannot recognize all tumor cells because tumor antigens expressed on individual cancer cells in cancer tissues are not uniform. As a strategy for solving this problem, not only CAR-expressing cells but also endogenous immunocytes may be activated to develop an antitumor effect in coordination with endogenous immunocytes, and one of approaches thereof may be therapy in which a CD137 agonist antibody activating immunocytes is used in combination. However, there is a problem that clinically and nonclinically, a C137 agonist antibody induces activation of immunocytes in nontumor tissues such as those in the liver to develop toxicity.

As another problem of treatment of cancer by adoptive cell immunotherapy using CAR, the following is pointed out. First, an antigen recognition site of CAR needs to be constructed for each targeted tumor antigen because the tumor antigen expressed on the surface of a tumor cell varies depending on a cancer cell. Secondly, tumor antigens targeted by CAR may cause tumor antigen escape because treatment decreases their expression levels or mutates the tumor antigens. Most of existing CAR-expressing cells recognize only a single target antigen. Therefore, the tumor antigen escape deteriorates therapeutic effects. Thirdly, economical cost and labor burden for identifying antigen-binding molecules for various tumor antigens and establishing CAR-expressing cells fused with these molecules are problems. Therefore, there is need for preparation of a versatile CAR-expressing cell capable of universally recognizing various target antigens and changing the recognized tumor antigen depending on the properties of target antigen cells and the treatment stage in a patient, and development of a treatment method using the CAR-expressing cell.

SOLUTION TO PROBLEM

The present disclosure provides a treatment method using a multispecific antigen-binding molecule having a site exhibiting agonist activity on an immunoreceptor and a site recognizing a target antigen in combination with immunocytes transduced with a chimeric receptor having an immunoreceptor recognized by the antigen-binding molecule in an extracellular domain. In one aspect, the antigen-binding molecule in the present disclosure accumulates in lesion sites expressing a target antigen, and therefore the agonist activity of the molecules is selectively expressed in the legion sites, so that it is possible to avoid development of toxicity. The chimeric receptor in the present disclosure has no target antigen-binding activity unlike conventional CAR, and therefore does not inhibit binding of the antigen-binding molecule in the present disclosure to the target antigen. Further, the chimeric receptor in the present disclosure has binding activity against the antigen-binding molecule in the present disclosure, so that activation of endogenous immunocytes and activation of immunocytes expressing a chimeric receptor can be simultaneously induced by an antigen-binding molecule single agent.
That is, the present invention discloses the following.
More specifically, one aspect of the present disclosure provides the following invention.

[1] A pharmaceutical composition comprising a cell expressing a chimeric receptor, for use in combination with the administration of an antigen-binding molecule, wherein

the chimeric receptor comprises an extracellular domain, and the extracellular domain comprises an extracellular domain of an immunoreceptor, an extracellular domain variant of an immunoreceptor, or a portion thereof, and the antigen-binding molecule is a multispecific antigen-binding molecule having a target antigen recognition site and an immunoreceptor recognition site recognizing the immunoreceptor.

[2] A pharmaceutical composition comprising an antigen-binding molecule, for use in combination with the administration of a cell expressing a chimeric receptor, wherein

the chimeric receptor comprises an extracellular domain, and the extracellular domain comprises an extracellular domain of an immunoreceptor, an extracellular domain variant of an immunoreceptor, or a portion thereof, and the antigen-binding molecule is a multispecific antigen-binding molecule having a target antigen recognition site and an immunoreceptor recognition site recognizing the immunoreceptor.

[3] The pharmaceutical composition according to [1] or [2], wherein the immunoreceptor recognition site recognizes the extracellular domain of the chimeric receptor and an endogenous immunoreceptor.

[4] The pharmaceutical composition according to any of [1] to [3], wherein the extracellular domain of the chimeric receptor comprises an extracellular domain variant of an immunoreceptor, or a portion thereof, having attenuated binding to a ligand for the endogenous immunoreceptor.

[5] The pharmaceutical composition according to any of [1] to [4], wherein the immunoreceptor is a costimulatory molecule.

[6] The pharmaceutical composition according to any of [1] to [5], wherein the immunoreceptor is human CD137.

[7] The pharmaceutical composition according to any of [1] to [6], wherein the extracellular domain of the chimeric receptor comprises a human CD137 extracellular domain variant, or a portion thereof, in which at least a part or all of a cysteine-rich domain 3 and a cysteine-rich domain 4 of a human CD137 extracellular domain are deleted.

[8] The pharmaceutical composition according to any of [1] to [7], wherein the chimeric receptor comprises an intracellular signaling domain, and the intracellular signaling domain comprises an intracellular signaling domain of CD3 zeta.

[9] The pharmaceutical composition according to any of [1] to [8], wherein the target antigen is a receptor, a tumor antigen, an MHC antigen, or a differentiation antigen.

[10] The pharmaceutical composition according to any of [1] to [9], wherein the target antibody is a tumor antigen.

[11] The pharmaceutical composition according to any of [1] to [10], wherein the antigen-binding molecule is a bispecific antibody.

[12] The pharmaceutical composition according to any of [1] to [11] for use in the treatment or prevention of a cancer.

[13] A chimeric receptor comprising an extracellular domain comprising an extracellular domain of an immunoreceptor, an extracellular domain variant of an immunoreceptor, or a portion thereof.

[14] The chimeric receptor according to [13], wherein the extracellular domain of the chimeric receptor is an extracellular domain variant of an immunoreceptor, or a portion thereof, having attenuated binding to a ligand for the endogenous immunoreceptor.

[15] The chimeric receptor according to [13] or [14], wherein the immunoreceptor is a costimulatory molecule.

[16] The chimeric receptor according to any of [13] to [15], wherein the immunoreceptor is human CD137.

[17] The chimeric receptor according to any of [13] to [16], wherein the extracellular domain of the chimeric receptor comprises a human CD137 variant, or a portion thereof, in which at least a part or all of a cysteine-rich domain 3 and a cysteine-rich domain 4 of a human CD137 extracellular domain are deleted.

[18] The chimeric receptor according to any of [13] to [17], wherein the chimeric receptor comprises an intracellular signaling domain, and the intracellular signaling domain comprises an intracellular signaling domain of CD3 zeta.

[19] A cell expressing the chimeric receptor according to any one of [13] to [18].

[20] A composition comprising the cell according to [19].

[21] A nucleic acid encoding the chimeric receptor according to any of [13] to [18].

[22] A vector harboring the nucleic acid according to [21].

[23] A method for producing the cell according to [19], comprising transfecting or transducing a cell with the nucleic acid according to [21] or the vector according to [22].

[24] The pharmaceutical composition according to any of [1] to [12] or the chimeric receptor according to any of [13] to [18], wherein the chimeric receptor further comprises a transmembrane domain.

[A1] A pharmaceutical composition comprising a cell expressing a chimeric receptor, for use in combination with the administration of an antigen-binding molecule, wherein

the chimeric receptor comprises an extracellular domain, a transmembrane domain and an intracellular signaling domain, and the extracellular domain comprises an extracellular domain of an immunoreceptor, an extracellular domain variant of an immunoreceptor, or a portion thereof, and
the antigen-binding molecule comprises a bispecific antigen-binding site having a target antigen recognition site and an immunoreceptor recognition site recognizing the immunoreceptor.

[A2] A pharmaceutical composition comprising an antigen-binding molecule, for use in combination with the administration of a cell expressing a chimeric receptor, wherein

the chimeric receptor comprises an extracellular domain, a transmembrane domain and an intracellular signaling domain, and the extracellular domain comprises an extracellular domain of an immunoreceptor, an extracellular domain variant of an immunoreceptor, or a portion thereof, and
the antigen-binding molecule comprises a bispecific antigen-binding site having a target antigen recognition site and an immunoreceptor recognition site which recognizes the immunoreceptor.

[A3] The pharmaceutical composition according to [A1] or [A2], wherein the immunoreceptor recognition site recognizes the extracellular domain of the chimeric receptor and an endogenous immunoreceptor.

[A4] The pharmaceutical composition according to any of [A1] to [A3], wherein the extracellular domain of the chimeric receptor comprises an extracellular domain variant of an immunoreceptor, or a portion thereof, having attenuated binding to an endogenous immune ligand.

[A5] The pharmaceutical composition according to any of [A1] to [A4], wherein the immunoreceptor is a costimulatory molecule.

[A6] The pharmaceutical composition according to any of [A1] to [A5], wherein the immunoreceptor is human CD137.

[A7] The pharmaceutical composition according to any of [A1] to [A6], wherein the extracellular domain of the chimeric receptor comprises an extracellular domain variant, or a portion thereof, in which at least a part or all of a cysteine-rich domain 3 and a cysteine-rich domain 4 of a human CD137 extracellular domain are deleted.

[A8] The pharmaceutical composition according to any of [A1] to [A7], wherein the intracellular signaling domain comprises an intracellular signaling domain of CD3 zeta.

[A9] The pharmaceutical composition according to any of [A1] to [A8], wherein the target antigen is a receptor, a tumor antigen, an MHC antigen, or a differentiation antigen.

[A10] The pharmaceutical composition according to any of [A1] to [A9], wherein the target antibody is a tumor antigen.

[A11] The pharmaceutical composition according to any of [A1] to [A10], wherein the antigen-binding molecule is a bispecific antibody.

[A12] The pharmaceutical composition according to any of [A1] to [A11] for use in the treatment or prevention of a cancer.

[A13] A chimeric receptor comprising an extracellular domain, a transmembrane domain and an intracellular signaling domain, the extracellular domain comprising an extracellular domain of an immunoreceptor, an extracellular domain variant of an immunoreceptor, or a portion thereof.

[A14] The chimeric receptor according to [A13], wherein the extracellular domain of the chimeric receptor comprises an extracellular domain variant of an immunoreceptor, or a portion thereof, having attenuated binding to an endogenous immune ligand.

[A15] The chimeric receptor according to [A13] or [A14], wherein the immunoreceptor is a costimulatory molecule.

[A16] The chimeric receptor according to any of [A13] to [A15], wherein the immunoreceptor is human CD137.

[A17] The chimeric receptor according to any of [A13] to [A16], wherein the extracellular domain of the chimeric receptor comprises an extracellular domain variant, or a portion thereof, in which at least a part or all of a cysteine-rich domain 3 and a cysteine-rich domain 4 of a human CD137 extracellular domain are deleted.

[A18] The chimeric receptor according to any of [A13] to [A17], wherein the intracellular signaling domain comprises an intracellular signaling domain of CD3 zeta.

[A19] A cell expressing the chimeric receptor according to any of [A13] to [A18].

[A20] A composition comprising the cell according to [A19].

[A21] A nucleic acid encoding a chimeric receptor according to any of [A13] to [A17].

[A22] A vector harboring the nucleic acid according to [A21].

[A23] A method for producing the cell according to [A18], comprising transfecting or transducing a cell with the nucleic acid according to [A21] or the vector according to [A22].

[B1] A method for treating a disease, the method comprising:

administering an effective amount of an antigen-binding molecule to a subject in need of treatment; and
administering a cell expressing a chimeric receptor to the subject, wherein
the chimeric receptor comprises an extracellular domain, and the extracellular domain comprises an extracellular domain of an immunoreceptor, an extracellular domain variant of an immunoreceptor, or a portion thereof, and
the antigen-binding molecule is a multispecific antigen-binding molecule having a target antigen recognition site and an immunoreceptor recognition site which recognizes the immunoreceptor.

[B2] The method according to [B 1], wherein the chimeric receptor further comprises a transmembrane domain and an intracellular signaling domain.

[B3] The method according to [B1] or [B2], wherein the immunoreceptor recognition site recognizes the extracellular domain of the chimeric receptor and an endogenous immunoreceptor.

[B4] The method according to any of [B1] to [B3], wherein the extracellular domain of the chimeric receptor comprises an extracellular domain variant of an immunoreceptor, or a portion thereof, having attenuated binding to a ligand for the endogenous immunoreceptor.

[B5] The method according to any of [B1] to [B4], wherein the immunoreceptor is a costimulatory molecule.

[B6] The method according to any of [B1] to [B5], wherein the immunoreceptor is human CD137.

[B7] The method according to any of [B1] to [B6], wherein the extracellular domain of the chimeric receptor comprises a human CD137 extracellular domain variant, or a portion thereof, in which at least a part or all of a cysteine-rich domain 3 and a cysteine-rich domain 4 of a human CD137 extracellular domain are deleted.

[B8] The method according to any of [B1] to [B7], wherein the chimeric receptor comprises an intracellular signaling domain, and the intracellular signaling domain comprises an intracellular signaling domain of CD3 zeta.

[B9] The method according to any of [B1] to [B8], wherein the target antigen is a receptor, a tumor antigen, an MHC antigen, or a differentiation antigen.

[B10] The method according to any of [B1] to [B9], wherein the target antibody is a tumor antigen.

[B11] The method according to any of [B1] to [B10], wherein the antigen-binding molecule is a bispecific antibody.

[B12] The method according to any of [B1] to [B11], wherein the disease is cancer.

[B13] The method according to [B 12], wherein the cancer is selected from primary or metastatic melanoma, thymoma, lymphoma, sarcoma, lung cancer, colorectal cancer, liver cancer, non-Hodgkin's lymphoma, Hodgkin's lymphoma, leukemias, uterus cancer, cervical cancer, bladder cancer, kidney cancer and adenocarcinoma, for example, breast cancer, prostate cancer, ovary cancer and pancreatic cancer.

[C1] A method for treating a disease, the method comprising:

administering an effective amount of an antigen-binding molecule to a subject in need of treatment; and
administering a cell expressing a chimeric receptor to the subject, wherein
the chimeric receptor comprises an extracellular domain, a transmembrane domain and an intracellular signaling domain, and the extracellular domain comprises an extracellular domain of an immunoreceptor, an extracellular domain variant of an immunoreceptor, or a portion thereof, and
the antigen-binding molecule comprises a bispecific antigen-binding site having a target antigen recognition site and an immunoreceptor recognition site which recognizes the immunoreceptor.

[C2] The method according to [C1], wherein the chimeric receptor further comprises a transmembrane domain and an intracellular signaling domain.

[C3] The method according to [C1] or [C2], wherein the immunoreceptor recognition site recognizes the extracellular domain of the chimeric receptor and an endogenous immunoreceptor.

[C4] The method according to any of [C1] to [C3], wherein the extracellular domain of the chimeric receptor comprises an extracellular domain variant of an immunoreceptor, or a portion thereof, having attenuated binding to an endogenous immune ligand.

[C5] The method according to any of [C1] to [C4], wherein the immunoreceptor is a costimulatory molecule.

[C6] The method according to any of [C1] to [C5], wherein the immunoreceptor is human CD137.

[C7] The method according to any of [C1] to [C6], wherein the extracellular domain of the chimeric receptor comprises an extracellular domain variant, or a portion thereof, in which at least a part or all of a cysteine-rich domain 3 and a cysteine-rich domain 4 of a human CD137 extracellular domain are deleted.

[C8] The method according to any of [C1] to [C7], wherein the intracellular signaling domain comprises an intracellular signaling domain of CD3 zeta.

[C9] The method according to any of [C1] to [C8], wherein the target antigen is a receptor, a tumor antigen, an MHC antigen, or a differentiation antigen.

[C10] The pharmaceutical composition according to any of [C1] to [C9], wherein the target antibody is a tumor antigen.

[C11] The method according to any of [C1] to [C10], wherein the antigen-binding molecule is a bispecific antibody.

[C12] The method according to any of [C1] to [C11] for use in the treatment or prevention of cancer.

[C13] The method according to [C12], wherein the cancer is selected from primary or metastatic melanoma, thymoma, lymphoma, sarcoma, lung cancer, liver cancer, colorectal cancer, non-Hodgkin's lymphoma, Hodgkin's lymphoma, leukemias, uterus cancer, cervical cancer, bladder cancer, kidney cancer and adenocarcinoma, for example, breast cancer, prostate cancer, ovary cancer and pancreatic cancer.

BRIEF DESCRIPTION OF DRAWINGS

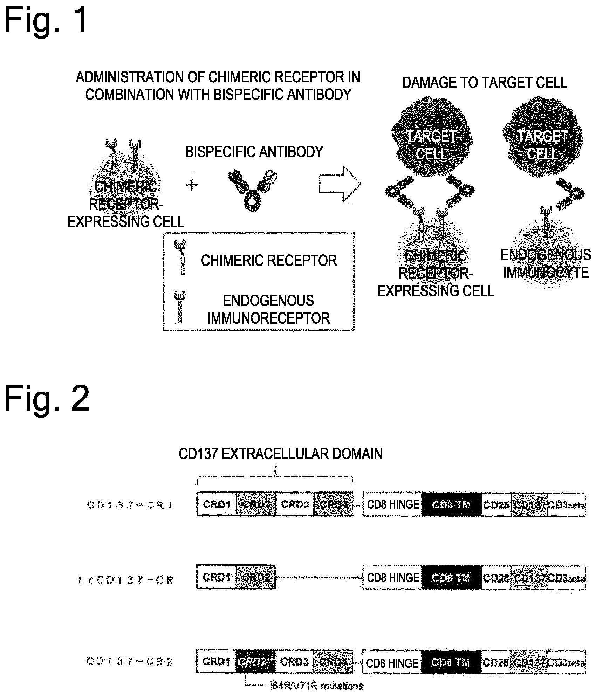

Figure 1 is a conceptual diagram of a treatment method using an antigen-binding molecule exhibiting agonist activity on an immunoreceptor (e.g. a bispecific antibody) in combination with immunocytes transduced with a chimeric receptor having an immunoreceptor recognized by the antigen-binding molecule in an extracellular domain, and shows the feature of the present invention which is recruitment of endogenous immunocytes in addition to chimeric receptor-expressing cells.

Figure 2 is a schematic diagram showing the chimeric receptor CD137-CR2 having I64R and V71R mutations in the extracellular domain of CD137, compared with the chimeric receptors CD137-CD8-CD28-CD137-CD3 zeta (CD137-CR1), trCD137-CD8-CD28-CD137-CD3 zeta (trCD137-CR) and CD137-CD1.

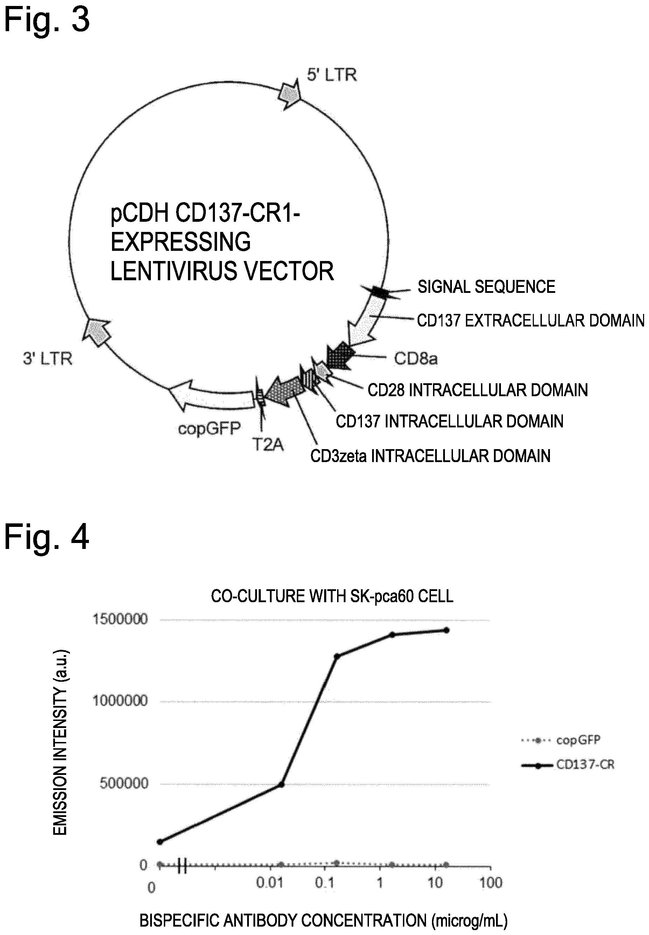

Figure 3 is a schematic diagram showing a lentivirus vector construct expressing pCDH-CD137-CR1 as described in Example 3-1 and the order of arrangement of components in frame units from the 5' end to the 3' end.

Figure 4 shows the results of evaluating the ability to activate CD137-CR1 Jurkat cells when cells expressing human GPC3 as a target antigen are co-cultured with a bispecific antibody consisting of an anti-GPC3 antibody and an anti-CD137 antibody, by the reporter assay described in Example 3-4. The abscissa depicts the concentration of the bispecific antibody (H0000-F760nN17/GL4-k0a//hCD137VH-F760mnP17/hCD137VL-k0), and the ordinate depicts the emission intensity of luciferase.

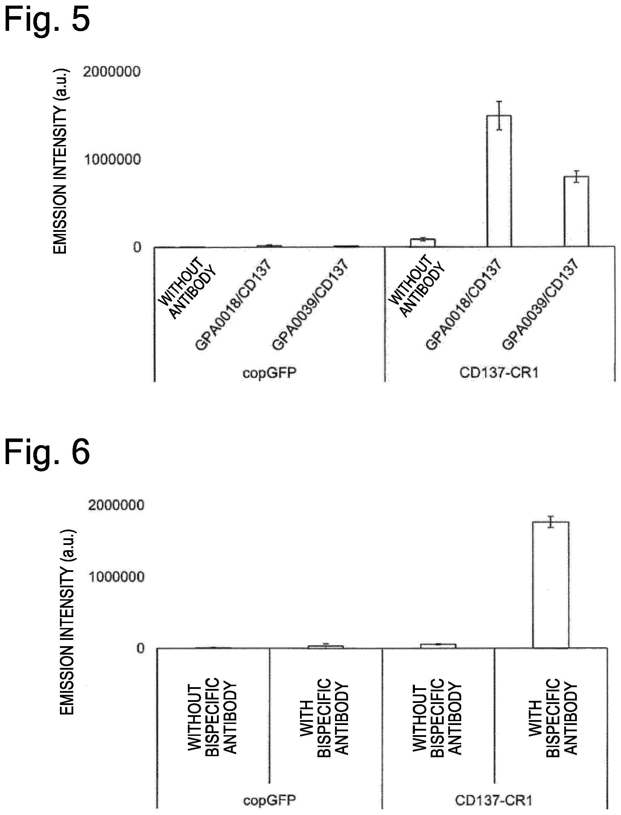

Figure 5 shows the results of evaluating the ability to activate CD137-CR1-copGFP Jurkat cells when cells expressing human GPRC5D as a target antigen are co-cultured with a bispecific antibody consisting of an anti-GPRC5D antibody and an anti-CD137 antibody, by the reporter assay described in Example 3-5. Two bispecific antibodies, i.e. GPA0018H-F760mnN17/GPA0018L-k0C//hCD137VH-F760mnP17/hCD137VL-k0(GPA0018/CD137) and GPA0039H-F760mnN17/GPA0039L-k0C//hCD137VH-F760mnP17/hCD137VL-k0(GPA0039/CD137) are used. The abscissa depicts the presence or absence thereof, and the ordinate depicts the emission intensity of luciferase.

Figure 6 shows the results of evaluating the ability to activate NFAT-RE-luc2 Jurkat cells (copGFP) expressing only copGFP without expressing a chimeric receptor and CD137-CR1-copGFP Jurkat cells (CD137-CR1) when cells expressing human IL-6R as a target antigen are co-cultured with a bispecific antibody consisting of an anti-IL-6R antibody and an anti-CD137 antibody, by the reporter assay described in Example 3-6. The abscissa depicts the presence or absence of the bispecific antibody (MRAH.v1-F760mnN17/MRAL.v1-k0.v1//hCD137VH-F760mnP17/hCD137VL-k0), and the ordinate depicts the emission intensity of luciferase.

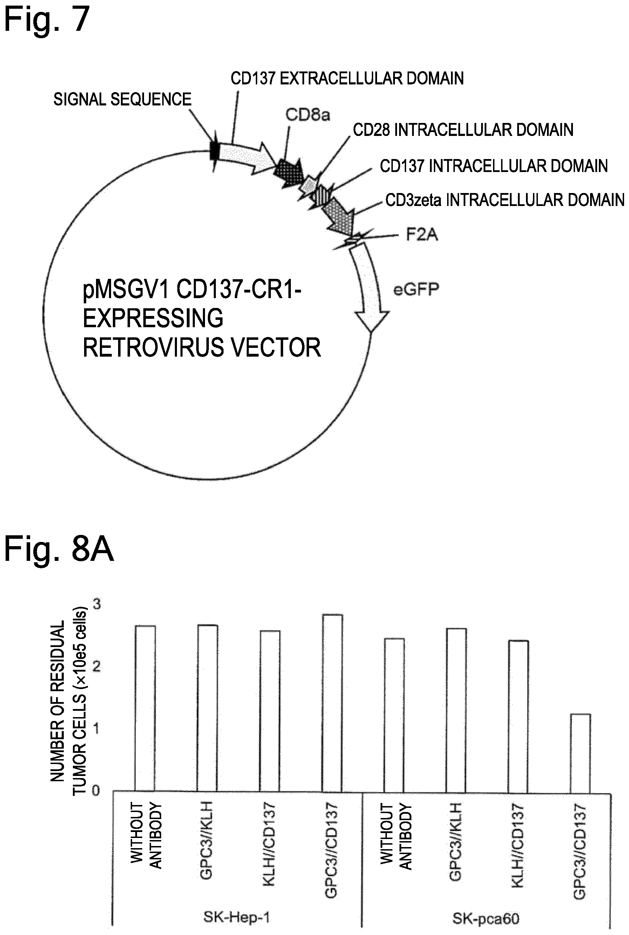

Figure 7 is a schematic diagram showing a retrovirus vector construct expressing pMSGV1-CD 13 7 -CR1 as described in Example 4-1 and the order of arrangement of components in frame units from the 5' end to the 3' end.

Figure 8A is a graph showing the results of evaluating the cytotoxic activity of CD137-CR1-eGFP-expressing T cells using the number of residual tumor cells as an index when a human liver cancer cell line (SK-Hep-1) and SK-pca60 cells expressing human GPC3 as a target antigen are used, and co-cultured with three bispecific antibodies (consisting of an anti-GPC3 antibody and an anti-KLH antibody, an anti-KLH antibody and an anti-CD137 antibody, and an anti-GPC3 antibody and an anti-CD137 antibody) as described in Example 4-4. The ordinate depicts the number of residual tumor cells, and the abscissa depicts the presence or absence of three bispecific antibodies.

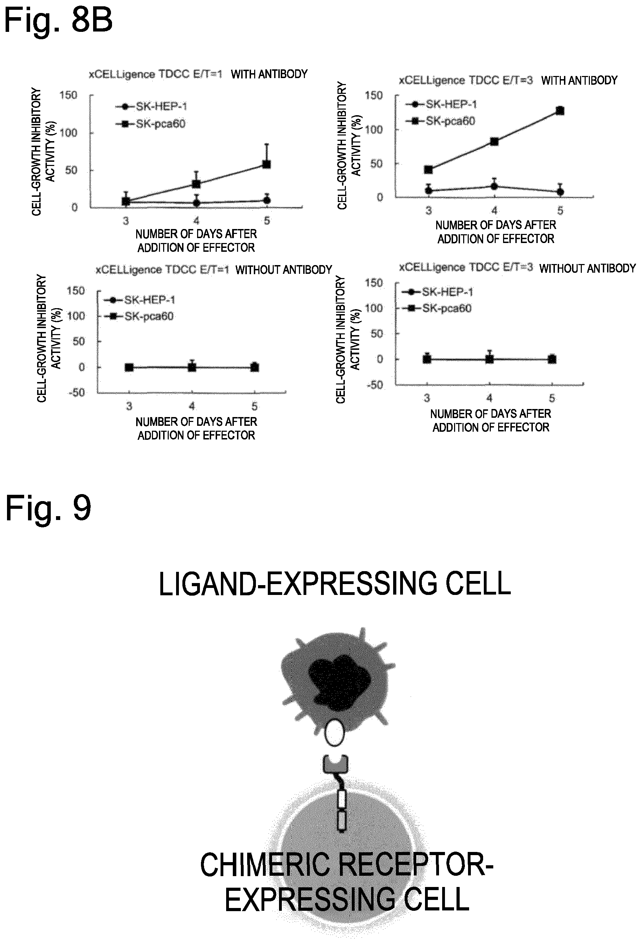

Figure 8B is a graph showing the results of evaluating the cytotoxic activity of CD137-CR1-eGFP-expressing T cells using the xCELLigence system when a human liver cancer cell line (SK-Hep-1) and SK-pca60 cells expressing human GPC3 as a target antigen are used, and co-cultured with a bispecific antibody consisting of an anti-GPC3 antibody and an anti-CD137 antibody as described in Example 4-5. The evaluation is performed while the ratio of effector cells to target cells (E : T) and the presence or absence of the bispecific antibody (written as "with antibody" or "without antibody" in Figure 8B) are changed. The ordinate depicts the cell-growth activity (%), and the abscissa depicts the number of days after addition of the effector cells and the antibody.

Figure 9 is a schematic diagram showing a target antigen-independent activation mechanism of chimeric receptor-expressing cells by binding to cells expressing a ligand.

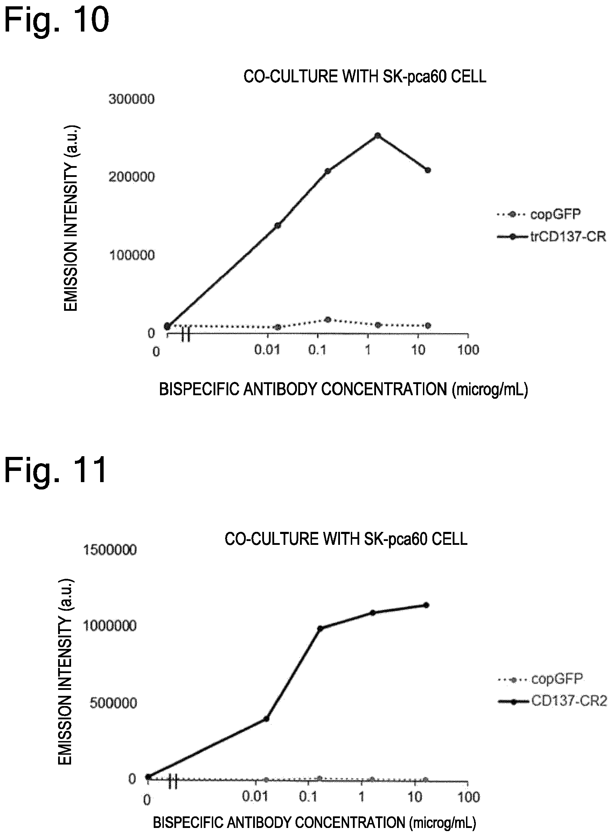

Figure 10 is a graph showing the results of evaluating the ability to activate trCD137-CR-copGFP Jurkat cells (trCD137-CR) when cells expressing human GPC3 as a target antigen are co-cultured with a bispecific antibody consisting of an anti-GPC3 antibody and an anti-CD137 antibody, by the reporter assay described in Example 6-4. The abscissa depicts the concentration of the bispecific antibody (H0000-F760nN17/GL4-k0al/hCD137VH-F760mnP17/hCD137VL-k0), and the ordinate depicts the emission intensity of luciferase.

Figure 11 is a graph showing the results of evaluating the ability to activate CD137-CR2-copGFP Jurkat cells (CD137-CR2) when cells expressing human GPC3 as a target antigen are co-cultured with a bispecific antibody consisting of an anti-GPC3 antibody and an anti-CD137 antibody, by the reporter assay described in Example 7-4. The abscissa depicts the concentration of the bispecific antibody (H0000-F760nN17/GL4-k0al/hCD137VH-F760mnP17/hCD137VL-k0), and the ordinate depicts the emission intensity of luciferase.

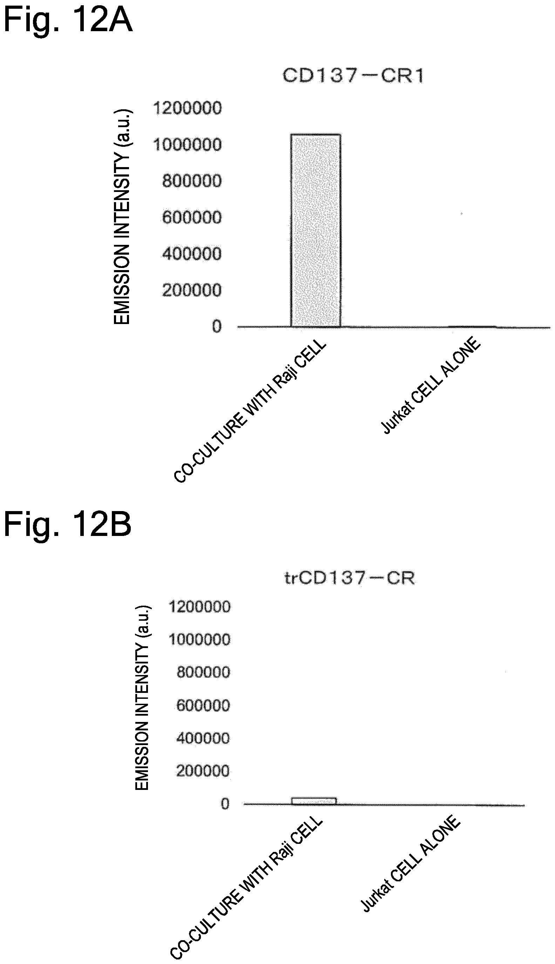

Figure 12A is graph showing the results of evaluating the ability to activate chimeric receptor-expressing cells in the presence or absence of the ligand (human CD137L)-expressing cells (Raji) described in Example 8 for the CD137-CR1-copGFP Jurkat cells (CD137-CR1) prepared in Example 3-2. The ordinate depicts the emission intensity of luciferase.

Figure 12B is graph showing the results of evaluating the ability to activate chimeric receptor-expressing cells in the presence or absence of the ligand (human CD137L)-expressing cells (Raji) described in Example 8 for the trCD137-CR-copGFP Jurkat cells (trCD137-CR) prepared in Example 6-2. The ordinate depicts the emission intensity of luciferase.

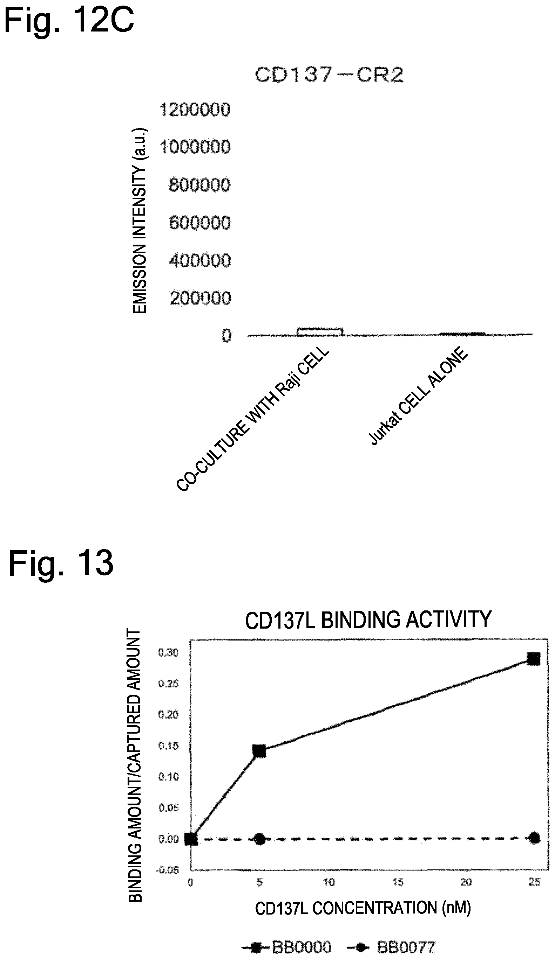

Figure 12C is graph showing the results of evaluating the ability to activate chimeric receptor-expressing cells in the presence or absence of the ligand (human CD137L)-expressing cells (Raji) described in Example 8 for the CD137-CR2-copGFP Jurkat cells (CD137-CR2) prepared in Example 7-2. The ordinate depicts the emission intensity of luciferase.

Figure 13 is a graph showing the results of measuring the binding activity of BB0000 and BB0077 against an *in vivo* ligand (human CD 137L) as described in Example 9-3-1. The abscissa depicts the concentration of CD137L, and the ordinate depicts the amount of CD137L bound with respect to the amounts of B0000 and BB0077 added.

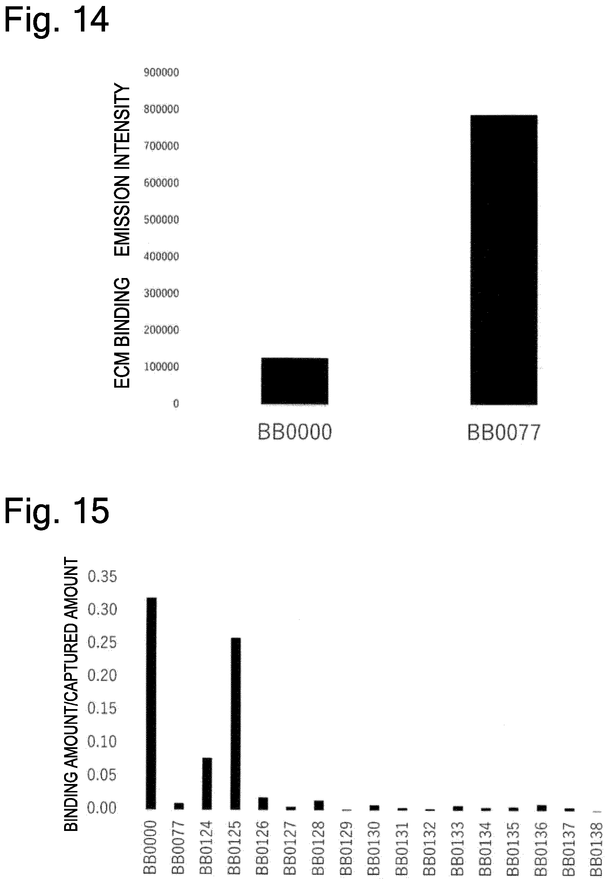

Figure 14 is a graph showing the results of evaluating the ECM binding of BB0000 and BB0077 as described in Example 9-3-2. The ordinate depicts the emission intensity showing ECM binding.

Figure 15 is a graph showing the results of measuring the binding activity of BB0124 to BB0138 against an *in vivo* ligand (human CD137L) as described in Example 9-3-4. The abscissa depicts CD137L variants evaluated, and the ordinate depicts the amount of CD 137L bound with respect to the amounts of the variants added.

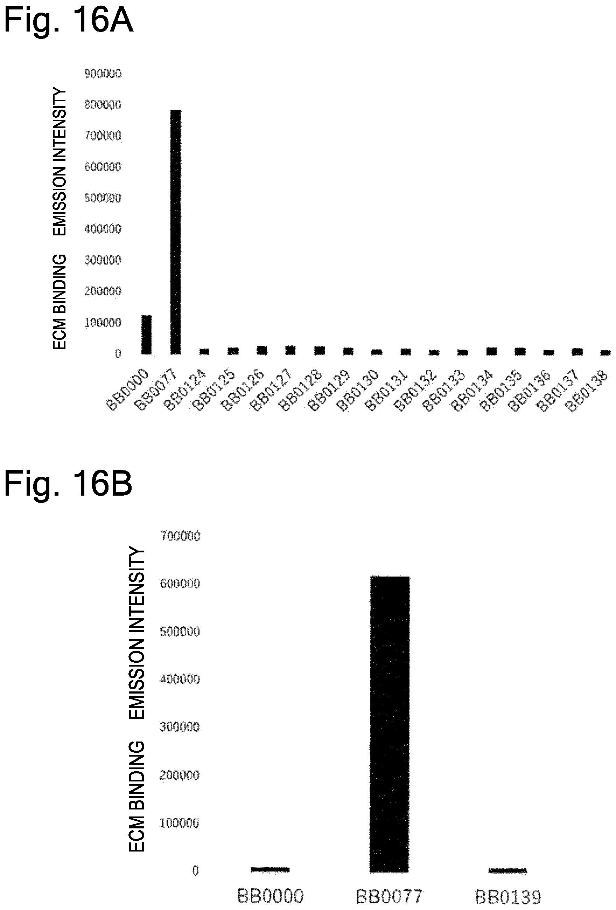

Figure 16A is a graph showing the results of evaluating the ECM binding of the CD137 variants BB0124 to BB0138 as described in Example 9-3-5. The ordinate depicts the emission intensity showing ECM binding.

Figure 16B is a graph showing the results of evaluating the ECM binding of the CD137 variant BB0139 as described in Example 9-3-3. The ordinate depicts the emission intensity showing ECM binding.

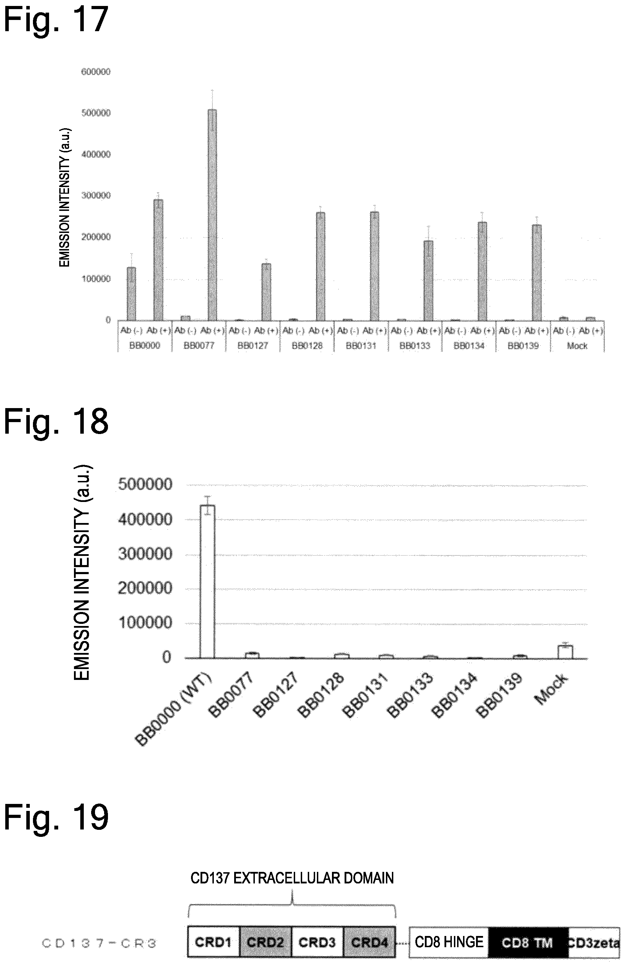

Figure 17 is a graph showing the results of evaluating the ability to activate the cells in a manner dependent on a bispecific antibody consisting of an anti-GPC3 antibody and an anti-CD137 antibody (expressed as Ab in Figure 17) in the presence of target cells by the reporter assay described in Example 10-4. The abscissa depicts Jurkat cells expressing a chimeric receptor in which eight CD137 variants BB0000, BB0077, BB0127, BB0128, BB0131, BB0133, BB0134 and BB0139 are extracellular domains and the cytoplasmic regions of CD8-CD28-CD137-CD3 zeta are linked, and a parent strain of NFAT-RE-luc2 Jurkat cells that are not subjected to gene introduction (Mock). The ordinate depicts the emission intensity of luciferase.

Figure 18 is a graph showing the results of evaluating the ability to activate Jurkat cells having a CD137 variant in an extracellular domain in the presence of ligand-expressing cells by the reporter assay described in Example 10-5. The abscissa depicts Jurkat cells expressing a chimeric receptor in which eight variants BB0000, BB0077, BB0127, BB0128, BB0131, BB0133, BB0134 and BB0139 are extracellular domains and the cytoplasmic regions of CD8-CD28-CD137-CD3 zeta are linked, and a parent strain of NFAT-RE-luc2 Jurkat cells that are not subjected to gene introduction (Mock). The ordinate depicts the emission intensity of luciferase.

Figure 19 is a schematic diagram showing the chimeric receptor CD137-CD8-CD3 zeta encoded by a lentivirus vector described in Example 11-1.

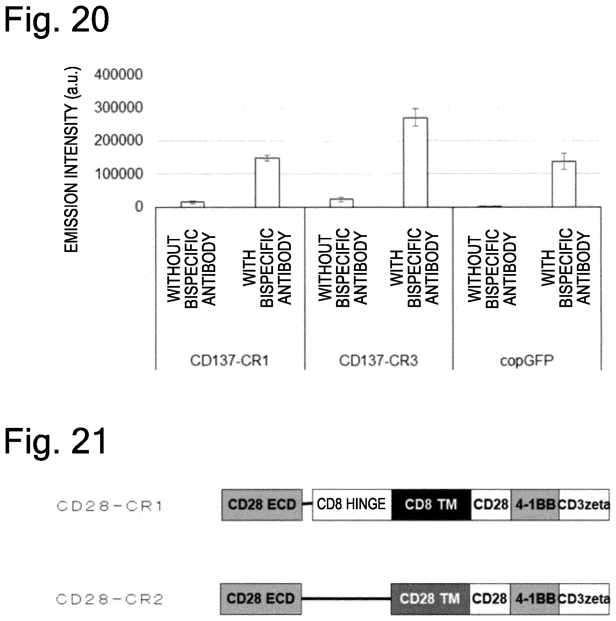

Figure 20 is a graph showing the results of evaluating the ability to activate the three types of Jurkat cells by CD137 in a manner dependent on a bispecific antibody consisting of an anti-GPC3 antibody and an anti-CD137 antibody in the presence of target cells by the reporter assay described in Example 11-4. The abscissa depicts three types of Jurkat cells prepared in Example 11-2, i.e. CD137-CR1 Jurkat cells (CD137-CR1), CD137-CR3-copGFP Jurkat cells (CD137-CR3), and NF-kB-Luc2/4-1BB Jurkat cells (copGFP) expressing only copGFP without expressing a chimeric receptor, and the presence or absence of a bispecific antibody. The ordinate depicts the emission intensity of luciferase.

Figure 21 is a schematic diagram showing a construct using human CD8 for a transmembrane domain encoded by a lentivirus vector (CD28-CR1) and a construct using human CD28 (CD28-CR2) for the transmembrane domain as described in Example 12-1.

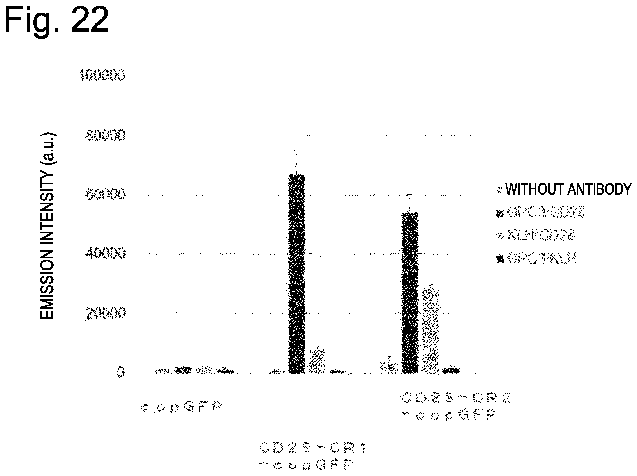

Figure 22 is a graph showing the results of evaluating the ability to activate CD28-CR1 Jurkat cells and CD28-CR2 Jurkat cells described in Example 12-4, in the presence of the target cells. The abscissa depicts three types of Jurkat cells, i.e. NFAT-RE-luc2 Jurkat cells expressing only copGFP without expressing a chimeric receptor (copGFP), Jurkat cells expressing CD28-CR1-copGFP prepared in Example 12-2 (CD28-CR1-copGFP), and Jurkat cells expressing CD28-CR2-copGFP (CD28-CR2-copGFP), and the presence or absence of three bispecific antibodies, i.e. a bispecific antibody consisting of an anti-GPC3 antibody and an anti-CD28 antibody (GPC3/CD28), a bispecific antibody consisting of an anti-KLH antibody and an anti-CD28 antibody (KLH/CD28), and a bispecific antibody consisting of an anti-GPC3 antibody and an anti-KLH antibody (GPC3/KLH). The ordinate depicts the emission intensity of luciferase.

DESCRIPTION OF EMBODIMENTS

Other features and advantages of the present disclosure will be evident from the detailed description given below. However, it is obvious to those skilled in the art from this detailed description that various changes or modifications can be made in the present disclosure without departing from the spirit and scope of the present disclosure. Therefore, the detailed description and specific examples showing preferred embodiments of the present disclosure should be construed as being given merely for illustrative purposes.

Hereinafter, embodiments of the present disclosure will be described with reference to the drawings.

The polypeptide according to the present invention usually refers to a peptide having a length on the order of 4 amino acids or longer, and a protein. Also, the polypeptide according to the present invention is usually a polypeptide consisting of an artificially designed sequence, but is not limited thereto. For example, an organism-derived polypeptide may be used. Alternatively, the polypeptide according to the present invention may be any of a natural polypeptide, a synthetic polypeptide, a recombinant polypeptide, and the like. Furthermore, portions of these polypeptides are also included in the polypeptide of the present invention.

In the present specification, each amino acid is indicated by one-letter code or three-letter code, or both, as represented by, for example, Ala/A, Leu/L, Arg/R, Lys/K, Asn/N, Met/M, Asp/D, Phe/F, Cys/C, Pro/P, Gln/Q, Ser/S, Glu/E, Thr/T, Gly/G, Trp/W, His/H, Tyr/Y, Ile/I, or Val/V. For expressing an amino acid located at a particular position, an expression using a number representing the particular position in combination with the one-letter code or the three-letter code of the amino acid can be appropriately used. For example, an amino acid 37V, which is an amino acid contained in a single-domain antibody, represents Val located at position 37 defined by the Kabat numbering.

For the alteration of an amino acid in the amino acid sequence of a polypeptide such as an antibody, a method known in the art such as site-directed mutagenesis (Kunkel et al. (Proc. Natl. Acad. Sci. USA (1985) 82, 488-492)) or overlap extension PCR can be appropriately adopted. A plurality of methods known in the art can also be adopted as alteration methods for substituting an amino acid by an amino acid other than a natural amino acid (Annu. Rev. Biophys. Biomol. Struct. (2006) 35, 225-249; and Proc. Natl. Acad. Sci. U.S.A. (2003) 100 (11), 6353-6357). For example, a cell-free translation system (Clover Direct (ProteinExpress Co., Ltd.)) including tRNA in which a non-natural amino acid is bonded to amber suppressor tRNA complementary to a UAG codon (amber codon), one of stop codons, is also preferably used. In the present specification, examples of the alteration include, but are not limited to, substitution.

In the present specification, the term "and/or" that is used in representing an amino acid alteration position is meant to include every combination appropriately represented by "and" and "or". Specifically, for example, the phrase "amino acids at positions 37, 45, and/or 47 are substituted" includes the following variations of amino acid alteration position: (a) position 37, (b) position 45, (c) position 47, (d) positions 37 and 45, (e) positions 37 and 47, (f) positions 45 and 47, and (g) positions 37, 45 and 47.

In the present specification, expression in which the one-letter codes or three-letter-codes of amino acids before and after alteration are used previous and next to a number representing a particular position can be appropriately used for representing amino acid alteration. For example, an alteration F37V or Phe37Val used for substituting an amino acid contained in an antibody variable region or a single-domain antibody represents the substitution of Phe at position 37 defined by the Kabat numbering by Val. Specifically, the number represents an amino acid position defined by the Kabat numbering; the one-letter code or three-letter code of the amino acid previous to the number represents the amino acid before the substitution; and the one-letter code or three-letter code of the amino acid next to the number represents the amino acid after the substitution. Likewise, an alteration P238A or Pro238Ala used for substituting an amino acid in a Fc region contained in an antibody constant region represents the substitution of Pro at position 238 defined by the EU numbering by Ala. Specifically, the number represents an amino acid position defined by the EU numbering; the one-letter code or three-letter code of the amino acid previous to the number represents the amino acid before the substitution; and the one-letter code or three-letter code of the amino acid next to the number represents the amino acid after the substitution.

In the present specification, the term "antibody" is used in the broadest sense and encompasses various antibody structures including, but are not limited to, a monoclonal antibody, a polyclonal antibody, a multispecific antibody (e.g., a bispecific antibody), a single-domain antibody, and an antibody portion as long as the antibody exhibits the desired antigen-binding activity.

In the present invention, the "multispecific antigen-binding molecule" refers to a molecule which can bind specifically to multiple different antigens, specifically multiple epitopes contained in multiple antigens. That is, the multispecific antigen-binding molecule is a molecule having specificity to at least two different epitopes, and includes molecules which bind to different antigens, and molecules which bind to different epitopes on the same antigen. The multispecific antigen-binding molecule conceptually encompasses a bispecific antigen-binding molecule, and examples thereof include multispecific antibodies, and bispecific antibodies. The molecule of the bispecific antibody binds to two antigens, and the multispecific antibody may have specificity to two antigens or more antigens (e.g. three antigens). The bispecific antibodies and the multispecific antibodies can be prepared as full-length antibodies or as molecules containing antibody portions.

The "antibody portion" refers to a molecule, other than a complete antibody, which contains a part of the complete antibody and binds to an antigen to which the complete antibody binds. Examples of the antibody portion include, but are not limited to, Fv, Fab, Fab', Fab'-SH, F(ab')$_2$, diabody, linear antibodies, single-chain antibody molecules (e.g., scFv), and multispecific antibodies formed from antibody portions.

The terms "full-length antibody" refers to an antibody having a structure substantially similar to a natural antibody structure, or having heavy chains containing a Fc region defined in the present specification.

In the present specification, the term "homo-antibody" refers to an antibody which binds to a single antigen and having a four-chain structure in which two light chain polypeptide chains and two heavy chain polypeptide chains are present.

The term "variable region" or "variable domain" refers to a domain of the heavy chain or light chain of an antibody, the domain involved in allowing the antibody to bind to the antigen. The variable domains of the heavy chain and the light chain (VH and VL, respectively) of an antibody normally have similar structure including a framework region (FR) including four conserved domains and three complementarity determining regions (CDR) (e.g., see Kindt et al. Kuby Immunology, 6th ed., W.H. Freeman and Co., page 91 (2007)). One VH or VL domain provides antigen-binding specificity.

The term "complementarity determining region" or "CDR" used in the present specification is hypervariable in the sequence, and/or forms a structurally determined loop ("hypervariable loop"), and/or refers to antigen contact residues ("antigen contacts") or each region of an antibody variable domain. Normally, an antibody comprises six CDRs: three CDRs (H1, H2 and H3) in VH and three CDRs (L1, L2 and L3) in VL. Exemplary CDRs herein include the following:

(a) hypervariable loops formed of amino acid residues 26 to 32 (L1), 50 to 52 (L2), 91 to 96 (L3), 26 to 32 (H1), 53 to 55 (H2), and 96 to 101 (H3) (Chothia and Lesk, J. Mol. Biol. 196:901-917 (1987));
(b) CDRs formed of amino acid residues 24 to 34 (L1), 50 to 56 (L2), 89 to 97 (L3), 31 to 35b (H1), 50 to 65 (H2), and 95 to 102 (H3) (Kabat et al., Sequences of Proteins of Immunological Interest, 5th Ed. Public Health Service, National Institutes of Health, Bethesda, MD (1991));
(c) antigen contacts formed of amino acid residues 27c to 36 (L1), 46 to 55 (L2), 89 to 96 (L3), 30 to 35b (H1), 47 to 58 (H2), and 93 to 101 (H3) (MacCallum et al. J. Mol. Biol. 262: 732-745 (1996)); and
(d) combinations of (a), (b) and/or (c) including HVR amino acid residues 46-56 (L2), 47-56 (L2), 48-56 (L2), 49-56 (L2), 26-35 (H1), 26-35b (H1), 49-65 (H2), 93-102 (H3) and 94-102 (H3).

Unless otherwise specified, CDR residues and other residues in a variable domain (e.g. FR residues) are herein numbered according to Kabat et al. in the above.

The term "framework" or "FR" refers to variable domain residues other than complementarity determining region (CDR) residues. FR in a variable domain normally consists of four FR domains: FR1, FR2, FR3, and FR4. According to this, CDR and FR sequences normally appear in VH (or VL) in the following order: FR1-H1(L1)-FR2-H2(L2)-FR3-H3(L3)-FR4.

In the present specification, the term "constant region" or "constant domain" refers to a region or a domain other than variable regions in an antibody. For example, an IgG antibody is a heterotetrameric glycoprotein of approximately 150,000 Da constituted by two identical light chains and two identical heavy chains connected through disulfide bonds. Each heavy chain has a variable region (VH) also called variable heavy chain domain or heavy chain variable domain, followed by a heavy chain constant region (CH) containing a CH1 domain, a hinge region, a CH2 domain, and a CH3 domain, from the N terminus toward the C terminus. Likewise, each light chain has a variable region (VL) also called variable light chain domain or light chain variable domain, followed by a constant light chain (CL) domain, from the N terminus toward the C terminus. The light chains of natural antibodies may be attributed to one of two types called kappa ($\kappa$) and lambda ($\lambda$) on the basis of the amino acid sequences of their constant domains.

In the present specification, the term "Fc region" is used for defining the C-terminal region of immunoglobulin heavy chains, including at least a portion of constant regions. This term includes a Fc region having a natural sequence and a mutant Fc region. In one embodiment, the heavy chain Fc region of human IgG1 spans from Cys226 or Pro230 to the carboxyl terminus of the heavy chain. However, the C-terminal lysine (Lys447) or glycinelysine (Gly446-Lys447) of the Fc region may be present or absent. In the present specification, amino acid residues in a Fc region or a constant region are numbered according to the EU numbering system (also called EU index) described in Kabat et al., Sequences of Proteins of Immunological Interest, 5th Ed. Public Health Service, National Institutes of Health, Bethesda, MD 1991, unless otherwise specified.

The "class" of an antibody refers to the type of a constant domain or a constant region carried by the heavy chain of the antibody. Antibodies have 5 major classes: IgA, IgD, IgE, IgG, and IgM. Some of these classes may be further divided into subclasses (isotypes). Examples thereof include IgG1, IgG2, IgG3, IgG4, IgA1, and IgA2. Heavy chain constant domains corresponding to immunoglobulins of different classes are called alpha, delta, epsilon, gamma, and mu, respectively.

In the present specification, the "target antigen recognition site" is a site of an antigen-binding molecule capable of recognizing an antigen of interest (target antigen) and binding specifically to the target antigen, and can also be referred to as a target antigen-binding domain. The target antigen recognition site may be a site having any structure as long as it binds to an antigen of interest. Examples of such a site include, but are not limited to, an antibody heavy chain variable region (VH), an antibody light chain variable region (VL), a single-domain antibody (sdAb), a module called A domain of approximately 35 amino acids contained in an *in vivo* cell membrane protein avimer (International Publication Nos. WO2004/044011 and WO2005/040229), adnectin containing a 10Fn3 domain serving as a protein binding domain derived from a glycoprotein fibronectin expressed on cell membranes (International Publication No. WO2002/032925), Affibody containing an IgG binding domain scaffold constituting a three-helix bundle composed of 58 amino acids of protein A (International Publication No. WO1995/001937), DARPins (designed ankyrin repeat proteins) which are molecular surface-exposed regions of ankyrin repeats (AR) each having a 33-amino acid residue structure folded into a subunit of a turn, two antiparallel helices, and a loop (International Publication No. WO2002/020565), anticalin having four loop regions connecting eight antiparallel strands bent toward the central axis in one end of a barrel structure highly conserved in lipocalin molecules such as neutrophil gelatinase-associated lipocalin (NGAL) (International Publication No. WO2003/029462), and a depressed region in the internal parallel sheet structure of a horseshoe-shaped fold composed of repeated leucine-rich-repeat (LRR) modules of an immunoglobulin structure-free variable lymphocyte receptor (VLR) as seen in the acquired immune systems of jawless vertebrates such as lamprey or hagfish (International Publication No. WO2008/016854).

Preferred examples of the target antigen recognition site in the present invention include a heavy chain variable region (VH) of an antibody and a light chain variable region (VL) of an antibody, and particularly preferred examples thereof include a target antigen recognition site in a multispecific antibody having an immunoreceptor recognition site, for example, a target antigen recognition site in a bispecific antibody. The site capable of recognizing and binding to a specific antigen of the antigen-binding molecule in the present invention is sometimes referred to as an antigen-binding domain. For example, the multispecific antibody has a plurality of antigen-binding domains. Among them, antigen-binding domains capable of recognizing and binding to a target antigen function as target antigen-binding sites, and antigen-binding domains capable of recognizing and binding to an immunoreceptor function as immunoreceptor recognition sites. Therefore, the target antigen-binding site and the immunoreceptor recognition site of the multispecific antibody both correspond to antigen-binding domains of the multispecific antibody. The phrase "comprising a bispecific antigen-binding site" means having two types of antigen-binding domains.

In the present specification, the "antigen" means a substance recognized as a target by a molecule such as an antibody, and in the present invention, the "target antigen" means an antigen to which an antigen-binding molecule can bind through a target antigen recognition site. The target antigen includes an epitope to which a target antigen recognition site can bind. The target antigen according to the present invention is an antigen for use in the treatment of a disease caused by a target tissue. Preferred examples thereof include, but are not limited to, molecules expressed on the surface of target cells (e.g., cancer cells and inflammatory cells), molecules expressed on the surface of other cells in tissues containing target cells, molecules expressed on the surface of cells having an immunological role against target cells and tissues containing target cells, and large molecules present in the stromata of tissues containing target cells. Examples of the target antigen can include the following antigens.

In one embodiment, examples of the target antigen according to the present invention preferably include receptors, tumor antigens, MHC antigens, and differentiation antigens.

Examples of the target antigen can include the following molecules: 17-IA, 4-1BB, 4Dc, 6-keto-PGF1a, 8-iso-PGF2a, 8-oxo-dG, A1 adenosine receptor, A33, ACE, ACE-2, activin, activin A, activin AB, activin B, activin C, activin RIA, activin RIA ALK-2, activin RIB ALK-4, activin RIIA, activin RIIB, ADAM, ADAM10, ADAM12, ADAM15, ADAM17/TACE, ADAM8, ADAM9, ADAMTS, ADAMTS4, ADAMTS5, addressin, aFGF, ALCAM, ALK, ALK-1, ALK-7, alpha-1-antitrypsin, alpha-V/beta-1 antagonist, ANG, Ang, APAF-1, APE, APJ, APP, APRIL, AR, ARC, ART, artemin, anti-Id, ASPARTIC, atrial natriuretic factor, av/b3 integrin, Axl, b2M, B7-1, B7-2, B7-H, B-lymphocyte stimulator (BlyS), BACE, BACE-1, Bad, BAFF, BAFF-R, Bag-1, BAK, Bax, BCA-1, BCAM, Bcl, BCMA, BDNF, b-ECGF, bFGF, BID, Bik, BIM, BLC, BL-CAM, BLK, BMP, BMP-2 BMP-2a, BMP-3 osteogenin, BMP-4 BMP-2b, BMP-5, BMP-6 Vgr-1, BMP-7 (OP-1), BMP-8 (BMP-8a, OP-2), BMPR, BMPR-IA (ALK-3), BMPR-IB (ALK-6), BRK-2, RPK-1, BMPR-II (BRK-3), BMP, b-NGF, BOK, bombesin, bone-derived neurotrophic factor, BPDE, BPDE-DNA, BTC, complement factor 3 (C3), C3a, C4, C5, C5a, C10, CA125, CAD-8, calcitonin, cAMP, carcinoembryonic antigen (CEA), cancer-associated antigens, cathepsin A, cathepsin B, cathepsin C/DPPI, cathepsin D, cathepsin E, cathepsin H, cathepsin L, cathepsin O, cathepsin S, cathepsin V, cathepsin X/Z/P, CBL, CCI, CCK2, CCL, CCL1, CCL11, CCL12, CCL13, CCL14, CCL15, CCL16, CCL17, CCL18, CCL19, CCL2, CCL20, CCL21, CCL22, CCL23, CCL24, CCL25, CCL26, CCL27, CCL28, CCL3, CCL4, CCL5, CCL6, CCL7, CCL8, CCL9/10, CCR, CCR1, CCR10, CCR10, CCR2, CCR3, CCR4, CCR5, CCR6, CCR7, CCR8, CCR9, CD1, CD2, CD3, CD3E, CD4, CD5, CD6, CD7, CD8, CD10, CD11a, CD11b, CD11c, CD13, CD14, CD15, CD16, CD18, CD19, CD20, CD21, CD22, CD23, CD25, CD27L, CD28, CD29, CD30, CD30L, CD32, CD33 (p67 protein), CD34, CD38, CD40, CD40L, CD44, CD45, CD46, CD49a, CD52, CD54, CD55, CD56, CD61, CD64, CD66e, CD74, CD89, CD95, CD123, CD137, CD138, CD140a, CD146, CD147, CD148, CD152, CD164, CEACAM5, CFTR, cGMP, CINC, botulinum toxin, Clostridium perfringens toxin, CKb8-1, CLC, CMV, CMV UL, CNTF, CNTN-1, COX, C-Ret, CRG-2, CT-1, CTACK, CTGF, CTLA-4, PD1, PDL1, LAG3, TIM3, galectin-9, CX3CL1, CX3CR1, CXCL, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCR, CXCR1, CXCR2, CXCR3, CXCR4, CXCR5, CXCR6, cytokeratin tumor-associated antigens, DAN, DCC, DcR3, DC-SIGN, decay accelerating factor, des(1-3)-IGF-I (brain IGF-1), Dhh, digoxin, DNAM-1, Dnase, Dpp, DPPIV/CD26, Dtk, ECAD, EDA, EDA-A1, EDA-A2, EDAR, EGF, EGFR (ErbB-1), EMA, EMMPRIN, ENA, endothelin receptor, enkephalinase, eNOS, Eot, eotaxin 1, EpCAM, ephrin B2/EphB4, EPO, ERCC, E-selectin, ET-1, factor IIa, factor VII, factor VIIIc, factor IX, fibroblast-activating protein (FAP), Fas, FcR1, FEN-1, ferritin, FGF, FGF-19, FGF-2, FGF3, FGF-8, FGFR, FGFR-3, fibrin, FL, FLIP, Flt-3, Flt-4, follicle-stimulating hormone, fractalkine, FZD1, FZD2, FZD3, FZD4, FZD5, FZD6, FZD7, FZD8, FZD9, FZD10, G250, Gas6, GCP-2, GCSF, GD2, GD3, GDF, GDF-1, GDF-3 (Vgr-2), GDF-5 (BMP-14, CDMP-1), GDF-6 (BMP-13, CDMP-2), GDF-7 (BMP-12, CDMP-3), GDF-8 (myostatin), GDF-9, GDF-15 (MIC-1), GDNF, GDNF, GFAP, GFRa-1, GFR-alpha 1, GFR-alpha 2, GFR-alpha 3, GITR, glucagon, Glut4, glycoprotein IIb/IIIa (GPIIb/IIIa), GM-CSF, gp130, gp72, GPR20, GRO, growth hormone-releasing factor, hapten (NP-cap or NIP-cap), HB-EGF, HCC, HCMV gB envelope glycoprotein, HCMV gH envelope glycoprotein, HCMV UL, hematopoietic growth factor (HGF), Hep B gp120, heparanase, Her2/neu (ErbB-2), Her3 (ErbB-3), Her4 (ErbB-4), herpes simplex virus (HSV) gB glycoprotein, HSV gD glycoprotein, HGFA, high-molecular-weight melanoma-associated antigen (HMW-MAA), HIV gp120, HIV IIIB gp 120 V3 loop, HLA, HLA-DR, HM1.24, HMFG PEM, HRG, Hrk, human heart myosin, human cytomegalovirus (HCMV), human growth hormone (HGH), HVEM, I-309, IAP, ICAM, ICAM-1, ICAM-3, ICE, ICOS, IFNg, Ig, IgA receptor, IgE, IGF, IGF binding protein, IGF-1R, IGFBP, IGF-I, IGF-II, IL, IL-1, IL-1R, IL-2, IL-2R, IL-4, IL-4R, IL-5, IL-5R, IL-6, IL-6R, IL-8, IL-9, IL-10, IL-12, IL-13, IL-15, IL-18, IL-18R, IL-21, IL-23, IL-27, interferon (INF)-alpha, INF-beta, INF-gamma, inhibin, iNOS, insulin chain A, insulin chain B, insulin-like growth factor 1, integrin alpha 2, integrin alpha 3, integrin alpha 4, integrin alpha 4/beta 1, integrin alpha 4/beta 7, integrin alpha 5 (alpha V), integrin alpha 5/beta 1, integrin alpha 5/beta 3, integrin alpha 6, integrin beta 1, integrin beta 2, interferon gamma, IP-10, I-TAC, JE, kallikrein 2, kallikrein 5, kallikrein 6, kallikrein 11, kallikrein 12, kallikrein 14, kallikrein 15, kallikrein L1, kallikrein L2, kallikrein L3, kallikrein L4, KC, KDR, keratinocyte growth factor (KGF), laminin 5, LAMP, LAP, LAP (TGF-1), latent TGF-1, latent TGF-1 bp1, LBP, LDGF, LECT2, lefty, Lewis-Y antigen, Lewis-Y-related antigen, LFA-1, LFA-3, Lfo, LIF, LIGHT, lipoprotein, LIX, LKN, Lptn, L-selectin, LT-a, LT-b, LTB4, LTBP-1, lung surface, luteinizing hormone, lymphotoxin beta receptor, Mac-1, MAdCAM, MAG, MAP2, MARC, MCAM, MCAM, MCK-2, MCP, M-CSF, MDC, Mer, metalloproteinases, MGDF receptor, MGMT, MHC (HLA-DR), MIF, MIG, MIP, MIP-1-alpha, MK, MMAC1, MMP, MMP-1, MMP-10, MMP-11, MMP-12, MMP-13, MMP-14, MMP-15, MMP-2, MMP-24, MMP-3, MMP-7, MMP-8, MMP-9, MPIF, Mpo, MSK, MSP, mucin (Muc1), MUC18, mullerian-inhibiting substance, Mug, MuSK, NAIP, NAP, NCAD, N-C adheren, NCA 90, NCAM, NCAM, neprilysin, neurotrophin-3, -4, or -6, neurturin, nerve growth factor (NGF), NGFR, NGF-beta, nNOS, NO, NOS, Npn, NRG-3, NT, NTN, OB, OGG1, OPG, OPN, OSM, OX40L, OX40R, p150, p95, PADPr, parathyroid hormone, PARC, PARP, PBR, PBSF, PCAD, P-cadherin, PCNA, PDGF, PDGF, PDK-1, PECAM, PEM, PF4, PGE, PGF, PGI2, PGJ2, PIN, PLA2, placental alkaline phosphatase (PLAP), PIGF, PLP, PP14, proinsulin, prorelaxin, protein C, PS, PSA, PSCA, prostate-specific membrane antigen (PSMA), PTEN, PTHrp, Ptk, PTN, R51, RANK, RANKL, RANTES, RANTES, relaxin A chain, relaxin B chain, renin, respiratory syncytial virus (RSV) F, RSV Fgp, Ret, rheumatoid factor, RLIP76, RPA2, RSK, S100, SCF/KL, SDF-1, SERINE, serum albumin, sFRP-3, Shh, SIGIRR, SK-1, SLAM, SLPI, SMAC, SMDF, SMOH, SOD, SPARC, Stat, STEAP, STEAP-II, TACE, TACI, TAG-72 (tumor-associated glycoprotein-72), TARC, TCA-3, T cell receptor (e.g., T cell receptor alpha/beta), TdT, TECK, TEM1, TEM5, TEM7, TEM8, TERT, testicular PLAP-like alkaline phosphatase, TfR, TGF, TGF-alpha, TGF-beta, TGF-beta Pan Specific, TGF-beta RI (ALK-5), TGF-beta RII, TGF-beta RIIb, TGF-beta RIII, TGF-beta 1, TGF-beta 2, TGF-beta 3, TGF-beta 4, TGF-beta 5, thrombin, thymus Ck-1, thyroid stimulating hormone, Tie, TIMP, TIQ, tissue factor, TMEFF2, Tmpo, TMPRSS2, TNF, TNF-alpha, TNF-alpha/beta, TNF-beta 2, TNFc, TNF-RI, TNF-RII, TNFRSF10A (TRAIL R1 Apo-2, DR4), TNFRSF10B (TRAIL R2 DR5, KILLER, TRICK-2A, TRICK-B), TNFRSF10C (TRAIL R3 DcR1, LIT, TRID), TNFRSF10D (TRAIL R4 DcR2, TRUNDD), TNFRSF11A (RANK ODF R, TRANCE R), TNFRSF11B (OPG OCIF, TR1), TNFRSF12 (TWEAK R FN14), TNFRSF13B (TACI), TNFRSF13C (BAFF R), TNFRSF14 (HVEM ATAR, HveA, LIGHT R, TR2), TNFRSF16 (NGFR p75NTR), TNFRSF17 (BCMA), TNFRSF18 (GITR AITR), TNFRSF19 (TROY TAJ, TRADE), TNFRSF19L (RELT), TNFRSF1A (TNF RI CD120a, p55-60), TNFRSF1B (TNF RII CD120b, p75-80), TNFRSF26 (TNFRH3), TNFRSF3 (LTbR TNF RIII, TNFC R), TNFRSF4 (OX40 ACT35, TXGP1 R), TNFRSF5 (CD40 p50), TNFRSF6 (Fas Apo-1, APT1, CD95), TNFRSF6B (DcR3 M68, TR6), TNFRSF7 (CD27), TNFRSF8 (CD30), TNFRSF9 (4-1BB CD137, ILA), TNFRSF21 (DR6), TNFRSF22 (DcTRAIL, R2 TNFRH2), TNFRST23 (DcTRAIL R1 TNFRH1), TNFRSF25 (DR3 Apo-3, LARD, TR-3, TRAMP, WSL-1), TNFSF10 (TRAIL Apo-2 ligand, TL2), TNFSF11 (TRANCE/RANK ligand ODF, OPG ligand), TNFSF12 (TWEAK Apo-3 ligand, DR3 ligand), TNFSF13 (APRIL TALL2), TNFSF13B (BAFF BLYS, TALL1, THANK, TNFSF20), TNFSF14 (LIGHT HVEM ligand, LTg), TNFSF15 (TL1A/VEGI), TNFSF18 (GITR ligand AITR ligand, TL6), TNFSF1A (TNF-a Conectin, DIF, TNFSF2), TNFSF1B (TNF-b LTa, TNFSF1), TNFSF3 (LTb TNFC, p33), TNFSF4 (OX40 ligand gp34, TXGP1), TNFSF5 (CD40 ligand CD154, gp39, HIGM1, IMD3, TRAP), TNFSF6 (Fas ligand Apo-1 ligand, APT1 ligand), TNFSF7 (CD27 ligand CD70), TNFSF8 (CD30 ligand CD153), TNFSF9 (4-1BB ligand CD137 ligand), TP-1, t-PA, Tpo, TRAIL, TRAIL R, TRAIL-R1, TRAII,-R2, TRANCE, transferrin receptor, TRF, Trk, TROP-2, TLR (toll-like receptor) 1, TLR2, TLR3, TLR4, TLR5, TLR6, TLR7, TLR8, TLR9, TLR10, TSG, TSLP, tumor-associated antigen CA125, tumor-associated antigen-expressing Lewis-Y-related carbohydrate, TWEAK, TXB2, Ung, uPAR, uPAR-1, urokinase, VCAM, VCAM-1, VECAD, VE-cadherin, VE-cadherin-2, VEFGR-1 (flt-1), VEGF, VEGFR, VEGFR-3 (flt-4), VEGI, VIM, viral antigens, VLA, VLA-1, VLA-4, VNR integrin, von Willebrand factor, WIF-1, WNT1, WNT2, WNT2B/13, WNT3, WNT3A, WNT4, WNT5A, WNT5B, WNT6, WNT7A, WNT7B, WNT8A, WNT8B, WNT9A, WNT9A, WNT9B, WNT10A, WNT10B, WNT11, WNT16, XCL1, XCL2, XCR1, XCR1, XEDAR, XIAP, XPD, HMGB1, IgA, Aβ, CD81, CD97, CD98, DDR1, DDR2, DKK1, EREG, Hsp90, IL-17/IL-17R, IL-20/IL-20R, oxidized LDL, PCSK9, prekallikrein, RON, TMEM16F, SOD1, chromogranin A, chromogranin B, tau, VAP1, high-molecular-weight kininogen, IL-31, IL-31R, Nav1.1, Nav1.2, Nav1.3, Nav1.4, Nav1.5, Nav1.6, Nav1.7, Nav1.8, Nav1.9, EPCR, C1, C1q, C1r, C1s, C2, C2a, C2b, C3, C3a, C3b, C4, C4a, C4b, C5, C5a, C5b, C6, C7, C8, C9, factor B, factor D, factor H, properdin, sclerostin, fibrinogen, fibrin, prothrombin, thrombin, tissue factor, factor V, factor Va, factor VII, factor VIIa, factor VIII, factor VIIIa, factor IX, factor IXa, factor X, factor Xa, factor XI, factor XIa, factor XII, factor XIIa, factor XIII, factor XIIIa, TFPI, antithrombin III, EPCR, thrombomodulin, TAPI, tPA, plasminogen, plasmin, PAI-1, PAI-2, GPC3, syndecan-1, syndecan-2, syndecan-3, syndecan-4, LPA, S1P, and receptors for hormones or growth factors.

Although the examples of the target antigen listed above also include receptors, these receptors even existing in a soluble form in a body fluid can be used as the antigen to which the target antigen recognition site in the present invention binds. One non-limiting example of the soluble form of such a receptor can include the protein which is soluble IL-6R as described by Mullberg et al. (J. Immunol. (1994) 152 (10), 4958-4968).

Examples of the receptor can include receptors belonging to receptor families such as hematopoietic factor receptor family, cytokine receptor family, tyrosine kinase receptor family, serine/threonine kinase receptor family, TNF receptor family, G protein-coupled receptor family, GPI-anchored receptor family, tyrosine phosphatase receptor family, adhesion factor family, and hormone receptor family. The receptors belonging to these receptor families, and their features are described in many literatures, for example, reviews of Cooke BA., King RJB., van der Molen HJ. ed. New Comprehensive Biochemistry Vol. 18B "Hormones and their Actions Part II" pp. 1-46 (1988) Elsevier Science Publishers BV., or Masayuki Miyasaka ed., Cell Technology suppl. Handbook series "Adhesion Factor Handbook" (1994) (Gakken Medical Shujunsha Co., Ltd., Tokyo, Japan) as well as Patthy (Cell (1990) 61 (1), 13-14), Ullrich et al. (Cell (1990) 61 (2), 203-212), Massague (e with acute accent code) (Cell (1992) 69 (6), 1067-1070), Miyajima et al. (Annu. Rev. Immunol. (1992) 10, 295-331), Taga et al. (FASEB J. (1992) 6, 3387-3396), Fantl et al. (Annu. Rev. Biochem. (1993), 62, 453-481), Smith et al. (Cell (1994) 76 (6) 959-962), and Flower DR. (Biochim. Biophys. Acta (1999) 1422 (3) 207-234).

Specific examples of the receptors belonging to the receptor families preferably include human or mouse erythropoietin (EPO) receptor (Blood (1990) 76 (1), 31-35; and Cell (1989) 57 (2), 277-285), human or mouse granulocyte colony-stimulating factor (G-CSF) receptor (Proc. Natl. Acad. Sci. USA. (1990) 87 (22), 8702-8706, mG-CSFR, Cell (1990) 61 (2), 341-350), human or mouse thrombopoietin (TPO) receptor (Proc Natl Acad Sci USA. (1992) 89 (12), 5640-5644; and EMBO J. (1993) 12 (7), 2645-53), human or mouse insulin receptor (Nature (1985) 313 (6005), 756-761), human or mouse Flt-3 ligand receptor (Proc. Natl. Acad. Sci. USA. (1994) 91 (2), 459-463), human or mouse platelet-derived growth factor (PDGF) receptor (Proc. Natl. Acad. Sci. USA. (1988) 85 (10) 3435-3439), human or mouse interferon (IFN)-alpha, beta receptor (Cell (1990) 60 (2), 225-234; and Cell (1994) 77 (3), 391-400), human or mouse leptin receptor, human or mouse growth hormone (GH) receptor, human or mouse interleukin (IL)-10 receptor, human or mouse insulin-like growth factor (IGF)-I receptor, human or mouse leukemia inhibitory factor (LIF) receptor, and human or mouse ciliary neurotrophic factor (CNTF) receptor.

Examples of the target antigen include membrane-associated molecules expressed on cell membranes, and soluble molecules extracellularly secreted by cells. When the antigen-binding domain of the present invention binds to a soluble molecule secreted from cells, the antigen-binding domain preferably has neutralizing activity.

The solution containing the soluble molecule is not limited, and this soluble molecule may exist in a body fluid, i.e., every vascular liquid or every liquid filling between tissues or cells in living bodies. In a non-limiting aspect, the soluble molecule to which the target antigen recognition site in the present invention binds can exist in an extracellular fluid. The extracellular fluid refers to a generic name for plasma, intercellular fluid, lymph, tight connective tissues, cerebrospinal fluid, spinal fluid, aspirates, synovial fluid, or such components in the bone and cartilage, alveolar fluid (bronchoalveolar lavage fluid), ascitic fluid, pleural effusion, cardiac effusion, cyst fluid, aqueous humor (hydatoid), or such transcellular fluids (various fluids in glandular cavities resulting from the active transport or secretory activity of cells, and fluids in the lumen of the gut and other body cavities) in vertebrates.

The term "tumor antigen" refers to an antigen expressed on a cancer cell, and means a biological molecule having antigenicity, the expression of which becomes recognized in association with the malignant alteration of cells. The tumor antigen includes a tumor-specific antigen (antigen that is present only in tumor cells and is not found in other normal cells), and a tumor-associated antigen (antigen that is also present in other organs and tissues or heterogeneous and allogeneic normal cells, or antigen that is expressed during development and/or differentiation). Also, an aberrant sugar chain that appears on cell surface or a protein molecule during cell canceration is the tumor antigen and is also called cancer sugar chain antigen. In one aspect of the present invention, the target antigen is a tumor antigen.

Examples of the tumor antigen preferably include GPC3 which belongs as the receptor described above to the GPI-anchored receptor family and is expressed in some cancers including liver cancer (Int J Cancer. (2003) 103 (4), 455-65), EpCAM which is expressed in a plurality of cancers including lung cancer (Proc Natl Acad Sci USA. (1989) 86 (1), 27-31) (its polynucleotide sequence and polypeptide sequence are described in RefSeq registration Nos. NM_002354.2 and NP_002345.2, respectively), EGFR, CA19-9, CA15-3, sialyl SSEA-1 (SLX), Her2, Her3, prostate stem cell antigen (PSCA), alphafetoprotein (AFP), cancer embryonic antigen (CEA), tumor antigen-125 (CA-125), calretinin, MUC-1, MUC-16, epithelial membrane protein (EMA), epithelial tumor antigen (ETA), tyrosinase, melanoma-associated antigen (MAGE), chromogranin, cytokeratin, desmin, glial fibrillary acidic protein (GFAP), gross cystic disease fluid protein (GCDFP-15), HMB-45 antigen, protein melan-A (melanoma antigen that is recognized by T lymphocytes; MART-1), myo-D1, muscle-specific actin (MSA), neurofilament, neuron-specific enolase (NSE), placental alkaline phosphatase, synaptophysin, thyroglobulin, thyroid transcription factor-1, a dimer form of pyruvate kinase isozyme type M2 (tumor M2-PK), GD2 (ganglioside G2), EGFRvIII (epidermal growth factor variant III), sperm protein 17 (Sp17), mesothelin, PAP (prostatic acid phosphatase), prostein, TARP (T cell receptor gamma alternate reading frame protein), Trp-p8, STEAP1 (six-transmembrane epithelial antigen of prostate member 1), TROP-2, Claudin 6, RNF43a, aberrant ras protein or aberrant p53 protein, integrin alpha v beta 3 (CD61), galectin, K-Ras (V-Ki-ras2 Kirsten rat sarcoma viral oncogene), and Ral-B.

Further examples thereof include: thyroid-stimulating hormone receptor (TSHR); CD171; CS-1 (CD2 subset 1, CRACC, SLAMF7, CD319 and 19A24); C-type lectin-like molecule-1 (CLL-1); ganglioside GD3 (aNeu5Ac(2-8)aNeu5Ac(2-3)bDGalp(1-4)bDGlcp(1-1)Cer); Tn antigen (Tn Ag); T antigen (T Ag); Fms-like tyrosine kinase 3 (FLT3); CD38; CD44v6; B7H3 (CD276); KIT (CD117); interleukin-13 receptor subunit alpha-2 (IL-13Ra2); interleukin 11 receptor alpha (IL-11Ra); interleukin 2 receptor alpha (IL-2Ra); prostate stem cell antigen (PSCA); protease serine 21 (PRSS21); vascular endothelial cell growth factor receptor 2 (VEGFR2); Lewis (Y) antigen; CD24; platelet-derived growth factor receptor beta (PDGFR- beta); stage-specific embryonic antigen-4 (SSEA-4); neural cell adhesion molecule (NCAM); carbonic anhydrase IX (CAIX); proteasome (prosome, macropain) subunit, beta, 9 (LMP2); ephrin type-A receptor 2 (EphA2); fucosyl GM1; sialyl Lewis adhesion molecule (sLe); ganglioside GM3 (aNeu5Ac(2-3)bDGalp(1-4)bDGlcp(1-1)Cer; TGS5; high-molecular-weight melanoma-associated antigen (HMWMAA); o-acetyl-GD2 ganglioside (OAcGD2); folate receptor beta; tumor endothelial marker 1 (TEM1/CD248); tumor endothelial marker 7 related (TEM7R); claudin 6 (CLDN6); G protein-coupled receptor glass C group 5, member D (GPRC5D); chromosome X open reading frame 61 (CXORF61); CD97; CD179a; anaplastic lymphoma kinase (ALK); polysialic acid; placenta specific 1 (PLAC1); a hexasaccharide moiety of globoH glycoceramide (GloboH); mammary gland differentiation antigen (NY-BR-1); uroplakin 2 (UPK2); hepatitis A virus cellular receptor 1 (HAVCR1); adrenaline receptor beta 3 (ADRB3); pannexin 3 (PANX3); G protein-coupled receptor 20 (GPR20); lymphocyte antigen 6 complex, locus K9 (LY6K); olfactory receptor 51E2 (OR51E2); TCR gamma alternate reading frame protein (TARP); Wilms' tumor protein (WT1); ETS translocation variant gene 6, located on chromosome 12p (ETV6-AML); sperm protein 17 (SPA17); X antigen family, member 1A (XAGE1); angiopoietin binding cell surface receptor 2 (Tie 2); melanoma cancer-testis antigen-1 (MAD-CT-1); melanoma cancer-testis antigen-2 (MAD-CT-2); Fos-related antigen 1; p53 mutants; human telomerase reverse transcriptase (hTERT); sarcoma translocation breakpoint; melanoma inhibitor of apoptosis (ML-IAP); ERG (transmembrane protease, serine 2 (TMPRSS2)-ETS fusion gene); N-acetylglucosaminyl-transferase V (NA17); paired box protein Pax-3 (PAX3); androgen receptor; cyclin B1; v-myc avian myelocytomatosis viral oncogene neuroblastoma-derived homolog (MYCN); Ras homolog family member C (RhoC); cytochrome P450 1B1 (CYP1B1); CCCTC binding factor (zinc finger protein)-like (BORIS); squamous cell carcinoma antigen recognized by T cells 3 (SART3); paired box protein Pax-5 (PAX5); proacrosin binding protein p32 (OY-TES1); lymphocyte-specific protein tyrosine kinase (LCK); A kinase anchoring protein 4 (AKAP-4); synovial sarcoma, X breakpoint 2 (SSX2); CD79a; CD79b; CD72; leukocyte-associated immunoglobulin like receptor 1 (LAIR1); Fc fragment of IgA receptor (FCAR); leukocyte immunoglobulin-like receptor subfamily A member 2 (LILRA2); CD300 molecule-like family member f (CD300LF); C-type lectin domain family 12 member A (CLEC12A); bone marrow stromal antigen 2 (BST2); EGF-like module-containing mucin-like hormone receptor-like 2 (EMR2); lymphocyte antigen 75 (LY75); glypican-3 (GPC3); Fc receptor-like 5 (FCRL5); and immunoglobulin lambda-like polypeptide 1 (IGLL1).

The "MHC antigen" is a gene product of major histocompatibility complex (MHC). Among such antigens, glycoproteins expressed on cell membrane are mainly classified into MHC class I antigens and MHC class II antigens. The MHC class I antigens include HLA-A, -B, -C, -E, -F, -G, and -H, and the MHC class II antigens include HLA-DR, -DQ, and -DP. Tumor antigen-derived peptides presented on these MHC antigens are also included therein. A tumor antigen such as GP100, MART-1, or MAGE-1, MAGE-A4, NY-ESO-1 or a complex with MHC presenting a mutated site, such as RAS or p53, is also regarded as one of the tumor antigens.

The "differentiation antigen" is a generic name for cell surface molecules that appear or disappear in association with the differentiation of bone marrow stem cells into macrophages, T cells, B cells or the like. The differentiation antigen may include CD1, CD2, CD4, CD5, CD6, CD7, CD8, CD10, CD11a, CD11b, CD11c, CD13, CD14, CD15s, CD16, CD18, CD19, CD20, CD21, CD22, CD23, CD25, CD27, CD28, CD29, CD30, CD32, CD33, CD34, CD35, CD38, CD40, CD41a, CD41b, CD42a, CD42b, CD43, CD44, CD45, CD45RO, CD48, CD49a, CD49b, CD49c, CD49d, CD49e, CD49f, CD51, CD54, CD55, CD56, CD57, CD58, CD61, CD62E, CD62L, CD62P, CD64, CD69, CD70, CD71, CD73, CD95, CD99, CD102, CD106, CD117, CD122, CD126, and CDw130.

In general, the term "tumor" is a generic name for tissue masses that are formed by autonomous and excessive growth of tissues and cells against *in vivo* control. The tumor includes malignant tumor having three features, autonomous growth, infiltration and metastasis, and cachexia, and benign tumor characterized only by autonomous growth. The malignant tumor "cancer" refers to a disease characterized by the uncontrollable growth of aberrant cells. Cancer cells can spread locally or via bloodstream and the lymphatic system to other parts of the body. Examples of various cancers are described in the present disclosure and include, but are not limited to, breast cancer, prostate cancer, ovary cancer, cervical cancer, skin cancer, pancreatic cancer, colorectal cancer, kidney cancer, liver cancer, brain cancer, lymphoma, leukemia, lung cancer and the like. The terms "tumor" and "cancer" are interchangeably used in the present disclosure. Both the terms encompass, for example, solid and liquid, for example, diffuse or circulating, tumors. As used in the present disclosure, the term "cancer" or "tumor" encompasses premalignant as well as malignant cancers and tumors.

Examples of the cancer for the anticancer agent or the method for treating a cancer as mentioned later according to the present disclosure can include cancers such as adenocarcinoma, squamous cell carcinoma, adenosquamous cancer, undifferentiated cancer, large-cell cancer, small-cell cancer, skin cancer, breast cancer, prostate cancer, bladder cancer, vaginal cancer, neck cancer, uterus cancer, liver cancer, kidney cancer, pancreatic cancer, spleen cancer, lung cancer, tracheal cancer, bronchial cancer, colorectal cancer, small intestine cancer, stomach cancer, esophageal cancer, gallbladder cancer, testis cancer, and ovary cancer, cancers of bone tissues, cartilage tissues, fat tissues, muscle tissues, vascular tissues and hematopoietic tissues as well as sarcoma such as chondrosarcoma, Ewing's sarcoma, malignant hemangioendothelioma, malignant schwannoma, osteosarcoma, and soft tissue sarcoma, blastoma such as hepatoblastoma, medulloblastoma, nephroblastoma, neuroblastoma, pancreatoblastoma, pleuropulmonary blastoma, and retinoblastoma, germ cell tumor, lymphoma, and leukemia.

In one embodiment, the tumor antigen in association with a cancer type is a marker expressed by both normal cells and cancer cells, for example, a lineage marker such as CD19 on B cells. In a certain aspect, the tumor antigen of the present disclosure is derived from a cancer including, but is not limited to, primary or metastatic melanoma, thymoma, lymphoma, sarcoma, lung cancer, colorectal cancer, liver cancer, non-Hodgkin's lymphoma, Hodgkin's lymphoma, leukemias, uterus cancer, cervical cancer, bladder cancer, kidney cancer and adenocarcinoma, for example, breast cancer, prostate cancer, ovary cancer, pancreatic cancer, and the like. In one embodiment, the tumor antigen is an antigen common in particular proliferative disorders. In one embodiment, the tumor-associated antigen is a cell surface molecule that is overexpressed in cancer cells compared with normal cells, for example, by 1-fold overexpression, 2-fold overexpression, 3-fold overexpression or more as compared with normal cells. In some embodiments, the tumor-associated antigen is a cell surface molecule that is inappropriately synthesized in cancer cells, for example, a molecule containing deletion, addition or mutation as compared with a molecule expressed in normal cells. In one embodiment, the tumor-associated antigen is expressed exclusively on the cell surface of cancer cells, as a whole or as a fragment (e.g., MHC/peptide), and neither synthesized nor expressed on the surface of normal cells. Usually, peptides derived from endogenous proteins fill the pockets of major histocompatibility complex (MHC) class I molecules, and are recognized by T cell receptors (TCRs) on $CD8^+$ T lymphocytes. The MHC class I complex is constitutively expressed by all nucleated cells. In cancers, virusspecific and/or tumor-specific peptide/MHC complexes are representative of a unique class of cell surface targets for immunotherapy. TCR-like antibodies targeting virus- or tumor antigen-derived peptides in the context of human leukocyte antigen (HLA)-A1 or HLA-A2 are described [see e.g., Sastry et al., J Virol.2011 85(5): 1935-1942; Sergeeva et al., Bood, 2011 117(16): 4262-4272; Verma et al., J Immunol 2010 184(4): 2156-2165; Willemsen et al., Gene Ther 2001 8(21): 1601-1608; Dao et al., Sci Transl Med 2013 5(176): 176ra33;Tassev et al., Cancer Gene Ther 2012 19(2): 84-100]. The TCR-like antibody can be identified, for example, by screening a library such as a human scFv phage display library.

Examples of the antigen to which the antigen-binding molecule in the present disclosure binds include viral antigens, bacterial (particularly, infectious bacteria) antigens, parasite antigens, cell surface markers on target cells related to particular pathological conditions (e.g., tumor antigens), and surface molecules of immunocytes causing autoimmunity.

One aspect of the present disclosure provides a chimeric receptor capable of binding to an antigen derived from the family *Retroviridae* (e.g., human immunodeficiency virus, for example, HIV-1 and HIV-LP), the family *Picornaviridae* (e.g., poliovirus, hepatitis A virus, enterovirus, human coxsackievirus, rhinovirus, and echovirus), rubella virus, coronavirus, vesicular stomatitis virus, rabies virus, Ebola virus, parainfluenza virus, mumps virus, measles virus, respiratory syncytial virus, influenza virus, hepatitis B virus, parvovirus, the family *Adenoviridae,* the family *Herpesviridae* [e.g., type 1 and type 2 herpes simplex virus (HSV), varicella-zoster virus, cytomegalovirus (CMV), and herpes virus], the family *Poxviridae* (e.g. smallpox virus, vaccinia virus, and pox virus), or hepatitis C virus, via the antigen-binding molecule.

Another aspect of the present disclosure provides a chimeric receptor capable of binding to an antigen derived from a bacterial strain of the genus *Staphylococci,* the genus *Streptococcus, Escherichia coli, Pseudomonas,* or the genus *Salmonella,* via the antigen-binding molecule. Particularly, the present disclosure provides a chimeric receptor capable of binding to an antigen derived from an infectious bacterium, for example, *Helicobacter pyloris, Legionella pneumophilia*, a bacterial strain of *Mycobacteria* sps. (e.g. *M. tuberculosis, M. avium, M. intracellulare, M. kansasii*, or *M. gordonae, Staphylococcus aureus, Neisseria gonorrhoeae, Neisseria meningitides, Listeria monocytogenes, Streptococcus pyogenes,* Group A *Streptococcus,* Group B *Streptococcus* (*Streptococcus agalactiae*), *Streptococcus pneumoniae,* or *Clostridium tetani,* via the antigen-binding molecule.

In another aspect of the present disclosure, the antigen-binding molecule is capable of binding to a tumor antigen such as 5T4, alpha 5 beta 1-integrin, 707-AP, AFP, ART-4, B7H4, B7H3, BAGE, beta-catenin/m, Bcr-abl, MN/C IX antibody, CA125, CAMEL, CAP-1, CASP-8, CD4, CD19, CD20, CD22, CD25, CDC27/m, CD30, CD33, CD52, CD56, CD80, CDK4/m, CEA, CT, Cyp-B, DAM, EGFR, ErbB3, ELF2M, EMMPRIN, EpCam, ETV6-AML1, G250, GAGE, GnT-V, Gp100, Gpc3, Gpr20, HAGE, HER-2/neu, HLA-A*0201-R170I, HPV-E7, HSP70-2M, HST-2, hTERT (or hTRT), iCE, IGF-1R, IL-2R, IL-5, KIAA0205, LAGE, LDLR/FUT, MAGE, MART-1/melan-A, MART-2/Ski, MC1R, myosin/m, MUC1, MUC-16, MUM-1, MUM-2, MUM-3, NA88-A, PAP, proteinase-3, p190 minor bcr-abl, Pml/RAR alpha, PRAME, PSA, PSM, PSMA, RAGE, RU1 or RU2, SAGE, SART-1 or SART-3, survivin, TEL/AML1, TGF beta, TPI/m, Trop-2, TRP-1, TRP-2, TRP-2/INT2, VEGF, WT1, NY-Eso-1 or NY-Eso-B. In the present disclosure, the antibody is also capable of binding to a cell surface adhesion molecule, a surface molecule of inflammatory cells found in autoimmune diseases, or a TCR causing autoimmunity.

In the present specification, the "immunoreceptor recognition site" is a site of an antigen-binding molecule capable of recognizing an immunoreceptor of interest and binding specifically to the immunoreceptor, and can also be referred to as an immunoreceptor binding domain. The immunoreceptor recognition site may be a site having any structure as long as it binds to an immunoreceptor of interest. Examples of such a site include, but are not limited to, a heavy chain variable region (VH) of an antibody and a light chain variable region (VL) of the antibody, which recognize an immunoreceptor as an antigen, a single-domain antibody (sdAb), a module called A domain of approximately 35 amino acids contained in an *in vivo* cell membrane protein avimer (International Publication Nos. WO2004/044011 and WO2005/040229), adnectin containing a 10Fn3 domain serving as a protein binding domain derived from a glycoprotein fibronectin expressed on cell membranes (International Publication No. WO2002/032925), Affibody containing an IgG binding domain scaffold constituting a three-helix bundle composed of 58 amino acids of protein A (International Publication No. WO1995/001937), DARPins (designed ankyrin repeat proteins) which are molecular surface-exposed regions of ankyrin repeats (AR) each having a 33-amino acid residue structure folded into a subunit of a turn, two antiparallel helices, and a loop (International Publication No. WO2002/020565), anticalin having four loop regions connecting eight antiparallel strands bent toward the central axis in one end of a barrel structure highly conserved in lipocalin molecules such as neutrophil gelatinase-associated lipocalin (NGAL) (International Publication No. WO2003/029462), and a depressed region in the internal parallel sheet structure of a horseshoe-shaped fold composed of repeated leucine-rich-repeat (LRR) modules of an immunoglobulin structure-free variable lymphocyte receptor (VLR) as seen in the acquired immune systems of jawless vertebrates such as lamprey or hagfish (International Publication No. WO2008/016854).

Preferred examples of the immunoreceptor recognition site in the present invention include a heavy chain variable region (VH) of an antibody and a light chain variable region (VL) of the antibody, which recognizes an immunoreceptor as an antigen, and particularly preferred examples thereof include an immunoreceptor recognition site in a multispecific antibody having a target antigen recognition site, for example, an immunoreceptor recognition site in a bispecific antibody. When the site capable of recognizing and binding to a specific immunoreceptor of the antigen-binding molecule in the present invention is a part of the antibody which recognizes an immunoreceptor as an antigen, the immunoreceptor recognition site is sometimes referred to as an antigen-binding domain. For example, the multispecific antibody has a plurality of antigen-binding domains. Among them, antigen-binding domains capable of recognizing and binding to a target antigen function as target antigen-binding sites, and antigen-binding domains capable of recognizing and binding to an immunoreceptor function as immunoreceptor recognition sites. Therefore, the target antigen-binding site and the immunoreceptor recognition site of the multispecific antibody both correspond to antigen-binding domains of the multispecific antibody.

Examples of the immunoreceptor recognized by the antigen-binding molecule include an immunoreceptor on a T cell, and an immunoreceptor on a natural killer (NK) cell. Specific examples thereof include TIGIT, PD-1, PD-L1, TIM-3, LAG-3, BTLA, CD268, CD267, CD266, CD226, CD160, CD137, CD96, CD70, CD47, CD40, CD30, CD28, CD27, CD18, NKG2D, VISTA, ICOS, B7-HE, GITR, OX40, KIR, SLAM7, CTLA-4, RANK, osteoprotegerin, BCMA, TNFR1, TNFR2, Fas, DR1, DR2, DR3, DR4, DR5, DR6, HVEM, NGFR, EDA2R, TR6, and TROY.

In one aspect, the immunoreceptor recognition site of the antigen-binding molecule which is used may be an agonist antibody for a costimulatory molecule belonging to a tumor necrosis factor receptor superfamily (TNFRSF) (hereinafter, referred to as a TNFRSF agonist antibody), or an antigen-binding portion thereof. The TNFRSF agonist means an antibody which, when added to a cell, a tissue or a living organism expressing a TNF receptor superfamily, activates the cell expressing the TNF receptor superfamily by at least about 5%, specifically by at least about 10%, more specifically by at least about 15%, where the activation level achieved under physiological conditions with an equimolar amount of a binding partner is defined as 100% activation. In various specific examples, the TNFRSF agonist antibody used as the pharmaceutical composition of the present invention can activate the cell by at least about 20%, 30%, 40%, 50%, 60%, 70%, 80%, 90%, 100%, 125%, 150%, 175%, 200%, 250%, 300%, 350%, 400%, 450%, 500%, 750%, 1000% or more. In one aspect of the present invention, the immunoreceptor recognition site of the antigen-binding molecule binds as an agonist to the endogenous immunoreceptor, and also binds as an agonist to the chimeric receptor of the present invention.

The target molecule of the TNFRSF agonist antibody is not particularly limited as long as it is a factor which activates cells expressing the TNF receptor superfamily (e.g. T cells and NK cells). Examples of the preferred factor include CD137 and CD40. Examples of the more preferred factor include CD137. Examples of the CD137 agonist antibody include Urelumab (CAS Registry Number: 934823-49-1), and various known CD137 agonist antibodies.

Examples of the CD137 agonist antibody include the following antibodies represented by SEQ ID NOS described in, for example, WO2015/156268:

[1] an antibody having an amino acid sequence set forth as SEQ ID NO: 66 as a heavy chain variable region and an amino acid sequence set forth as SEQ ID NO: 85 as a light chain variable region;

[2] an antibody having an amino acid sequence set forth as SEQ ID NO: 67 as a heavy chain variable region and an amino acid sequence set forth as SEQ ID NO: 86 as a light chain variable region;

[3] an antibody having an amino acid sequence set forth as SEQ ID NO: 70 as a heavy chain variable region and an amino acid sequence set forth as SEQ ID NO: 89 as a light chain variable region;

[4] an antibody having an amino acid sequence set forth as SEQ ID NO: 76 as a heavy chain variable region and an amino acid sequence set forth as SEQ ID NO: 95 as a light chain variable region;

[5] an antibody having an amino acid sequence set forth as SEQ ID NO: 77 as a heavy chain variable region and an amino acid sequence set forth as SEQ ID NO: 96 as a light chain variable region;

[6] an antibody having an amino acid sequence set forth as SEQ ID NO: 78 as a heavy chain variable region and an amino acid sequence set forth as SEQ ID NO: 97 as a light chain variable region;

[7] the antibody according to any of [1] to [6], which has an amino acid sequence set forth as SEQ ID NO: 99 as a heavy chain constant region and an amino acid sequence set forth as SEQ ID NO: 59 or an amino acid sequence set forth as SEQ ID NO: 60 as a light chain constant region;

[8] an antibody equivalent in activity to the antibody according to any of [1] to [7]; and

[9] an antibody which binds to an epitope identical to an epitope to which the antibody according to any of [1] to [7] binds.

In the antibody according to [8], the term "equivalent in activity" means that the agonist activity on CD137 is 70% or more, preferably 80% or more, more preferably 90% or more of the binding activity of the antibody according to any of [1] to [7].

The present invention also provides the antibody according to [9], which binds to an epitope identical to an epitope to which the anti-CD137 antibody disclosed in the present invention binds. Such an antibody can be obtained by, for example, the following method.

Preferred examples of the antibody which binds to an epitope identical to an epitope to which the antibody according to any of [1] to [7] binds include an antibody which recognizes a region having the sequence of SPCPPNSFSSAGGQRTCDICRQCKGVFRTRKECSSTSNAECDCTPGFHCLGAGCSMCE QDCKQGQELTKKGC (SEQ ID NO: 15 described in WO2015/156268) in the CD137 protein. Further, examples thereof include an antibody which recognizes a region having the sequence of DCTPGFHCLGAGCSMCEQDCKQGQELTKKGC (SEQ ID NO: 16 described in WO2015/156268) in the CD137 protein.

By altering the anti-human CD137 antibody to a bispecific antibody with a tumor-specific antigen antibody (e.g. anti-human GPC3 antibody) and evaluating the tumor-specific antigen-dependent CD137 agonist ability, an anti-tumor antigen/anti-human CD137 bispecific antibody exhibiting a desired effect can be provided. Such an antibody can be used as the antigen-binding molecule according to the present invention.

The epitope, which means an antigenic determinant, present in the antigen means a site on the antigen (target antigen or immunoreceptor) to which the antigen-binding molecule disclosed in the present specification binds. Accordingly, for example, the epitope can be defined by its structure. Alternatively, the epitope can be defined by the antigen-binding activity of the antigen-binding molecule which recognizes the epitope. When the antigen is a peptide or a polypeptide, it is also possible to identify the epitope by amino acid residues constituting the epitope. When the epitope is a sugar chain, it is also possible to identify the epitope by a particular sugar chain structure.

A linear epitope refers to an epitope comprising an epitope that is recognized by its primary sequence of amino acids. The linear epitope contains typically at least 3 and most commonly at least 5, for example, approximately 8 to approximately 10 or 6 to 20 amino acids, in its unique sequence.

In contrast to the linear epitope, a conformational epitope refers to an epitope that is contained in a primary sequence of amino acids containing a component other than the single defined component of the epitope to be recognized (e.g., an epitope whose primary sequence of amino acids may not be recognized by an antibody that determines the epitope). The conformational epitope may contain an increased number of amino acids, as compared with the linear epitope. As for the recognition of the conformational epitope, the target antigen recognition site or the immunoreceptor recognition site of the antigen-binding molecule recognizes the three-dimensional structure of the peptide or the protein. For example, when a protein molecule is folded to form a three-dimensional structure, certain amino acids and/or polypeptide backbone constituting the conformational epitope are arranged in parallel to allow the antibody to recognize the epitope. Examples of the method for determining the conformation of the epitope include, but are not limited to, X-ray crystallography, twodimensional nuclear magnetic resonance spectroscopy, and site-specific spin labeling and electron paramagnetic resonance spectroscopy. See, for example, Epitope Mapping Protocols in Methods in Molecular Biology (1996), Vol. 66, Morris ed.

The structure of the target antigen recognition site or the immunoreceptor recognition site of the antigen-binding molecule which binds to the epitope of the target antigen or the immunoreceptor is called a paratope. The paratope stably binds to the epitope through a hydrogen bond, electrostatic force, van der Waals' forces, a hydrophobic bond, or the like acting between the epitope and the paratope. This binding force between the epitope and the paratope is called affinity. The total binding force when a plurality of antigen-binding domains bind to a plurality of antigens is called avidity. The affinity works synergistically when, for example, an antibody comprising a plurality of antigen-binding domains (i.e., a polyvalent antibody) bind to a plurality of epitopes. Therefore, the avidity is higher than the affinity.

In a particular embodiment, the antigen-binding molecule provided in the present specification has a dissociation constant (Kd) of $\leq 1\ \mu M$, $\leq 100$ nM, $\leq 10$ nM, $\leq 1$ nM, $\leq 0.1$ nM, $\leq 0.01$ nM or $\leq 0.001$ nM (e.g. $10^{-8}$ (1E-08) M or less, for example, $10^{-8}$ (1E-08) M to $10^{-13}$ (1E-13) M, for example, $10^{-9}$ (1E-09) M to $10^{-13}$ (1E-13) M).

A method for confirming the binding of an antigen-binding domain directed to the antigen, or an antigen-binding molecule comprising the antigen-binding domain as a target antigen recognition site or an immunoreceptor recognition site to the epitope can be appropriately carried out according to the example given below.

For example, whether the antigen-binding domain directed to an antigen recognizes a linear epitope present in the certain antigen molecule when the antigen is a receptor expressed on a cell surface can be confirmed, for example, as follows: a linear peptide comprising an amino acid sequence constituting the extracellular domain of a certain antigen is synthesized for the purpose described above. The peptide can be chemically synthesized. Alternatively, the peptide is obtained by a genetic engineering approach using a region encoding an amino acid sequence corresponding to the extracellular domain in a certain antigen cDNA. Next, the antigen-binding domain directed to a certain antigen is evaluated for its binding activity against the linear peptide comprising an amino acid sequence constituting the extracellular domain. For example, the binding activity of the antigen-binding domain against the peptide can be evaluated by ELISA using an immobilized linear peptide as an antigen. Alternatively, the binding activity against the linear peptide may be determined on the basis of a level at which the linear peptide inhibits the binding of the antigen-binding domain to a certain antigen-expressing cells. These tests can determine the binding activity of the antigen-binding domain against the linear peptide.

Also, whether the target antigen recognition site or the immunoreceptor recognition site recognizes the conformational epitope can be confirmed as follows: Cells expressing a target antigen or an immunoreceptor are prepared. The recognition of the conformational epitope by the target antigen recognition site or the immunoreceptor recognition site is confirmed, for example, when the target antigen recognition site or the immunoreceptor recognition site directed to the target antigen or the immunoreceptor strongly binds to the certain cells upon contact with the cells, whereas the recognition site does not substantially bind to an immobilized linear peptide comprising an amino acid sequence constituting a certain target antigen or immunoreceptor, or a linear peptide denatured with a general denaturant such as guanidine and comprising an amino acid sequence constituting the extracellular domain of a certain target antigen or immunoreceptor. In this context, the term "not substantially bind" means that the binding activity of the latter is 80% or less, usually 50% or less, preferably 30% or less, particularly preferably 15% or less of binding activity against the target antigen or immunoreceptor expressing cells.

The method for confirming the target antigen or immunoreceptor binding activity of the antigen-binding molecule also includes a method of measuring a Kd value by, for example, radiolabeled antigen binding assay (RIA). In one embodiment, RIA is carried out using the antigen-binding molecule, and the target antigen, the immunoreceptor or a portion thereof. For example, the binding affinity in a solution of the antigen-binding molecule is measured by equilibrating the antigen-binding domain with the smallest concentration of a (125I)-labeled antigen in the presence of a titration series of an unlabeled antigen, and subsequently capturing the bound antigen by a plate coated with the antigen-binding domain (see e.g., Chen et al., J. Mol. Biol. 293: 865-881 (1999)).

According to an alternative embodiment, Kd is measured by a surface plasmon resonance method using BIACORE (registered trademark). For example, assay using BIACORE (registered trademark)-2000 or BIACORE (registered trademark)-3000 (BIAcore, Inc., Piscataway, NJ) is carried out at 25°C using a CM5 chip with about 10 response units (RU) of the antigen immobilized thereon. In one embodiment, a carboxymethylated dextran biosensor chip (CM5, BIAcore, Inc.) is activated using N-ethyl-N'-(3-dimethylaminopropyl)-carbodiimide hydrochloride (EDC) and N-hydroxysuccinimide (NHS) according to the supplier's instruction. The antigen is diluted to 5 $\mu$g/ml (about 0.2 $\mu$M) with 10 mM sodium acetate (pH 4.8) and then injected thereto at a flow rate of 5 $\mu$l/min so as to attain protein binding at about 10 response units (RU). After the antigen injection, 1 M ethanolamine is injected thereto in order to block unreacted groups. For kinetic measurement, 2-fold dilutions (0.78 nM to 500 nM) of the antigen-binding molecule (target antigen recognition site or immunoreceptor recognition site) in PBS containing 0.05% Polysorbate 20 (TWEEN-20 (registered trademark)) as a surfactant (PBST) are injected thereto at a flow rate of about 25 $\mu$l/min at 25°C. An association rate (kon) and a dissociation rate (koff) are calculated by fitting sensorgrams of association and dissociation at the same time using a simple 1: 1 Langmuir binding model (BIACORE (registered trademark) evaluation software version 3.2). An equilibrium dissociation constant (Kd) is calculated as a koff/kon ratio. Furthermore, an apparent dissociation constant (Kd) may be determined by use of equilibrium analysis. For these procedures, see the protocol attached to BIACORE (registered trademark). See, for example, Chen et al., J. Mol. Biol. 293: 865-881 (1999) and Methods Enzymol. 2000; 323: 325-40. In the surface plasmon resonance assay, the amount of the protein immobilized, the amount of the protein used in reaction, temperature, and solution composition can be variously changed by those skilled in the art. When the on-rate in the surface plasmon resonance assay described above exceeds $10^6 M^{-1}s^{-1}$, the on-rate can be determined by use of a fluorescence quenching technique of using a spectrometer (e.g. a stopped-flow spectrophotometer (Aviv Instruments, Inc.) or SLM-AMINCO(TM) spectrophotometer 8000 series (Thermo Spectronic/Thermo Fisher Scientific Inc.) using a stirring cuvette) to measure increase or decrease in fluorescence intensity (excitation = 295 nm; emission = 340 nm, band path: 16 nm) at 25°C for 20 nM antigen-binding domain in PBS (pH 7.2) in the presence of gradually increased concentrations of the antigen.

Furthermore, the target antigen or immunoreceptor binding activity of the antigen-binding molecule can also be measured by a known molecule-molecule interaction measurement method such as electrogenerated chemiluminescence. Instead of the antigen-binding molecule used in the assay described in the present specification, a portion corresponding to the target antigen recognition site or the immunoreceptor recognition site can be used, and as the target antigen recognition site or the immunoreceptor recognition site, a portion corresponding to the target antigen recognition site or the immunoreceptor recognition site, or an antigen-binding molecule can be used.

Examples of the method for measuring the binding activity of the antigen-binding molecule against cells expressing a target antigen or an immunoreceptor include methods described in Antibodies: A Laboratory Manual (Ed Harlow, David Lane, Cold Spring Harbor Laboratory (1988) 359-420). Specifically, the binding activity can be evaluated on the basis of the principle of ELISA or FACS (fluorescence activated cell sorting) using cells expressing a target antigen or an immunoreceptor as an antigen.

In the ELISA format, the binding activity of an antigen-binding molecule is quantitatively evaluated by comparing the levels of signals generated through enzymatic reaction. Specifically, a test antigen-binding molecule is added to an ELISA plate with the certain antigen-expressing cells immobilized thereon. Then, the test antigen-binding molecule bound with the cells is detected through the use of an enzyme-labeled antibody which recognizes the test antigen-binding domain. Alternatively, in the FACS, a dilution series of a test antigen-binding molecule is prepared, and the antibody binding titer for the certain antigen-expressing cells can be determined to compare the binding activity of the test antigen-binding molecule against the certain antigen-expressing cells.

The binding of the test antigen-binding molecule to the target antigen or the immunoreceptor expressed on the surface of cells suspended in a buffer solution or the like can be detected using a flow cytometer. For example, the following apparatuses are known as the flow cytometer:

FACSCanto™II
FACSAria™
FACSArray™
FACSVantage™SE
FACSCalibur™ (all are trade names of BD Biosciences)
EPICS ALTRA HyPerSort
Cytomics FC 500
EPICS XL-MCL ADC EPICS XL ADC
Cell Lab Quanta/Cell Lab Quanta SC (all are trade names of Beckman Coulter, Inc.)

One preferred example of the method for measuring the target antigen or immunoreceptor binding activity of the antigen-binding molecule includes the following method: first, a certain antigen-expressing cells reacted with a test antigen-binding molecule are stained with a FITC-labeled secondary antibody which recognizes the test antigen-binding domain. The test antigen-binding molecule is appropriately diluted with a suitable buffer solution to prepare the antigen-binding molecule at the desired concentration for use. The antigen-binding molecule can be used, for example, at any concentration from 10 μg/ml to 10 ng/ml. Next, fluorescence intensity and the number of cells are measured using FACSCalibur™ (Becton, Dickinson and Company). The amount of the antigen-binding molecule bound to the cells is reflected in the fluorescence intensity obtained by analysis using CELL QUEST Software (Becton, Dickinson and Company), i.e., a geometric mean value. In short, the binding activity of the test antigen-binding molecule indicated by the amount of the test antigen-binding molecule bound can be determined by obtaining the geometric mean value.

Whether a certain antigen-binding molecule shares an epitope with another antigen-binding molecule can be confirmed by the competition between these antigen-binding molecules for the same epitope. The competition between the antigen-binding molecules is detected by cross-blocking assay or the like. The cross-blocking assay is preferably,

**EP 4 197 545 A1**

for example, competitive ELISA assay.

Specifically, in the cross-blocking assay, a certain target antigen- or immunoreceptor-derived antigen protein applied onto wells of a microtiter plate are preincubated in the presence or absence of a candidate competitor antigen-binding molecule. Then, the test antigen-binding molecule is added thereto. The amount of the test antigen-binding molecule bound with the certain antigen protein in the wells indirectly correlates with the binding capacity of the candidate competitor antigen-binding molecule that competes for the binding to the same epitope. In short, larger affinity of the competitor antigen-binding molecule for the same epitope means lower binding activity of the test antigen-binding molecule against the certain antigen protein-coated wells.

The amount of the test antigen-binding molecule bound with the wells via the antigen protein can be easily measured by labeling the antigen-binding molecule in advance. For example, a biotin-labeled antigen-binding molecule is assayed by using an avidinperoxidase conjugate and an appropriate substrate. In particular, cross-blocking assay that utilizes enzyme labels such as peroxidase is called competitive ELISA assay. The antigen-binding molecule can be labeled with an alternative detectable or measurable labeling material. Specifically, radiolabels, fluorescent labels, and the like are known in the art.

Provided that the competitor antigen-binding molecule can block the binding of the antigen-binding molecule directed to the certain antigen by at least 20%, preferably at least 20 to 50%, more preferably at least 50% as compared with binding activity obtained in a control test carried out in the absence of the candidate competitor antigen-binding molecule aggregate, the test antigen-binding molecule is determined as an antigen-binding molecule which substantially binds to the same epitope as that for the competitor antigen-binding molecule, or competes for the binding to the same epitope.

When the epitope to which the antigen-binding molecule binds has an identified structure, whether the test antigen-binding molecule and a control antigen-binding molecule share an epitope can be evaluated by comparing the binding activity of these antigen-binding molecules against a peptide or a polypeptide prepared by introducing an amino acid mutation to a peptide constituting the epitope.

In such a method for measuring binding activity, for example, the binding activity of a test antigen-binding molecule and a control antigen-binding molecule against a linear peptide containing an introduced mutation can be compared in the ELISA format described above to be measured. In a method other than ELISA, the binding activity against the mutated peptide bound with a column may be measured by flowing the test antigen-binding molecule and the control antigen-binding molecule in the column, and then quantifying the antigen-binding molecule eluted in the eluate. A method for adsorbing a mutated peptide, for example, as a fusion peptide with GST, to a column is known in the art.

When the identified epitope is a conformational epitope, whether a test antigen-binding molecule and a control antigen-binding molecule share an epitope can be evaluated by the following method: first, a certain antigen-expressing cells and cells expressing the certain antigen with a mutation introduced to the epitope are prepared. The test antigen-binding molecule and the control antigen-binding molecule are added to cell suspensions containing these cells suspended in an appropriate buffer solution such as PBS. Subsequently, the cell suspensions are appropriately washed with a buffer solution, and a FITC-labeled antibody capable of recognizing the test antigen-binding molecule and the control antigen-binding molecule is then added thereto. The fluorescence intensity and the number of cells stained with the labeled antibody are measured using FACSCalibur™ (Becton, Dickinson and Company). The test antigen-binding molecule and the control antigen-binding molecule are appropriately diluted with a suitable buffer solution and used at concentrations thereby adjusted to the desired ones. These antigen-binding molecules are used, for example, at any concentration from 10 μg/ml to 10 ng/ml. The amount of the labeled antibody bound to the cells is reflected in the fluorescence intensity obtained by analysis using CELL QUEST™ Software (Becton, Dickinson and Company), i.e., a geometric mean value. In short, the binding activity of the test antigen-binding molecule and the control antigen-binding molecule indicated by the amount of the labeled antibody bound can be determined by obtaining the geometric mean value.

The competition of the antigen-binding molecule with another antigen-binding molecule for the same epitope can also be confirmed by use of radiolabeled antigen binding assay (RIA), BIACORE(R) surface plasmon resonance assay, electrogenerated chemiluminescence, or the like, in addition to ELISA or FACS described above.

The geometric mean comparison value (ΔGeo-Mean value for the mutated certain antigen molecule) thus obtained by analysis, which reflects the amount of the test antigen-binding molecule bound with the cells expressing the mutated certain antigen, is compared with the ΔGeo-Mean comparison value that reflects the amount of the test antigen-binding molecule bound to the certain antigen-expressing cells. In this case, the concentrations of the test antigen-binding molecule used for determining the ΔGeo-Mean comparison values for the cells expressing the mutated certain antigen and the certain antigen-expressing cells are particularly preferably adjusted to equal or substantially equal concentrations. An antigen-binding molecule already confirmed to recognize an epitope in certain antigen is used as the control antigen-binding molecule.

Provided that the ΔGeo-Mean comparison value of the test antigen-binding molecule for the cells expressing the mutated certain antigen is smaller than at least 80%, preferably 50%, more preferably 30%, particularly preferably 15% of the ΔGeo-Mean comparison value of the test antigen-binding molecule for the certain antigen-expressing cells, the test antigen-binding molecule "does not substantially bind to cells expressing the mutated certain antigen". The calculation expression for determining the Geo-Mean (geometric mean) value is described in the CELL QUEST Software User's Guide (BD biosciences). The epitope for the test antigen-binding molecule and the control antigen-binding molecule can be assessed as being the same when their comparison values can be regarded as being substantially equivalent as a result of comparison.

In the present specification, the term "carrying moiety" refers to a moiety other than a target antigen recognition site and an immunoreceptor recognition site in an antigen-binding molecule. The carrying moiety of the present invention is usually a peptide or a polypeptide constituted by amino acids. In a specific embodiment, the carrying moiety in the antigen-binding molecule is linked to the target antigen recognition site and the immunoreceptor recognition site. The carrying moiety of the present invention may be a series of peptides or polypeptides connected through an amide bond, or may be a complex formed from a plurality of peptides or polypeptides through a covalent bond such as a disulfide bond or a noncovalent bond such as a hydrogen bond or hydrophobic interaction.

When the antigen-binding molecule is an antibody, the approach of extending the half-life in blood of the antigen-binding molecule in one embodiment includes the imparting of FcRn binding activity to the antigen-binding molecule. As a method for imparting FcRn, normally, a FcRn binding region is established in the antigen-binding molecule. The FcRn binding region refers to a region having binding activity against FcRn and may have any structure as long as the region used has binding activity against FcRn.

The antigen-binding molecule containing a FcRn binding region is capable of being taken up into cells and then brought back into plasma through the salvage pathway of FcRn. For example, an IgG molecule has a relatively long circulation time in plasma (slow disappearance) because FcRn known as a salvage receptor of the IgG molecule functions. An IgG molecule taken up into the endosome through pinocytosis binds to FcRn expressed in the endosome under intraendosomal acidic conditions. An IgG molecule that has failed to bind to FcRn is moved to the lysosome and degraded therein, whereas the IgG molecule bound with FcRn is transferred to cell surface, then dissociated from the FcRn under neutral conditions in plasma, and thereby brought back into plasma.

The FcRn binding region is preferably a region binding directly to FcRn. Preferred examples of the FcRn binding region can include antibody Fc regions. However, a region capable of binding to a polypeptide, such as albumin or IgG, which has FcRn binding capacity is capable of binding indirectly to FcRn via albumin, IgG, or the like. Therefore, the FcRn binding region according to the present invention may be a region which binds to such a polypeptide having FcRn binding capacity.

The binding activity of the FcRn binding region according to the present invention against FcRn, particularly, human FcRn may be measured by a method known to those skilled in the art, as mentioned in the above section about binding activity. The conditions therefor may be appropriately determined by those skilled in the art. The binding activity against human FcRn can be evaluated as KD (dissociation constant), apparent KD (apparent dissociation constant), kd (dissociation rate), or apparent kd (apparent dissociation rate), etc. These values can be measured by methods known to those skilled in the art. For example, Biacore (GE Healthcare Japan Corp.), Scatchard plot, a flow cytometer, and the like can be used.

The conditions for measuring the binding activity of the FcRn binding region against FcRn are not particularly limited and may be appropriately selected by those skilled in the art. The binding activity can be measured under conditions involving, for example, a MES buffer and 37°C, as described in WO2009/125825. Also, the binding activity of the FcRn binding region of the present invention against FcRn may be measured by a method known to those skilled in the art and can be measured using, for example, Biacore (GE Healthcare Japan Corp.).

As for pH for use in the measurement conditions, the binding affinity of the FcRn binding region for FcRn may be evaluated at any pH of 4.0 to 6.5. Preferably, a pH of 5.8 to 6.0, which is close to pH in the early endosome *in vivo,* is used for determining the binding affinity of the FcRn binding region for human FcRn. As for temperature for use in the measurement conditions, the binding affinity of the FcRn binding region for FcRn may be evaluated at any temperature of 10°C to 50°C. Preferably, a temperature of 15°C to 40°C is used for determining the binding affinity of the FcRn binding region for human FcRn. More preferably, any temperature from 20°C to 35°C, for example, any one of 20, 21, 22, 23, 24, 25, 26, 27, 28, 29, 30, 31, 32, 33, 34, and 35°C, is also used for determining the binding affinity of the FcRn binding region for FcRn. The temperature of 25°C is one non-limiting example of the temperature of the present invention.

One example of the FcRn binding region includes, but is not limited to, an IgG antibody Fc region. In the case of using an IgG antibody Fc region, its type is not limited, and for example, IgG1, IgG2, IgG3, or IgG4 Fc region may be used.

A natural IgG antibody Fc region as well as an altered Fc region having one or more amino acid substitutions may be used as long as the Fc region has FcRn binding activity.

For example, an altered Fc region containing an amino acid sequence derived from an IgG antibody Fc region by the substitution of at least one amino acid selected from EU numbering positions 237, 238, 239, 248, 250, 252, 254, 255, 256, 257, 258, 265, 270, 286, 289, 297, 298, 303, 305, 307, 308, 309, 311, 312, 314, 315, 317, 325, 332, 334, 360, 376, 380, 382, 384, 385, 386, 387, 389, 424, 428, 433, 434 and 436 by another amino acid may be used.

More specifically, an altered Fc region containing at least one amino acid substitution selected from

an amino acid substitution to substitute Gly at position 237 by Met,
an amino acid substitution to substitute Pro at position 238 by Ala,
an amino acid substitution to substitute Ser at position 239 by Lys,
an amino acid substitution to substitute Lys at position 248 by Ile,
an amino acid substitution to substitute Thr at position 250 by Ala, Phe, Ile, Met, Gln, Ser, Val, Trp, or Tyr,
an amino acid substitution to substitute Met at position 252 by Phe, Trp, or Tyr,
an amino acid substitution to substitute Ser at position 254 by Thr,
an amino acid substitution to substitute Arg at position 255 by Glu,
an amino acid substitution to substitute Thr at position 256 by Asp, Glu, or Gln,
an amino acid substitution to substitute Pro at position 257 by Ala, Gly, Ile, Leu, Met, Asn, Ser, Thr, or Val,
an amino acid substitution to substitute Glu at position 258 by His,
an amino acid substitution to substitute Asp at position 265 by Ala,
an amino acid substitution to substitute Asp at position 270 by Phe,
an amino acid substitution to substitute Asn at position 286 by Ala or Glu,
an amino acid substitution to substitute Thr at position 289 by His,
an amino acid substitution to substitute Asn at position 297 by Ala,
an amino acid substitution to substitute Ser at position 298 by Gly,
an amino acid substitution to substitute Val at position 303 by Ala,
an amino acid substitution to substitute Val at position 305 by Ala,
an amino acid substitution to substitute Thr at position 307 by Ala, Asp, Phe, Gly, His, Ile, Lys, Leu, Met, Asn, Pro, Gln, Arg, Ser, Val, Trp, or Tyr,
an amino acid substitution to substitute Val at position 308 by Ala, Phe, Ile, Leu, Met, Pro, Gln, or Thr,
an amino acid substitution to substitute Leu or Val at position 309 by Ala, Asp, Glu, Pro, or Arg,
an amino acid substitution to substitute Gln at position 311 by Ala, His, or Ile,
an amino acid substitution to substitute Asp at position 312 by Ala or His,
an amino acid substitution to substitute Leu at position 314 by Lys or Arg,
an amino acid substitution to substitute Asn at position 315 by Ala or His,
an amino acid substitution to substitute Lys at position 317 by Ala,
an amino acid substitution to substitute Asn at position 325 by Gly,
an amino acid substitution to substitute Ile at position 332 by Val,
an amino acid substitution to substitute Lys at position 334 by Leu,
an amino acid substitution to substitute Lys at position 360 by His,
an amino acid substitution to substitute Asp at position 376 by Ala,
an amino acid substitution to substitute Glu at position 380 by Ala,
an amino acid substitution to substitute Glu at position 382 by Ala,
an amino acid substitution to substitute Asn or Ser at position 384 by Ala,
an amino acid substitution to substitute Gly at position 385 by Asp or His,
an amino acid substitution to substitute Gln at position 386 by Pro,
an amino acid substitution to substitute Pro at position 387 by Glu,
an amino acid substitution to substitute Asn at position 389 by Ala or Ser,
an amino acid substitution to substitute Ser at position 424 by Ala,
an amino acid substitution to substitute Met at position 428 by Ala, Asp, Phe, Gly, His, Ile, Lys, Leu, Asn, Pro, Gln, Ser, Thr, Val, Trp, or Tyr,
an amino acid substitution to substitute His at position 433 by Lys,
an amino acid substitution to substitute Asn at position 434 by Ala, Phe, His, Ser, Trp, or Tyr, and
an amino acid substitution to substitute Tyr or Phe at position 436 by His
(all according to the EU numbering)
in an IgG antibody Fc region may be used.

From another viewpoint, a Fc region containing at least one amino acid selected from

Met as the amino acid at position 237,
Ala as the amino acid at position 238,
Lys as the amino acid at position 239,
Ile as the amino acid at position 248,

Ala, Phe, Ile, Met, Gln, Ser, Val, Trp, or Tyr as the amino acid at position 250,

Phe, Trp, or Tyr as the amino acid at position 252,

Thr as the amino acid at position 254,

Glu as the amino acid at position 255,

Asp, Glu, or Gln as the amino acid at position 256,

Ala, Gly, Ile, Leu, Met, Asn, Ser, Thr, or Val as the amino acid at position 257,

His as the amino acid at position 258,

Ala as the amino acid at position 265,

Phe as the amino acid at position 270,

Ala or Glu as the amino acid at position 286,

His as the amino acid at position 289,

Ala as the amino acid at position 297,

Gly as the amino acid at position 298,

Ala as the amino acid at position 303,

Ala as the amino acid at position 305,

Ala, Asp, Phe, Gly, His, Ile, Lys, Leu, Met, Asn, Pro, Gln, Arg, Ser, Val, Trp, or Tyr as the amino acid at position 307,

Ala, Phe, Ile, Leu, Met, Pro, Gln, or Thr as the amino acid at position 308,

Ala, Asp, Glu, Pro, or Arg as the amino acid at position 309,

Ala, His, or Ile as the amino acid at position 311,

Ala or His as the amino acid at position 312,

Lys or Arg as the amino acid at position 314,

Ala or His as the amino acid at position 315,

Ala as the amino acid at position 317,

Gly as the amino acid at position 325,

Val as the amino acid at position 332,

Leu as the amino acid at position 334,

His as the amino acid at position 360,

Ala as the amino acid at position 376,

Ala as the amino acid at position 380,

Ala as the amino acid at position 382,

Ala as the amino acid at position 384,

Asp or His as the amino acid at position 385,

Pro as the amino acid at position 386,

Glu as the amino acid at position 387,

Ala or Ser as the amino acid at position 389,

Ala as the amino acid at position 424,

Ala, Asp, Phe, Gly, His, Ile, Lys, Leu, Asn, Pro, Gln, Ser, Thr, Val, Trp, or Tyr as the amino acid at position 428,

Lys as the amino acid at position 433,

Ala, Phe, His, Ser, Trp, or Tyr as the amino acid at position 434, and

His as the amino acid at position 436

(all according to the EU numbering) in an IgG antibody Fc region may be used.

In one embodiment, the method for extending the half-life in blood involves binding the antigen-binding molecule to albumin. Since albumin does not undergo renal excretion and has FcRn binding activity, its half-life in blood is as long as 17 to 19 days (J Clin Invest. 1953 Aug; 32 (8): 746-768). Hence, it has been reported that a protein bound with albumin becomes bulky and capable of binding indirectly to FcRn and therefore has an increased half-life in blood (Antibodies 2015, 4 (3), 141-156).

In one embodiment, the alternative method for extending the half-life in blood involves PEGylating the antigen-binding molecule. The PEGylation of a protein is considered to render the protein bulky and also suppress its degradation by protease in blood, thereby extending the half-life in blood of the protein (J Pharm Sci. 2008 Oct; 97 (10): 4167-83).

In some embodiments of the present invention, the antigen-binding molecule contains an antibody Fc region. In a specific embodiment, the antigen-binding molecule contains a CH2 domain and a CH3 domain of a human IgG antibody. In a specific embodiment, the antigen-binding molecule contains a moiety spanning from human IgG1 antibody heavy chain Cys226 or Pro230 to the carboxyl terminus of the heavy chain. However, the C-terminal lysine (Lys447) or glycine-lysine (Gly446-Lys447) of the Fc region may be present or absent.

In some embodiments of the present invention, the antigen-binding molecule contains an antibody constant region. In a more preferred embodiment, the antigen-binding molecule contains an IgG antibody constant region. In a more preferred embodiment, the antigen-binding molecule contains a human IgG antibody constant region. In a more preferred embodiment, the antigen-binding molecule is a bispecific antibody, and contains a human IgG antibody constant region.

In some embodiments of the present invention, the antigen-binding molecule contains: a region substantially similar in structure to an antibody heavy chain constant region; and a region substantially similar in structure to an antibody light chain, connected to the region via a covalent bond such as a disulfide bond or a noncovalent bond such as a hydrogen bond or hydrophobic interaction.

The term "IgG antibody-like molecule" used in the present specification is used to define a molecule having moieties substantially similar in structure to constant domains or constant regions as in an IgG antibody, and moieties substantially similar in structure to variable domains or variable regions as in the IgG antibody, and having conformation substantially similar to that of the IgG antibody. However, in the present specification, the "IgG antibody-like molecule" may or may not exert antigen-binding activity while retaining the structures similar to those of the IgG antibody.

When the antigen-binding molecule is an IgG antibody-like molecule, a target antigen recognition site and an immunoreceptor recognition site may be respectively established at moieties corresponding to two variable regions of the IgG antibody. Such an embodiment is encompassed by the present invention.

In the present specification, the term "specificity" refers to a property by which the target antigen recognition site or the immunoreceptor recognition site of the antigen-binding molecule does not substantially bind to a molecule other than a particular target antigen or immunoreceptor. This term is also used when the antigen-binding molecule has specificity for an epitope contained in a particular target antigen or immunoreceptor. The term "not substantially bind" is determined according to the method described for measurement of binding activity and means that the binding activity of a specific binding molecule for a molecule other than the particular target antigen or immunoreceptor is 80% or less, usually 50% or less, preferably 30% or less, particularly preferably 15% or less, of its binding activity for the binding partner molecule(s).

Usually, a nucleic acid encoding the antigen-binding molecule in the present invention can be carried by (or inserted in) an appropriate vector, and introduced into host cells, followed by application of a usual method to prepare the antigen-binding molecule. The vector is not particularly limited as long as the vector can stably retain an inserted nucleic acid. For example, when *E. coli* is used as the host, various commercially available vectors, preferably a pBluescript vector (manufactured by Stratagene Corp.) or the like can be used as a vector for cloning. In the case of using the vector for the purpose of producing a polypeptide (e.g., a chimeric receptor, an IgG antibody, a bispecific antibody, and an antigen-binding molecule) for use in carrying out the present invention, an expression vector is particularly useful. The expression vector is not particularly limited as long as the vector permits expression of the polypeptide *in vitro,* in *E. coli,* in cultured cells, or in organism individuals. The expression vector is preferably, for example, a pBEST vector (manufactured by Promega Corp.) for *in vitro* expression, a pET vector (manufactured by Invitrogen Corp.) for *E. coli,* a pME18S-FL3 vector (GenBank Accession No. AB009864) for cultured cells, and a pME18S vector (Mol Cell Biol. 8: 466-472 (1988)) for organism individuals. The insertion of the DNA of the present invention into the vector can be performed by a routine method, for example, ligase reaction using restriction sites (Current protocols in Molecular Biology edit. Ausubel et al. (1987) Publish. John Wiley & amp; Sons. Section 11.4-11.11).

The host cells are not particularly limited, and various host cells are used according to the purpose. Examples of the cells for expressing the antigen-binding molecule include bacterial cells (e.g., *Streptococcus, Staphylococcus, E. coli, Streptomyces,* and *Bacillus subtilis*), fungal cells (e.g., yeasts and *Aspergillus*), insect cells (e.g. *Drosophila* S2 and *Spodoptera* SF9), animal cells (e.g., CHO, COS, HeLa, C127, 3T3, BHK, HEK293, and Bowes melanoma cells) and plant cells. The introduction of the vector to the host cells may be performed by a method known in the art, for example, a calcium phosphate precipitation method, an electroporation method (Current protocols in Molecular Biology edit. Ausubel et al., (1987) Publish. John Wiley & amp; Sons. Section 9.1-9.9), a Lipofectamine method (manufactured by GIBCO-BRL/Thermo Fisher Scientific Inc.), or a microinjection method.

An appropriate secretory signal can be incorporated into a nucleic acid encoding an antigen-binding molecule of interest in order to secrete the antigen-binding molecule expressed in the host cells to the lumen of the endoplasmic reticulum, periplasmic space, or an extracellular environment. The signal may be endogenous to the polypeptide of interest or may be a foreign signal.

When the antigen-binding molecule of the present invention is secreted into a medium, the recovery of the antigen-binding molecule in the production method is performed by the recovery of the medium. When the antigen-binding molecule of the present invention is produced into cells, the cells are first lysed, followed by the recovery of the antigen-binding molecule.

A method known in the art including ammonium sulfate or ethanol precipitation, acid extraction, anion- or cation-exchange chromatography, phosphocellulose chromatography, hydrophobic interaction chromatography, affinity chromatography, hydroxyapatite chromatography, and lectin chromatography can be used for recovering and purifying the antigen-binding molecule of the present invention from the recombinant cell cultures.

An amino acid contained in each amino acid sequence described in the present invention may be posttranslationally modified (e.g., the modification of N-terminal glutamine to pyroglutamic acid by pyroglutamylation is a modification well known to those skilled in the art). Such an amino acid sequence containing the posttranslationally modified amino acid is also included in the amino acid sequence described in the present invention, as a matter of course.

A method for preparing an antibody having the desired binding activity is known to those skilled in the art. In the present invention, an antigen-binding molecule against a molecule expressed on the surface of target cells (lesion cells) as an antigen (target antigen) can be used. When the target cells are tumor cells or cancer cells, the antigen is illustrated as a tumor antigen in the present specification. A method for preparing an antibody which binds to the tumor antigen will be illustrated below.

The antibody which binds to the tumor antigen can be obtained as a polyclonal or a monoclonal antibody by use of an approach known in the art. A mammal-derived monoclonal antibody can be preferably prepared as the antibody. The mammal-derived monoclonal antibody encompasses, for example, those produced by hybridomas and those produced by host cells transformed with expression vectors containing an antibody gene by a genetic engineering approach.

The monoclonal antibody-producing hybridomas can be prepared by use of a technique known in the art, for example, as follows: mammals are immunized with a tumor antigen protein used as a sensitizing antigen according to a usual immunization method. Immunocytes thus obtained are fused with parental cells known in the art by a usual cell fusion method. Next, cells producing a monoclonal antibody can be screened for by a usual screening method to select hybridomas producing the anti-tumor antigen antibody.

Specifically, the monoclonal antibody is prepared, for example, as follows: first, a tumor antigen gene can be expressed to obtain a tumor antigen protein for use as a sensitizing antigen for antibody obtainment. Specifically, a gene sequence encoding the tumor antigen is inserted into expression vectors known in the art, with which appropriate host cells are then transformed. The desired human tumor antigen protein is purified from the host cells or from a culture supernatant thereof by a method known in the art. In order to obtain a soluble tumor antigen from a culture supernatant, for example, a protein lacking a moiety constituting a hydrophobic region in a tumor antigen polypeptide sequence can be used. Also, purified natural GPC3 protein may be used as a sensitizing antigen.

This purified tumor antigen protein can be used as the sensitizing antigen for use in the immunization of mammals. A partial peptide of the tumor antigen can also be used as the sensitizing antigen. This partial peptide may be obtained by chemical synthesis from the amino acid sequence of the human tumor antigen. Alternatively, the partial peptide may be obtained by the incorporation of a portion of the tumor antigen gene into expression vectors followed by its expression. Furthermore, the partial peptide can also be obtained by the degradation of the tumor antigen protein with a proteolytic enzyme. The region and size of the tumor antigen peptide for use as such a partial peptide are not particularly limited by specific embodiments. The number of amino acids constituting the peptide used as the sensitizing antigen is preferably at least 5 or more, for example, 6 or more or 7 or more. More specifically, a peptide of 8 to 50, preferably 10 to 30 residues can be used as the sensitizing antigen.

Also, a fusion protein comprising a desired partial polypeptide or peptide of the tumor antigen protein fused with a different polypeptide can be used as the sensitizing antigen. For example, an antibody Fc portion or a peptide tag can be preferably used for producing the fusion protein for use as the sensitizing antigen. Two or more types of genes respectively encoding the desired polypeptide portions are fused in frame, and the fusion gene can be inserted into expression vectors as described above to prepare vectors for the expression of the fusion protein. The method for preparing the fusion protein is described in Molecular Cloning 2nd ed. (Sambrook, J. et al., Molecular Cloning 2nd ed., 9.47-9.58 (1989), Cold Spring Harbor Lab. Press). As one example, a method for obtaining GPC3 for use as a sensitizing antigen and an immunization method using this sensitizing antigen are also specifically described in WO2003/000883, WO2004/022754, and WO2006/006693.

The mammals to be immunized with the sensitizing antigen are not limited to specific animals. The mammals to be immunized are preferably selected in consideration of compatibility with the parental cells for use in cell fusion. In general, rodents, for example, mice, rats, or hamsters, rabbits, monkeys, or the like are preferably used.

These animals are immunized with the sensitizing antigen according to a method known in the art. For example, a general immunization method involves administering the sensitizing antigen to the mammals by intraperitoneal or subcutaneous injection to thereby perform immunization. Specifically, the sensitizing antigen diluted with PBS (phosphate-buffered saline), saline, or the like at an appropriate dilution ratio is mixed with a usual adjuvant, for example, a Freund's complete adjuvant, if desired, and emulsified. Then, the resulting sensitizing antigen is administered to the mammals several times at 4- to 21-day intervals. Also, an appropriate carrier may be used in the immunization with the sensitizing antigen. Particularly, in the case of using a partial peptide having a small molecular weight as the sensitizing antigen, immunization with the sensitizing antigen peptide bound with a carrier protein such as albumin or keyhole limpet hemocyanin may be desirable in some cases.

Alternatively, the hybridomas producing the desired antibody can also be prepared as described below by use of DNA immunization. The DNA immunization is an immunization method which involves immunostimulating immunized animals by expressing *in vivo* the sensitizing antigen in the immunized animals given vector DNAs that have been constructed in a form capable of expressing the antigenic protein-encoding gene in the immunized animals. The DNA immunization can be expected to be superior to the general immunization method using the administration of the protein antigen to animals to be immunized as follows:

- the DNA immunization can provide immunostimulation with the membrane protein structure maintained; and
- the DNA immunization eliminates the need of purifying the immunizing antigen.

In order to obtain the monoclonal antibody of the present invention by the DNA immunization, first, a DNA expressing the tumor antigen protein is administered to the animals to be immunized. The DNA encoding the tumor antigen can be synthesized by a method known in the art such as PCR. The obtained DNA is inserted into appropriate expression vectors, which are then administered to the animals to be immunized. For example, commercially available expression vectors such as pcDNA3.1 can be preferably used as the expression vectors. A method generally used can be used as a method for administering the vectors to the organisms. For example, gold particles with the expression vectors adsorbed thereon can be transferred into the cells of animal individuals to be immunized using a gene gun to thereby perform the DNA immunization. Further, an antibody which recognizes the tumor antigen may be prepared by use of a method described in International Publication No. WO2003/104453.

A rise in the titer of the antibody which binds to the tumor antigen is confirmed in the serum of the mammals thus immunized. Then, immunocytes are collected from the mammals and subjected to cell fusion. Particularly, spleen cells can be used as preferred immunocytes.

Mammalian myeloma cells are used in the cell fusion with the immunocytes. The myeloma cells preferably have an appropriate selection marker for screening. The selection marker refers to a character that can survive (or cannot survive) under particular culture conditions. For example, hypoxanthine-guanine phosphoribosyltransferase deficiency (hereinafter, abbreviated to HGPRT deficiency) or thymidine kinase deficiency (hereinafter, abbreviated to TK deficiency) is known in the art as the selection marker. Cells having the HGPRT or TK deficiency is sensitive to hypoxanthine-aminopterin-thymidine (hereinafter, abbreviated to HAT-sensitive). The HAT-sensitive cells are killed in a HAT selective medium because the cells fail to synthesize DNA. By contrast, these cells, when fused with normal cells, become able to grow even in the HAT selective medium because the fused cells can continue DNA synthesis through the use of the salvage pathway of the normal cells.

The cells having the HGPRT or TK deficiency can be selected in a medium containing 6-thioguanine or 8-azaguanine (hereinafter, abbreviated to 8AG) for the HGPRT deficiency or 5'-bromodeoxyuridine for the TK deficiency. The normal cells are killed by incorporating these pyrimidine analogs into their DNAs. By contrast, the cells deficient in these enzymes can survive in the selective medium because the cells cannot incorporate the pyrimidine analogs therein. In addition, a selection marker called G418 resistance confers resistance to a 2-deoxystreptamine antibiotic (gentamicin analog) through a neomycin resistance gene. Various myeloma cells suitable for cell fusion are known in the art.

For example, P3 (P3x63Ag8.653) (J. Immunol. (1979) 123 (4), 1548-1550), P3x63Ag8U.1 (Current Topics in Microbiology and Immunology (1978) 81, 1-7), NS-1 (C. Eur. J. Immunol. (1976) 6 (7), 511-519), MPC-11 (Cell (1976) 8 (3), 405-415), SP2/0 (Nature (1978) 276 (5685), 269-270), FO (J. Immunol. Methods (1980) 35 (1-2), 1-21), S194/5.XX0.BU.1 (J. Exp. Med. (1978) 148 (1), 313-323), R210 (Nature (1979) 277 (5692), 131-133) can be preferably used as such myeloma cells.

Basically, the cell fusion of the immunocytes with the myeloma cells is carried out according to a method known in the art, for example, the method of Kohler and Milstein et al. (Methods Enzymol. (1981) 73, 3-46).

More specifically, the cell fusion can be carried out, for example, in a usual nutrient medium in the presence of a cell fusion promoter. For example, polyethylene glycol (PEG) or hemagglutinating virus of Japan (HVJ) is used as the fusion promoter. In addition, an auxiliary such as dimethyl sulfoxide is added thereto for use, if desired, for enhancing fusion efficiency.

The ratio between the immunocytes and the myeloma cells used can be arbitrarily set. For example, the amount of the immunocytes is preferably set to 1 to 10 times the amount of the myeloma cells. For example, an RPMI1640 medium or a MEM medium suitable for the growth of the myeloma cell line as well as a usual medium for use in this kind of cell culture is used as the medium for use in the cell fusion. Preferably, a solution supplemented with serum (e.g., fetal calf serum (FCS)) can be further added to the medium.

For the cell fusion, the immunocytes and the myeloma cells are well mixed in the predetermined amounts in the medium. A PEG solution (e.g., average molecular weight: on the order of 1000 to 6000) preheated to approximately 37°C is usually added thereto at a concentration of 30 to 60% (w/v). The mixed solution is gently mixed so that desired fusion cells (hybridomas) are formed. Subsequently, the appropriate medium listed above is sequentially added to the cell cultures, and its supernatant is removed by centrifugation. This operation can be repeated to remove the cell fusion agents or the like unfavorable for hybridoma growth.

The hybridomas thus obtained can be cultured in a usual selective medium, for example, a HAT medium (medium containing hypoxanthine, aminopterin, and thymidine), for selection. The culture using the HAT medium can be continued for a time long enough to kill cells (non-fused cells) other than the desired hybridomas (usually, the time long enough is several days to several weeks). Subsequently, hybridomas producing the desired antibody are screened for and single-cell cloned by a usual limiting dilution method.

The hybridomas thus obtained can be selected by use of a selective medium appropriate for the selection marker of the myeloma used in the cell fusion. For example, the cells having the HGPRT or TK deficiency can be selected by culture in a HAT medium (medium containing hypoxanthine, aminopterin, and thymidine). Specifically, when HAT-sensitive myeloma cells are used in the cell fusion, only cells successfully fused with normal cells can be grown selectively in the HAT medium. The culture using the HAT medium is continued for a time long enough to kill cells (non-fused cells) other than the desired hybridomas. Specifically, the culture can generally be carried out for several days to several weeks to select the desired hybridomas. Subsequently, hybridomas producing the desired antibody can be screened for and single-cell cloned by a usual limiting dilution method.

The screening of the desired antibody and the single-cell cloning can be preferably carried out by a screening method based on antigen-antibody reaction known in the art. For example, a monoclonal antibody which binds to GPC3 can bind to GPC3 expressed on cell surface. Such a monoclonal antibody can be screened for, for example, by FACS (fluorescence activated cell sorting). FACS is a system that can analyze the binding of an antibody to cell surface, by bringing cells into contact with a fluorescent antibody, analyzing the cells with laser light, and measuring fluorescence emitted from individual cells.

In order to screen for a hybridoma producing the monoclonal antibody of the present invention by FACS, first, cells expressing GPC3 are prepared. The cells for screening are preferably mammalian cells forced to express the tumor antigen used. Untransformed mammalian cells used as host cells can be used as a control to selectively detect the binding activity of an antibody against the tumor antigen on cell surface. Specifically, a hybridoma producing an antibody which binds to a cell forced to express GPC3 without binding to the host cells can be selected to obtain a hybridoma producing a monoclonal antibody against the tumor antigen.

Alternatively, the antibody can be evaluated for its binding activity against immobilized tumor antigen-expressing cells on the basis of the principle of ELISA. For example, GPC3-expressing cells are immobilized onto each well of, for example, an ELISA plate. The hybridoma culture supernatant is contacted with the immobilized cell in the well to detect an antibody which binds to the immobilized cell. In the case of a mouse-derived monoclonal antibody, the antibody bound with the cell can be detected using an anti-mouse immunoglobulin antibody. Hybridomas producing the desired antibody having the ability to bind to the antigen, thus selected by screening, can be cloned by a limiting dilution method or the like.

The monoclonal antibody-producing hybridomas thus prepared can be subcultured in a usual medium. The hybridomas can also be stored over a long period in liquid nitrogen.

The hybridomas are cultured according to a usual method, and the desired monoclonal antibody can be obtained from the culture supernatant thereof. Alternatively, the hybridomas may be administered to mammals compatible therewith and grown, and the monoclonal antibody can be obtained from the ascitic fluids thereof. The former method is suitable for obtaining highly pure antibodies.

An antibody encoded by an antibody gene cloned from the antibody-producing cells such as hybridomas may also be preferably used. The cloned antibody gene is incorporated in appropriate vectors, which are then transferred to hosts so that the antibody encoded by the gene is expressed. Methods for the antibody gene isolation, the incorporation into vectors, and the transformation of host cells have already been established by, for example, Vandamme et al. (Eur. J. Biochem. (1990) 192 (3), 767-775). A method for producing a recombinant antibody as mentioned below is also known in the art.

For example, cDNAs encoding the variable regions (V regions) of the antibody which binds to the tumor antigen are obtained from the hybridoma cells producing the antibody. For this purpose, usually, total RNA is first extracted from the hybridomas. For example, the following methods can be used as a method for mRNA extraction from the cells: - guanidine ultracentrifugation method (Biochemistry (1979) 18 (24), 5294-5299), and - AGPC method (Anal. Biochem. (1987) 162 (1), 156-159).

The extracted mRNAs can be purified using mRNA Purification Kit (manufactured by GE Healthcare Bio-Sciences Corp.) or the like. Alternatively, a kit for directly extracting total mRNA from cells is also commercially available, such as QuickPrep mRNA Purification Kit (manufactured by GE Healthcare Bio-Sciences Corp.). The mRNAs may be obtained from the hybridomas using such a kit. From the obtained mRNAs, the cDNAs encoding antibody V regions can be synthesized using reverse transcriptase. The cDNAs can be synthesized using, for example, AMV Reverse Transcriptase First-strand cDNA Synthesis Kit (manufactured by Seikagaku Corp.). Alternatively, a 5'-RACE method (Proc. Natl. Acad. Sci. USA (1988) 85 (23), 8998-9002; and Nucleic Acids Res. (1989) 17 (8), 2919-2932) using SMART RACE cDNA amplification kit (manufactured by Clontech Laboratories, Inc.) and PCR may be appropriately used for the cDNA synthesis and amplification. In the course of such cDNA synthesis, appropriate restriction sites mentioned later can be further introduced into both ends of the cDNAs.

The cDNA portions of interest are purified from the obtained PCR products and subsequently ligated with vector DNAs. The recombinant vectors thus prepared are transferred to *E. coli* or the like. After colony selection, desired recombinant vectors can be prepared from the *E. coli* that has formed the colony. Then, whether or not the recombinant vectors have the nucleotide sequences of the cDNAs of interest is confirmed by a method known in the art, for example, a dideoxynucleotide chain termination method.

The 5'-RACE method using primers for variable region gene amplification is conveniently used for obtaining the genes encoding variable regions. First, cDNAs are synthesized with RNAs extracted from the hybridoma cells as templates to obtain a 5'-RACE cDNA library. A commercially available kit such as SMART RACE cDNA amplification kit is appropriately used in the synthesis of the 5'-RACE cDNA library.

The antibody gene is amplified by PCR with the obtained 5'-RACE cDNA library as a template. Primers for mouse antibody gene amplification can be designed on the basis of an antibody gene sequence known in the art. These primers have nucleotide sequences differing depending on immunoglobulin subclasses. Thus, the subclass is desirably determined in advance using a commercially available kit such as Iso Strip mouse monoclonal antibody isotyping kit (Roche Diagnostics K.K.).

Specifically, primers capable of amplifying genes encoding γ1, γ2a, γ2b, and γ3 heavy chains and κ and λ light chains can be used, for example, for the purpose of obtaining a gene encoding mouse IgG. Primers that anneal to portions corresponding to constant regions close to variable regions are generally used as 3' primers for amplifying IgG variable region genes. On the other hand, primers included in the 5' RACE cDNA library preparation kit are used as 5' primers.

The PCR products thus obtained by amplification can be used to reshape an immunoglobulin composed of heavy chains and light chains in combination. The desired antibody can be screened for with the binding activity of the reshaped immunoglobulin against the antigen as an index. For example, the binding of the antibody to GPC3 is further preferably specific for the purpose of obtaining the antibody against GPC3. The antibody used in the present invention can be screened for, for example, by the following steps:

(1) contacting each antibody comprising V regions encoded by the cDNAs obtained from the hybridomas, with antigen-expressing cells;
(2) detecting the binding between the antigen-expressing cell and the antibody; and
(3) selecting the antibody which binds to the antigen-expressing cell.

A method for detecting the binding between the antibody and the tumor antigen-expressing cell is known in the art. Specifically, the binding between the antibody and the tumor antigen-expressing cell can be detected by an approach such as FACS mentioned above. A fixed preparation of tumor antigen-expressing cells can be appropriately used for evaluating the binding activity of the antibody.

A panning method using phage is also preferably used as a method for screening for the antibody with its binding activity as an index. When antibody genes are obtained as libraries of heavy chain and light chain subclasses from a polyclonal antibody-expressing cell population, a screening method using phage is advantageous. Genes encoding heavy chain and light chain variable regions can be linked via an appropriate linker sequence to form a gene encoding single-chain Fv (scFv). The gene encoding scFv can be inserted to phage vectors to obtain phages expressing scFv on their surface. The phages thus obtained are contacted with the desired antigen. Then, antigen-bound phages can be recovered to recover a DNA encoding scFv having the binding activity of interest. This operation can be repeated, if necessary, to enrich scFvs having the desired binding activity.

After the obtainment of the cDNA encoding each V region of the antibody which binds to the tumor antigen of interest, this cDNA is digested with restriction enzymes which recognize the restriction sites inserted in both ends of the cDNA. The restriction enzymes preferably recognize and digest a nucleotide sequence that appears low frequently in the nucleotide sequence constituting the antibody gene. The insertion of restriction sites that provide cohesive ends is preferred for inserting one copy of the digested portion in the correct direction in a vector. The thus-digested cDNAs encoding the V regions of the anti-GPC3 antibody can be inserted to appropriate expression vectors to obtain antibody expression vectors. In this case, genes encoding antibody constant regions (C regions) and the genes encoding the V regions are fused in frame to obtain a chimeric antibody. In this context, the chimeric antibody refers to an antibody comprising constant and variable regions of different origins. Thus, heterogeneous (e.g., mouse-human) chimeric antibodies as well as human-human homogeneous chimeric antibodies are also encompassed by the chimeric antibody according to the present invention. The V region genes can be inserted to expression vectors preliminarily having constant region genes to construct chimeric antibody expression vectors. Specifically, for example, recognition sequences for restriction enzymes that digest the V region genes can be appropriately located on the 5' side of an expression vector carrying the DNAs encoding the desired antibody constant regions (C regions). This expression vector having the C region genes and the V region genes are digested with the same combination of restriction enzymes and fused in frame to construct a chimeric antibody expression vector.

In order to produce the monoclonal antibody, the antibody gene is incorporated into expression vectors such that the antibody gene is expressed under the control of expression control regions. The expression control regions for antibody expression include, for example, an enhancer and a promoter. Also, an appropriate signal sequence can be added to the amino terminus such that the expressed antibody is extracellularly secreted. For example, a peptide having an amino acid sequence MGWSCIIIFLVATATGVHS is used as a signal sequence in Examples described later. Any other suitable signal sequence can be added. The expressed polypeptide is cleavable at the carboxyl terminal moiety of this sequence. The cleaved polypeptide can be extracellularly secreted as a mature polypeptide. Furthermore, appropriate host cells can be transformed with these expression vectors to obtain recombinant cells expressing the DNA encoding the antibody which binds to the targeted tumor antigen.

For the antibody gene expression, DNAs encoding the heavy chain (H chain) and the light chain (L chain) of the antibody are separately incorporated into different expression vectors. The same host cell can be co-transfected with these vectors carrying the H chain gene and the L chain gene and thereby allowed to express an antibody molecule comprising the H chain and the L chain. Alternatively, the DNAs encoding the H chain and L chain may be incorporated into a single expression vector, with which host cells can be transformed (see International Publication No. WO94/11523).

Many combinations of host cells and expression vectors are known in the art for preparing the antibody by transferring the isolated antibody gene into appropriate hosts. All of these expression systems can be applied to the isolation of the domain comprising antibody variable regions according to the present invention. In the case of using eukaryotic cells as the host cells, animal cells, plant cells, or fungus cells can be appropriately used. Specifically, examples of the animal cells can include the following cells:

(1) mammalian cells such as CHO, COS, myeloma, BHK (baby hamster kidney), Hela, and Vero,;
(2) amphibian cells such as *Xenopus* oocytes; and
(3) insect cells such as sf9, sf21, and Tn5.

Alternatively, antibody gene expression systems using cells derived from the genus *Nicotiana* (e.g., *Nicotiana tabacum*) as the plant cells are known in the art. Cultured callus cells can be appropriately used for the plant cell transformation.

The following cells can be used as the fungus cells:

cells derived from yeasts of the genus *Saccharomyces* (e.g., *Saccharomyces cerevisiae*) and the genus *Pichia* (e.g., *Pichia pastoris*), and
cells derived from filamentous fungi of the genus *Aspergillus* (e.g., *Aspergillus niger)*.

Antibody gene expression systems using prokaryotic cells are also known in the art. In the case of using, for example, bacterial cells, cells of bacteria such as *E. coli* and *Bacillus subtilis* can be appropriately used. The expression vectors containing the antibody gene of interest are transferred into these cells by transformation. The transformed cells are cultured *in vitro,* and the desired antibody can be obtained from the cultures of the transformed cells.

In addition to the host cells, transgenic animals may be used for the production of the recombinant antibody. Specifically, the desired antibody can be obtained from animals transfected with the gene encoding this antibody. For example, the antibody gene can be inserted in frame into a gene encoding a protein specifically produced in milk to thereby construct a fusion gene. For example, goat β casein can be used as the protein secreted into milk. A DNA portion containing the fusion gene having the antibody gene insert is injected into goat embryos, which are in turn introduced into female goats. From milk produced by transgenic goats (or progeny thereof) brought forth by the goats that have received the embryos, the desired antibody can be obtained as a fusion protein with the milk protein. In order to increase the amount of milk containing the desired antibody produced from the transgenic goats, hormone can be administered to the transgenic goats (Bio/Technology (1994) 12 (7), 699-702).

In the case of administering the antigen-binding molecule described in the present specification to humans, a domain derived from a genetically recombinant antibody that has been altered artificially can be appropriately adopted as the domain comprising antibody variable regions in the antigen-binding molecule, for example, for the purpose of reducing heteroantigenicity in humans. The genetically recombinant antibody encompasses, for example, humanized antibodies. Such an altered antibody is appropriately produced using a method known in the art.

Each antibody variable region that is used for preparing the domain comprising antibody variable regions in the antigen-binding molecule described in the present specification is usually constituted by three complementarity-determining regions (CDRs) flanked by four framework regions (FRs). The CDRs are regions that substantially determine the binding specificity of the antibody. The CDRs have highly diverse amino acid sequences. On the other hand, the amino acid sequences constituting the FRs often exhibit high identity even among antibodies differing in binding specificity. Therefore, in general, the binding specificity of an antibody can reportedly be transplanted to another antibody by CDR grafting.

The humanized antibody is also called reshaped human antibody. Specifically, for example, a humanized antibody comprising non-human animal (e.g., mouse) antibody CDRs grafted in a human antibody is known in the art. General gene recombination approaches are also known for obtaining the humanized antibody. Specifically, for example, overlap extension PCR is known in the art as a method for grafting mouse antibody CDRs to human FRs. In the overlap extension PCR, nucleotide sequences encoding mouse antibody CDRs to be grafted are added to primers for human antibody FR synthesis. The primers are prepared for each of the four FRs. In the mouse CDR grafting to the human FRs, in general, the selection of human FRs highly homologous to the mouse FRs is reportedly advantageous in maintaining the CDR functions. Specifically, in general, it is preferred to use human FRs comprising amino acid sequences highly identical to those of the mouse FRs adjacent to the mouse CDRs to be grafted.

The nucleotide sequences to be linked are designed such that the sequences are connected in frame. DNAs encoding human FRs are individually synthesized with their respective primers. The resulting PCR products contain the mouse CDR-encoding DNA added to each human FR-encoding DNA. The mouse CDR-encoding nucleotide sequences are designed such that the nucleotide sequence in each product overlaps with another. Subsequently, the overlapping CDR portions in the products synthesized with the human antibody gene as a template are annealed to each other for complementary strand synthesis reaction. Through this reaction, the human FRs are linked via the mouse CDR sequences.

Finally, the full-length gene of the V region comprising three CDRs and four FRs thus linked is amplified using primers that respectively anneal to the 5' and 3' ends thereof and have the added recognition sequences for appropriate restriction enzymes. The DNA thus obtained and the DNA encoding the human antibody C region can be inserted into expression vectors such that these DNAs are fused in frame to prepare vectors for human-type antibody expression. These vectors carrying the DNAs are transferred to hosts to establish recombinant cells. Then, the recombinant cells are cultured for the expression of the DNA encoding the humanized antibody to produce the humanized antibody into the cultures of the cultured cells (see European Patent Publication No. EP239400 and International Publication No. WO 1996/002576).

The humanized antibody thus prepared can be evaluated for its binding activity against the antigen by qualitative or quantitative assay to thereby select suitable human antibody FRs that allow the CDRs to form a favorable antigen-binding site when linked via the CDRs. If necessary, FR amino acid residue(s) may be substituted such that the CDRs of the resulting reshaped human antibody form an appropriate antigen-binding site. For example, a mutation can be introduced in the amino acid sequence of human FR by the application of the PCR method used in the mouse CDR grafting to the human FRs. Specifically, a mutation of a partial nucleotide sequence can be introduced to the primers annealing to a FR nucleotide sequence. The FR nucleotide sequence synthesized using such primers contains the mutation thus introduced. The mutant antibody having the substituted amino acid(s) can be evaluated for its binding activity against the antigen by assay in the same way as above to thereby select mutated FR sequences having the desired properties (Sato, K.et al., Cancer Res., (1993) 53, 851-856).

Furthermore, transgenic animals having all repertoires of human antibody genes (see International Publication Nos. WO1993/012227, WO1992/003918, WO1994/002602, WO1994/025585, WO1996/034096, and WO1996/033735) can be used as animals to be immunized by DNA immunization to obtain the desired human antibody.

In addition, a technique of obtaining a human antibody by panning using a human antibody library is also known. For example, human antibody V regions are expressed as a single-chain antibody (scFv) on the surface of phages by a phage display method. A phage expressing scFv which binds to the antigen can be selected. The gene of the selected phage can be analyzed to determine DNA sequences encoding the V regions of the human antibody which binds to the antigen. After the determination of the DNA sequence of the scFv which binds to the antigen, the V region sequences are fused in frame with the sequences of the desired human antibody C regions. Then, this fusion product can be inserted to appropriate expression vectors to prepare expression vectors. The expression vectors are transferred to the suitable expression cells as listed above. The cells are allowed to express the gene encoding the human antibody to obtain the human antibody. These methods have already been known in the art (see International Publication Nos. WO1992/001047, WO1992/020791, WO1993/006213, WO1993/011236, WO1993/019172, WO1995/001438, and WO1995/015388).

In the present specification, the term "Fv (variable fragment)" means the minimum unit of an antibody-derived antigen-binding domain consisting of a pair of a light chain variable region (VL) and a heavy chain variable region (VH) of an antibody. In 1988, Skerra and Pluckthun found that an antibody can be prepared from an *E. coli* periplasm fraction in a homogeneous state with activity retained, by inserting an antibody gene downstream of a bacterial signal sequence and inducing the expression of the gene in *E. coli* (Science (1988) 240 (4855), 1038-1041). In Fv prepared from the periplasm fraction, VH and VL were associated in a form binding to an antigen.

In the present specification, Fv preferably includes, for example, a pair of Fvs which is any of the following antigen-binding molecules:

antigen-binding molecules comprising (1) a bivalent antigen-binding domain which is bivalent scFv in which one monovalent scFv of the bivalent scFv is linked to one polypeptide constituting an Fc region via a heavy chain Fv portion constituting a CD3 binding domain, and the other monovalent scFv is linked to the other polypeptide constituting the Fc region via a light chain Fv portion forming the CD3 binding domain; (2) a domain comprising an Fc region having no binding activity against an Fc gamma receptor and having amino acids constituting an Fc region of IgG1, IgG2a, IgG3 or IgG4; and (3) at least a monovalent CD3 binding domain, wherein the light chain Fv portion and the heavy chain Fv portion are associated to constitute a CD3 binding domain in a form binding to the antigen CD3 .

In the present specification, the term "scFv", "single-chain antibody", or "sc(Fv)$_2$" means an antibody portion of a single polypeptide chain that contains variable regions derived from both heavy and light chains, but not constant regions. In general, a single-chain antibody further contains a polypeptide linker between the VH domain and the VL domain, which enables formation of the desired structure that presumably permits antigen binding. The single-chain antibody is discussed in detail by Pluckthun in The Pharmacology of Monoclonal Antibodies, Vol. 113, Rosenburg and Moore ed., Springer-Verlag, New York, 269-315 (1994). See also International Publication No. WO1988/001649 and U.S. Patent Nos. 4,946,778 and 5,260,203. In a particular aspect, the single-chain antibody can be bispecific and/or humanized.

scFv is an antigen-binding domain in which VH and VL constituting Fv are linked via a peptide linker (Proc. Natl. Acad. Sci. U.S.A. (1988) 85 (16), 5879-5883). VH and VL can be retained in close proximity by the peptide linker.

sc(Fv)$_2$ is a single-chain antibody in which four variable regions of two VLs and two VHs are linked via linkers such as peptide linkers to form a single chain (J Immunol. Methods (1999) 231 (1-2), 177-189). These two VHs and two VLs may be derived from different monoclonal antibodies. Such sc(Fv)$_2$ also preferably includes a bispecific sc(Fv)$_2$ which recognizes two epitopes present in the same antigen, for example, as disclosed in Journal of Immunology (1994) 152 (11), 5368-5374. sc(Fv)$_2$ can be produced by methods known to those skilled in the art. sc(Fv)$_2$ can be produced, for example, by linking scFvs by a linker such as a peptide linker.

Examples of the configuration of the antigen-binding domains constituting the sc(Fv)$_2$ described in the present specification include an antibody in which two VHs and two VLs are aligned as VH, VL, VH, and VL (i.e., [VH]-linker-[VL]-linker-[VH]-linker-[VL]) in this order starting at the N-terminus of the single-chain polypeptide. The order of two VHs and two VLs is not particularly limited to the configuration described above and may be any order of arrangement. Examples thereof can also include the following arrangements:

[VL]-linker-[VH]-linker-[VH]-linker-[VL],
[VH]-linker-[VL]-linker-[VL]-linker-[VH],
[VH]-linker-[VH]-linker-[VL]-linker-[VL],
[VL] -linker- [VL] -linker- [VH]-linker-[VH], and
[VL] -linker- [VH] -linker- [VL] -linker- [VH].

The molecular form of the sc(Fv)$_2$ is also described in detail in WO2006/132352. On the basis of the description therein, those skilled in the art can appropriately prepare the desired sc(Fv)$_2$ in order to prepare the antigen-binding molecule disclosed in the present specification.

The antigen-binding molecule of the present invention may be conjugated with a carrier polymer such as PEG or an organic compound such as an anticancer agent. Also, a sugar chain can be preferably added to the antigen-binding molecule of the present invention by the insertion of a glycosylation sequence for the purpose of producing the desired effects.

For example, an arbitrary peptide linker that can be introduced by genetic engineering, or a synthetic compound linker (e.g., a linker disclosed in Protein Engineering, 9 (3), 299-305, 1996) can be used as the linker to link the antibody variable regions. In the present invention, a peptide linker is preferred. The length of the peptide linker is not particularly limited and can be appropriately selected by those skilled in the art according to the purpose. The length is preferably 5 or more amino acids (the upper limit is not particularly limited and is usually 30 or less amino acids, preferably 20 or less amino acids), particularly preferably 15 amino acids. When the sc(Fv)$_2$ contains three peptide linkers, all of these peptide linkers used may have the same lengths or may have different lengths.

Examples of the peptide linker can include

Ser,
Gly-Ser,
Gly-Gly-Ser,
Ser-Gly-Gly,
Gly-Gly-Gly-Ser,
Ser-Gly-Gly-Gly,
Gly-Gly-Gly-Gly-Ser,
Ser-Gly-Gly-Gly-Gly,
Gly-Gly-Gly-Gly-Gly-Ser,
Ser-Gly-Gly-Gly-Gly-Gly,
Gly-Gly-Gly-Gly-Gly-Gly-Ser,
Ser-Gly-Gly-Gly-Gly-Gly-Gly,
(Gly-Gly-Gly-Gly-Ser)$_n$, and

EP 4 197 545 A1

(Ser-Gly-Gly-Gly-Gly)$_n$,

wherein n is an integer of 1 or larger. However, the length or sequence of the peptide linker can be appropriately selected by those skilled in the art according to the purpose.

The synthetic compound linker (chemical cross-linking agent) is a cross-linking agent usually used in the cross-linking of peptides, for example, N-hydroxysuccinimide (NHS), disuccinimidyl suberate (DSS), bis(sulfosuccinimidyl) suberate (BS3), dithiobis(succinimidyl propionate) (DSP), dithiobis(sulfosuccinimidyl propionate) (DTSSP), ethylene glycol bis(succinimidyl succinate) (EGS), ethylene glycol bis(sulfosuccinimidyl succinate) (sulfo-EGS), disuccinimidyl tartrate (DST), disulfosuccinimidyl tartrate (sulfo-DST), bis[2-(succinimidoxycarbonyloxy)ethyl]sulfone (BSOCOES), or bis[2-(sulfosuccinimidoxycarbonyloxy)ethyl]sulfone (sulfo-BSOCOES). These cross-linking agents are commercially available.

While three linkers are normally required to bind four antibody variable regions, the linkers to be used may the same linker, or different linkers.

"Fab" is constituted by a single light chain, and a CH1 region and a variable region of a single heavy chain. The heavy chain of the Fab molecule cannot form a disulfide bond with another heavy chain molecule.

"F(ab')$_2$" and "Fab'" are produced by treating an immunoglobulin (monoclonal antibody) with protease such as pepsin and papain, and mean an antibody fragment produced by digestion near a disulfide bond present between two H chains in a hinge region. For example, papain treatment cleaves IgG upstream of the disulfide bond present between two H chains in a hinge region to produce two homologous antibody fragments in which an L chain consisting of VL (L chain variable region) and CL (L chain constant region) is linked to an H chain fragment consisting of VH (H chain variable region) and CH gamma 1 (gamma 1 region in an H chain constant region) via a disulfide bond at their C-terminal regions. Each of these two homologous antibody fragments is called Fab'.

"F(ab')$_2$" comprises two light chains and two heavy chains containing a constant region (CH1 domains and a portion of CH2 domains) so as to form the interchain disulfide bond between these two heavy chains. The F(ab')$_2$ constituting the antigen-binding molecule disclosed in the present specification can be preferably obtained by the partial digestion of, for example, a full-length monoclonal antibody having the desired antigen-binding domains with a proteolytic enzyme such as pepsin followed by the removal of an Fc portion adsorbed on a protein A column. The protease is not particularly limited as long the protease is capable of digesting a full-length antibody to restrictively form F(ab')$_2$ under appropriately set reaction conditions (e.g., pH) of the enzyme. Examples thereof can include pepsin and ficin.

An Fc region constituting the antigen-binding molecule disclosed in the present specification can be preferably obtained by partially digesting an antibody such as a monoclonal antibody with protease such as pepsin, and then adsorbing the resulting portion onto a protein A or protein G column, followed by elution with an appropriate elution buffer or the like. The protease is not particularly limited as long as the protease is capable of digesting an antibody such as a monoclonal antibody under appropriately set reaction conditions (e.g., pH) of the enzyme. Examples thereof can include pepsin and ficin.

The antigen-binding molecule described in the present specification comprises an Fc region having reduced binding activity against an Fc gamma receptor and having amino acids constituting an IgG1, IgG2, IgG3 or IgG4 Fc region.

Antibody isotypes are determined according to the structures of constant regions. Constant regions of the isotypes IgG1, IgG2, IgG3, and IgG4 are called C gamma 1, C gamma 2, C gamma 3, and C gamma 4, respectively.

The Fc region refers to a region, except for F(ab')$_2$, comprising two light chains and two heavy chains comprising a portion of a constant region including a region between the CH1 and CH2 domains such that interchain disulfide bonds are formed between the two heavy chains. The Fc region constituting the antigen-binding molecule disclosed in the present specification can be preferably obtained by partially digesting an IgG1, IgG2, IgG3, or IgG4 monoclonal antibody or the like with protease such as pepsin, and then re-eluting a fraction adsorbed on a protein A column. The protease is not particularly limited as long as the protease is capable of digesting a full-length antibody to restrictively form F(ab')$_2$ under appropriately set reaction conditions (e.g., pH) of the enzyme. Examples thereof can include pepsin and ficin.

The Fcγ receptor refers to a receptor capable of binding to the Fc region of an IgG1, IgG2, IgG3, or IgG4 monoclonal antibody and means any member of the protein family substantially encoded by Fcγ receptor genes. In humans, this family includes, but is not limited to: FcγRI (CD64) including isoforms FcγRIa, FcγRIb, and FcγRIc; FcγRII (CD32) including isoforms FcγRIIa (including allotypes H131 and R131), FcγRIIb (including FcγRIIb-1 and FcγRIIb-2), and FcγRIIc; and FcγRIII (CD16) including isoforms FcγRIIIa (including allotypes V158 and F158) and FcγRIIIb (including allotypes FcγRIIIb-NA1 and FcγRIIIb-NA2); and any yet-to-be-discovered human FcγR or FcγR isoform or allotype. The FcγR includes those derived from humans, mice, rats, rabbits, and monkeys. The FcγR is not limited to these molecules and may be derived from any organism. The mouse FcγRs include, but are not limited to, FcγRI (CD64), FcγRII (CD32), FcγRIII (CD16), and FcγRIII-2 (CD16-2), and any yet-to-be-discovered mouse FcγR or FcγR isoform or allotype. Preferred examples of such Fcγ receptors include human FcγRI (CD64), FcγRIIa (CD32), FcγRIIB (CD32), FcγRIIIA (CD16), and/or FcγRIIIB (CD16). The polynucleotide sequence and amino acid sequence of FcγRI are described in RefSeq registration Nos. NM_000566.3 and NP_000557.1, respectively. The polynucleotide sequence and amino acid sequence

of FcγRIIA are described in RefSeq registration Nos. BC020823.1 and 30AAH20823.1, respectively. The polynucleotide sequence and amino acid sequence of FcγRIIB are described in RefSeq registration Nos. BC146678.1 and AAI46679.1, respectively. The polynucleotide sequence and amino acid sequence of FcγRIIIA are described in RefSeq registration Nos. BC033678.1 and AAH33678.1, respectively. The polynucleotide sequence and amino acid sequence of FcγRIIIB are described in RefSeq registration Nos. BC128562.1 and AAI28563.1, respectively. Whether or not the Fcγ receptor has binding activity against the Fc region of an IgG1, IgG2, IgG3, or IgG4 monoclonal antibody can be confirmed by FACS or the ELISA format described above as well as by ALPHAScreen (amplified luminescent proximity homogeneous assay screen), the BIACORE method based on a surface plasmon resonance (SPR) phenomenon, or the like (Proc. Natl. Acad. Sci. USA (2006) 103 (11), 4005-4010).

The "Fc ligand" or the "effector ligand" refers to a molecule derived from any organism, preferably a polypeptide, which binds to an antibody Fc region to form an Fc/Fc ligand complex. The binding of the Fc ligand to Fc preferably induces one or more effector functions. The Fc ligand includes, but is not limited to, Fc receptors, FcγR, FcαR, FcεR, FcRn, C1q, and C3, mannan binding lectin, mannose receptor, *Staphylococcus* protein A, *Staphylococcus* protein G, and viral FcγR. The Fc ligand also includes Fc receptor homologs (FcRH) (Davis et al., (2002) Immunological Reviews 190, 123-136) which are a family of Fc receptors homologous to FcγR. The Fc ligand may also include unfound molecules which binds to Fc.

Whether or not the Fc region has reduced binding activity against any Fcγ receptor of FcγI, FcγIIA, FcγIIB, FcγIIIA and/or FcγIIIB can be confirmed by FACS or the ELISA format described above as well as by ALPHAScreen (amplified luminescent proximity homogeneous assay screen), the BIACORE method based on a surface plasmon resonance (SPR) phenomenon, or the like (Proc. Natl. Acad. Sci. USA (2006) 103 (11), 4005-4010).

The ALPHAScreen method is carried out by the ALPHA technology using two types of beads (donor and acceptor) on the basis of the following principle: luminescence signals are detected only when these two beads are located in proximity through the biological interaction between a molecule bound with the donor bead and a molecule bound with the acceptor bead. A laser-excited photosensitizer in the donor bead converts ambient oxygen to singlet oxygen having an excited state. The singlet oxygen diffuses around the donor bead and reaches the acceptor bead located in proximity thereto to thereby cause chemiluminescent reaction in the bead, which finally emits light. In the absence of the interaction between the molecule bound with the donor bead and the molecule bound with the acceptor bead, singlet oxygen produced by the donor bead does not reach the acceptor bead. Thus, no chemiluminescent reaction occurs.

For example, a biotin-labeled antigen-binding molecule is allowed to bind to the donor bead, while a glutathione S transferase (GST)-tagged Fcγ receptor is allowed to bind to the acceptor bead. In the absence of a competing antigen-binding molecule having a mutated Fc region, an antigen-binding molecule having a wild-type Fc region interacts with the Fcγ receptor to generate signals of 520 to 620 nm. The untagged antigen-binding molecule having a mutated Fc region competes with the antigen-binding molecule having a wild-type Fc region for the interaction with the Fcγ receptor. Decrease in fluorescence caused as a result of the competition can be quantified to thereby determine relative binding affinity. The antigen-binding molecule (e.g., antibody) biotinylation using sulfo-NHS-biotin or the like is known in the art. The Fcγ receptor can be tagged with GST by an appropriately adopted method which involves, for example: fusing a polynucleotide encoding the Fcγ receptor in flame with a polynucleotide encoding GST; and allowing the resulting fusion gene to be expressed by cells or the like harboring vectors capable of expression thereof, followed by purification using a glutathione column. The obtained signals are preferably analyzed using, for example, software GRAPHPAD PRISM (GraphPad Software, Inc., San Diego) adapted to a one-site competition model based on nonlinear regression analysis.

One (ligand) of the substances between which the interaction is to be observed is immobilized onto a thin gold film of a sensor chip. The sensor chip is irradiated with light from the back such that total reflection occurs at the interface between the thin gold film and glass. As a result, a site having a drop in reflection intensity (SPR signal) is formed in a portion of reflected light. The other (analyte) of the substances between which the interaction is to be observed is injected on the surface of the sensor chip. Upon binding of the analyte to the ligand, the mass of the immobilized ligand molecule is increased to change the refractive index of the solvent on the sensor chip surface. This change in the refractive index shifts the position of the SPR signal (on the contrary, the dissociation of the bound molecules gets the signal back to the original position). The Biacore system plots on the ordinate the amount of the shift, i.e., change in mass on the sensor chip surface, and displays time-dependent change in mass as assay data (sensorgram). Kinetics, i.e., an association rate constant (ka) and a dissociation rate constant (kd), can be determined from the curve of the sensorgram, while affinity (KD) can be determined from the ratio between these constants. Inhibition assay is also preferably used in the BIACORE method. Examples of the inhibition assay are described in Proc. Natl. Acad. Sci. USA (2006) 103 (11), 4005-4010.

A plurality of therapeutic antibodies that exhibit an antitumor effect exert an antitumor effect on cancer cells through the inhibition of signals necessary for cancer cell growth, the induction of cell death signals, or ADCC (antibody dependent cell-mediated cytotoxicity or antibody-dependent cellular cytotoxicity) or CDC (complement dependent cytotoxicity). An antibody Fc region binds to an Fc receptor present on effector cells such as NK cells or macrophages so that these effector cells exert cytotoxicity to a target cancer cell bound with the antibody. This cytotoxicity is ADCC. A complement complex binds to a complement binding site present in an antibody structure. A complement component present in the complex forms a hole on the cell membrane of a cell bound with the antibody so that the influx of water or ions into the cell is promoted to disrupt the cell, causing cytotoxicity. This cytotoxicity is CDC. Among the Fc receptors, the Fcγ receptor refers to a receptor capable of binding to the Fc region of an IgG1, IgG2, IgG3, or IgG4 monoclonal antibody. When binding activity against the Fcγ receptor is low, T cells and the receptor expressed in NK cells, macrophages, or the like are not bridged in a cancer antigen-independent manner. Hence, cancer antigen-independent cytokine induction does not occur. An antibody having reduced binding activity against any of the Fcγ receptors of FcγI, FcγIIA, FcγIIB, FcγIIIA and/or FcγIIIB is desirable for an antigen-binding molecule.

In the present specification, the reduced binding activity against an Fcγ receptor means that the test antigen-binding molecule exhibits competitive activity of, for example, 50% or lower, preferably 45% or lower, 40% or lower, 35% or lower, 30% or lower, 20% or lower, or 15% or lower, particularly preferably 10% or lower, 9% or lower, 8% or lower, 7% or lower, 6% or lower, 5% or lower, 4% or lower, 3% or lower, 2% or lower, or 1% or lower, compared with the competitive activity of a control antigen-binding molecule on the basis of the analysis method described above.

An antigen-binding molecule having an IgG1, IgG2, IgG3, or IgG4 monoclonal antibody Fc region can be appropriately used as the control antigen-binding molecule. Examples of the structure of the Fc region include a sequence of RefSeq registration No. AAC82527.1 with A added to the N terminus, a sequence of RefSeq registration No. AAB59393.1 with A added to the N terminus, a sequence of RefSeq registration No. CAA27268.1 with A added to the N terminus, and a sequence of RefSeq registration No. AAB59394.1 with A added to the N terminus. In the case of using an antigen-binding molecule having a mutant of the Fc region of an antibody of a certain isotype as a test substance, an antigen-binding molecule having the Fc region of the antibody of this certain isotype is used as a control to test the effect of the mutation in the mutant on the binding activity against an Fcγ receptor. The antigen-binding molecule having the Fc region mutant thus confirmed to have reduced binding activity against an Fcγ receptor is appropriately prepared.

For example, a 231A-238S deletion (WO 2009/011941), C226S, C229S, P238S, (C220S) (J. Rheumatol (2007) 34, 11), C226S, C229S (Hum. Antibod. Hybridomas (1990) 1 (1), 47-54), C226S, C229S, E233P, L234V, or L235A (Blood (2007) 109, 1185-1192) (these amino acids are defined according to the EU numbering) mutant is known in the art as such a mutant.

Preferred examples thereof include antigen-binding molecules having an Fc region derived from the Fc region of an antibody of a certain isotype by the substitution of any of the following constituent amino acids: amino acids at positions 220, 226, 229, 231, 232, 233, 234, 235, 236, 237, 238, 239, 240, 264, 265, 266, 267, 269, 270, 295, 296, 297, 298, 299, 300, 325, 327, 328, 329, 330, 331, and 332 defined according to the EU numbering. The isotype of the antibody from which the Fc region is originated is not particularly limited, and an Fc region originated from an IgG1, IgG2, IgG3, or IgG4 monoclonal antibody can be appropriately used. An Fc region originated from an IgG1 antibody is preferably used.

For example, an antigen-binding molecule having an Fc region derived from an IgG1 antibody Fc region by any of the following substitution groups of the constituent amino acids (the number represents the position of an amino acid residue defined according to the EU numbering; the one-letter amino acid code positioned before the number represents an amino acid residue before the substitution; and the one-letter amino acid code positioned after the number represents an amino acid residue after the substitution):

(a) L234F, L235E, and P331S,
(b) C226S, C229S, and P238S,
(c) C226S and C229S,
(d) C226S, C229S, E233P, L234V, and L235A,
(e) L234A, L235A or L235R, and N297A, and
(f) L235A or L235R, S239K, and N297A or by the deletion of an amino acid sequence from positions 231 to 238 defined according to the EU numbering can also be appropriately used.

An antigen-binding molecule having an Fc region derived from an IgG2 antibody Fc region by any of the following substitution groups of the constituent amino acids (the number represents the position of an amino acid residue defined according to the EU numbering; the one-letter amino acid code positioned before the number represents an amino acid residue before the substitution; and the one-letter amino acid code positioned after the number represents an amino acid residue after the substitution):

(g) H268Q, V309L, A330S, and P331S,
(h) V234A,
(i) G237A,
(j) V234A and G237A,
(k) A235E and G237A,

(1) V234A, A235E, and G237A defined according to the EU numbering can also be appropriately used.

An antigen-binding molecule having an Fc region derived from an IgG3 antibody Fc region by any of the following substitution groups of the constituent amino acids (the number represents the position of an amino acid residue defined according to the EU numbering; the one-letter amino acid code positioned before the number represents an amino acid residue before the substitution; and the one-letter amino acid code positioned after the number represents an amino acid residue after the substitution):

(m) F241A,
(n) D265A, and
(o) V264A defined according to the EU numbering can also be appropriately used.

An antigen-binding molecule having an Fc region derived from an IgG4 antibody Fc region by any of the following substitution groups of the constituent amino acids (the number represents the position of an amino acid residue defined according to the EU numbering; the one-letter amino acid code positioned before the number represents an amino acid residue before the substitution; and the one-letter amino acid code positioned after the number represents an amino acid residue after the substitution):

(p) L235A, G237A, and E318A,
(q) L235E, and
(r) F234A and L235A defined according to the EU numbering can also be appropriately used.

Other preferred examples thereof include antigen-binding molecules having an Fc region derived from the Fc region of an IgG1 antibody by the substitution of any of the following constituent amino acids: amino acids at positions 233, 234, 235, 236, 237, 327, 330, and 331 defined according to the EU numbering, by an amino acid at the corresponding EU numbering position in the Fc region of the counterpart IgG2 or IgG4.

Other preferred examples thereof include antigen-binding molecules having an Fc region derived from the Fc region of an IgG1 antibody by the substitution of any one or more of the following constituent amino acids: amino acids at positions 234, 235, and 297 defined according to the EU numbering, by a different amino acid. The type of the amino acid present after the substitution is not particularly limited. An antigen-binding molecule having an Fc region with any one or more of amino acids at positions 234, 235, and 297 substituted by alanine is particularly preferred.

Other preferred examples thereof include antigen-binding molecules having an Fc region derived from the Fc region of an IgG1 antibody by the substitution of the constituent amino acid at position 265 defined according to the EU numbering, by a different amino acid. The type of the amino acid present after the substitution is not particularly limited. An antigen-binding molecule having an Fc region with an amino acid at position 265 substituted by alanine is particularly preferred.

Many studies have been made so far on antibody-dependent cellular cytotoxicity (ADCC) and complement-dependent cytotoxicity (CDC) which are the effector functions of antibodies of IgG class. Among the antibodies of human IgG class, antibodies of IgG1 subclass have the highest ADCC activity and CDC activity. Also, antibody-dependent cellular phagocytosis (ADCP) which is the phagocytosis of target cells via antibodies of IgG class is suggested as one of the effector functions of antibodies. Antibodies of IgG1 subclass are capable of exerting these effector functions against tumor. Therefore, the antibodies of IgG1 subclass are used as most of antibody drugs for cancer antigens.

Meanwhile, IgG antibodies for mediating an antibody effector function ADCC or ADCP, or antibody-dependent cellular phagocytosis (ADCP) activity for the phagocytosis of target cells require the binding of the Fc region of the IgG antibody to an Fcγ receptor (FcγR) present on the surface of effector cells such as killer cells, natural killer cells, or activated macrophages.

Enhancement in cytotoxic effector functions such as ADCC and ADCP is a promising approach for enhancing the antitumor effects of anticancer antibodies. It is suggested that an antibody having an Fc region that exhibits the optimized binding to an Fcγ receptor mediates a stronger effector function and thereby exerts an effective antitumor effect. Accordingly, various reports (e.g., WO2013047752) have been made so far as antibody engineering approaches of enhancing or improving the antitumor activity of antibody drugs against cancer antigens.

For the binding between an Fc region and an Fcγ receptor, some amino acid residues in an antibody hinge region and CH2 domain and sugar chains added to Asn 297 (EU numbering) of the CH2 domain have been found to be important (Clark, M., Chemical Immunology (1997) 65, 88-110; Greenwood J, Clark M, Waldmann H., Eur. J. Immunol. (1993) 23, 1098-1104; and Morgan A, Jones ND, Nesbitt AM, Chaplin L, Bodmer MW, Emtage JS., Immunology (1995) 86, 319-324). Fc region mutants having various Fcγ receptor binding characteristics have been studied so far by focusing on this binding site, to obtain Fc region mutants having higher affinity for activating Fcγ receptors (WO2000/042072 and WO2006/019447).

Since binding activity against an Fcγ receptor plays an important role in the cytotoxic activity of antibodies targeting membrane antigens as mentioned above, human IgG1 isotype having high binding activity against FcγR is used when cytotoxic activity is necessary. Further, enhancement in cytotoxic activity by enhancing binding activity against an Fcγ receptor is a technique widely used. An attempt has also been made to enhance the Fcγ receptor binding activity of antibodies targeting soluble antigens (WO2013047752).

The secondary molecule antibody having ADCC activity is preferably an IgG antibody or an IgG antibody-like molecule having an Fc region containing at least one or more amino acids selected from the group consisting of:

Lys or Tyr at amino acid position 221,
Phe, Trp, Glu or Tyr at amino acid position 222,
Phe, Trp, Glu or Lys at amino acid position 223,
Phe, Trp, Glu or Tyr at amino acid position 224,
Glu, Lys or Trp at amino acid position 225,
Glu, Gly, Lys or Tyr at amino acid position 227,
Glu, Gly, Lys or Tyr at amino acid position 228,
Ala, Glu, Gly or Tyr at amino acid position 230,
Glu, Gly, Lys, Pro or Tyr at amino acid position 231,
Glu, Gly, Lys or Tyr at amino acid position 232,
Ala, Asp, Phe, Gly, His, Ile, Lys, Leu, Met, Asn, Gln, Arg, Ser, Thr, Val, Trp or Tyr at amino acid position 233,
Ala, Asp, Glu, Phe, Gly, His, Ile, Lys, Met, Asn, Pro, Gln, Arg, Ser, Thr, Val, Trp or Tyr at amino acid position 234,
Ala, Asp, Glu, Phe, Gly, His, Ile, Lys, Met, Asn, Pro, Gln, Arg, Ser, Thr, Val, Trp or Tyr at amino acid position 235,
Ala, Asp, Glu, Phe, His, Ile, Lys, Leu, Met, Asn, Pro, Gln, Arg, Ser, Thr, Val, Trp or Tyr at amino acid position 236,
Asp, Glu, Phe, His, Ile, Lys, Leu, Met, Asn, Pro, Gln, Arg, Ser, Thr, Val, Trp or Tyr at amino acid position 237,
Asp, Glu, Phe, Gly, His, Ile, Lys, Leu, Met, Asn, Gln, Arg, Ser, Thr, Val, Trp or Tyr at amino acid position 238,
Asp, Glu, Phe, Gly, His, Ile, Lys, Leu, Met, Asn, Pro, Gln, Arg, Thr, Val, Trp or Tyr at amino acid position 239,
Ala, Ile, Met or Thr at amino acid position 240,
Asp, Glu, Leu, Arg, Trp or Tyr at amino acid position 241,
Leu, Glu, Leu, Gln, Arg, Trp or Tyr at amino acid position 243,
His at amino acid position 244,
Ala at amino acid position 245,
Asp, Glu, His or Tyr at amino acid position 246,
Ala, Phe, Gly, His, Ile, Leu, Met, Thr, Val or Tyr at amino acid position 247,
Glu, His, Gln or Tyr at amino acid position 249,
Glu or Gln at amino acid position 250,
Phe at amino acid position 251,
Phe, Met or Tyr at amino acid position 254,
Glu, Leu or Tyr at amino acid position 255,
Ala, Met or Pro at amino acid position 256,
Asp, Glu, His, Ser or Tyr at amino acid position 258,
Asp, Glu, His or Tyr at amino acid position 260,
Ala, Glu, Phe, Ile or Thr at amino acid position 262,
Ala, Ile, Met or Thr at amino acid position 263,
Asp, Glu, Phe, Gly, His, Ile, Lys, Leu, Met, Asn, Pro, Gln, Arg, Ser, Thr, Trp or Tyr at amino acid position 264,
Ala, Leu, Phe, Gly, His, Ile, Lys, Leu, Met, Asn, Pro, Gln, Arg, Ser, Thr, Val, Trp or Tyr at amino acid position 265,
Ala, Ile, Met or Thr at amino acid position 266,
Asp, Glu, Phe, His, Ile, Lys, Leu, Met, Asn, Pro, Gln, Arg, Thr, Val, Trp or Tyr at amino acid position 267,
Asp, Glu, Phe, Gly, Ile, Lys, Leu, Met, Pro, Gln, Arg, Thr, Val or Trp at amino acid position 268,
Phe, Gly, His, Ile, Lys, Leu, Met, Asn, Pro, Arg, Ser, Thr, Val, Trp or Tyr at amino acid position 269,
Glu, Phe, Gly, His, Ile, Leu, Met, Pro, Gln, Arg, Ser, Thr, Trp or Tyr at amino acid position 270,
Ala, Asp, Glu, Phe, Gly, His, Ile, Lys, Leu, Met, Asn, Gln, Arg, Ser, Thr, Val, Trp or Tyr at amino acid position 271,
Asp, Phe, Gly, His, Ile, Lys, Leu, Met, Pro, Arg, Ser, Thr, Val, Trp or Tyr at amino acid position 272,
Phe or Ile at amino acid position 273,
Asp, Glu, Phe, Gly, His, Ile, Leu, Met, Asn, Pro, Arg, Ser, Thr, Val, Trp or Tyr at amino acid position 274,
Leu or Trp at amino acid position 275,
Asp, Glu, Phe, Gly, His, Ile, Leu, Met, Pro, Arg, Ser, Thr, Val, Trp or Tyr at amino acid position 276,
Asp, Glu, Gly, His, Ile, Lys, Leu, Met, Asn, Pro, Gln, Arg, Ser, Thr, Val or Trp at amino acid position 278,
Ala at amino acid position 279,
Ala, Gly, His, Lys, Leu, Pro, Gln, Trp or Tyr at amino acid position 280,

Asp, Lys, Pro or Tyr at amino acid position 281,

Glu, Gly, Lys, Pro or Tyr at amino acid position 282,

Ala, Gly, His, Ile, Lys, Leu, Met, Pro, Arg or Tyr at amino acid position 283,

Asp, Glu, Leu, Asn, Thr or Tyr at amino acid position 284,

Asp, Glu, Lys, Gln, Trp or Tyr at amino acid position 285,

Glu, Gly, Pro or Tyr at amino acid position 286,

Asn, Asp, Glu or Tyr at amino acid position 288,

Asp, Gly, His, Leu, Asn, Ser, Thr, Trp or Tyr at amino acid position 290,

Asp, Glu, Gly, His, Ile, Gln or Thr at amino acid position 291,

Ala, Asp, Glu, Pro, Thr or Tyr at amino acid position 292,

Phe, Gly, His, Ile, Leu, Met, Asn, Pro, Arg, Ser, Thr, Val, Trp or Tyr at amino acid position 293,

Phe, Gly, His, Ile, Lys, Leu, Met, Asn, Pro, Arg, Ser, Thr, Val, Trp or Tyr at amino acid position 294,

Asp, Glu, Phe, Gly, His, Ile, Lys, Met, Asn, Pro, Arg, Ser, Thr, Val, Trp or Tyr at amino acid position 295,

Ala, Asp, Glu, Gly, His, Ile, Lys, Leu, Met, Asn, Gln, Arg, Ser, Thr or Val at amino acid position 296,

Asp, Glu, Phe, Gly, His, Ile, Lys, Leu, Met, Pro, Gln, Arg, Ser, Thr, Val, Trp or Tyr at amino acid position 297,

Ala, Asp, Glu, Phe, His, Ile, Lys, Met, Asn, Gln, Arg, Thr, Val, Trp or Tyr at amino acid position 298,

Ala, Asp, Glu, Phe, Gly, His, Ile, Lys, Leu, Met, Asn, Pro, Gln, Arg, Ser, Val, Trp or Tyr at amino acid position 299,

Ala, Asp, Glu, Gly, His, Ile, Lys, Leu, Met, Asn, Pro, Gln, Arg, Ser, Thr, Val or Trp at amino acid position 300,

Asp, Glu, His or Tyr at amino acid position 301,

Ile at amino acid position 302,

Asp, Gly or Tyr at amino acid position 303,

Asp, His, Leu, Asn or Thr at amino acid position 304,

Glu, Ile, Thr or Tyr at amino acid position 305,

Ala, Asp, Asn, Thr, Val or Tyr at amino acid position 311,

Phe at amino acid position 313,

Leu at amino acid position 315,

Glu or Gln at amino acid position 317,

His, Leu, Asn, Pro, Gln, Arg, Thr, Val or Tyr at amino acid position 318,

Asp, Phe, Gly, His, Ile, Leu, Asn, Pro, Ser, Thr, Val, Trp or Tyr at amino acid position 320,

Ala, Asp, Phe, Gly, His, Ile, Pro, Ser, Thr, Val, Trp or Tyr at amino acid position 322,

Ile at amino acid position 323,

Asp, Phe, Gly, His, Ile, Leu, Met, Pro, Arg, Thr, Val, Trp or Tyr at amino acid position 324,

Ala, Asp, Glu, Phe, Gly, His, Ile, Lys, Leu, Met, Pro, Gln, Arg, Ser, Thr, Val, Trp or Tyr at amino acid position 325,

Ala, Asp, Glu, Gly, Ile, Leu, Met, Asn, Pro, Gln, Ser, Thr, Val, Trp or Tyr at amino acid position 326,

Ala, Asp, Glu, Phe, Gly, His, Ile, Lys, Leu, Met, Asn, Pro, Arg, Thr, Val, Trp or Tyr at amino acid position 327,

Ala, Asp, Glu, Phe, Gly, His, Ile, Lys, Met, Asn, Pro, Gln, Arg, Ser, Thr, Val, Trp or Tyr at amino acid position 328,

Asp, Glu, Phe, Gly, His, Ile, Lys, Leu, Met, Asn, Gln, Arg, Ser, Thr, Val, Trp or Tyr at amino acid position 329,

Cys, Glu, Phe, Gly, His, Ile, Lys, Leu, Met, Asn, Pro, Arg, Ser, Thr, Val, Trp or Tyr at amino acid position 330,

Asp, Phe, His, Ile, Leu, Met, Gln, Arg, Thr, Val, Trp or Tyr at amino acid position 331,

Ala, Asp, Glu, Phe, Gly, His, Lys, Leu, Met, Asn, Pro, Gln, Arg, Ser, Thr, Val, Trp or Tyr at amino acid position 332,

Ala, Asp, Glu, Phe, Gly, His, Ile, Leu, Met, Pro, Ser, Thr, Val or Tyr at amino acid position 333,

Ala, Glu, Phe, Ile, Leu, Pro or Thr at amino acid position 334,

Asp, Phe, Gly, His, Ile, Leu, Met, Asn, Pro, Arg, Ser, Val, Trp or Tyr at amino acid position 335,

Glu, Lys or Tyr at amino acid position 336,

Glu, His or Asn at amino acid position 337,

Asp, Phe, Gly, Ile, Lys, Met, Asn, Gln, Arg, Ser or Thr at amino acid position 339,

Ala or Val at amino acid position 376,

Gly or Lys at amino acid position 377,

Asp at amino acid position 378,

Asn at amino acid position 379,

Ala, Asn or Ser at amino acid position 380,

Ala or Ile at amino acid position 382,

Glu at amino acid position 385,

Thr at amino acid position 392,

Leu at amino acid position 396,

Lys at amino acid position 421,

Asn at amino acid position 427,

Phe or Leu at amino acid position 428,

Met at amino acid position 429,

Trp at amino acid position 434,

Ile at amino acid position 436, and

Gly, His, Ile, Leu or Tyr at amino acid position 440, among sites represented by EU numbering in the Fc region.

Examples of the antigen-binding molecule according to the present invention include a multispecific antibody. In this context, the multispecific antibody is an antibody having a plurality of different specificities. Examples of the multispecific antibody include a bispecific antibody. An IgG bispecific antibody can be secreted by a hybrid hybridoma (quadroma) resulting from the fusion of two IgG antibody-producing hybridomas (Milstein C et al., Nature (1983) 305, 537-540). In the case of using an Fc region having reduced binding activity against an Fcγ receptor as an Fc region of the bispecific antibody, an Fc region originating from a bispecific antibody is also appropriately used.

Examples of the method for preparing a multispecific antibody include, but are not limited to, recombinant coexpression of two immunoglobulin heavy chain-light chain pairs having different specificities (see Milstein and Cuello, Nature 305: 537 (1983), WO93/08829, and Traunecker et al., EMBO J. 10: 3655 (1991)), and the knob-in-hole technique (see e.g., U.S. Patent No. 5,731,168). The multispecific antibody may also be prepared by manipulating electrostatic steering effects for preparing a Fc heterodimer molecule (WO2009/089004A1); crosslinking two or more antibodies or portions (see U.S. Patent No. 4,676,980 and Brennan et al., Science, 229: 81 (1985)); preparing an antibody having two specificities using a leucine zipper (see Kostelny et al., J. Immunol., 148(5):1547-1553 (1992)); preparing a bispecific antibody portion using the "diabody" technique (see Hollinger et al., Proc. Natl. Acad. Sci. USA, 90:6444-6448 (1993)); using a single chain Fv (scFv) dimer (see Gruber et al., J. Immunol., 152:5368 (1994)); and preparing a trispecific antibody as described in, for example, Tutt et al. J. Immunol. 147: 60 (1991).

In the present specification, the antibodies also include an altered antibody with three or more functional antigen-binding sites which includes an " octopus antibody" (see e.g., U.S.2006/0025576 A1).

In one aspect of the present invention, the antigen-binding molecule comprises a bispecific antigen-binding site having a target antigen recognition site and an immunoreceptor recognition site. In this context, the antigen-binding molecule may have an additional antigen-binding site in addition to a bispecific antigen-binding site. Examples of the antigen-binding molecule in the aspect include a bispecific antigen-binding molecule, and a multispecific antigen-binding molecule having three or more antigen-binding sites, and more specific examples thereof include a bispecific antibody, and a multispecific antibody having three or more antigen-binding sites.

The IgG bispecific antibody is secreted by introducing a total of four genes, genes of L chains and H chains constituting the two IgG antibodies of interest, to cells, and coexpressing these genes. However, the number of combinations of IgG H and L chains produced by such a method is theoretically as many as 10. It is difficult to purify IgG consisting of H and L chains in the combination of interest from the ten IgG types. In addition, the amount of a secreted bispecific antibody having the combination of interest is considerably reduced theoretically. This requires a large culture scale and further increases production cost.

A technique for promoting the association between H chains and between L and H chains in the combination of interest can be applied to the bispecific antibody of the present invention.

For example, a technique of suppressing the unintended association between H chains by introducing charge repulsion to the interface between the second constant regions (CH2) or the third constant regions (CH3) of the antibody H chains (WO2006/106905) can be applied to association for the multispecific antibody.

In the technique of suppressing the unintended association between H chains by introducing charge repulsion to the CH2 or CH3 interface, examples of amino acid residues contacting with each other at the interface between the H chain constant regions can include a residue at EU numbering position 356, a residue at EU numbering position 439, a residue at EU numbering position 357, a residue at EU numbering position 370, a residue at EU numbering position 399, and a residue at EU numbering position 409 in one CH3 region, and their partner residues in another CH3 region. More specifically, for example, an antibody comprising two H chain CH3 regions can be prepared as an antibody in which one to three pairs of amino acid residues selected from the following amino acid residue pairs (1) to (3) in the first H chain CH3 region have the same charge: (1) amino acid residues at EU numbering positions 356 and 439 contained in the H chain CH3 region; (2) amino acid residues at EU numbering positions 357 and 370 contained in the H chain CH3 region; and (3) amino acid residues at EU numbering positions 399 and 409 contained in the H chain CH3 region.

The antibody can be further prepared as an antibody in which one to three pairs of amino acid residues are selected from the amino acid residue pairs (1) to (3) in the second H chain CH3 region different from the first H chain CH3 region so as to correspond to the amino acid residue pairs (1) to (3) having the same charge in the first H chain CH3 region and to have opposite charge from their corresponding amino acid residues in the first H chain CH3 region.

Each amino acid residue described in the pairs (1) to (3) is located close to its partner in the associated H chains. Those skilled in the art can find positions corresponding to the amino acid residues described in each of the pairs (1) to (3) as to the desired H chain CH3 regions or H chain constant regions by homology modeling or the like using commercially available software and can appropriately alter amino acid residues at the positions.

In the antibody described above, each of the "amino acid residues having charge" is preferably selected from, for example, amino acid residues included in any of the following groups (a) and (b):

(a) glutamic acid (E) and aspartic acid (D); and
(b) lysine (K), arginine (R), and histidine (H).

In the antibody described above, the phrase "having the same charge" means that, for example, all of two or more amino acid residues are amino acid residues included in any one of the groups (a) and (b). The phrase "carrying opposite charge" means that, for example, at least one amino acid residue among two or more amino acid residues may be an amino acid residue included in any one of the groups (a) and (b), while the residual amino acid residue(s) is amino acid residue(s) included in the other group.

In a preferred embodiment, the antibody may have the first H chain CH3 region and the second H chain CH3 region cross-linked through a disulfide bond.

The amino acid residue to be altered according to the present invention is not limited to the amino acid residues in the antibody variable region or the antibody constant region mentioned above. Those skilled in the art can find amino acid residues constituting the interface as to a polypeptide mutant or a heteromultimer by homology modeling or the like using commercially available software and can alter amino acid residues at the positions so as to regulate the association.

The association for the bispecific antibody of the present invention can also be carried out by an alternative technique known in the art. An amino acid side chain present in the Fc region of one antibody H chain is substituted by a larger side chain (knob), and its partner amino acid side chain present in the Fc region of the other H chain is substituted by a smaller side chain (hole). The knob can be placed into the hole to efficiently associate the polypeptides of the Fc domains differing in amino acid sequence (WO1996/027011; Ridgway JB et al., Protein Engineering (1996) 9, 617-621; Merchant AM et al. Nature Biotechnology (1998) 16, 677-681; and US20130336973).

In addition to this technique, a further alternative technique known in the art can be used for forming the bispecific antibody of the present invention. A portion of CH3 of one antibody H chain is converted to its counterpart IgA-derived sequence, and its complementary portion in CH3 of the other H chain is converted to its counterpart IgA-derived sequence. Use of the resulting strand-exchange engineered domain CH3 can cause efficient association between the polypeptides differing in sequence through complementary CH3 association (Protein Engineering Design & Selection, 23; 195-202, 2010). By use of this technique known in the art, the bispecific antibody of interest can also be efficiently formed.

Alternatively, the bispecific antibody may be formed by, for example, an antibody preparation technique using antibody CH1-CL association and VH-VL association as described in WO2011/028952, WO2014/018572, or Nat Biotechnol. 2014 Feb; 32 (2): 191-8, a technique of preparing a bispecific antibody using separately prepared monoclonal antibodies (Fab arm exchange) as described in WO2008/119353 and WO2011/131746, a technique of controlling the association between antibody heavy chain CH3 domains as described in WO2012/058768 and WO2013/063702, a technique of preparing a bispecific antibody constituted by two types of light chains and one type of heavy chain as described in WO2012/023053, or a technique of preparing a bispecific antibody using two bacterial cell lines each expressing an antibody half-molecule consisting of one H chain and one L chain as described in Christoph et al. (Nature Biotechnology Vol. 31, p. 753-758 (2013)).

Even if the bispecific antibody of interest cannot be formed efficiently, the bispecific antibody of the present invention may be obtained by the separation and purification of the bispecific antibody of interest from among produced antibodies. For example, the previously reported method involves introducing amino acid substitution to the variable domains of two types of H chains to impart thereto difference in isoelectric point (pI) so that two types of homodimers and the heterodimerized antibody of interest can be separately purified by ion-exchanged chromatography (WO2007114325). A method using protein A to purify a heterodimerized antibody consisting of a mouse IgG2a H chain capable of binding to protein A and a rat IgG2b H chain incapable of binding to protein A has previously been reported as a method for purifying the heterodimer (WO98050431 and WO95033844). Alternatively, amino acid residues at EU numbering positions 435 and 436 that constitute the protein A binding site of IgG may be substituted by amino acids, such as Tyr and His, which offer the different strength of protein A binding, and the resulting H chain, or H chains differing in strength of protein A binding, obtained according to a method described in Reference Example 5, are used to change the interaction of each H chain with protein A. As a result, only the heterodimerized antibody can be efficiently purified by use of a protein A column.

A common L chain capable of imparting binding ability to a plurality of different H chains may be obtained and used as a common L chain for the bispecific antibody. Bispecific IgG can be efficiently expressed by introducing such a common L chain and a plurality of different H chains to cells for IgG expression (Nature Biotechnology (1998) 16, 677-681). A method for selecting a common L chain that exhibits high binding ability in response to any different H chains can also be utilized for the selection of a common H chain (WO2004/065611).

An Fc region having improved C-terminal heterogeneity can be appropriately used as the Fc region of the present invention. More specifically, the present invention provides an Fc region lacking glycine 446 and lysine 447 defined according to the EU numbering in the amino acid sequences of two polypeptides constituting an Fc region originating from IgG1, IgG2, IgG3 or IgG4.

A plurality, for example, two or more, of these techniques may be used in combination. These techniques may be appropriately separately applied to two H chains to be associated. These techniques can further be used in combination with the Fc region having reduced binding activity against an Fcγ receptor mentioned above. The antigen-binding molecule of the present invention may be prepared as a separately produced antigen-binding molecule having the same amino acid sequence based on such a variant.

In the present invention, antibody variable regions that are "functionally equivalent" are not particularly limited as long as the antibody variable regions are antibody H chain and/or L chain variable regions that satisfy the conditions mentioned above. Such antibody variable regions may have the substitution, deletion, addition, and/or insertion of one or more amino acids (e.g., 1, 2, 3, 4, 5, or 10 amino acids) in the amino acid sequences of the variable regions described in Tables 1 to 3 mentioned above. A method well known to those skilled in the art for introducing the substitution, deletion, addition, and/or insertion of one or more amino acids into an amino acid sequence is a method of introducing mutations into proteins. Those skilled in the art can prepare variable regions that are functionally equivalent to the antibody variable regions having the functions mentioned above by appropriately introducing mutations into amino acid sequences using, for example, site-directed mutagenesis (Hashimoto-Gotoh, T, Mizuno, T, Ogasahara, Y, and Nakagawa, M. (1995) An oligodeoxyribonucleotide-directed dual amber method for site-directed mutagenesis. Gene 152, 271-275, Zoller, MJ, and Smith, M. (1983) Oligonucleotide-directed mutagenesis of DNA fragments cloned into M13 vectors. Methods Enzymol. 100, 468-500, Kramer, W, Drutsa, V, Jansen, HW, Kramer, B, Pflugfelder, M, and Fritz, HJ (1984) The gapped duplex DNA approach to oligonucleotide-directed mutation construction. Nucleic Acids Res. 12, 9441-9456, Kramer W, and Fritz HJ (1987) Oligonucleotide-directed construction of mutations via gapped duplex DNA Methods. Enzymol. 154, 350-367, Kunkel, TA (1985) Rapid and efficient site-specific mutagenesis without phenotypic selection. Proc Natl Acad Sci USA. 82, 488-492).

In the case of altering an amino acid residue, the amino acid is desirably mutated into a different amino acid having the conserved properties of the amino acid side chain. Examples of the amino-acid side chain properties can include hydrophobic amino acids (A, I, L, M, F, P, W, Y, and V), hydrophilic amino acids (R, D, N, C, E, Q, G, H, K, S, and T), amino acids having aliphatic side chains (G, A, V, L, I, and P), amino acids containing hydroxy group-containing side chains (S, T, and Y), amino acids having sulfur atom-containing side chains (C and M), amino acids having carboxylic acid- and amide-containing side chains (D, N, E, and Q), amino acids having base-containing side chains (R, K, and H), and amino acids having aromatic side chains (H, F, Y, and W) (amino acids are represented by one-letter codes in parentheses). Amino acid substitution within each of these groups is called conservative substitution. It has already been known that a polypeptide having an amino acid sequence modified from a certain amino acid sequence by the deletion and/or addition of one or more amino acid residues and/or the substitution thereof by other amino acids maintains its biological activity (Mark, D. F. et al., Proc. Natl. Acad. Sci. USA (1984) 81: 5662-6; Zoller, M. J. and Smith, M., Nucleic Acids Res. (1982) 10: 6487-500; Wang, A. et al., Science (1984) 224: 1431-3; Dalbadie-McFarland, G. et al., Proc. Natl. Acad. Sci. USA (1982) 79: 6409-13). The variable region of the present invention having such amino acid alteration has an amino acid sequence identity of at least 70%, more preferably at least 75%, still more preferably at least 80%, further preferably at least 85%, still further preferably at least 90%, most preferably at least 95%, to the amino acid sequence of the CDR sequences, the FR sequences, or the whole variable region of the variable region before the alteration. In the present specification, the sequence identity is defined as the percentage of residues identical to the residues of the original amino acid sequence of the H chain or L chain variable region determined by aligning the sequences and appropriately introducing gaps so as to maximize the sequence identity, as necessary. The identity of amino acid sequences can be determined by a method mentioned later.

The "functionally equivalent antibody variable region" may be obtained, for example, from a nucleic acid that hybridizes under stringent conditions to a nucleic acid consisting of a nucleotide sequence encoding any of the amino acid sequences of the variable regions described in Tables 1 to 3 mentioned above. Examples of the stringent hybridization conditions for isolating a nucleic acid that hybridizes under stringent conditions to a nucleic acid consisting of a nucleotide sequence encoding the amino acid sequence of a variable region can include conditions of 6 M urea, 0.4% SDS, 0.5 × SSC, and 37°C, and hybridization conditions with stringency equivalent thereto. The isolation of a more highly homologous nucleic acid can be expected using more stringent conditions, for example, conditions of 6 M urea, 0.4% SDS, 0.1 × SSC, and 42°C. The washing conditions after the hybridization are, for example, washing with 0.5 × SSC (1 × SSC is 0.15 M NaCl and 0.015 M sodium citrate, pH 7.0) and 0.1% SDS at 60°C, more preferably washing with 0.2 × SSC and 0.1% SDS at 60°C, still more preferably washing with 0.2 × SSC and 0.1% SDS at 62°C, even more preferably washing using 0.2 × SSC and 0.1% SDS at 65°C, yet more preferably washing using 0.1 × SSC and 0.1% SDS at 65°C. The isolated nucleic acid can be sequenced by a method known in the art mentioned later. The homology of the isolated nucleic acid has at least 50% or higher, preferably 70% or higher, more preferably 90% or higher (e.g., 95%, 96%, 97%, 98%, 99%, or higher) sequence identity, in terms of the whole nucleotide sequence.

The nucleic acid that hybridizes under stringent conditions to a nucleic acid consisting of a nucleotide sequence encoding the amino acid sequence of a variable region may be isolated by using, instead of the method based on the hybridization technique described above, a gene amplification method, for example, polymerase chain reaction (PCR), using primers synthesized on the basis of information on the nucleotide sequence encoding the amino acid sequence of the variable region.

The identity of nucleotide sequences or amino acid sequences can be determined using algorithm BLAST by Karlin and Altschul (Proc. Natl. Acad. Sci. USA (1993) 90: 5873-7). Programs called BLASTN and BLASTX have been developed on the basis of this algorithm (Altschul et al., J. Mol. Biol. (1990) 215: 403-10). In the case of analyzing nucleotide sequences according to BLASTN based on BLAST, the parameters are set to, for example, score = 100 and wordlength = 12. In the case of analyzing amino acid sequences according to BLASTX based on BLAST, the parameters are set to, for example, score = 50 and wordlength = 3. In the case of using BLAST and Gapped BLAST programs, default parameters for each program are used. Specific approaches for these analysis methods are known in the art (see the website of NCBI (National Center for Biotechnology Information), BLAST (Basic Local Alignment Search Tool); http://www.ncbi.nlm.nih.gov).

The Fc region contained in the bispecific antibody of the present invention is not particularly limited as long as the Fc region has reduced binding activity against an Fcγ receptor. Preferred examples of the Fc region of the present invention can include a combination of an Fc region moiety of E22Hh and an Fc region moiety of E22Hk, a combination of an Fc region moiety of E2702GsKsc and an Fc region moiety of E2704sEpsc, and combination of an Fc region moiety of E2702sKsc and an Fc region moiety of E2704sEpsc described in WO2016/047722A1.

Alternatively, a gene encoding the antigen-binding molecule according to the present invention may be incorporated into a vector for gene treatment to perform gene treatment. As an administration method, the gene can be directly administered by naked plasmid. Alternatively, the gene can be administered in the state of being packaged in ribosome etc., formed as any of various virus vectors such as a retrovirus vector, an adenovirus vector, a vaccinia virus vector, a poxvirus vector, an adenovirus-related vector and a HVJ vector (see Adolph "Viral Genome Method", CRC Press, Florid (1996)), or coated on a bead support such as colloidal gold particles (WO93/17706 etc.). The nucleic acid encoding the antigen-binding molecule according to the present invention may be directly administered to a living organism, or may be directly administered to a living organism by an electroporation method. For example, the antigen-binding molecule according to the present invention can be administered by a method in which mRNA encoding the antigen-binding molecule according to the present invention is subjected to chemical modification for enhancing the stability of mRNA *in vivo,* and the mRNA is directly administered to a human to express the antigen-binding molecule according to the present invention *in vivo* (see EP2101823B, WO2013/120629). However, the mRNA may be administered by any method as long as the antigen-binding molecule is expressed *in vivo,* and the action thereof can be exhibited. Preferably, a sufficient amount of the mRNA is administered through an appropriate parenteral administration (e.g., injection or infusion via an intravenous, intraperitoneal, subcutaneous, intracutaneous, fat tissue, lacteal tissue, inhalational or intramuscular route, or gas inducing particle bombardment method (by electron gun etc.), or a method via a mucosal route for nasal drops etc.). A gene encoding the antigen-binding molecule according to the present invention may be administered to blood cells, bone-marrow-derived cells and the like by liposomal transfection, a particle bombardment method (U.S. Patent No. 4,945,050) or virus infection *ex vivo,* followed by reintroduction of the cells into an animal to administer the gene.

The "treatment" (and its grammatically derived words, for example, "treat" and "treating") used in the present specification means clinical intervention that intends to alter the natural course of an individual to be treated and can be carried out both for prevention and during the course of a clinical pathological condition. The desirable effect of the treatment includes, but is not limited to, the prevention of the development or recurrence of a disease, the alleviation of symptoms, the attenuation of any direct or indirect pathological influence of the disease, the prevention of metastasis, reduction in the rate of progression of the disease, recovery from or alleviation of a disease condition, and ameliorated or improved prognosis. In some embodiments, the pharmaceutical composition of the present invention is used for delaying the onset of a disease or delaying the progression of the disease.

In the present invention, the pharmaceutical composition usually refers to a drug for the treatment or prevention of a disease or for examination or diagnosis. In the present invention, in the case of using a pharmaceutical composition in combination with the administration of an additional component, the pharmaceutical composition can be administered concurrently with, separately from, or continuously with the administration of the additional component. The pharmaceutical composition of the present invention may contain the additional component as a component.

The pharmaceutical composition comprising the antigen-binding molecule according to the present invention can be formulated by use of a method known to those skilled in the art. For example, the pharmaceutical composition can be parenterally used in an injection form of a sterile solution or suspension with water or any of other pharmaceutically acceptable liquids. The pharmaceutical composition can be formulated, for example, by appropriately in combination with a pharmacologically acceptable carrier or medium, specifically, sterile water or physiological saline, a plant oil, an emulsifier, a suspending agent, a surfactant, a stabilizer, a flavoring agent, an excipient, a vehicle, an antiseptic, a binder, etc. and mixing into a unit dosage form required for generally accepted pharmaceutical practice. The amount of the active ingredient in these formulations is set so as to give an appropriate volume in a prescribed range.

A sterile composition for injection can be formulated according to usual pharmaceutical practice using a vehicle such as injectable distilled water. Examples of the injectable aqueous solution include isotonic solutions containing physiological saline, glucose, or other adjuvants (e.g., D-sorbitol, D-mannose, D-mannitol, and sodium chloride). The aqueous solution can be used in combination with an appropriate solubilizer, for example, an alcohol (ethanol, etc.), a polyalcohol (propylene glycol, polyethylene glycol, etc.), or a nonionic surfactant (Polysorbate 80(TM), HCO-50, etc.).

Examples of the oil solution include sesame oil and soybean oil. The oil solution can also be used in combination with benzyl benzoate and/or benzyl alcohol as a solubilizer. The oil solution can be supplemented with a buffer (e.g., a phosphate buffer solution and a sodium acetate buffer solution), a soothing agent (e.g., procaine hydrochloride), a stabilizer (e.g., benzyl alcohol and phenol), and an antioxidant. The prepared injection solution is usually filled into an appropriate ampule.

The pharmaceutical composition comprising the antigen-binding molecule according to the present invention is preferably parenterally administered. For example, a composition having an injection, transnasal, transpulmonary, or percutaneous dosage form is administered. The pharmaceutical composition can be administered systemically or locally by, for example, intravenous injection, intra-arterial injection, intramuscular injection, intraperitoneal injection, intramedullary injection, intra-articular injection, intrasynovial injection, intracranial injection, intraspinal injection, intrathecal injection, intracutaneous injection, subcutaneous injection, intracardial injection, intralesional injection to tumors or the like, a method using a catheter or the like.

The administration method can be appropriately selected according to the age and symptoms of a patient. The dose of the pharmaceutical composition of the present invention can be set to the range of, for example, 0.0001 mg to 1000 mg per kg body weight per dose. Alternatively, the dose of the pharmaceutical composition can be set to a dose of, for example, 0.001 to 100000 mg per patient. However, the present invention is not necessarily limited by these numerical values. Although the dose and the administration method vary depending on the body weight, age, symptoms, etc. of a patient, those skilled in the art can set an appropriate dose and administration method in consideration of these conditions.

In the present invention, the pharmaceutical composition of the present invention may be encapsulated, if necessary, in microcapsules (e.g., hydroxymethylcellulose, gelatin, and poly(methyl methacrylate) microcapsules), and used in a colloidal drug delivery system (liposomes, albumin microspheres, microemulsion, nanoparticles, and nanocapsules, etc.) (see e.g., Remington's Pharmaceutical Science 16th edition, Oslo Ed. (1980)). Methods for preparing agents into sustained-release agents are also known in the art, and such methods may be applied to the bispecific antigen-binding molecule of the present invention (J. Biomed. Mater. Res. (1981) 15, 267-277, Chemtech. (1982) 12, 98-105; U.S. Patent No. 3773719; EP Patent Publication Nos. EP58481 and EP133988; and Biopolymers (1983) 22, 547-556).

The term "chimeric receptor" refers to a recombinant polypeptide that comprises at least an extracellular domain, a transmembrane domain and an intracellular signaling domain and induces specificity and intracellular signal production for target cells, for example, cancer cells, when expressed in immune effector cells.

The extracellular domain of the chimeric receptor means any proteinous molecule which can bind specifically to a predetermined molecule, or a part thereof. In the present invention, the extracellular domain of the chimeric receptor is a polypeptide comprising a sequence of an extracellular domain of an immunoreceptor or a variant thereof. In one aspect of the present invention, the extracellular domain of the chimeric receptor may be a polypeptide consisting of only a sequence of an extracellular domain of an immunoreceptor or a variant thereof, or may comprise a polypeptide or amino acid residues in addition to the sequence of an extracellular domain of an immunoreceptor, or a variant thereof. In another aspect of the present invention, the extracellular domain of the chimeric receptor may comprise a sequence of an extracellular domain of an immunoreceptor or a portion of a variant thereof.

The extracellular domain of an immunoreceptor in the present invention is an extracellular part of an immunoreceptor which can bind to an endogenous immune ligand. For example, in CD137, the extracellular domain of an immunoreceptor refers to SEQ ID NO: 129, SEQ ID NO: 130, SEQ ID NO: 131 and SEQ ID NO: 132.

In the present invention, the extracellular domain variant of an immunoreceptor means a polypeptide in which a part of the extracellular domain of an immunoreceptor is subjected to addition, deletion or substitution. The extracellular domain variant of an immunoreceptor is preferably one having attenuated binding to an endogenous immune ligand, where a binding site of the extracellular domain of the immunoreceptor for the endogenous ligand is altered, for example, on the basis of a binding site of an immunoreceptor for the endogenous ligand, identified by X-ray crystallographic analysis described in literature. Examples of the extracellular domain variant of an immunoreceptor having attenuated binding to an endogenous immune ligand include an extracellular domain variant in which a cysteine-rich domain 3, a cysteine-rich domain 4, and a stalk connected to a transmembrane region (Genbank NM001561.6, Cys 88 to Gln 186), in the extracellular domain of the immunoreceptor of CD 137, are deleted.

In the present invention, the portion of the extracellular domain of an immunoreceptor may be a portion of a polypeptide constituting the extracellular domain of an immunoreceptor, and the number of constituent amino acids, and the like are not particularly limited as long as the antigen-binding molecule can be recognized. Examples of the portion include a polypeptide constituting an epitope recognized by an antibody having agonist activity on an immunoreceptor, or a portion thereof. In the present invention, the extracellular domain of the chimeric receptor may comprise a variant of the epitope, and the portion of a polypeptide constituting an extracellular of an immunoreceptor contained in the epitope also corresponds to a "portion of an extracellular domain of an immunoreceptor".

The term "transmembrane domain" is positioned between the extracellular domain and the intracellular signaling domain and comprises a polypeptide having a function of penetrating a cell membrane.

The term "intracellular signaling domain" means any oligopeptide domain or polypeptide domain known to work to transduce signals that cause activation or inhibition of an intracellular biological process, for example, activation of immunocytes such as T cells or NK cells, and comprises at least one "stimulatory molecule signaling domain" derived from a stimulatory molecule of T cells mentioned later, and may further comprise at least one "costimulatory molecule signaling domain" derived from a costimulatory molecule of T cells mentioned later.

As used in the present disclosure, the "domain" means, for example, one region in a polypeptide that is folded into a particular structure independently of other regions and/or has a particular function. The domain can be, for example, a cytoplasmic moiety of a molecule or a portion thereof. As used in the present disclosure, the "cytoplasmic domain" of a molecule means a full-length cytoplasmic domain or a portion thereof that transduces intracellular signals when activated.

In one embodiment, the chimeric receptor is a molecule comprising domains defined below.

In one embodiment, the chimeric receptor comprises a chimeric fusion protein comprising an extracellular domain, an extracellular hinge domain, a transmembrane domain, and an intracellular signaling domain comprising a stimulatory molecule signaling domain derived from a stimulatory molecule.

In one embodiment, the chimeric receptor comprises a chimeric fusion protein comprising an extracellular domain, an extracellular hinge domain, a transmembrane domain, and an intracellular signaling domain comprising a costimulatory molecule signaling domain derived from a costimulatory molecule and a functional signaling domain derived from a stimulatory molecule.

In one embodiment, the chimeric receptor comprises a chimeric fusion protein comprising an extracellular domain, a transmembrane domain, and an intracellular signaling domain comprising at least two functional signaling domains derived from one or more costimulatory molecules and a functional signaling domain derived from a stimulatory molecule.

In one embodiment, the chimeric receptor comprises a chimeric fusion protein comprising an extracellular domain, a transmembrane domain, and an intracellular signaling domain comprising at least two costimulatory molecule signaling domains derived from one or more costimulatory molecules, a stimulatory molecule signaling domain derived from a stimulatory molecule, and an additional functional domain and/or motif.

One embodiment discloses a chimeric receptor comprising i) an extracellular domain capable of binding to an immunoreceptor recognition site of an antigen-binding molecule, ii) a transmembrane domain, and iii) an intracellular segment comprising one or more intracellular signaling domains selected from a cytoplasmic costimulatory domain and/or a cytoplasmic domain of an interleukin receptor chain and a CD3 zeta intracellular signaling domain comprising an exogenous STAT3 association motif (wherein the intracellular segment comprises an endogenous or exogenous JAK binding motif and STATS association motif). In an embodiment, these domains are optionally fused directly or indirectly in the foregoing order starting from the N terminus. In an embodiment, these domains within the intracellular segment are fused in the inverse order.

In one embodiment, an extracellular hinge domain and a transmembrane domain may be contained between the extracellular domain and the intracellular signaling domain. The term "extracellular hinge domain" means a domain that links the extracellular domain to the transmembrane domain. The extracellular hinge domain is not particularly limited as long as the extracellular hinge domain can link the extracellular domain to the transmembrane domain. The extracellular hinge domain may be derived from a natural protein or may be artificially designed. The extracellular hinge domain can be constituted by, for example, approximately 10 to 300 amino acids, preferably approximately 20 to 100 amino acids. The extracellular hinge domain preferably neither hinders the ability of the extracellular domain to bind to the antigen-binding molecule according to the present disclosure nor hinders signaling mediated by the intracellular signaling domain. The term "transmembrane domain" is not particularly limited as long as the transmembrane domain is positioned between the extracellular domain and the intracellular signaling domain and is a polypeptide having a function of penetrating a cell membrane. The transmembrane domain may be derived from a natural protein or may be artificially designed. The transmembrane domain derived from a natural protein can be obtained from any membrane-associated protein or transmembrane protein.

In one embodiment, the chimeric receptor comprises an additional leader sequence at the amino terminus (N terminus) of the chimeric receptor fusion protein.

In one embodiment, the chimeric receptor further comprises a leader sequence at the N terminus of the extracellular domain. In this context, the leader sequence may be cleaved from the extracellular domain during cell processing and the localization of the chimeric receptor to a cell membrane.

The term "immunoreceptor" means a receptor which is expressed on immunocytes, and involved in activation or inhibition of immunocytes. Examples of the immunocytes include T cells, dendritic cells, B cells, hematopoietic stem cells, macrophages, monocytes, NK cells or hematopoietic cells (neutrophils and basophils). In the present invention, the chimeric receptor has an extracellular domain of an immunoreceptor or a variant thereof as an extracellular domain. In this contrast, the immunoreceptor is preferably a receptor which is involved in activation of immunocytes, and examples thereof include costimulatory molecules belonging to a tumor necrosis factor receptor superfamily (TNFRSF), specifically CD137, CD40, OX40, RANK, and GITR. The immunoreceptor may be a stimulatory molecule or a costimulatory molecule, and specific examples thereof are as described later.

The immunoreceptor recognition site of the antigen-binding molecule in the present invention can recognize an extracellular domain of an immunoreceptor contained in an extracellular binding domain of a chimeric receptor, or a portion thereof. More preferably, the immunoreceptor recognition site of the antigen-binding molecule recognizes a part different from the binding site of the endogenous ligand. The endogenous ligand binding site on the immunoreceptor is identified by, for example, structural-biological analysis described in literature. For example, in CD137, the X-ray crystal structure of a complex with CD137L has been clarified (Chin SM et al (2018) Nat Commun. 9, 4679), and F36 on CRD1, P49, S52, Q59, T61, C62, D63, I64, Q67, K69, V71 and F72 on CRD2, and S100, M101 and C102 on CRD3 have been reported to be involved in interaction with CD137L. Mutants have been prepared by mutation of I64 or V71 among the above-mentioned sites into Arg, and shown to exhibit attenuated binding to CD137L. A mutant obtained by mutation of an amino acid residue involved in interaction with CD137L into another amino acid residue can be used as an extracellular domain variant having attenuated binding to CD137L.

In one aspect, the extracellular domain of a chimeric receptor comprises an extracellular domain of a costimulatory molecule belonging to TNFRSF, or a portion thereof, and the immunoreceptor recognition site of the antigen-binding molecule which is used may be an agonist antibody for the costimulatory molecule belonging to TNFRSF (hereinafter, referred to as a TNFRSF agonist antibody), or an antigen-binding portion thereof can be used. In various specific examples, the antigen-binding molecule used as the pharmaceutical composition of the present invention can activate the chimeric receptor-expressing cell by at least about 20%, 30%, 40%, 50%, 60%, 70%, 80%, 90%, 100%, 125%, 150%, 175%, 200%, 250%, 300%, 350%, 400%, 450%, 500%, 750%, 1000% or more.

In one aspect of the present invention, the extracellular domain of the chimeric receptor comprises a target molecule of the TNFRSF agonist antibody, or a portion thereof. The target molecule of the TNFRSF agonist antibody is not particularly limited as long as it is a factor which activates cells expressing the TNF receptor superfamily (e.g. T cells and NK cells). Examples of the preferred factor include CD137 and CD40. Examples of the more preferred factor include CD137. Examples of the CD137 agonist antibody include Urelumab (CAS Registry Number: 934823-49-1), and various known CD137 agonist antibodies.

In one aspect of the present invention, the extracellular domain of the chimeric receptor comprises an epitope of an antibody having agonist activity on the immunoreceptor as a portion of the immunoreceptor. Examples of the immunoreceptor are as described above, and CD137 is preferable.

In one aspect of the present invention, the extracellular domain of the chimeric receptor comprises a target molecule of the CD137 agonist antibody, or a portion thereof. Examples of the CD137 agonist antibody include the following antibodies represented by SEQ ID NOS described in, for example, WO2015/156268:

[1] an antibody having an amino acid sequence set forth as SEQ ID NO: 66 as a heavy chain variable region and an amino acid sequence set forth as SEQ ID NO: 85 as a light chain variable region;
[2] an antibody having an amino acid sequence set forth as SEQ ID NO: 67 as a heavy chain variable region and an amino acid sequence set forth as SEQ ID NO: 86 as a light chain variable region;
[3] an antibody having an amino acid sequence set forth as SEQ ID NO: 70 as a heavy chain variable region and an amino acid sequence set forth as SEQ ID NO: 89 as a light chain variable region;
[4] an antibody having an amino acid sequence set forth as SEQ ID NO: 76 as a heavy chain variable region and an amino acid sequence set forth as SEQ ID NO: 95 as a light chain variable region;
[5] an antibody having an amino acid sequence set forth as SEQ ID NO: 77 as a heavy chain variable region and an amino acid sequence set forth as SEQ ID NO: 96 as a light chain variable region;
[6] an antibody having an amino acid sequence set forth as SEQ ID NO: 78 as a heavy chain variable region and an amino acid sequence set forth as SEQ ID NO: 97 as a light chain variable region;
[7] the antibody according to any of [1] to [6], which has an amino acid sequence set forth as SEQ ID NO: 99 as a heavy chain constant region and an amino acid sequence set forth as SEQ ID NO: 59 or an amino acid sequence set forth as SEQ ID NO: 60 as a light chain constant region;
[8] an antibody equivalent in activity to the antibody according to any of [1] to [7]; and
[9] an antibody which binds to an epitope identical to an epitope to which the antibody according to any of [1] to [7] binds.

In the antibody according to [8], the term "equivalent in activity" means that the agonist activity on CD137 is 70% or more, preferably 80% or more, more preferably 90% or more of the binding activity of the antibody according to any of [1] to [7].

Preferred examples of the antibody which binds to an epitope identical to an epitope to which the antibody according to any of [1] to [7] binds include an antibody which recognizes a region having the sequence of SPCPPNSFSSAGGQRTCDICRQCKGVFRTRKECSSTSNAECDCTPGFHCLGAGCSMCE QDCKQGQELTKKGC in the CD137 protein. Further, examples thereof include an antibody which recognizes a region having the sequence of DCTPGFHCLGAGCSMCEQDCKQGQELTKKGC in the CD137 protein.

In one embodiment, examples of the extracellular hinge domain include extracellular hinge domains of CD8 alpha, CD8 beta, CD28, CD4, NKp30, NKp44, and NKp46. Alternatively, a hinge region of an immunoglobulin (e.g., IgG4) may be used.

In one embodiment, examples of the protein from which the transmembrane domain is derived can include T cell receptor alpha and beta chains, CD3 zeta, CD28, CD3 epsilon, CD45, CD4, CD5, CD8 alpha, CD8 beta, CD9, CD16, CD22, CD33, CD37, CD64, CD80, CD86, CD134, CD137, ICOS, CD154, GITR, NKp30, NKp44, and NKp46. In one embodiment, the protein from which the transmembrane domain is derived is CD8 alpha or CD28.

The term "signaling domain" refers to a functional moiety of a protein that acts by conveying intracellular information in order to regulate cell activity via a defined signaling pathway through the production of a second messenger or through the functionalization of an effector in response to such a messenger.

As used in the present disclosure, the "intracellular signaling domain" refers to an intracellular moiety of a molecule. The intracellular signaling domain can generate signals that promote an immune effector function of cells containing a chimeric receptor, for example, chimeric receptor-expressing T cells. Examples of the immune effector function of chimeric receptor-expressing T cells include cytolytic activity and helper activity, for example, cytokine secretion. In an embodiment, the intracellular signaling domain is a moiety of a protein that transduces effector function signals and allows cells to carry out a specified function. Although the whole intracellular signaling domain may be adopted, it is not necessarily required to use the whole chain in many cases. A truncated moiety that transduces effector function signals can be used instead of an intact chain as long as a truncated moiety of the intracellular signaling domain is used. Thus, the term "intracellular signaling domain" is meant to include every truncated moiety of the intracellular signaling domain sufficient for transducing effector function signals.

In an embodiment, the intracellular signaling domain may comprise a primary intracellular signaling domain. Typical examples of the primary intracellular signaling domain include those derived from molecules involved in primary stimulation or antigen dependent stimulation. In an embodiment, the intracellular signaling domain may comprise a costimulatory intracellular domain. Typical examples of the costimulatory intracellular signaling domain include those derived from molecules involved in costimulatory signals or antigen independent stimulation. In the case of, for example, chimeric receptor-expressing cells, the primary intracellular signaling domain may comprise an intracytoplasmic sequence of a T cell receptor, or the costimulatory intracellular signaling domain may comprise an intracytoplasmic sequence of a co-receptor or a costimulatory molecule.

As used in the present disclosure, the term "CD3 zeta" means cluster of differentiation 3 (CD3) T cell coreceptor of every mammalian species, preferably a human. In mammals, CD3 comprises a CD3 zeta chain, a CD3 delta chain and two CD3 epsilon chains. The CD3 zeta chain (e.g., NCBI RefSeq: NP_932170.1) comprises an intracellular signaling domain that can be used for engineering the chimeric receptor. In the particular chimeric receptor of the present disclosure, the primary signaling sequence of CD3 zeta is the full-length cytoplasmic region sequence of GenBank NM000734.3 (nucleotides 299 to 634) or a portion thereof, or equivalent residues from a non-human species, for example, a mouse, a rodent, a monkey, an anthropoid and the like.

In one embodiment, the intracellular signaling domain may comprise a cytoplasmic domain of an interleukin receptor chain. In one embodiment, the intracellular signaling domain used may be CD28, 4-1BB, ICOS, or CD3 zeta-CD28-4-1BB or CD3 zeta-CD28-OX40 in which a plurality of signaling domains are connected. In one embodiment, the protein from which the transmembrane domain is derived is CD8 alpha or CD28, and the intracellular signal transduction domain may be CD28, 4-1BB, ICOS, or CD3 zeta-CD28-4-1BB or CD3 zeta-CD28-OX40 in which a plurality of signal transduction domains are connected.

One embodiment discloses a chimeric receptor comprising i) an extracellular domain capable of binding to an immunoreceptor recognition site of an antigen-binding molecule, ii) a transmembrane domain, and iii) an intracellular segment comprising one or more intracellular signaling domains selected from a cytoplasmic costimulatory domain and/or a cytoplasmic domain of an interleukin receptor chain and a CD3 zeta intracellular signaling domain comprising an exogenous STAT3 association motif (wherein the intracellular segment comprises an endogenous or exogenous JAK binding motif and STAT5 association motif). In an embodiment, these domains are optionally fused directly or indirectly in the foregoing order starting from the N terminus. In an embodiment, these domains within the intracellular segment are fused in the inverse order.

The term "stimulation" refers to primary response that is induced by the binding of a stimulatory molecule (e.g.,

a TCR/CD3 complex or a chimeric receptor) to its ligand of the same origin (or an immunoreceptor recognition site of an antigen-binding molecule for chimeric receptor), and thereby mediates a signal transduction event, for example, but not limited to, signal transduction mediated by a TCR/CD3 complex, or signal transduction mediated by an appropriate NK receptor or signaling domain of chimeric receptor. The stimulation may mediate changed expression of a certain molecule.

The term "stimulatory molecule" refers to a molecule that is expressed by immunocytes, for example, T cells, NK cells or B cells, which provide an intracytoplasmic signaling sequence regulating the activation of the immunocytes in the form of stimulation, in at least some aspects of an immunocyte signaling pathway. In one aspect, the signal is a primary signal that is triggered, for example, by the binding between a TCR/CD3 complex and an MHC molecule presenting a peptide, and thereby mediates T cell response including, but not limited to, growth, activation, differentiation, and the like. The primary intracytoplasmic signaling sequence (also referred to as a "primary signaling domain") which acts in the form of stimulation may contain a signaling motif, which is known as an immunoreceptor tyrosine-based activation motif or ITAM. In the present disclosure, examples of ITAM containing an intracytoplasmic signaling sequence having particular application include, but are not limited to, those derived from CD3 zeta, common FcR gamma (FCER1G), Fc gamma RIIa, FcR beta (Fc epsilon R1b), CD3 gamma, CD3 delta, CD3 epsilon, CD22, CD79a, CD79b, CD278 ("ICOS"), Fc epsilon RI, CD66d, CD32, DAP10, DAP12, CLEC2, CLEC7A (Dectin 1), CLEC9A, EZRIN, RADIXIN and MOESIN. In particular chimeric receptors of the present disclosure, the intracellular signaling domain in any one or more chimeric receptors comprises an intracellular signaling sequence, for example, a primary signaling sequence of CD3 zeta. The extracellular domain of the chimeric receptor according to the present invention may be the extracellular domain of the stimulatory molecule.

The term "costimulatory molecule" refers to a cognate binding partner on a T cell that specifically binds to a costimulatory ligand and thereby mediates the costimulatory response, for example, but not limited to, growth, of the T cell (secondary signal). The costimulatory molecule is a cell surface molecule other than an antigen receptor or its ligand that is responsible for an efficient immune response. The term "costimulatory intracellular signaling domain" refers to an intracellular moiety of the costimulatory molecule. The intracellular signaling domain may comprise the whole intracellular moiety, or the whole natural intracellular signaling domain of the molecule from which the intracellular moiety is obtained, or a functional fragment or derivative thereof. Examples of the costimulatory molecule include, but are not limited to, ligands that specifically bind to MHC class I molecule, TNF receptor protein, immunoglobulin-like protein, cytokine receptor, integrin, signaling lymphocytic activation molecule (SLAM protein), activating NK cell receptor, BTLA, Toll ligand receptor, OX40 (CD134), CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD5, CD8 alpha, CD8 beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, CD154 and CD83. The extracellular domain of the chimeric receptor according to the present invention may be the extracellular domain of the costimulatory molecule.

In one embodiment, the costimulatory molecule is selected from, for example, 4-1BB (i.e., CD137), CD27, CD28 and/or OX40, and enhances T cell receptor stimulation.

The term "4-1BB"or "CD137" refers to a member of the TNFR superfamily having an amino acid sequence provided as GenBank accession No. AAA62478.2, or equivalent residues from a non-human species, for example, a mouse, a rodent, a monkey, an anthropoid and the like. The term "4-1BB costimulatory domain" is defined as amino acid residues 214 to 255 of GenBank accession No. AAA62478.2, or equivalent residues from a non-human species, for example, a mouse, a rodent, a monkey, an anthropoid and the like. In one aspect, the "4-1BB costimulatory domain" is the full-length sequence from nucleotides 886 to 1026 of GenBank NM001561.5 or a portion thereof, or equivalent residues from a non-human species, for example, a mouse, a rodent, a monkey, an anthropoid and the like.

The term "nucleic acid" or "polynucleotide" refers to deoxyribonucleic acid (DNA) or ribonucleic acid (RNA), and a polymer thereof in either single- or double-stranded form. The term "nucleic acid" includes a gene, cDNA, or mRNA. In one embodiment, the nucleic acid molecule is a synthetic (e.g., chemically synthesized) nucleic acid molecule or a recombinant nucleic acid molecule. This term encompasses a nucleic acid containing analogs or derivatives of natural nucleotides that have binding characteristics similar to those of a reference nucleic acid and are metabolized in a manner similar to that of naturally occurring nucleotides, unless particularly limited. A particular nucleic acid sequence also virtually includes conservatively altered mutants thereof (e.g., by degenerate codon substitution), alleles, orthologs, SNPs, and complementary sequences as well as explicitly presented sequences, unless otherwise specified. Specifically, the degenerate codon substitution may be achieved by producing a sequence in which the third position of one or more selected (or all) codons is substituted with mixed base and/or deoxyinosine residues [Batzer et al., Nucleic Acid Res. 19: 5081 (1991); Ohtsuka et al., J. Biol. Chem. 260: 2605-2608 (1985); and Rossolini et al., Mol. Cell. Probes 8: 91-98 (1994)].

As used in the present disclosure, the term "nucleic acid sequence" refers to a sequence of nucleoside or nucleotide monomers consisting of natural bases, sugars and intersugar (backbone) bonds. This term also includes a modified or substituted sequence comprising a non-natural monomer or a portion thereof. The nucleic acid sequence of the present application can be a deoxyribonucleic acid sequence (DNA) or a ribonucleic acid sequence (RNA) and contains natural bases including adenine, guanine, cytosine, thymidine and uracil. These sequences may also contain modified bases. Examples of such a modified base include aza and deaza adenine, guanine, cytosine, thymidine and uracil; and xanthine and hypoxanthine.

As used in the present disclosure, the term "isolated nucleic acid" refers to a nucleic acid substantially free of cellular materials or culture media when produced by a recombinant DNA technique, or free of chemical precursors or other chemicals when chemically synthesized. The isolated nucleic acid is also substantially free of sequences naturally flanking the nucleic acid (i.e., sequences positioned at the 5' and 3' ends of the nucleic acid) from which the nucleic acid is derived. The term "nucleic acid" includes DNA and RNA, can be either double-stranded or single-stranded, and corresponds to a sense or antisense strand. The term "nucleic acid" further includes a complementary nucleic acid sequence, for example, cDNA.

The term "coding (encoding)" refers to the inherent characteristics of a specific sequence of nucleotides in a polynucleotide, such as a gene, a cDNA, or an mRNA, useful as a template for the synthesis of other polymers and high molecules in biological processes having either a defined sequence of nucleotides (e.g., rRNA, tRNA and mRNA) or a defined sequence of amino acids, and biological characteristics resulting therefrom. Thus, a gene, cDNA, or RNA encodes a protein when the transcription and translation of mRNA corresponding to the gene produce the protein in cells or other biological systems. Both a coding strand, the nucleotide sequence of which is identical to a mRNA sequence and is usually shown in a sequence listing, and a non-coding strand which is used as a template for the transcription of a gene or cDNA, can be regarded as encoding a protein or other products of the gene or the cDNA.

The "nucleotide sequence encoding an amino acid sequence" encompasses all of nucleotide sequences that are degenerate forms of each other and nucleotide sequences encoding the same amino acid sequence, unless otherwise specified. The phrase "nucleotide sequence encoding a protein or RNA" may encompass an intron, in some cases, to the extent that the nucleotide sequence encoding the protein is capable of containing the intron. The nucleic acid molecule is operably linkable to at least one regulatory element for the expression of the chimeric receptor.

The terms "peptide", "polypeptide", and "protein" are interchangeably used and refer to a compound constituted by amino acid residues covalently linked through peptide bonds. The protein or the peptide must contain at least two amino acids, with no limitation on the maximum number of amino acids capable of constituting the sequence of the protein or the peptide. The polypeptide includes every peptide or protein comprising two or more amino acids linked to each other through peptide bonds. As used in the present disclosure, this term refers to both a short chain also generally called peptide, oligopeptide and oligomer in the art, and a longer chain generally called protein in the art, of which there are many types. Examples of the "polypeptide" particularly include biologically active fragments, substantially homologous polypeptides, oligopeptides, homodimers, heterodimers, mutants of polypeptides, altered polypeptides, derivatives, analogs, and fusion protein. Examples of the peptide include natural peptides, recombinant peptides, and combinations thereof.

The polypeptide according to the present disclosure usually refers to a peptide having a length on the order of 10 amino acids or longer, and a protein. When a linkage of amino acids connected through a peptide bond from the N terminus to the C terminus is regarded as a single peptide chain, the polypeptide of the present disclosure may be a complex protein formed from a plurality of single peptide chains through interaction such as a S-S bond, hydrophobic interaction, or an ion bond.

The term "isolated polypeptide", also called "isolated protein", refers to a polypeptide substantially free of cellular materials or culture media when produced by a recombinant DNA technique, or free of chemical precursors or other chemicals when chemically synthesized.

The term "amino acid" includes all of natural amino acids and modified amino acids.

As used in the present disclosure, the term "conservative amino acid variation" is the substitution of an amino acid residue by another amino acid residue without impairing the desired characteristics of the protein.

The amino acid sequence specifically shown in the present application may include conservative sequence alteration. In this context, the "conservative sequence variation" refers to amino acid mutation that does not significantly influence or change the binding characteristics of an antibody or an antibody fragment containing the amino acid sequence. Examples of such conservative alteration include amino acid substitution, addition and deletion. The alteration can be introduced into the antibody or the antibody fragment of the present disclosure by standard techniques known in the art, such as site-directed mutagenesis and PCR-mediated mutagenesis. The conservative amino acid substitution is substitution that replaces an amino acid residue with an amino acid residue having a similar side chain. Families of amino acid residues having similar side chains are defined in the art. These families include amino acids having a basic side chain (e.g., lysine, arginine, and histidine), amino acids having an acidic side chain (e.g., aspartic acid and glutamic acid), amino acids having an uncharged polar side chain (e.g., glycine, asparagine, glutamine, serine, threonine, tyrosine, cysteine, and tryptophan), amino acids having a nonpolar side chain (e.g., alanine, valine, leucine, isoleucine, proline, phenylalanine, and methionine), amino acids having a beta-branched side chain (e.g., threonine, valine, and isoleucine) and amino acids having an aromatic side chain (e.g., tyrosine, phenylalanine, tryptophan, and histidine). Thus, one or more amino acid residues within the chimeric receptor of the present disclosure may be replaced with other amino acid residues of the same side chain family, and the chimeric receptor thus changed can be tested by use of functional assay described in the present disclosure.

The term "expression" refers to the transcription and/or translation of a particular nucleotide sequence driven by a promoter.

The "host cell", the "host cell line", and the "host cell cultures" in the present disclosure are interchangeably used and refer to cells harboring a foreign nucleic acid (including the progeny of such cells). The host cell includes a "transformant" and a "transformed cell", which include a primary transformed cell and progeny derived from the cell, regardless of the number of passages. The progeny may not be completely identical in terms of nucleic acid contents to a parent cell, and may have a mutation. Mutant progeny having the same function or biological activity as that used for screening or selecting the original transformed cells for are also included in the present disclosure.

The "vector" in the present disclosure refers to a nucleic acid molecule that can propagate another nucleic acid to which the vector is linked. This term includes a vector as a self-replicating nucleic acid structure, and a vector integrated into the genomes of host cells harboring the vector. A certain vector can bring about the expression of a nucleic acid operatively link to the vector itself. Such a vector is also referred to as an "expression vector" in the present disclosure.

The transfection means the uptake of an expression vector by a host cell for which whether or not to actually express any coding sequence is not clear. Many transfection methods are known to the ordinarily skilled artisan, for example, $CaPO_4$ precipitation and electroporation. In general, successful transfection is recognized when a sign of the operation of the vector appears within the host cell.

The term "promoter" refers to a DNA sequence that is recognized by the synthetic mechanism of cells, or an introduced synthetic mechanism, necessary for starting the specific transcription of a polynucleotide sequence.

The term "lentivirus" refers to a genus of the family *Retroviridae.* The lentivirus is unique, among the retroviruses, in being able to infect non-dividing cells. The lentivirus can deliver a significant amount of genetic information into DNA of host cells and is therefore one of the most efficient methods of a gene delivery vector. HIV, SIV, and FIV are all examples of the lentivirus.

The term "lentivirus vector" refers to a vector derived from at least a portion of the lentivirus genome. Examples thereof particularly include self-inactivating lentivirus vectors as disclosed in Milone et al., Mol. Ther. 17 (8): 1453-1464 (2009). Other examples of the lentivirus vector that may be used in clinics include, but are not limited to, LENTIVECTOR(R) gene delivery technology manufactured by Oxford BioMedica plc, LENTIMAX(TM) vector system manufactured by Lentigen Technology, Inc., and the like. Nonclinical types of lentivirus vectors are also available, and these vectors can be appropriately selected and prepared by those skilled in the art.

In the present disclosure, the term "pharmaceutically acceptable" refers to a molecular entity and other ingredients of such a composition that are physiologically tolerable and do not typically produce undesirable reactions when the composition is administered to a mammal (e.g., a human). Preferably, in the present disclosure, the "pharmaceutically acceptable" means approved by a regulatory agency of the Federal or a state government or listed in the U.S. Pharmacopeia or other generally recognized pharmacopeia for use in mammals, more specifically, humans.

In one aspect of the present disclosure, the chimeric receptor of the present disclosure comprises two signaling domains described herein, i.e., CD3 zeta and 4-1BB/CD137, or CD3 zeta as well as one or more signaling domains. In a particular aspect, several signaling domains are fused to each other for additive or synergistic effects. Non-limiting examples of the useful additional signaling domain include a portion or the whole of one or more of TCR zeta chain, CD27, CD28, OX40/CD134, 4-1BB/CD137, Fc epsilon RIy, ICOS/CD278, ILRB/CD122, IL-2RG/CD132, DAP-10 and CD40.

The present disclosure discloses a chimeric receptor comprising i) an extracellular domain capable of binding to a predetermined antigen via an antigen-binding molecule, ii) a transmembrane domain, and iii) an intracellular segment comprising one or more intracellular signaling domains selected from a cytoplasmic costimulatory domain and/or a cytoplasmic domain of an interleukin receptor chain and a CD3 zeta intracellular signaling domain comprising an exogenous STAT3 association motif (wherein the intracellular segment comprises an endogenous or exogenous JAK binding motif and STATS association motif). In an embodiment, these domains are optionally fused directly or indirectly in the foregoing order starting from the N terminus. In an embodiment, these domains within the intracellular segment are fused in the inverse order.

The present disclosure also includes a chimeric receptor comprising i) an extracellular domain capable of binding via the antigen-binding molecule, ii) a transmembrane domain, and iii) an intracellular segment comprising one or more intracellular signal transduction domains including a cytoplasmic domain of an interleukin (IL) receptor chain and optionally at least one supplementary cytoplasmic domain. In an embodiment, these domains are optionally fused directly or indirectly in the foregoing order starting from the N terminus. In one embodiment, these domains within the intracellular segment are fused in the inverse order.

In some embodiments, the IL receptor chain is proximal to the transmembrane domain and/or is near or forms the N terminus of the intracellular segment of the chimeric receptor. In other embodiments, the IL receptor chain is near or forms the C terminus of the intracellular segment of the chimeric receptor. In some embodiments, the IL receptor chain is upstream or N-terminal from a CD3 zeta intracellular signaling domain comprising an exogenous STAT3 association motif YXXQ.

In an embodiment where the intracellular segment comprises only a signaling domain of the IL receptor chain, cells expressing the chimeric receptor can be activated by a predetermined antigen presented in an MHC complex via endogenous TCR and/or by a CD80/86 molecule via endogenous CD28, for example by B cells.

The present disclosure also provides a cell expressing a chimeric receptor. Such a cell can have, for example, high cytotoxic activity against a cell having, on its surface, a predetermined/preselected antigen to which the chimeric receptor binds via an antigen-binding molecule, as compared with a parent cell that does not express the chimeric receptor. For example, as shown in Examples, cells expressing the chimeric receptor of the present disclosure provide an antigen- binding molecule-dependent and a cancer antigen- or tumor antigen-dependent antitumor effect when given an antigen-binding molecule. Accordingly, an antitumor effect in humans is also expected.

In cells expressing the chimeric receptor of the present disclosure, the chimeric receptor of the present disclosure may be expressed, and another transduction may be applied.

The intracellular segment of the chimeric receptor according to the present disclosure is a proteinous molecule that can comprise one or more intracellular signaling domains and is capable of transducing signals into cells when the extracellular domain present in the same molecule binds to (interacts with) its cognate antigen/ligand.

In an aspect, the intracellular segment of the chimeric receptor comprises a CD3 zeta intracellular signaling domain comprising an exogenous STAT3 association motif. In addition, the intracellular segment of the chimeric receptor comprises one or more intracellular signaling domains selected from a cytoplasmic domain of an IL receptor chain and/or a cytoplasmic costimulatory domain. The intracellular segment comprises an endogenous or exogenous JAK binding motif and a STATS association motif.

A primary cytoplasmic signaling sequence regulates the primary activation of a TCR complex. For example, the CD3 zeta intracellular signaling domain provides a primary cytoplasmic signal. The primary cytoplasmic signaling sequence may comprise a signaling motif known as an immunoreceptor tyrosine-based activation motif (ITAM) [Nature, vol. 338, pp. 383-384 (1989)]. On the other hand, a primary cytoplasmic signaling sequence that acts in an inhibitory manner may comprise a signaling motif known as an immunoreceptor tyrosine-based inhibition motif (ITIM) [J Immunol., vol. 162, No. 2, pp. 897-902 (1999)]. In the present disclosure, an intracellular signaling domain having ITAM and/or ITIM can be used.

In the present disclosure, the CD3 zeta intracellular domain comprises an immunoreceptor tyrosine-based activation motif (ITAM). Examples of the intracellular signaling domain having ITAM that can be used instead of CD3 zeta or to replace CD3 zeta include intracellular signaling domains having ITAM derived from FcR gamma, FcR beta, CD3 gamma, CD3 delta, CD3 epsilon, CD5, CD22, CD79a, CD79b, and CD66d. Specifically, examples of the intracellular domain comprising one or more ITAM motifs include peptides having sequences from amino acids 52 to 164 of CD3 zeta (NCBI RefSeq: NP_932170.1), from amino acids 45 to 86 of Fc epsilon RI gamma (NCBI RefSeq: NP_004097.1), from amino acids 201 to 244 of Fc epsilon RI beta (NCBI RefSeq: NP_000130.1), from amino acids 139 to 182 of CD3 gamma (NCBI RefSeq: NP_000064.1), from amino acids 128 to 171 of CD3 delta (NCBI RefSeq: NP_000723.1), from amino acids 153 to 207 of CD3 epsilon (NCBI RefSeq: NP_000724.1), from amino acids 402 to 495 of CD5 (NCBI RefSeq: NP_055022.2), from amino acids 707 to 847 of CD22 (NCBI RefSeq: NP_001762.2), from amino acids 166 to 226 of CD79a (NCBI RefSeq: NP_001774.1), from amino acids 182 to 229 of CD79b (NCBI RefSeq: NP_000617.1), and from amino acids 177 to 252 of CD66d (NCBI RefSeq: NP_001806.2), and mutants having the same functions as those of these peptides. The amino acids based on amino acid sequence information of NCBI RefSeq ID or GenBank described in the present disclosure are numbered on the basis of the full length of a precursor (comprising a signal peptide sequence, etc.) of each protein.

In an embodiment, the amino acid residue represented by "X" in the STAT3 association motif YXXQ can be any natural amino acid including any modified natural amino acid that retains STAT3 binding. In one embodiment, the amino acid X is independently selected from leucine, arginine, histidine, phenylalanine, lysine, proline, methionine, valine, glutamine, threonine, and aspartic acid. The amino acid X is, for example, arginine. The amino acid X is, for example, histidine.

In an embodiment, the two amino acid residues flanking the tyrosine residue in the STAT3 association motif YXXQ are arginine-histidine. In yet another embodiment, the exogenous STAT3 association motif is YRHQ.

The exogenous STAT3 association motif YXXQ may be introduced in any moiety of the intracellular domain of CD3 zeta. In an embodiment, the YXXQ association motif is inserted near a C-terminal region. Without wishing to be bound by a particular theory, many endogenous YXXQ motifs are probably located near or within 100 aa from the C terminus. Also, the YXXQ motif located near the C-terminal region has been shown to be more functional at a more proximal site in GP130 and LIFR studies (Schmitz J et al., J Immunol. 2000; 164: 848-54; and Tomida M et al., Blood. 1999; 93: 1934-41).

In an embodiment, the exogenous STAT3 association motif YXXQ is introduced in any moiety of the intracellular domain of CD3 zeta located within 200 amino acid residues from the C terminus of the chimeric receptor. For example, the STAT3 association motif is introduced within 200 amino acid residues, within 150 amino acid residues, within 100 amino acid residues, within 90 amino acid residues, within 80 amino acid residues, within 70 amino acid residues, within 60 amino acid residues, within 50 amino acid residues, within 40 amino acid residues, within 30 amino acid residues, within 20 amino acid residues or within 10 amino acid residues from the C terminus of the chimeric receptor. In one embodiment, the exogenous STAT3 association motif is introduced at a location other than ITAM.

In an embodiment, the CD3 zeta intracellular domain comprising an exogenous STAT3 association motif comprises at least one ITAM motif. In one embodiment, the CD3 zeta intracellular domain comprising an exogenous STAT3 association motif comprises two ITAM motifs. In a further embodiment, the CD3 zeta intracellular domain comprising an exogenous STAT3 association motif comprises three ITAM motifs.

Those skilled in the art will appreciate that several methods may be used to introduce a STAT3 association motif into the intracellular signaling domain of CD3 zeta. For example, the exogenous STAT3 association motif can be introduced by substituting amino acid residues Leu-His-Met at amino acid residues 104 to 106 of the intracellular signaling domain of CD3 zeta by tyrosine at residue 104 and any other two amino acid residues at positions 105 and 106 flanking the tyrosine residue. The amino acid residues 104-105-106 of the intracellular signaling domain of CD3 zeta correspond to amino acid residues 156-157-158 of the full-length CD3 zeta (e.g., NCBI RefSeq: NP_932170.1).

As described, the chimeric receptor according to an embodiment comprises an intracellular segment comprising one or more intracellular signaling domains selected from a cytoplasmic domain of an IL receptor chain and a cytoplasmic costimulatory domain.

In the present disclosure, the cytoplasmic domain of an IL receptor chain can be selected from any chain of the IL receptor. For example, a cytoplasmic domain comprising amino acids 266 to 551 of an IL-2 receptor beta chain (NCBI REFSEQ: NP_000869.1) (amino acids 256 to 538 of an IL-21 receptor alpha chain (NCBI REFSEQ: NP_068570.1)), amino acids 284 to 369 of a common IL-2 receptor gamma chain (NCBI REFSEQ: NP_000197.1), amino acids 265 to 459 of IL-7R alpha (NCBI REFSEQ: NP_002176.2), amino acids 292 to 521 of IL-9R alpha (NCBI REFSEQ: NP_002177.2) or amino acids 257 to 825 of IL-4R alpha (NCBI REFSEQ: NP_000409.1) may be used. The whole region of the cytoplasmic domain of the IL receptor chain may be used.

Alternatively, a truncated fragment of the cytoplasmic domain of the IL receptor (ILR) chain may also be used. The truncated fragment comprises, for example, up to 250 amino acids of the ILR cytoplasmic domain, or is 50 to 200 amino acids or 80 to 150 amino acids of the ILR cytoplasmic domain.

In an embodiment, the cytoplasmic domain of the IL receptor chain, optionally the truncated fragment of the cytoplasmic domain of the IL receptor chain, comprises at least a STAT association motif, optionally a STAT5 association motif, and a JAK binding motif (also known as a box-1 motif). In an embodiment, the cytoplasmic domain of the IL receptor chain or the truncated fragment thereof comprises a STAT5 association motif and a JAK binding motif.

In an embodiment, the cytoplasmic domain and/or the truncated fragment of the IL receptor chain includes mutants having the same function, for example, mutants that induce STAT signaling, optionally STAT5 signaling and/or JAK signaling.

In one aspect of the present disclosure, a cytoplasmic domain of an IL-2 receptor (IL-2R) beta chain may be used. Examples of the cytoplasmic domain of an IL-2R beta chain that may be used in the present disclosure include amino acids 266 to 551 of an IL-2R beta chain (NCBI RefSeq: NP_000869.1). One embodiment includes a peptide having any of sequences from amino acid positions 266 to 337 and from amino acid positions 530 to 551. In one aspect of the present disclosure, a truncated fragment of the cytoplasmic domain of the IL-2R beta chain may be used. This truncated fragment may comprise i) a JAK binding motif (e.g., amino acids 278 to 286 of NCBI RefSeq: NP_000869.1), also called BOX-1 motif, which permits association with tyrosine kinase JAK1, and ii) a STAT association motif, optionally a STAT5 or STAT3 association motif. Other moieties of the IL receptor chain can be changed, for example, by conservative amino acid variation.

In an embodiment, the intracellular segment may comprise an exogenous JAK binding motif, or a signaling molecule comprising a JAK binding motif. The JAK binding motif is derived from, for example, IL2R gamma (IL2RG), erythropoietin receptor (EpoR), thrombopoietin receptor (TpoR), granulocyte macrophage colony stimulating factor receptor (GM-CSFR), and growth hormone receptor (GHR).

The IL-2R beta chain comprises three functional STAT5 binding motifs, YFFF, YCTF and YLSL, for use in STAT5 association. Mutations of these tyrosine residues can abolish the IL-2 reactivity of the IL-2R beta chain (Friedmann et al., 1996). The erythropoietin receptor (EpoR) comprises two tyrosine residues that mediate STAT5 activation, i.e., Y343 and Y401 both of which have a YXXL motif as described above (Klingmuller et al., 1996). Thus, YXXL can be a preferred motif for STAT5 recruitment. Other amino acid residues are also functional, for example, as shown with the IL-2R beta chain STAT5 binding motif. In one embodiment, the STAT5 association motif is an IL-2R beta chain STAT5 association motif and comprises tyrosine residue-510 (tyrosine residue 510 is amino acid 536 of NCBI RefSeq: NP_000869.1).

In an embodiment, the STAT5 association motif can be derived from IL2R gamma, EpoR, TpoR, GM-CSFR and GHR.

In an embodiment, the STAT5 association motif of the IL-2R beta chain comprises amino acid residues YXXL. In an embodiment, the amino acid residue represented by "X" in the STAT5 association motif can be any natural amino acid including any modified natural amino acid that retains STAT5 binding.

Likewise, the intracellular segment comprises one or more JAK binding motifs that may be located or introduced in any of the intracellular signaling domains.

In one aspect of the present disclosure, a cytoplasmic domain of an IL-21 receptor (IL-21R) alpha chain may be used. Examples of the cytoplasmic domain of the IL-21R alpha chain used in the present disclosure include intracellular signaling domains comprising amino acid positions 256 to 538 (NCBI RefSeq: NP_068570.1) of the IL-21R alpha chain. In one aspect of the present disclosure, a truncated fragment of the cytoplasmic domain of the IL-21R alpha chain may be used. The truncated fragment comprises a box-1 motif (amino acids 266 to 274 of NCBI RefSeq: NP_068570.1) necessary for association with tyrosine kinase JAK1, and comprises a STAT association motif. In an embodiment, the STAT association motif comprises tyrosine residue-500 (amino acid 519 of NCBI RefSeq: NP_000869.1) and three residues flanking at the C-terminal side of tyrosine residue 500, i.e., YLRQ, necessary for STAT1/3 association.

Other examples of the intracellular signaling domain include cytoplasmic regions derived from a TCR complex and/or a costimulatory molecule, and any mutant having the same functions as those of these sequences. Other examples thereof include cytoplasmic signaling domains listed in Table 2 of Sadelain et al., 2009, which is incorporated herein by reference.

The activation of natural T cells is transduced by two different types of intracellular signaling domains, i.e., a domain for inducing antigen-dependent primary activation via a TCR complex (e.g., a primary cytoplasmic signal provided by, for example, CD3 zeta) and a domain that acts in an antigen-independent manner to provide a secondary or costimulatory signal (secondary cytoplasmic signal).

Examples of the intracellular domain comprising the secondary or costimulatory cytoplasmic signaling domain that may be used in the present disclosure include sequences derived from CD2, CD4, CD5, CD8 alpha, CD8 beta, CD28, CD134, CD137 (4-1BB), ICOS, and CD154, for example, truncated fragments thereof comprising a signaling motif. Specific examples thereof include peptides having any of sequences from amino acids 236 to 351 of CD2 (NCBI RefSeq: NP_001758.2), from amino acids 421 to 458 of CD4 (NCBI RefSeq: NP_000607.1), from amino acids 402 to 495 of CD5 (NCBI RefSeq: NP_055022.2), from amino acids 207 to 235 of CD8 alpha (NCBI RefSeq: NP_001759.3), from amino acids 196 to 210 of CD8 beta (GenBank: AAA35664.1), from amino acids 180 to 220 of CD28 (NCBI RefSeq: NP_006130.1), from amino acids 214 to 255 of CD137 (4-1BB, NCBI RefSeq: NP_001552.2), from amino acids 241 to 277 of CD134 (OX40, NCBI RefSeq: NP_003318.1), and from amino acids 166 to 199 of ICOS (NCBI RefSeq: NP_036224.1), and mutants having the same functions as those of these peptides.

In one aspect, the disclosure preferably includes a chimeric receptor comprising an intracellular segment having one or more, for example 2 or 3 intracellular signaling domains in addition to the intracellular signaling domain of CD3 zeta comprising an exogenous STAT3 association motif.

The present disclosure also includes a chimeric receptor comprising an intracellular segment having two or more same intracellular signaling domains linked in series. In one aspect, the present disclosure provides a chimeric receptor having a cytoplasmic domain of an IL receptor located on the N-terminal side of an intracellular signaling domain of CD3 zeta, i.e., a chimeric receptor comprising a cytoplasmic domain of an IL receptor and an intracellular signaling domain of CD3 zeta linked in this order from the N-terminal side. The present disclosure also includes a chimeric receptor obtained by further adding an intracellular domain of CD28 (e.g., a cytoplasmic costimulatory domain of CD28) to the chimeric receptor mentioned above, i.e., a chimeric receptor comprising an intracellular signaling domain of CD28, a cytoplasmic domain of an IL receptor, and an intracellular signaling domain of CD3 zeta comprising an exogenous STAT3 motif, linked in this order from the N-terminal side.

In an embodiment, the chimeric receptor comprises an intracellular segment comprising a CD3 zeta intracellular signaling domain comprising an exogenous STAT3 association motif and an intracellular signaling domain selected from a cytoplasmic domain of an interleukin receptor chain and a cytoplasmic costimulatory domain, wherein at least one intracellular signaling domain comprises an endogenous or exogenous JAK binding motif and a STAT5 association motif.

In one embodiment, the chimeric receptor comprises a CD3 zeta intracellular signaling domain having an exogenous STAT3 association motif, a cytoplasmic domain of an IL receptor chain fragment comprising an endogenous or exogenous JAK binding motif and a STAT5 association motif, and a cytoplasmic costimulatory domain of CD28.

In the chimeric receptor of the present disclosure, an oligopeptide linker or a polypeptide linker can be inserted between the domains of the intracellular segment so as to link the domains therein and/or to link these domains to other domains. For example, a linker having a length of 2 to 10 amino acids can be used. Particularly, a linker having a glycine-serine continuous sequence can be used. For example, a linker IDGGGGSGGGGSGGGGS can be inserted between the CD28 cytoplasmic domain and the partial cytoplasmic IL-2 receptor beta domain. For example, a linker KLGGSGP can be inserted between the partial cytoplasmic IL-2 receptor beta domain and the intracellular domain of the CD3 zeta chain.

Another aspect provides a chimeric receptor comprising i) an extracellular domain capable of binding to an immunoreceptor recognition site of an antigen-binding molecule, ii) a transmembrane domain, and iii) an intracellular segment comprising one or more intracellular signaling domains including a cytoplasmic domain of an interleukin receptor chain and optionally a supplementary cytoplasmic domain.

The cytoplasmic domain of an IL receptor chain may be selected from any chain of the IL receptor described in the present specification. The whole region of the cytoplasmic domain of the IL receptor chain may be used. Alternatively, a truncated fragment of the cytoplasmic domain of the IL receptor chain may also be used. Examples of the full length and the truncated fragment thereof are given in the present specification.

In an embodiment, the truncated fragment may comprise at least one tyrosine kinase association motif (also known as a box-1 motif) and a STAT (signal transducer and activator of transcription) association motif described in the present specification. The truncated fragment comprises, for example, up to 250 amino acids of the ILR cytoplasmic domain, or is 50 to 200 amino acids or 80 to 150 amino acids of the ILR cytoplasmic domain.

The STAT association motif of the IL-2R beta chain comprises tyrosine residue-510 (tyrosine residue 510 is amino acid 536 of NCBI RefSeq: NP_000869.1). In an embodiment, the STAT association motif comprises tyrosine residue 510 and 4 residues flanking at the C-terminal side of tyrosine residue 510, i.e., YLSLQ.

Other STAT association motifs are also known and include, for example, YXXQ, optionally YXPQ, of IL-6, YXXQ of IL-10, YLPSNID of IL-12, YLSLQ, YCTFP and YFFFH of IL-2, YVTMS of IL-7, YLPQE of IL-9, and YKAFS and YKPFQ of IL-4. Any of the STAT signaling domains may be used and/or can be introduced into the ILR chain.

In an embodiment, the intracellular segment of the chimeric receptor comprises at least one supplementary signaling domain other than those present in the IL receptor, in addition to the cytoplasmic domain of the IL receptor. Examples of the intracellular signaling domain include a cytoplasmic region derived from a TCR complex and/or a costimulatory molecule, and any mutant having the same functions as those of these sequences. Other examples thereof include cytoplasmic signal transduction domains listed in Table 2 of Sadelain et al., 2009, which is incorporated herein by reference.

The present disclosure includes a chimeric receptor comprising an intracellular segment comprising one or more, for example, 2 or 3 intracellular signaling domains in addition to the cytoplasmic domain of the IL receptor. The chimeric receptor comprises, for example, a cytoplasmic domain of an IL receptor and an intracellular signaling domain of CD3 zeta. The chimeric receptor comprises, for example, a cytoplasmic domain of an IL receptor, an intracellular signaling domain of CD3 zeta and a cytoplasmic costimulatory domain of CD28.

In an embodiment, the chimeric receptor comprises an intracellular segment comprising a CD3 zeta intracellular signaling domain, and one or more cytoplasmic costimulatory domains, wherein the intracellular segment comprises a JAK binding motif, a STATS and/or STAT3 association motif.

The chimeric receptor of the present disclosure comprises a transmembrane domain. The transmembrane domain may be derived from a natural polypeptide or may be artificially designed. The transmembrane domain derived from a natural polypeptide can be obtained from a membrane-associated or transmembrane protein. For example, a transmembrane domain of a T cell receptor alpha or beta chain, a CD3 zeta chain, CD28, CD3 epsilon, CD45, CD4, CD5, CD8, CD9, CD16, CD22, CD33, CD37, CD64, CD80, CD86, CD134, CD137, ICOS, CD154, or GITR can be used. The artificially designed transmembrane domain is a polypeptide mainly comprising hydrophobic residues such as leucine and valine. For example, a triplet of phenylalanine, tryptophan and valine can be found at each end of the synthetic transmembrane domain. Optionally, a short-chain oligopeptide linker or a polypeptide linker, for example, a linker having a length of 2 to 10 amino acids can be arranged between the transmembrane domain and the intracellular segment as described in the present specification. Particularly, a linker sequence having a glycine-serine continuous sequence can be used.

The transmembrane domain used can be, for example, a transmembrane domain having any of sequences from amino acids 153 to 179 of CD28 (NCBI RefSeq: NP_006130.1).

In the chimeric receptor of the present disclosure, a spacer domain can be arranged between the extracellular domain and the transmembrane domain, or between the intracellular segment and the transmembrane domain. The spacer domain means any oligopeptide or polypeptide that works to link the transmembrane domain to the extracellular domain and/or the transmembrane domain to the intracellular segment. The spacer domain comprises up to 300 amino acids, for example, approximately 10 to 100 amino acids, or approximately 25 to 50 amino acids.

The spacer domain preferably has a sequence that promotes the binding of the chimeric receptor to an antigen via the antigen-binding molecule and enhances signal transduction into cells. Examples of the amino acid that is expected to promote the binding include cysteine, charged amino acids, and serine and threonine within a potential glycosylation site. These amino acids can be used as amino acids constituting the spacer domain.

In an embodiment, the spacer domain is a polypeptide comprising or consisting of amino acids 118 to 178 of CD8 alpha (NCBI RefSeq: NP_001759.3), i.e., a hinge region of CD8 alpha, amino acids 135 to 195 of CD8 beta (GenBank: AAA35664.1), amino acids 315 to 396 of CD4 (NCBI RefSeq: NP_000607.1), amino acids 114 to 152 of CD28 (NCBI RefSeq: NP_006130.1), or a portion thereof. Further, the spacer domain may be an artificially synthesized sequence.

The chimeric receptor of the present disclosure can be designed so as to form a polymer, particularly, a dimer. For example, in order to polymerize (dimerize) the chimeric receptor, for example, via a disulfide bond, cysteine is inserted into the spacer domain and/or the transmembrane domain.

Further, in the chimeric receptor of the present disclosure, a signal peptide sequence can be linked to the N terminus. The signal peptide sequence resides at the N termini of many secretory proteins and membrane proteins, and has a length of 15 to 30 amino acids. Most protein molecules having an intracellular domain described in the present specification are membrane proteins, and have a signal peptide sequence. The signal peptide derived from such a secretory protein or a membrane protein can be used as the signal peptide for the chimeric receptor of the present disclosure. Any signal peptide can be used. The signal peptide can be, for example, an oncostatin M. signal peptide. The signal peptide can be derived from a human and may be derived from a non-human source, for example, insect cells or a virus. In an embodiment, the signal peptide is a human signal peptide.

The present disclosure provides a nucleic acid encoding the chimeric receptor described in the present specification. The nucleic acid encoding the chimeric receptor can be easily prepared from the amino acid sequence of the defined chimeric receptor by a routine method. A nucleotide sequence encoding an amino acid sequence can be obtained from NCBI RefSeq ID or GenBank accession number mentioned above for the amino acid sequence of each domain, and the nucleic acid of the present disclosure can be prepared by use of standard molecular biological and/or chemical procedures. For example, a nucleic acid can be synthesized on the basis of the nucleotide sequence, and the nucleic acid of the present disclosure can be prepared by combining DNA fragments obtained from a cDNA library through polymerase chain reaction (PCR).

The nucleic acid of the present disclosure can be linked to another nucleic acid so as to be expressed under the control of a preferred promoter. Examples of the promoter include promoters that constitutively promote the expression of a gene or an operably linked construct, and promoters that induce the expression of a gene or an operably linked construct by the action of a drug or the like (e.g., tetracycline or doxorubicin). The nucleic acid of the present disclosure can be also linked to a nucleic acid comprising other regulatory elements, for example, an enhancer sequence or a terminator sequence, which cooperate with a promoter or a transcription initiation site, in order to obtain the efficient transcription of the nucleic acid. In addition to the nucleic acid of the present disclosure, a gene capable of serving as a marker for confirming expression of the nucleic acid (e.g., a drug resistance gene, a gene encoding a reporter enzyme, or a gene encoding a fluorescent protein) may be incorporated.

In an embodiment, the nucleic acid is a codon-optimized nucleic acid for expression in a particular host.

A method for producing a cell expressing the chimeric receptor of the present disclosure comprises the step of introducing a nucleic acid encoding the chimeric receptor described in the present specification into a cell. This step is performed *ex vivo*. For example, a cell can be transformed *ex vivo* with a virus vector or a non-virus vector carrying the nucleic acid of the present disclosure so as to produce a cell expressing the chimeric receptor of the present disclosure.

In the method of the present disclosure, a cell derived from a mammal, for example, a human cell, or a cell derived from a non-human mammal such as a monkey, a mouse, a rat, a pig, a horse, or a dog can be used.

In one embodiment, the mammal is a human.

The present disclosure provides a chimeric receptor, a nucleic acid encoding the chimeric receptor, a cell expressing the chimeric receptor, and a composition comprising the cells. In an embodiment, one or more of the foregoing may be used in the field of adoptive gene immunotherapy targeting antigens such as tumor antigens, and/or in screening or other *in vitro* assays. The chimeric receptor of the present disclosure can be introduced into cells to obtain, for example, enhancement or elevation in the expression level of the chimeric receptor in the cells. Such cells are capable of exerting cytotoxic activity against cells expressing a target antigen.

The present invention provides a method for preparing a cell expressing the chimeric receptor of the present disclosure, comprising:

a) isolating an immunocyte from a mammal (optionally, the immunocyte is a T cell);
b) transfecting or transducing the isolated immunocyte (optionally, T cell) with a nucleic acid encoding the chimeric receptor described in the present specification; and
c) optionally isolating and/or culturing and expanding a chimeric receptor-expressing cell (optionally, a chimeric receptor-expressing T cell) after the transfection or the transduction.

In one embodiment, autologous T lymphocytes, autologous NK cells, or autologous macrophages are activated and/or grown *ex vivo* before re-introduction to a subject. In one embodiment, the T lymphocytes or the NK cells are allogeneic T lymphocytes or allogeneic NK cells. In one embodiment, the allogeneic T lymphocytes are T lymphocytes whose expression of an endogenous T cell receptor is blocked or eliminated. In one embodiment, allogeneic T lymphocytes or allogeneic NK cells are activated and/or grown *ex vivo* before introduction to a subject. In one embodiment, the chimeric receptor is introduced to T lymphocytes, NK cells or macrophages by a method selected from the group consisting of retroviral transduction, lentiviral transduction, DNA electroporation and RNA electroporation, DNA or RNA transfection, and genetic alteration by gene editing.

The NK cells used in the method of the present disclosure are devoid of or rarely express a major histocompatibility complex I and/or II molecule, and are capable of growing preferentially by exposure to cells genetically modified so as to express membrane-associated IL-15 and 4-1BB ligand (CDI37L). Such a cell line includes, but is not limited to, K562 [ATCC, CCL 243; Lozzio et al., Blood 45 (3): 321-334 (1975); and Klein et al., Int. J. Cancer 18: 421-431 (1976)] and Wilms' tumor cell line HFWT [Fehniger T A, Caligiuri M A. Int Rev Immunol 20 (3-4): 503-534 (2001); and Harada H, et al., Exp Hematol 32 (7): 614-621 (2004)], endometrial tumor cell line HHUA, melanoma cell line HMV-II, hepatoblastoma cell line HuH-6, lung small-cell cancer cell lines Lu-130 and Lu-134-A, neuroblastoma cell lines NB 19 and N1369, testicular NEC 14-derived embryonic cancer cell lines, neck cancer cell line TCO-2 and bone marrow-metastatic neuroblastoma cell line TNB 1[Harada H., et al., Jpn. J. Cancer Res 93: 313-319 (2002)]. Preferably, the cell line used is devoid of or rarely expresses both the MHC I and II molecules, as in the K562 and HFWT cell lines. A solid support may be used instead of the cell line. Such a support preferably is attached on its surface to at least one molecule capable of binding to NK cells and inducing an initial activation event and/or proliferative response or capable of binding a molecule having such an affect, thereby acting as a scaffold. The support may be attached on its surface to CD137 ligand protein, an anti-CD137 antibody, IL-15 protein or an anti-IL-15 receptor antibody. Preferably, the support has an anti-IL-15 receptor antibody and an anti-CD137 antibody attached on its surface.

In one embodiment, in any method of the present disclosure involving T lymphocyte activation, T lymphocytes can be activated in the presence of one or more agents selected from the group consisting of anti-CD3/CD28, IL-2 and phytohemagglutinin. In any method of the present disclosure involving NK cell activation, NK cells can be activated in the presence of one or more agents selected from the group consisting of CD137 ligand protein, anti-CD137 antibody, IL-15 protein, an anti-IL-15 receptor antibody, IL-2 protein, IL-12 protein, IL-21 protein and a K562 cell line.

The cell used in the method of the present disclosure is not particularly limited, and any cell can be used. For example, a cell collected, isolated, or purified from a body fluid, a tissue or an organ, for example, blood (peripheral blood, umbilical cord blood etc.) or bone marrow, or a cell obtained by differentiating or reprogramming (in order to prepare induce pluripotent stem cells (iPSCs)) the cell mentioned above can be used (see e.g., Themeli et al., 2013). A peripheral blood mononuclear cell (PBMC), an immune cell [including, for example, a T cell, a dendritic cell, a B cell, a hematopoietic stem cell, a macrophage, a monocyte, a NK cell or a hematopoietic cell (a neutrophil or a basophil)], an umbilical cord blood mononuclear cell, a fibroblast, a precursor adipocyte, a hepatocyte, a skin keratinocyte, a mesenchymal stem cell, an adipose stem cell, various cancer cell lines, or a neural stem cell can be used. For example, a NK cell or a T cell, a precursor cell of a T cell (a hematopoietic stem cell, a lymphocyte precursor cell etc.) or a cell population containing these cells can be used. Examples of the T cell include CD8-positive T cells, CD4-positive T cells, regulatory T cells, cytotoxic T cells, and tumor infiltrating lymphocytes. The cell population containing a T cell and a precursor cell of a T cell includes PBMCs. These cells may be collected from a living body, may be obtained by culturing and expanding cells collected from a living body, or may be established as a cell line. If the transplantation of the produced chimeric receptor-expressing cell or a cell differentiated from the produced chimeric receptor-expressing cell into a living body is desired, the nucleic acid can be introduced into a cell collected from the living body itself or a conspecific living body thereof.

In conjunction with the polynucleotide, the present disclosure also provides a vector comprising such a polynucleotide (including a vector in which such a polynucleotide is operably linked to at least one regulatory element for expression of the chimeric receptor). Non-limiting examples of the useful vector of the present disclosure include virus vectors such as retrovirus vectors and lentivirus vectors.

In a particular aspect, such a vector also comprises a suicide gene. The term "suicide gene" used herein refers to a gene that causes a cell expressing the suicide gene to die. The suicide gene can be a gene that imparts sensitivity to an agent, e.g., a drug, to a cell in which the gene is expressed, and causes the cell to die when the cell is contacted with or exposed to the agent. The suicide gene is known in the art (see e.g., Suicide Gene Therapy: Methods and Reviews, Springer, Caroline J. (Cancer Research UK Centre for Cancer Therapeutics at the Institute of Cancer Research, Sutton, Surrey, UK), Humana Press, 2004) and includes, for example, herpes simplex virus (HSV) thymidine kinase (TK) gene, and genes of cytosine deaminase, purine nucleoside phosphorylase, nitroreductase and caspases such as caspase 8.

The nucleic acid encoding the chimeric receptor of the present disclosure can be introduced to a vector, and the vector can be introduced into cells. For example, a virus vector such as a retrovirus vector (including an oncoretrovirus vector, a lentivirus vector, and a pseudotyped vector), an adenovirus vector, an adeno-associated virus (AAV) vector, a simian virus vector, a vaccinia virus vector or a Sendai virus vector, an Epstein-Barr virus (EBV) vector, and a HSV vector can be used. For example, a virus vector devoid of replicating ability so as not to self-replicate in infected cells can be used.

In addition, a non-virus vector can also be used in the present disclosure in combination with a liposome or a condensing agent such as a cationic lipid, as described in WO96/10038, WO97/18185, WO97/25329, WO97/30170 and WO97/31934 (which are incorporated herein by reference). The nucleic acid of the present disclosure may be introduced into cells by calcium phosphate transduction, DEAE-dextran, electroporation, or particle bombardment.

In the case of using, for example, a retrovirus vector, the method of the present disclosure can be performed by selecting a suitable packaging cell based on a LTR sequence and a packaging signal sequence that is possessed by the vector, and preparing a retrovirus particle using the packaging cell. Examples of the packaging cell include PG13 (ATCC CRL-10686), PA317 (ATCC CRL-9078), GP+E-86 and GP+envAm-12 (U.S. Patent No. 5,278,056), and Psi-Crip [Proceedings of the National Academy of Sciences of the United States of America, vol. 85, pp. 6460-6464 (1988)]. The retrovirus particle may be prepared using a 293 cell or a 293 T cell having high transfection efficiency. Many types of retrovirus vectors produced on the basis of retrovirus and packaging cells that can be used for packaging of retrovirus vectors are widely commercially available from many companies.

The present disclosure also provides a host cell comprising the chimeric receptor. Non-limiting examples of the useful host cell include T lymphocytes and NK cells, which may be autologous or allogeneic (whose endogenous T cell receptor is removed or maintained). In a certain aspect, the host cell is an autologous T lymphocyte isolated from a patient having a cancer. In a particular aspect, such an autologous T lymphocyte is activated and grown *ex vivo.*

The chimeric receptor of the present disclosure can be introduced into the host cell by any method known in the art. Non-limiting examples of the particularly useful method include retroviral transduction, lentiviral transduction and DNA and mRNA electroporation. As shown in Examples below, mRNA electroporation causes effective expression of the chimeric receptor of the present disclosure in T lymphocytes. Examples of references describing the retroviral transduction include Anderson et al., U.S. Patent No. 5,399,346; Mann et al., Cell 33: 153 (1983); Temin et al., U.S. Pat. No. 4,650,764; Temin et al., U.S. Patent No. 5,124,263; Dougherty et al., International Publication No. WO 95/07358 (published on March 16, 1995); and Kuo et al., Blood 82: 845 (1993). International Publication No. WO 95/07358 describes high efficiency transduction of primary B lymphocytes. For example, for specific techniques of retroviral transduction and mRNA electroporation that can be used, see also the Examples section below.

Host cell activation and growth can usually be used to attain integration of a virus vector into the genome and expression of the gene encoding the chimeric receptor of the present disclosure. However, provided that mRNA electroporation is used, neither activation nor growth is required (though electroporation is more effective when carried out on activated cells). As a result of viral transduction, the host cell (T lymphocyte or NKT cell) expresses the chimeric receptor of the present disclosure for a long period while potentially producing a stronger effect than that upon mRNA electroporation when the receptor is transiently expressed (typically for 3 to 5 days). However, the viral transduction is complicated, expensive and difficult to carry out, whereas the mRNA electroporation is much simpler and much easier to carry out. Further, the transient expression is useful if there is potential toxicity and should be helpful in the initial phases of clinical trials for possible adverse reactions.

One aspect is a method of preparing the cell disclosed in the present specification, comprising transfecting or transducing a cell with the nucleic acid or the vector described in the present specification.

In one embodiment, isolated immune cells are isolated T cells.

In an embodiment, isolated cells are CD3$^+$ and are optionally stimulated with an anti-CD3 antibody, optionally in a soluble or membrane-bound form, for example, OKT3 or mOKT3, and/or with APC before transduction or transfection. In one embodiment, the APC are artificial APC (aAPC). In another embodiment, the APC expresses a membrane form of an anti-CD3 monoclonal antibody.

In one embodiment, the transfection or transduction step is repeated. For example, the transfection or transduction step can be performed twice, or three times, or four times, or until, for example, an adequate level of expression is achieved. The transfection or transduction step can be performed, for example, five times.

In one embodiment, cells are transfected or transduced for two or more consecutive days. Cells are transfected or transduced, for example, for two consecutive days, three consecutive days, or four consecutive days.

In one embodiment, the chimeric receptor-transduced cells are stimulated with irradiated cells expressing a predetermined antigen. The chimeric receptor-transduced cells are stimulated with irradiated cells, for example, at an effector:target ratio of 100:1, 75:1, 50:1, 25:1, 20:1, 15:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:15, 1:20, 1:25, 1:50 or 1:100. The above-mentioned ratio is preferably 1:1.

The cell expressing the chimeric receptor of the present disclosure is a cell allowed to harbor or express a nucleic acid encoding the chimeric receptor described in the present specification by the production method described in the present specification.

The cell of the present disclosure binds to a particular antigen via the chimeric receptor and the antigen-binding molecule. Signals are thereby transduced into the cell, and as a result, the cell is activated. The activation of the cell expressing the chimeric receptor differs depending on the type of host cells and the intracellular domains of the chimeric receptor, and can be confirmed on the basis of, for example, cytokine release, improvement in cell growth rate, change in cell surface molecule, as an index. For example, the release of a cytotoxic cytokine (a tumor necrosis factor, lymphotoxin, etc.) from the activated cell causes destruction of target cells expressing an antigen. In addition, the cytokine release or the change in cell surface molecule stimulates other immunocytes, for example, B cells, dendritic cells, NK cells, and macrophages.

The T lymphocytes used in the method of the present disclosure are most preferably patient's own cells (i.e., autologous cells) that are isolated in advance from a blood sample and preferably activated and grown *ex vivo* (e.g., for 3 to 5 days) by use of a standard method, for example, anti-CD3/CD28 beads, IL-2 or phytohemagglutinin. Alternatively, allogeneic T lymphocytes can be used (preferably allogeneic T lymphocytes whose expression of an endogenous T cell receptor is blocked or eliminated). See Torikai et al., Blood, 2012 119: 5697-5705. T lymphocytes and NK cells thus isolated (and, if desired, activated and/or grown) from a patient are transduced (or electroporated) with a polynucleotide encoding the chimeric receptor of the present disclosure (or a vector comprising such a polynucleotide) so that the chimeric receptor is expressed on the cell surface of the T cells or the NK cells. The modified cells can then be administered to the patient (e.g., 1 day after drip infusion of a therapeutic antibody).

One aspect provides use of the chimeric receptor-expressing cell, the nucleic acid, the vector, the cell or the composition described in the present specification for treating a disease.

Another aspect is a method for treating or preventing a disease in a mammal, comprising administering an effective amount of the cell or the composition disclosed in the present specification to the mammal in need thereof.

A pharmaceutical composition comprising cells expressing a chimeric receptor as an active ingredient can be administered intradermally, intramuscularly, subcutaneously, intraperitoneally, intranasally, intraarterially, intravenously, intratumorally, or into an afferent lymph vessel, by parenteral administration, for example, by injection or infusion, though the administration route is not limited.

A further aspect is a method comprising integrating a nucleic acid encoding the chimeric receptor of the present disclosure into a living body using a virus vector or the like, and directly expressing the chimeric receptor. Examples of the virus vector include, but are not limited to, adenovirus vectors. Alternatively, the nucleic acid encoding the chimeric receptor may be integrated directly into a living body by electroporation, an approach of directly administering the nucleic acid, or the like without the use of the virus vector, and the chimeric receptor may be intermittently secreted in a living body by administering cells genetically altered so as to secrete and express the chimeric receptor to the living body.

According to the present disclosure, patients can be treated by a procedure which involves infusing a therapeutically effective dose of T cells or NK cells comprising the chimeric receptor of the present disclosure in the range of approximately $10^5$ (1E + 05) to $10^{10}$ (1E + 10) or more cells per kilogram body weight (cells/kg). The infusion can be repeated as frequently and as many times as possible as long as the patients can tolerate until the desired response is achieved. The appropriate infusion dosage and schedule may differ among patients, but can be determined by a physician who treats a particular patient. Typically, an initial dose of approximately $10^6$ (1E + 06) cells/kg is infused and increased to $10^8$ (1E + 08) or more cells/kg. IL-2 can also be used in combination therewith for the post-infusion growth of the infused cells. The amount of IL-2 can be approximately 1 to $5 \times 10^6$ (1 to 5E + 06) international units per square meter of body surface.

The pharmaceutical composition comprises the cell expressing the chimeric receptor as an active ingredient and may further comprise a preferred excipient. Examples of the excipient include the pharmaceutically acceptable excipients mentioned above for the composition comprising the nucleic acid of the present disclosure as an active ingredient, various cell culture media, and isotonic sodium chloride.

A further aspect of the present disclosure provides a pharmaceutical composition. In one aspect, the present disclosure provides a pharmaceutical composition comprising (i) a polynucleotide encoding the chimeric receptor of the present disclosure or a vector comprising such a polynucleotide and (ii) a pharmaceutically acceptable carrier or additive.

Appropriate additives for use in the pharmaceutical composition of the present disclosure are well known to those skilled in the art and can include, for example, a tissue culture medium (e.g., for the *ex vivo* survival of cells) or an aqueous salt solution (e.g., upon injection of cells to patients). Detailed description on the pharmaceutically acceptable additives is available from Remington's Pharmaceutical Sciences (Mack Pub. Co., N.J. 1991).

The disease against to which the pharmaceutical composition of the present invention is administered is not particularly limited as long as the disease exhibits sensitivity to the therapy used. In one embodiment, the disease is a cancer. In one embodiment, the subject is suspected of having a cancer, or has a cancer.

The cancer is, for example, blood cancer or solid tumor. The blood cancer is, for example, leukemia, lymphoma or myeloma. The solid tumor is, for example, cancers such as adenocarcinoma, squamous cell carcinoma, adenosquamous cancer, undifferentiated cancer, large-cell cancer, small-cell cancer, skin cancer, breast cancer, prostate cancer, bladder cancer, vaginal cancer, neck cancer, uterus cancer, liver cancer, kidney cancer, pancreatic cancer, spleen cancer, lung cancer, tracheal cancer, bronchial cancer, colon cancer, small intestine cancer, stomach cancer, esophageal cancer, gallbladder cancer, testis cancer, and ovary cancer, cancers of bone tissues, cartilage tissues, fat tissues, muscle tissues, vascular tissues and hematopoietic tissues as well as sarcoma such as chondrosarcoma, Ewing's sarcoma, malignant hemangioendothelioma, malignant schwannoma, osteosarcoma, and soft tissue sarcoma, blastoma such as hepatoblastoma, medulloblastoma, nephroblastoma, neuroblastoma, pancreatoblastoma, pleuropulmonary blastoma, and retinoblastoma, and germ cell tumor.

In an embodiment, the disease is an inflammatory disease/autoimmune disease (e.g., asthma and eczema), hepatitis, and an infectious disease, the cause of which is a virus such as influenza and HIV, a bacterium, or a fungus, for example, tuberculosis, MRSA, VRE, or deep mycosis. In one embodiment, the subject is suspected of having such an inflammatory disease, or has such an inflammatory disease.

The pharmaceutical composition of the present disclosure which binds to an antigen possessed by cells desired to be reduced or eliminated for the treatment of the diseases mentioned above, i.e., a tumor antigen, a viral antigen, a bacterial antigen, or the like, is administered for the treatment of these diseases.

Accordingly, one aspect includes a method for decreasing the number of cells expressing a predetermined antigen in a subject, comprising administering an effective amount of the pharmaceutical composition described in the present specification to the subject in need thereof, wherein the chimeric receptor-expressing cell specifically binds to the predetermined antigen via the antigen-binding molecule which binds to the extracellular domain.

The cell of the present disclosure can also be utilized for the prevention of an infectious disease after bone marrow transplantation or exposure to radiation, donor lymphocyte transfusion for the purpose of remission of recurrent leukemia, and the like.

The chimeric receptor of the present disclosure promotes T cell therapy that allows a single receptor to be used for diverse cancer cell types. This strategy can be eventually advantageous when an immune escape mechanism exploited by tumor is taken into consideration. At the same time, diverse antigens can be targeted (Grupp et al., N. Engl. J. Med. 2013; 368 (16):1509-1518). Antibody-mediated cytotoxicity can be stopped anytime by the mere discontinuation of antibody administration. Since T cells expressing the chimeric receptor of the present disclosure are activated only by an antibody bound to target cells, unbound immunoglobulins do not exert any stimulation into the infused T cells. Clinical safety can be further enhanced by using mRNA electroporation for the transient expression of the chimeric receptor in order to limit any potential autoimmune reactivity.

In one aspect of the method, the T lymphocytes or the NK cells are autologous T cells or NK cells isolated from the subject. In a particular aspect, the autologous T cells or NK cells are activated and/or grown *ex vivo* before re-introduction to the subject. In another aspect, the T cells or the NK cells are allogeneic T lymphocytes or NK cells. In a certain aspect, the T cells are allogeneic T cells whose expression of an endogenous T cell receptor is blocked or eliminated. In a particular aspect, the allogeneic T cells are activated and/or grown *ex vivo* before introduction to the subject. The T lymphocytes can be activated by any method known in the art in the presence of, for example, anti-CD3/CD28, IL-2 and/or phytohemagglutinin. The NK cells can be activated by any method known in the art in the presence of one or more agents selected from the group consisting of, for example, CD137 ligand protein, anti-CD137 antibody, IL-15 protein, an anti-IL-15 receptor antibody, IL-2 protein, IL-12 protein, IL-21 protein and a K562 cell line. For example, for description on useful methods for growing NK cells, see U.S. Patent Nos. 7,435,596 and 8,026,097.

In one aspect of the method, following introduction (or re-introduction) of the T cells or the NK cells to the subject, a therapeutically effective amount of a PD-1/PD-L1 signal inhibitor and/or a VEGF signal inhibitor is administered to the subject.

The composition and the method described in the present specification may be used in combination with another type of treatment for a cancer, such as chemotherapy, surgery, radiation, or gene therapy. Such treatment can be applied concurrently or sequentially (in any order) with the immunotherapy according to the present disclosure.

In combined use with an additional pharmaceutical composition, an appropriate therapeutically effective dose of each pharmaceutical composition can be decreased owing to additive or synergistic effects.

The treatment of the present disclosure can be combined with, for example, another immunomodulatory treatment such as a therapeutic vaccine (including, but not limited to, GVAX, DC vaccines, etc.), a checkpoint inhibitor (including, but not limited to, pharmaceutical composition blocking CTLA4, PD1, LAG3, TIM3, etc.) or an activator (including, but not limited to, pharmaceutical composition enhancing 41BB, OX40, etc.).

The pharmaceutical composition of the present disclosure may also comprise one or more additional active compounds, preferably compounds having complementary activity without adversely affecting each other, if necessary for a particular indication to be treated. Non-limiting examples of the possible additional active compounds include PD-1/PD-L1 signal inhibitors and VEGF signal inhibitors.

In another aspect, the pharmaceutical composition of the present disclosure further comprises a monoclonal antibody (e.g., rituximab, trastuzumab, or hu14.18K322A) that can exert cytotoxicity against cancer cells, or another antitumor molecule comprising an Fc moiety (e.g., a composite molecule comprising a ligand (e.g., a cytokine or an immune cell receptor) bound to a tumor surface receptor combined with an immunoglobulin Fc moiety or Fc-containing DNA or RNA).

An appropriate dose of the antibody used depends on the type of cancer to be treated, the severity and course of the disease, previous treatment, patient's clinical history and response to the antibody, and the discretion of the treating physician. The antibody can be administered to the patient at once or over a series of treatments. The progress of the treatment according to the present disclosure can be easily monitored by a conventional technique and assay.

The administration of the antibody can be carried out by any appropriate route including systemic administration and direct administration to a site of the disease (e.g., to primary tumor).

Non-limiting examples of the additional therapeutic agent useful for combination with the immunotherapy of the present disclosure include the following:

(i) anti-angiogenic agents (e.g., TNP-470, platelet factor 4, thrombospondin-1, tissue inhibitors of metalloproteases (TIMP1 and TIMP2), prolactin (16 kD fragment), angiostatin (38 kD fragment of plasminogen), endostatin, bFGF soluble receptor, transforming growth factor beta, interferon alpha, soluble KDR and FLT-1 receptor, placental proliferin-related protein, and those described in Carmeliet and Jain (2000));

(ii) VEGF antagonists or VEGF receptor antagonists such as anti-VEGF antibodies, VEGF variants, soluble VEGF receptor fragments, aptamers capable of blocking VEGF or VEGFR, neutralizing anti-VEGFR antibodies, inhibitors of VEGFR tyrosine kinase and any combination thereof;

(iii) chemotherapeutic compounds such as pyrimidine analogs (e.g., 5-fluorouracil, floxuridine, capecitabine, gemcitabine and cytarabine), purine analogs, folate antagonists and related inhibitors (e.g., mercaptopurine, thioguanine, pentostatin and 2-chlorodeoxyadenosine (cladribine)); antiproliferative/antimitotic agents including natural products such as vinca alkaloids (e.g., vinblastine, vincristine, and vinorelbine), microtubule disruptors such as taxane (e.g., paclitaxel and docetaxel), vincristine, vinblastine, nocodazole, epothilones and navelbine, epipodophyllotoxin (e.g., etoposide and teniposide), and DNA damaging agents (e.g., actinomycin, amsacrine, anthracyclines, bleomycin, busulfan, camptothecin, carboplatin, chlorambucil, cisplatin, cyclophosphamide, Cytoxan, dactinomycin, daunorubicin, doxorubicin, epirubicin, hexamethylmelamine, oxaliplatin, ifosfamide, melphalan, mechlorethamine, mitomycin, mitoxantrone, nitrosourea, plicamycin, procarbazine, taxol, taxotere, teniposide, triethylene thiophosphoramide and etoposide (VP16)); antibiotics such as dactinomycin (actinomycin D), daunorubicin, doxorubicin (adriamycin), idarubicin, anthracyclines, mitoxantrone, bleomycins, plicamycin (mithramycin) and mitomycin; enzymes (e.g., L-asparaginase which systemically metabolizes L-asparagine and removes cells lacking the ability to synthesize their own asparagine); antiplatelet agents; antiproliferative/antimitotic alkylating agents such as nitrogen mustards (e.g., mechlorethamine, cyclophosphamide and analogs, melphalan, and chlorambucil), ethylenimines and methylmelamines (e.g., hexamethylmelamine and thiotepa), alkyl sulfonates-busulfan, nitrosoureas (e.g., carmustine (BCNU) and analogs, and streptozocin), and trazenes-dacarbazinine (DTIC); antiproliferative/antimitotic antimetabolites such as folic acid analogs (e.g., methotrexate); platinum coordination complexes (e.g., cisplatin and carboplatin), procarbazine, hydroxyurea, mitotane, and aminoglutethimide; hormones, hormone analogs (e.g., estrogen, tamoxifen, goserelin, bicalutamide, and nilutamide) and aromatase inhibitors (e.g., letrozole and anastrozole); anticoagulants (e.g., heparin, synthetic heparin salts and other thrombin inhibitors); fibrinolytic agents (e.g., tissue plasminogen activator, streptokinase and urokinase), aspirin, dipyridamole, ticlopidine, clopidogrel, and abciximab; antimigratory agents; antisecretory agents (e.g., brefeldin); immunosuppressive agents (e.g., cyclosporine, tacrolimus (FK-506), sirolimus (rapamycin), azathioprine, and mycophenolate mofetil); anti-angiogenic compounds (e.g., TNP-470, genistein, and bevacizumab) and growth factor inhibitors (e.g., fibroblast growth factor (FGF) inhibitors); angiotensin receptor blockers; nitric oxide donors; anti-sense oligonucleotides; antibodies (e.g., trastuzumab); cell cycle inhibitors and differentiation inducers (e.g., tretinoin); mTOR inhibitors, topoisomerase inhibitors (e.g., doxorubicin (adriamycin), amsacrine, camptothecin, daunorubicin, dactinomycin, teniposide, epirubicin, etoposide, idarubicin and mitoxantrone, topotecan, and irinotecan), corticosteroids (e.g., cortisone, dexamethasone, hydrocortisone, methylprednisolone, prednisone, and prednisolone); growth factor signaling kinase inhibitors; mitochondrial dysfunction inducers and caspase activators; and chromatin disruptors.

For further examples of useful agents, see also Physician's Desk Reference, 59.sup.th edition, (2005), Thomson P D R, Montvale N.J.; Gennaro et al., Eds. Remington's The Science and Practice of Pharmacy 20.sup.th edition, (2000), Lippincott Williams and Wilkins, Baltimore Md.; Braunwald et al., Eds. Harrison's Principles of Internal Medicine, 15.sup.th edition, (2001), McGraw Hill, NY; Berkow et al., Eds. The Merck Manual of Diagnosis and Therapy, (1992), Merck Research Laboratories, Rahway N.J.

The above disclosure generally describes the present application. More complete understanding can be obtained by reference to specific examples given below. These examples are described merely for the purpose of illustration and are not intended to limit the scope of the present application. Change in form and substitution of equivalents are also contemplated as circumstances might suggest or render expedient. Although particular terms are used in the present disclosure, such terms are intended in a descriptive sense and not for purposes of limitation.

All references cited herein are incorporated herein by reference.

EXAMPLES

Hereinafter, the present invention will be described in more detail with reference to Examples. However, these Examples do not limit the scope of the present invention.

[Example 1] Chimeric receptor having extracellular domain of immunoreceptor in extracellular domain

It is known that by using an antigen-binding molecule having a binding domain exhibiting agonist activity on an immunoreceptor which exists on the surfaces of immunocytes and is involved in activation of the immunocytes, and a binding domain on a target antigen on target cells, the immunocytes expressing the immunoreceptor and the target cells expressing the target antigen are crosslinked by the antigen-binding molecule to activate the immunocytes. In the present invention, a treatment method using an antigen-binding molecule exhibiting agonist activity on an immunoreceptor in combination with immunocytes transduced with a chimeric receptor (hereinafter, also referred to as "CR") having an immunoreceptor recognized by the antigen-binding molecule in an extracellular domain was devised (Figure 1).

Specifically, the treatment method comprises: 1) administering a first composition comprising one or more types of antigen-binding molecules having a binding domain exhibiting agonist activity on an immunoreceptor which exists on the surfaces of immunocytes and is involved in activation of the immunocytes, and a binding domain directed to a target antigen specifically or selectively expressed on a target cell in a subject in need of treatment; and 2) administering immunocytes transduced with CR having an extracellular domain of an immunoreceptor, to which the antigen-binding molecule contained in the first composition can bind, in an extracellular domain, wherein the antigen-binding molecule crosslinks the immunocytes expressing CR and the target cells to activate the CR-expressing cells. Here, when the type of target antigen is to be changed, the type of target antigen can be changed by changing the antigen-binding molecule of the first composition, and it is not necessary to reprepare immunocytes expressing CR. The preparation of such CR cells is more versatile and useful than preparation of immunocytes that directly recognize an antigen specifically or selectively expressed in target cells.

In addition, in this treatment method, not only immunocytes in which CR is transformed, but also endogenous immunocytes endogenously expressing an immunoreceptor are recruited in a manner dependent on a target antigen by the antigen-binding molecule. It is considered that as a result, not only immunocytes in which CR is transformed are activated, but also endogenous immunocytes are activated by the agonist activity of the antigen-binding molecule, and enhancement of the drug efficacy is expected.

[Example 2] Preparation of bispecific antibody

(2-1) Preparation of homo-antibody

The antibodies shown in Table 1 were prepared. A heavy chain constant region exhibits reduced binding to a Fcγ receptor, and is altered so as to attain hetero-association of two heavy chains. Full-length genes having nucleotide sequences encoding a heavy chain and a light chain of each antibody were prepared by a method known to those skilled in the art using PCR or the like. The obtained plasmid portions were inserted to expression vectors for animal cells to prepare a heavy chain expression vector and a light chain expression vector. The nucleotide sequences of the obtained expression vectors were determined by a method known to those skilled in the art. The prepared plasmids were transiently introduced into human embryonic kidney cancer cell-derived FreeStyle293 cells (Invitrogen Corp.), or Expi293 cells (Invitrogen Corp.) so that the antibody was expressed. The obtained culture supernatant was recovered and then passed through 0.22 μm filter (Merck Millipore) to obtain a culture supernatant. From the obtained culture supernatant, the antibody was purified by a method known to those skilled in the art using rProtein A Sepharose (R) Fast Flow (GE Healthcare Japan Corp.) or Protein G Sepharose (R) 4 Fast Flow (GE Healthcare Japan Corp.). The concentration of the purified antibody was measured as absorbance at 280 nm using a spectrophotometer, and the antibody concentration was calculated using an absorption coefficient calculated by a method such as PACE from the obtained value (Protein Science 1995; 4: 2411-2423).

Antibodies in the present specification were designated according to the following rule:
(heavy chain variable region)-(heavy chain constant region)/(light chain variable region)-(light chain constant region).

For example, antibody name H0000-F760nN17/GL4-k0a means that the antibody has heavy chain variable region H0000, heavy chain constant region F760nN17, light chain variable region GL4, and light chain constant region k0a.

Likewise, variable regions of antibodies may be represented according to the following rule:
(heavy chain variable region)/(light chain variable region).

[Table 1]

Table 1: Structure of homo-antibody

| Antibody name | Variable region | | Constant region | | Target antigen |
|---|---|---|---|---|---|
| | Heavy chain SEQ ID NO: | Light chain SEQ ID NO: | Heavy chain SEQ ID NO: | Light chain SEQ ID NO: | |
| H0000-F760nN 17/ GL4-k0a | 1 | 2 | 3 | 4 | Human GPC3 |
| hCD137VH-F760mnP17/ hCD137VL-k0 | 5 | 6 | 7 | 8 | Human CD137 |
| GPA0018H-F760mnN 17/GPA0018L-k0C | 9 | 10 | 11 | 12 | Human GPRC5D |
| GPA0039H-F760mnN17/GPA0039L-k0C | 13 | 14 | 11 | 12 | Human GPRC5D |
| MRAH.v1-F760mnN17/MRAL.v1-k0.v1 | 15 | 16 | 11 | 17 | Human IL-6R |
| H0000-F760mnN17/ GL4-k0a | 1 | 2 | 11 | 4 | Human GPC3 |
| IC17HdK-F760mnN17/IC17L-k0 | 18 | 19 | 11 | 8 | KLH |
| H0000-F760mnP 17/GL4-k0a | 1 | 2 | 7 | 4 | Human GPC3 |
| TGN1412VH-F760nmP17.v1/TGN1412VL-KT0.v1 | 20 | 21 | 22 | 23 | Human CD28 |

(2-2) Preparation of bispecific antibody

Using the antibodies prepared in Example 2-1, the bispecific antibodies shown in Table 2 were prepared by a method known to those skilled in the art that utilized difference in charge among their constant regions (Labrijn et al, Proc. Natl. Acad. Sci., (2013) 110, 5145).

Reaction conditions: [2-MEA (Sigma)] = 25 mM, 37°C, 90 min in PBS (pH 7.4)

Bispecific antibodies in the present specification were designated according to the following rule:

AA (first antibody heavy chain)/XX (first antibody light chain)/BB (second antibody heavy chain)/YY (second antibody light chain).

[Table 2-1]

Table 2: Structure of bispecific antibody

| Antibody name | | | | |
|---|---|---|---|---|
| H0000-F760nN17/ GL4-k0a// hCD137VH-F760mnP17/ hCD 137VL-k0 | First arm | | | |
| | Variable region | | Constant region | |
| | Heavy chain SEQ ID NO: | Light chain SEQ ID NO: | Heavy chain SEQ ID NO: | Light chain SEQ ID NO: |
| | 1 | 2 | 3 | 4 |
| | Second arm | | | |
| | Variable region | | Constant region | |
| | Heavy chain SEQ ID NO: | Light chain SEQ ID NO: | Heavy chain SEQ ID NO: | Light chain SEQ ID NO: |
| | 5 | 6 | 7 | 8 |
| GPA0018H-F760mnN17/GPA00 18L-k0C//hCD137VH-F760mnP17/hCD137 VL-k0 | First arm | | | |
| | Variable region | | Constant region | |
| | Heavy chain SEQ ID NO: | Light chain SEQ ID NO: | Heavy chain SEQ ID NO: | Light chain SEQ ID NO: |
| | 9 | 10 | 11 | 12 |
| | Second arm | | | |
| | Variable region | | Constant region | |
| | Heavy chain SEQ ID NO: | Light chain SEQ ID NO: | Heavy chain SEQ ID NO: | Light chain SEQ ID NO: |
| | 5 | 6 | 7 | 8 |
| GPA0039H-F760mnN17/GPA00 39L-k0C//hCD137VH-F760mnP17/hCD137 VL-k0 | First arm | | | |
| | Variable region | | Constant region | |
| | Heavy chain SEQ ID NO: | Light chain SEQ ID NO: | Heavy chain SEQ ID NO: | Light chain SEQ ID NO: |
| | 13 | 14 | 11 | 12 |
| | Second arm | | | |
| | Variable region | | Constant region | |
| | Heavy chain SEQ ID NO: | Light chain SEQ ID NO: | Heavy chain SEQ ID NO: | Light chain SEQ ID NO: |
| | 5 | 6 | 7 | 8 |

[Table 2-2]

| | First arm | | | |
|---|---|---|---|---|
| | Variable region | | Constant region | |
| | Heavy chain SEQ ID NO: | Light chain SEQ ID NO: | Heavy chain SEQ ID NO: | Light chain SEQ ID NO: |
| MRAH.v1-F760mnN17/MRAL. v1-k0.v1//hCD137VH-F760mnP17/hCD137 VL-k0 | 15 | 16 | 11 | 17 |
| | Second arm | | | |
| | Variable region | | Constant region | |
| | Heavy chain SEQ ID NO: | Light chain SEQ ID NO: | Heavy chain SEQ ID NO: | Light chain SEQ ID NO: |
| | 5 | 6 | 7 | 8 |
| | First arm | | | |
| | Variable region | | Constant region | |
| | Heavy chain SEQ ID NO: | Light chain SEQ ID NO: | Heavy chain SEQ ID NO: | Light chain SEQ ID NO: |
| IC17HdK-F760mnN17/IC17L-k0//hCD137VH-F760mnP17/hCD137 VL-k0 | 18 | 19 | 11 | 8 |
| | Second arm | | | |
| | Variable region | | Constant region | |
| | Heavy chain SEQ ID NO: | Light chain SEQ ID NO: | Heavy chain SEQ ID NO: | Light chain SEQ ID NO: |
| | 5 | 6 | 7 | 8 |
| | First arm | | | |
| | Variable region | | Constant region | |
| | Heavy chain SEQ ID NO: | Light chain SEQ ID NO: | Heavy chain SEQ ID NO: | Light chain SEQ ID NO: |
| IC17HdK-F760mnN17/IC17L-k0//H0000-F760mnP17/GL4-k0a | 18 | 19 | 11 | 8 |
| | Second arm | | | |
| | Variable region | | Constant region | |
| | Heavy chain SEQ ID NO: | Light chain SEQ ID NO: | Heavy chain SEQ ID NO: | Light chain SEQ ID NO: |
| | 1 | 2 | 7 | 4 |

[Table 2-3]

| | First arm | | | |
|---|---|---|---|---|
| | Variable region | | Constant region | |
| | Heavychain SEQ ID NO: | Light chain SEQ ID NO: | Heavychain SEQ ID NO: | Light chain SEQ ID NO: |
| H0000-F760mnN17/GL4-k0a//TGN1412VH-F760mnP 17.v 1/TGN 1412VL-KT0.v1 | 1 | 2 | 11 | 4 |
| | Second arm | | | |
| | Variable region | | Constant region | |
| | Heavychain SEQ ID NO: | Light chain SEQ ID NO: | Heavychain SEQ ID NO: | Light chain SEQ ID NO: |
| | 20 | 21 | 22 | 23 |
| | First arm | | | |
| | Variable region | | Constant region | |
| | Heavychain SEQ ID NO: | Light chain SEQ ID NO: | Heavychain SEQ ID NO: | Light chain SEQ ID NO: |
| IC17HdK-F760mnN17/IC17L-k0//TGN1412VH-F760mnP 17.v 1/TGN 1412VL-KT0.v1 | 18 | 19 | 11 | 8 |
| | Second arm | | | |
| | Variable region | | Constant region | |
| | Heavychain SEQ ID NO: | Light chain SEQ ID NO: | Heavychain SEQ ID NO: | Light chain SEQ ID NO: |
| | 20 | 21 | 22 | 23 |
| | First arm | | | |
| | Variable region | | Constant region | |
| | Heavychain SEQ ID NO: | Light chain SEQ ID NO: | Heavychain SEQ ID NO: | Light chain SEQ ID NO: |
| IC17HdK-F760mnN17/IC17L-k0//H0000-F760mnP17/GL4-k0a | 18 | 19 | 11 | 8 |
| | Second arm | | | |
| | Variable region | | Constant region | |
| | Heavychain SEQ ID NO: | Light chain SEQ ID NO: | Heavychain SEQ ID NO: | Light chain SEQ ID NO: |
| | 1 | 2 | 7 | 4 |

[Example 3] Evaluation of ability to activate chimeric receptor-expressing cells having extracellular domain of CD137 in extracellular domain

(3-1) Construction of lentivirus vector

A lentivirus vector expressing chimeric receptor CD137-CD8-CD28-CD137-CD3 zeta consisting of a human CD137 extracellular domain, a human CD8 hinge, a transmembrane domain, and a third-generation chimeric antigen receptor intracellular signal domain (CD28-CD137-CD3 zeta) (Figure 2, referred to as CD137-CR1). pCDH-CMV-MCS-T2A-copGFP (System Biosciences) was used as a lentivirus vector skeleton. Figure 3 is a schematic view showing the vector construct and the order of arrangement of components in frame units from the 5' end to the 3' end. A hinge region and a transmembrane region of a human CD8 alpha-chain (Genbank NM001768.7, Phe128 to Asn210), and cytoplasmic regions of a human CD28 molecule (Genbank NM006139.4, Arg180 to Ser220), a human CD137 molecule (Genbank NM001561.6, Arg209 to Leu255) and a human CD3 zeta molecule (Genbank NM000734.4, Arg52 to Arg164) were linked to an extracellular domain (Genbank NM001561.6, Leu24 to Gln186) of codon-optimized human CD137. A gene encoding CD137-CR1 (SEQ ID NO: 24) was synthesized by a method known to those skilled in the art. This sequence was ligated into MCS of the pCDH-CMV-MCS-T2A-copGFP vector to construct a lentivirus vector coexpressing CD137-CR1 and copGFP.

(3-2) Introduction of gene into NFAT-RE-luc2 Jurkat cells

NFAT-RE-luc2 Jurkat cells (Promega Corporation) were transduced with a CD137-CR1-copGFP vector for analyzing the ability to activate T cells expressing CD137-CR1 by an *in vitro* reporter assay. The cells have luciferase gene inserted downstream of an element responsive to the transcriptional factor: nuclear factor of activated T cells (NFAT), and enable the activation of the NFAT pathway to be quantified by detecting the luminescence of luciferase.

The transduction was performed by a lentivirus method using the lentivirus vector constructed in Example 3-1. Specifically, first, 293Ta cell line (Genecopoeia) was transfected with the CD137-CR1-copGFP vector mentioned above and a packaging plasmid mix by Lenti-Pac™ HIV Expression Packaging Kit (Genecopoeia) to prepare lentivirus harboring the CD137-CR1-copGFP. 48 hours to 72 hours after the transfection, a supernatant containing the lentivirus was recovered, and concentrated using a Lenti-X™ concentrator (Takara Bio Inc.).

Next, the recovered supernatant containing lentivirus and a polybrene solution (Nacalai Tesque, Inc.) were added to NFAT-RE-luc2 Jurkat cells, and the mixture was centrifuges at 2000 rpm for 60 minutes to obtain NFAT-RE-luc2 Jurkat cells transduced with the CD137-CR1-copGFP vector (CD137-CR1-copGFP Jurkat cells). As a negative control for the experiment, NFAT-RE-luc2 Jurkat cells transduced with a pCDH-CMV-MCS-T2A-copGFP vector which does not ligate CD137-CR1 and expresses only copGFP were also prepared.

(3-3) Confirmation of CR protein expression rate

The expression of CD137-CR1 on the surfaces of the CD137-CR1-copGFP Jurkat cells prepared in Example 3-2 was analyzed by BD FACSAria™ III Cell Sorter (BD Biosciences) with CD137 antibody (BioLegend) used for staining. As a result, approximately 70% of the living cells were confirmed to express CD137-CR1.

(3-4) Evaluation of ability to activate CD137-CR1-copGFP Jurkat cells when target antigen is GPC3

CD137-CR1-copGFP Jurkat cells prepared in Example 3-2 were co-cultured with tumor cells expressing a tumor antigen, and the ability to activate Jurkat cells in the presence of a bispecific antibody consisting of an antibody to a tumor antigen and an anti-CD137 antibody was analyzed. Here, human GPC3 expressed on solid cancer was selected as a model tumor antigen, a bispecific antibody consisting of an anti-GPC3 antibody and an anti-CD137 antibody and prepared in Example 2-2 (H0000-F760nN17/GL4-k0a//hCD137VH-F760mnP17/hCD137VL-k0) was used in the experiment.

For the tumor cell line expressing human GPC3, SK-pca60 cells were used in which human GPC3 was stably expressed on SK-HEP1 (ATCC) that is a human liver cancer cell line. First, SK-pca60 cells were inoculated at 10 µL/well in a 384-well flat-bottom plate (Corning, Inc.) (1E + 04 ($1 \times 10^4$) cells/well). Subsequently, CD137-CR1-copGFP Jurkat cells, or NFAT-RE-luc2 Jurkat cells expressing only copGFP without expressing a chimeric receptor as a negative control were mixed at 5 µL/well (1E + 04 ($1 \times 10^4$) cells/well). Subsequently, the bispecific antibody prepared at each concentration was added at 5 µL/well (final concentration: 0, 16, 160, 1600, 16000 ng/mL). After 24 hours, the luciferase activity was measured using Bio-Glo™ Luciferase (Promega Corporation). The luciferase activity was measured by EnVision™ Xcite (PerkinElmer, Inc.). The results are shown in Figure 4.

As a result, the CD137-CR1-copGFP Jurkat cells were activated in a manner dependent on the antibody concentration of the bispecific antibody. On the other hand, the NFAT-RE-luc2 Jurkat cells expressing only copGFP without expressing a chimeric receptor were not activated even in the presence of the bispecific antibody. This indicates that CD137-CR1-copGFP-T cells are activated in a manner dependent on a bispecific antibody, and express cytotoxic activity on cells expressing GPC3 as a tumor antigen to induce an antitumor effect.

(3-5) Evaluation of ability to activate CD137-CR1-copGFP Jurkat cells when target antigen is GPRC5D.

CD137-CR1-copGFP Jurkat cells prepared in Example 3-2 were co-cultured with tumor cells expressing a tumor antigen, and the ability to activate Jurkat cells in the presence of a bispecific antibody consisting of an antibody to a tumor antigen and an anti-CD137 antibody was analyzed. Here, human GPRC5D expressed on blood cancer was selected as a model tumor antigen, a bispecific antibody consisting of an anti-GPRC5D antibody and an anti-CD137 antibody and prepared in Example 2-2 (GPA0018H-F760mnN17/GPA0018L-k0C//hCD137VH-F760mnP17/hCD137VL-k0, or GPA0039H-F760mnN17/GPA0039L-k0C//hCD137VH-F760mnP17/hCD137VL-k0) was used in the experiment.

For the tumor cell line expressing human GPRC5D, human plasmacytoma-derived NCI-H929 (ATCC) was used. First, NCI-H929 cells were inoculated at 10 µL/well in a 384-well flat-bottom plate (Corning, Inc.) (1E + 04 (1 ×

10$^4$) cells/well). Subsequently, CD137-CR1-copGFP Jurkat cells, or NFAT-RE-luc2 Jurkat cells expressing only copGFP without expressing a chimeric receptor as a negative control were mixed at 5 µL/well (1E + 04 (1 × 10$^4$) cells/well). Subsequently, the bispecific antibody was added at 5 µL/well with a final concentration of 0 or 10 µg/mL. After 24 hours, the luciferase activity was measured using Bio-Glo Luciferase (Promega Corporation). The luciferase activity was measured by EnVision™ Xcite (PerkinElmer, Inc.). The results are shown in Figure 5.

As a result, the CD137-CR1-copGFP Jurkat cells were activated only in the presence of the bispecific antibody. On the other hand, the NFAT-RE-luc2 Jurkat cells expressing only copGFP without expressing a chimeric receptor were not activated even in the presence of the bispecific antibody. This indicates that CD137-CR1-copGFP-T cells are activated in a manner dependent on a bispecific antibody, and express cytotoxic activity on cells expressing GPRC5D as a tumor antigen to induce an antitumor effect.

(3-6) Evaluation of ability to activate CD137-CR1-copGFP Jurkat cells when target antigen is IL-6R

CD137-CR1-copGFP Jurkat cells prepared in Example 3-2 were co-cultured with target cells expressing a target antigen, and the ability to activate Jurkat cells in the presence of a bispecific antibody consisting of an antibody to a target antigen and an anti-CD137 antibody was analyzed. Here, human IL-6R was selected as a model target antigen, a bispecific antibody consisting of an anti-IL-6R antibody and an anti-CD137 antibody and prepared in Example 2-2 (MRAH.v1-F760mnN17/MRAL.v1-k0.v1//hCD137VH-F760mnP17/hCD137VL-k0) was used in the experiment.

For the target cell line expressing human IL-6R, Z8AGBA-01-C26-CN-006 cells were used in which the full length of human IL-6R was stably expressed on CHO cells (IL-6R-CHO cells). First, IL-6R-CHO cells were inoculated at 10 µL/well in a 384-well flat-bottom plate (Corning, Inc.) (1E + 04 (1 × 10$^4$) cells/well). Subsequently, CD137-CR1-copGFP Jurkat cells, or NFAT-RE-luc2 Jurkat cells expressing only copGFP without expressing a chimeric receptor as a negative control were mixed at 5 µL/well (1E + 04 (1 × 10$^4$) cells/well). Subsequently, the bispecific antibody was added at 5 µL/well with a final concentration of 0 or 10 µg/m. After 24 hours, the luciferase activity was measured using Bio-Glo™ Luciferase (Promega Corporation). The luciferase activity was measured by EnVision™ Xcite (PerkinElmer, Inc.). The results are shown in Figure 6.

As a result, the CD137-CR1-copGFP Jurkat cells were activated only in the presence of the bispecific antibody. On the other hand, the NFAT-RE-luc2 Jurkat cells expressing only copGFP without expressing a chimeric receptor were not activated even in the presence of the bispecific antibody. This indicates that CD137-CR1-copGFP-T cells are activated in a manner dependent on a bispecific antibody, and express cytotoxic activity on cells expressing IL-6R as a target antigen.

[Example 4] Evaluation of *in vitro* cytotoxic activity of chimeric receptor-expressing cells having CD137 in extracellular region

(4-1) Construction of retrovirus vector

A retrovirus vector for a chimeric receptor was prepared for the expression of CD137-CR1. pMSGV1 (Tamada k et al., Clin Cancer Res 18:6436-6445 (2012)) was used as a retrovirus vector skeleton. Figure 7 is a schematic view showing the vector construct and the order of arrangement of components in frame units from the 5' end to the 3' end. F2A peptide (derived from foot and mouth disease virus) and an eGFP molecule (nucleotides 5521 to 6237bp, GenBankKF957646.1) were linked to CD137-CR1. The gene was synthesized by a method known to those skilled in the art, and ligated into pMSGV1 to generate CD137-CR1-eGFP retrovirus vector.

(4-2) Preparation of CD 13 7 -CR1-eGFP-expressing T cells

The retrovirus vector constructed in Example 4-1 was used in the retroviral transduction method of human T cells to prepare genetically altered T cells expressing CD137-CR1-eGFP.

Specifically, first, GP2-293 packaging cell line (Takara Bio Inc.) was transfected with the CD137-CR1-eGFP vector mentioned above and pAmpho plasmid (Takara Bio Inc.) using Lipofectamine (R) 2000 or 3000 (Thermo Fisher Scientific) to prepare retrovirus harboring the CD137-CR1-eGFP vector. 48 hours after the transfection, a supernatant containing the retrovirus was recovered and adsorbed onto two 24-well plates to prepare plates for transductions.

Subsequently, for the transduction of human T cells, 2E + 06 human peripheral blood mononuclear cells per well were cultured for 72 hours in the presence of IL-2 on a 6-well plate with an anti-CD3 monoclonal antibody and RetroNectin(R) (Takara Bio Inc.) immobilized thereon. The cells thus cultured were recovered, and cultured overnight on one of the plates for transductions with the CD 13 7 -CR1-eGFP retrovirus adsorbed thereon, which were prepared as mentioned above, in the presence of IL-2. On the next day, the cells were transferred to the other plate for transduction and further cultured overnight to obtain human T cells harboring the CD137-CR1-eGFP vector (CD137-CR1-eGFP-expressing T cells).

(4-3) Confirmation of CR protein expression rate

The cells thus transduced were maintained in the presence of human IL-2 and used in an experiment 7 to 8 days after the start of culture of the peripheral blood mononuclear cells. The surface expression of the CD137-CR1-eGFP on the transduced human T cells was determined by the staining of the cells with the anti-CD3 antibody, the anti-CD8 antibody and the anti-CD137 antibody, followed by flow cytometry. 80% on average of all T cells expressed the CD137-CR1-eGFP.

(4-4) Evaluation of cytotoxic activity of CD137-CR1-eGFP-expressing T cells using number of residual tumor cells as index

The cytotoxic activity of CD137-CR1-eGFP-expressing T cells prepared in Example 4-2 was evaluated using CytoFLEX (Beckman Coulter Inc.). SK-Hep1 was provided as a negative control, and SK-pca60 obtained by allowing SK-Hep1 to stably express human GPC3 was provided as target cells. The negative control and the target cells were inoculated at 1E + 05 ($1 \times 10^5$) cells in a 6-well plate. CD137-CR1-eGFP-expressing cells were used as effector cells and mixed at 1E + 05 cells so as to attain an effector cell:target cell (E:T) ratio of 1:1. Next, a bispecific antibody consisting of an anti-GPC3 antibody and an anti-CD137 antibody and prepared in Example 2-2 (H0000-F760nN17/GL4-k0a//hCD137VH-F760mnP17/hCD137VL-k0) was added at 10 $\mu$g per well. In addition, as a negative control, a bispecific antibody with the first or second arm substituted by an anti-Keyhole limpet hemocyanin (KLH) antibody (IC17HdK-F760mnN17/IC17L-k0//hCD137VH-F760mnP17/hCD137VL-k0 or IC17HdK-F760mnN17/IC17L-k0//H0000-F760mnP17/GL4-k0a) was added at 10 $\mu$g per well. 48 hours after the addition, the CD137-CR1-eGFP-expressing T cells and the target cells were recovered. The recovered cells were applied to Zombie Aqua™ Fixable Viability Kit (BioLegend, Inc., 423104) to stain dead cells, and the T cells were stained with an anti-human CD45 antibody (BioLegend, Inc., 304039).

The cytotoxic activity was evaluated by the number of residual cancer cells. The number of residual cancer cells was calculated from the number of cells after the co-culture and the percentage of cells CD45-negative fractions in living cells.

The results are shown in Figure 8A. The number of residual tumor cells significantly decreased only when the target cell was SK-pca60 and a bispecific antibody consisting of an anti-GPC3 antibody and an anti-CD137 antibody was added.

This result demonstrated the added antibody-dependent cytotoxic activity of CD137-CR1-eGFP-expressing T cells on the target cells *in vitro*.

(4-5) Evaluation of cytotoxic activity of CD137-CR1-eGFP-expressing T cells using xCELLigence system

The cytotoxic activity of CD137-CR1-eGFP-expressing T cells prepared in Example 4-2 was also evaluated using xCELLigence (Aligent Technologies). SK-Hep1 was provided as a negative control, and SK-pca60 obtained by allowing SK-Hep1 to stably express human GPC3 was provided as target cells. The negative control and the target cells were inoculated at 1E + 04 ($1 \times 10^4$) cells in E-Plate 96 on the previous day. On the following day, CD137-CR1-eGFP-expressing cells were used as effector cells and mixed at 1E + 04 ($1 \times 10^4$) and 3E + 04 ($3 \times 10^4$) cells so as to attain an effector cell:target cell (E:T) ratio of 1:1 and 1:3. Next, a bispecific antibody consisting of an anti-GPC3 antibody and an anti-CD137 antibody and prepared in Example 2-2 (H0000-F760nN17/GL4-k0a//hCD137VH-F760mnP17/hCD137VL-k0) was added to a final concentration of 10 $\mu$g/mL per well. The cytotoxic activity of CD137-CR1-eGFP-expressing cells is monitored in real time by measuring the electric resistance over time.

The cell-growth inhibitory activity was calculated in accordance with the following expression: cell-growth inhibitory activity (%) = 100x(A-B)/(A-1) (A: average value of delta cell indices for wells with no antibody, B: delta cell index for specimen). The results are shown in Figure 8B. The cell-growth inhibitory activity was significantly exhibited only when the target cell was SK-pca60 and a bispecific antibody consisting of an anti-GPC3 antibody and an anti-CD137 antibody was added.

This result demonstrated the added antibody-dependent cytotoxic activity of CD137-CR1-eGFP-expressing T cells on the target cells *in vitro*.

[Example 5] Chimeric receptor having immunoreceptor having attenuated ability to bind to ligand in extracellular domain

Immunocytes in which a chimeric receptor having the extracellular full-length of an immunoreceptor is transformed can be activated in a target antigen-independent manner by binding to a ligand directed to the immunoreceptor (Figure 9). This is a problem because non-target cells are damaged. This problem was considered to be solved by artificially altering the immunoreceptor in the extracellular domain to attenuate the ability to bind to the ligand.

[Example 6] Evaluation of ability to activate chimeric receptor-expressing cells having CD137 portion in extracellular domain

(6-1) Construction of lentivirus vector expressing trCD137-CR

The extracellular domain of human CD137 comprises cysteine-rich domains (CRDs) 1 to 4, and CRD2 and CRD3 are known to play an important role in binding to CD137L that is a ligand. Thus, as altered CR having attenuated binding to CD137L, trCD137-CR was prepared in which CRD3 and CRD4 of the human CD 137 extracellular domain were deleted (SEQ ID NO: 25). Specifically, a hinge region and a transmembrane region of a human CD8 alpha-chain (Genbank NM001768.7, Phe128 to Asn210), and cytoplasmic regions of a human CD28 molecule (Genbank NM006139.4, Arg180 to Ser220), a human CD137 molecule (Genbank NM001561.6, Arg209 to Leu255) and a human CD3 zeta molecule (Genbank NM000734.4, Arg52 to Arg164) were linked to CRD1 and CRD2 of a codon-optimized human CD137 extracellular domain (Genbank NM001561.6, Leu24 to Asp87) (Figure 2). A gene encoding trCD137-CR was synthesized by a method known to those skilled in the art. This sequence was ligated into MCS of the pCDH-CMV-MCS-T2A-copGFP vector to construct a lentivirus vector coexpressing trCD137-CR and copGFP.

(6-2) Introduction of gene into NFAT-RE-luc2 Jurkat cells

NFAT-RE-luc2 Jurkat cells (Promega Corporation) were transduced with a trCD137-CR vector for analyzing the ability to activate trCD137-CR-T by an *in vitro* reporter assay.
The transduction was performed by a lentivirus method using the lentivirus vector constructed in Example 6-1. Specifically, first, 293Ta cell line (Genecopoeia) was transfected with the trCD137-CR vector mentioned above and a packaging plasmid mix by Lenti-Pac™ HIV Expression Packaging Kit (Genecopoeia) to prepare lentivirus harboring the trCD137-CR-copGFP vector. 48 hours to 72 hours after the transfection, a supernatant containing the lentivirus was recovered, and concentrated using a Lenti-X™ concentrator (Takara Bio Inc.).
Next, the recovered supernatant containing lentivirus and a polybrene solution (Nacalai Tesque, Inc.) were added to NFAT-RE-luc2 Jurkat cells, and the mixture was centrifuged at 2000 rpm for 60 minutes to obtain NFAT-RE-luc2 Jurkat cells transduced with the trCD137-CR-copGFP vector (trCD137-CR-copGFP Jurkat cells). As a negative control for the experiment, NFAT-RE-luc2 Jurkat cells transduced with a pCDH-CMV-MCS-T2A-copGFP vector which does not ligate trCD137-CR and expresses only copGFP were also prepared.

(6-3) Confirmation of CR protein expression rate

The expression of trCD137-CR on the surfaces of the trCD137-CR-copGFP Jurkat cells prepared in Example 6-2 was analyzed by BD FACSAria™ III Cell Sorter (BD Biosciences) with CD137 antibody (BioLegend) used for staining. The epitope used for the analysis is known to be mainly CRD2 of CD137. As a result of the analysis, approximately 60% of the living cells were confirmed to express trCD137-CR.

(6-4) Evaluation of ability to activate trCD137-CR-copGFP Jurkat cells by reporter assay

trCD137-CR-copGFP Jurkat cells prepared in Example 5-2 were co-cultured with tumor cells expressing a tumor antigen, and the ability to activate Jurkat cells in the presence of a bispecific antibody consisting of an antibody to a tumor antigen and an anti-CD 137 antibody was analyzed. Here, human GPC3 was selected as a model tumor antigen, a bispecific antibody consisting of an anti-GPC3 antibody and an anti-CD137 antibody and prepared in Example 2-2 (H0000-F760nN17/GL4-k0a//hCD137VH-F760mnP17/hCD137VL-k0) was used in the experiment.
For the tumor cell line expressing human GPC3, SK-pca60 cells were used in which human GPC3 was stably expressed on SK-HEP1 (ATCC) that is a human liver cancer cell line. First, SK-pca60 cells were inoculated at 10 μL/well in a 384-well flat-bottom plate (Corning, Inc.) (1E + 04 ($1 \times 10^4$) cells/well). Subsequently, trCD137-CR-copGFP Jurkat cells, or NFAT-RE-luc2 Jurkat cells expressing only copGFP without expressing a chimeric receptor as a negative control were mixed at 5 μL/well (1E + 04 ($1 \times 10^4$) cells/well). Subsequently, the bispecific antibody prepared at each concentration was added at 5 μL/well (final concentration: 0, 16, 160, 1600, 16000 ng/mL). After 24 hours, the luciferase activity was measured using Bio-Glo Luciferase (Promega Corporation). The luciferase activity was measured by EnVision™ Xcite (PerkinElmer, Inc.). The results are shown in Figure 10.
As a result, the trCD137-CR-copGFP Jurkat cells were activated in a manner dependent on the antibody concentration of the bispecific antibody. On the other hand, the NFAT-RE-luc2 Jurkat cells expressing only copGFP without expressing a chimeric receptor were not activated even in the presence of the bispecific antibody. This indicates that trCD137-CR-copGFP-T cells are activated in a manner dependent on a bispecific antibody, and express cytotoxic activity on cells expressing a tumor antigen to induce an antitumor effect.

[Example 7] Evaluation of ability to activate chimeric receptor-expressing cells having CD137 variant in extracellular domain

(7-1) Construction of lentivirus vector

Mutants obtained by mutation of I64 or V71 on CRD2 of human CD137 into Arg are known to have attenuated binding to CD137L. Thus, as altered CR having attenuated binding to CD137L, CD137-CR2 (SEQ ID NO: 26) was prepared in which a hinge region and a transmembrane region of a human CD8 alpha-chain (Genbank NM001768.7, Phe128 to Asn210), and cytoplasmic regions of a human CD28 molecule (Genbank NM006139.4, Arg180 to Ser220), a human CD137 molecule (Genbank NM001561.6, Arg209 to Leu255) and a human CD3 zeta molecule (Genbank NM000734.4, Arg52 to Arg164) were linked to an extracellular domain of altered human CD137 obtained by mutation of I64 and V71 into Arg (Figure 2). A gene encoding CD137-CR2 was synthesized by a method known to those skilled in the art. This sequence was ligated into MCS of the pCDH-CMV-MCS-T2A-copGFP vector to construct a lentivirus vector coexpressing CD137-CR2 and copGFP.

(7-2) Introduction of gene into NFAT-RE-luc2 Jurkat cells

NFAT-RE-luc2 Jurkat cells (Promega Corporation) were transduced with a CD137-CR2 vector for analyzing the ability to activate CD137-CR2-T by an *in vitro* reporter assay.
The transduction was performed by a lentivirus method using the lentivirus vector constructed in Example 7-1. Specifically, first, 293Ta cell line (Genecopoeia) was transfected with the CD137-CR2 vector mentioned above and a packaging plasmid mix by Lenti-Pac™ HIV Expression Packaging Kit (Genecopoeia) to prepare lentivirus harboring the CD137-CR2-copGFP vector. 48 hours to 72 hours after the transfection, a supernatant containing the lentivirus was recovered, and concentrated using a Lenti-X™ concentrator (Takara Bio Inc.).
Next, the recovered supernatant containing lentivirus and a polybrene solution (Nacalai Tesque, Inc.) were added to NFAT-RE-luc2 Jurkat cells, and the mixture was centrifuged at 2000 rpm for 60 minutes to obtain NFAT-RE-luc2 Jurkat cells transduced with the CD137-CR2-copGFP vector (CD137-CR2-copGFP Jurkat cells). As a negative control for the experiment, NFAT-RE-luc2 Jurkat cells transduced with a pCDH-CMV-MCS-T2A-copGFP vector which does not ligate CD137-CR2 and expresses only copGFP were also prepared.

(7-3) Confirmation of CR protein expression rate

The expression of CD137-CR2 on the surfaces of the CD137-CR2-copGFP Jurkat cells prepared in Example 7-2 was analyzed by FACSAria™ III Cell Sorter (BD Biosciences) with CD137 antibody (BioLegend) used for staining. As a result, 95% or more of the living cells were confirmed to express CD137-CR2.

(7-4) Evaluation of ability to activate CD137-CR2-copGFP Jurkat cells by reporter assay

The CD137-CR2-copGFP Jurkat cells prepared in Example 7-2 were co-cultured with tumor cells expressing a tumor antigen, and the ability to activate Jurkat cells in the presence of a bispecific antibody consisting of an antibody to a tumor antigen and an anti-CD137 antibody was analyzed. Here, human GPC3 was selected as a model tumor antigen, a bispecific antibody consisting of an anti-GPC3 antibody and an anti-CD137 antibody and prepared in Example 2-2 (H0000-F760nN17/GL4-k0a//hCD137VH-F760mnP17/hCD137VL-k0) was used in the experiment.
For the tumor cell line expressing human GPC3, SK-pca60 cells were used in which human GPC3 was stably expressed on SK-HEP1 (ATCC) that is a human liver cancer cell line. First, SK-pca60 cells were inoculated at 10 μL/well in a 384-well flat-bottom plate (Corning, Inc.) (1E + 04 ($1 \times 10^4$) cells/well). Subsequently, CD137-CR2-copGFP Jurkat cells, or NFAT-RE-luc2 Jurkat cells expressing only copGFP without expressing a chimeric receptor as a negative control were mixed at 5 μL/well (1E + 04 ($1 \times 10^4$) cells/well). Subsequently, the bispecific antibody prepared at each concentration was added at 5 μL/well (final concentration: 0, 16, 160, 1600, 16000 ng/mL). After 24 hours, the luciferase activity was measured using Bio-Glo™ Luciferase (Promega Corporation). The luciferase activity was measured by EnVision™ Xcite (PerkinElmer, Inc.). The results are shown in Figure 11.
As a result, the CD137-CR2-copGFP Jurkat cells were activated in a manner dependent on the antibody concentration of the bispecific antibody. On the other hand, the NFAT-RE-luc2 Jurkat cells expressing only copGFP without expressing a chimeric receptor were not activated even in the presence of the bispecific antibody. This indicates that CD137-CR2-copGFP-T cells are activated in a manner dependent on a bispecific antibody, and express cytotoxic activity on cells expressing a tumor antigen to induce an antitumor effect.

[Example 8] Evaluation of ability to activate chimeric receptor-expressing cells in the presence of ligand-expressing cells.

Whether or not a chimeric receptor having an immunoreceptor in an extracellular domain was activated by binding to a ligand directed to the immunoreceptor, regardless of the presence or absence of a target antigen, was examined. Specifically, the CD137-CR1-copGFP Jurkat cells prepared in Example 3-2, the trCD137-CR-copGFP Jurkat cells prepared in Example 6-2, or the CD137-CR2-copGFP Jurkat cells prepared in Example 7-2 were co-cultured with cells expressing human CD137L, and whether or not Jurkat cells were activated was analyzed.

For the cell line expressing human CD137L, Raji cells (ATCC) derived from human B-cell lymphoma were used. First, a Raji cell suspension, or a medium free of Raji cells as a negative control was added at 10 $\mu$L/well in a 384-well flat-bottom plate (Corning, Inc.) (1E + 04 ($1 \times 10^4$) cells/well). Subsequently, CD137-CR1-copGFP Jurkat cells, trCD137-CR-copGFP Jurkat cells, or CD137-CR2-copGFP Jurkat cells were mixed at 5 $\mu$L/well (1E + 04 ($1 \times 10^4$) cells/well). After 24 hours, the luciferase activity was measured using Bio-Glo Luciferase (Promega Corporation). The luciferase activity was measured by EnVision™ Xcite (PerkinElmer, Inc.). The results are shown in Figures 12A to 12C.

As a result, the CD137-CR1-copGFP Jurkat cells were activated when the cells were co-cultured with Raji cells. On the other hand, the CD137-CR1-copGFP Jurkat cells were not activated when the cells were not co-cultured with Raji cells. The CD137-CR1-copGFP Jurkat cells were considered to have been activated in a target antigen-independent manner by direct binding to CD137L expressed on the Raji cells. On the other hand, trCD137-CR-copGFP Jurkat cells or CD137-CR2-copGFP Jurkat cells having attenuated binding to CD137L were not activated regardless of whether the cells were co-cultured with Raji cells. Thus, it was indicated that attenuation of binding to CD137L enabled suppression of target antigen-independent activation by binding to a ligand.

[Example 9] Optimization of CD137 variant having attenuated ability to bind to ligand.

As described in Example 5, immunocytes in which a chimeric receptor having the full-length of an extracellular domain of an immunoreceptor (CD137) is transformed can be activated in a target antigen-independent manner by binding to an *in vivo* ligand (CD137L) directed to the immunoreceptor, and thus may cause target antigen-non-specific cytotoxicity. This problem was considered to be solved by artificially altering the immunoreceptor in the extracellular domain to attenuate binding to the *in vivo* ligand. As one method thereof, a part of the extracellular domain is deleted as described in Example 6. Another method may be reduction of *in vivo* ligand binding activity by applying a gene mutation to a site where the ligand and the extracellular domain interact with each other. Thus, studies were conducted on a single mutation and a combination of mutations for the purpose of identifying an alteration that reduces *in vivo* ligand binding activity.

(9-1) Identification of site of interaction with *in vivo* ligand by alanine-scanning

CRD2 and CRD3 are known to play an important role as the site of interaction with CD137L. For the purpose of finding which residues directly interact or have a significant effect on the interaction, alanine-scanning mutagenesis was carried out for all the residues of CRD2 and CRD3 (C48 to K118) and some of the residues of CRD1 (N40 to P47). Specifically, first, Factor 10 cleavage sequence (FX, SEQ ID NO: 37), a portion of a human IgG1 antibody constant region (SEQ ID NO: 38) and a tag with biotin (BAP tag, SEQ ID NO: 39) were linked to a codon-optimized human CD137 extracellular domain (BB0000, SEQ ID NO: 36), followed by introduction into a mammalian expression vector (BB0000-FXFcBAP). Using this vector as a template, variants were prepared in which the residues of CRD1, CRD2 and CRD3 were each substituted by alanine (BB0001 to BB0076, SEQ ID NOS: 40 to 115).

A plasmid having a gene encoding the sequence of the prepared variant was introduced into a human embryonic kidney cell-derived Expi 293 line (Invitrogen Corp.) by lipofection. The supernatant after culture for 4 days was purified by a method known to those skilled in the art using rProtein A Sepharose(R) Fast Flow (Amersham Biosciences Ltd.) to obtain a CD137 variant of interest. The absorbance of the purified variant solution was measured at 280 nm using a spectrophotometer. The concentration of the purified antibody was calculated using an absorption coefficient calculated by PACE from the obtained measurement value (Protein Science 1995; 4: 2411-2423).

(9-2) Evaluation of binding of CD137 variant to *in vivo* ligand

Immunocytes in which a chimeric receptor having a CD137 extracellular domain or a variant thereof are required to have attenuated binding to an *in vivo* ligand for the sake of the safety of the immunocytes. Thus, the interaction of the prepared variant with the *in vivo* ligand (CD137) was analyzed.

(9-2-1) Preparation of CD137L as antigen

CD137L with a combination of heavy chain and light chain plasmids shown in Table 3 were expressed and purified by a method known to those skilled in the art, and was then cleaved by treatment with Lys-C (Roche, Ltd.) at 37°C for 1 hour. Each portion was made to pass through an affinity column of MabSelect SuRe (Cytiva), and non-bound components were subjected to gel permeation chromatography. As a result, CD137L was sorted out.

[Table 3]

Table 3: Preparation of CD137L

|  | Heavy chain 1 | Light chain 1 | Heavy chain 2 | Light chain 2 |
|---|---|---|---|---|
| CD137L | ss4G8VH-4B9CH SEQ ID NO: 116 | ss28H1VL-4B9CL SEQ ID NO: 117 | ssDimerichCD137L-CLFcknob SEQ ID NO: 118 | ssMonohCD137L-CH1 SEQ ID NO: 119 |

(9-2-2) Analysis of interaction using Biacore

Interaction between the CD137 variant prepared in Example 9-1 and CD137L was analyzed by the following method using Biacore™ T200 (Cytiva). The running buffers used were 20 mM ACES, 150 mM NaCl and 0.05% Tween 20 (registered trademark, pH 7.4), and the measurement was performed at 25°C. The sensor chip used was a chip with anti-Human IgG (Fc) antibody (Cytiva) immobilized on Series S CM4 (Cytiva). The CD137 variant of interest was captured to the chip, and CD137L diluted with the running buffer was provided as a binding molecule. The chip was regenerated using 3 M $MgCl_2$, and the antibody was repeatedly captured to perform the measurement. The dissociation constant KD (mol/L) of each variant with respect to the anti-CD137 antibody portion was calculated on the basis of the 1 : 1 binding model using Biacore™ T200 Evaluation Software Version 2.0.

As a result of the alanine scanning, N42, Q43 and I44 of CRD1, C48, P50, F53, D63, I64 and D87 of CRD2, and L95 and M101 of CRD3 were found as positions where the binding to the *in vivo* ligand was reduced to 90% or less of that in the wild type and it was possible to maintain the expression level at 50% or more of that in the wild type.

(9-2-3)

An alteration was searched which enabled the binding to CD137L to be reduced by wholly introducing a 1 amino acid alteration at the position M101 among the positions found in Example 9-2-2.

The CD137L binding activity of the prepared variant was evaluated by a method identical to that in the analysis of interaction described in Example 9-2-2.

As a result, an alteration M101D (BB0139: SEQ ID NO: 120) was newly found as an alteration capable of reducing CD137L binding.

(9-3) Preparation of variant reducing interaction with *in vivo* ligand

As described in Example 7, I64 and V71 on CRD2 of human CD137 are alterations capable of reducing the binding to CD137L. Thus, a variant with both I64 and V71 altered into Arg (BB0077, SEQ ID NO: 121) was prepared as a variant that is expected to be capable of further reducing interaction with an *in vivo* ligand. Here, BB0000-FXFcBAP was used as a template.

(9-3-1) Measurement of *in vivo* ligand binding activity of BB0000 and BB0077

The binding of BB0077 to an *in vivo* ligand was measured by the method described in Example 9-2-2. The binding was determined as a ratio obtained by dividing the amount of CD137 bound (RU: response unit) by the amount of the CD137 variant bound (RU). As a result, it was confirmed that BB0077 did not bind to the *in vivo* ligand (Figure 13).

(9-3-2) Evaluation of ECM binding of BB0000 and BB0077

When the prepared variant has non-specific binding activity against mammalian cells, cytotoxic activity may be exhibited to the cells in an antigen-non-specific manner. Thus, the binding activity against Extracellular Matrix (ECM) which is known as a system for evaluating the degree of non-specific binding of each variant *in vitro* was evaluated. In this assay system, the non-specific binding of BB0000 and BB0077 was evaluated (U.S.2014/0080153 A1). As a result, BB0077 was confirmed to very intensely bind to ECM, and shown to possibly cause a reaction in a non-specific manner *in vivo* (Figure 14).

(9-3-3) Evaluation of ECM binding of CD137 variant

As shown in Example 9-2-2, BB0077 did not bind to an *in vivo* ligand, but exhibited non-specific binding, and therefore was considered to raise a safety concern. Thus, effort was made to prepare a CD137 variant enabling suppression of both *in vivo* ligand binding and non-specific binding.

As a cause of non-specific binding, attention was given to the high isoelectric point of arginine. On the assumption that positive charge of arginine is attracted to negative charge induced on cell membrane surfaces or ECM, resulting in occurrence of non-specific binding, whether it was possible to reduce non-specific binding by alteration into aspartic acid, glutamine or glutamic acid as a bulky amino acid which has no positive charge and is capable of inhibiting binding to a ligand. Specifically, a variant with aspartic acid, glutamine or glutamic acid introduced into one or both of I64 and V71 mutated in BB0077 was prepared. The list of the names of the prepared variants, the alterations introduced into BB0000, and the sequence numbers is as shown in Table 4.

[Table 4]

Table 4: Prepared variant

| Name | Alteration | SEQ ID NO: |
|---|---|---|
| BB0124 | I64E | 122 |
| BB0125 | I64Q | 123 |
| BB0126 | I64D | 124 |
| BB0127 | V71E | 125 |
| BB0128 | V71Q | 126 |
| BB0129 | V71D | 127 |
| BB0130 | I64E/V71E | 128 |
| BB0131 | I64E/V71Q | 129 |
| BB0132 | I64E/V71D | 130 |
| BB0133 | I64Q/V71E | 131 |
| BB0134 | I64Q/V71Q | 132 |
| BB0135 | I64Q/V71D | 133 |
| BB0136 | I64D/V71E | 134 |
| BB0137 | I64D/V71Q | 135 |
| BB0138 | I64D/V71D | 136 |

(9-3-4) Evaluation of *in vivo* ligand binding activity of newly found variant

The *in vivo* ligand binding activity of the BB0124 to BB0138 variants prepared in Example 9-3-3 was evaluated by the same method as that described in Example 9-2-2. The binding was determined as a ratio obtained by dividing the amount of CD137 bound (RU: response unit) by the amount of the CD137 variant bound (RU). The result showed that the BB0127 to BB 138 variants significantly reduced the *in vivo* ligand binding. (Figure 15)

(9-3-5) Evaluation of ECM binding of variants

The non-specific binding of the variants found in Examples 9-2-3 and 9-3-4 was evaluated by the method described in Example 9-3-2. As a result, the variants were confirmed to exhibit further reduced non-specific binding as compared to the wild type (BB0000) (Figures 16A and 16B).

As is apparent from the above results, CD137 variants were newly found which do not bind to *in vivo* CD137L and exhibit reduced non-specific binding. It was expected that use of the variant as an extracellular region of a chimeric receptor would enable preparation of a chimeric receptor having less target-non-specific cytotoxicity.

[Example 10] Evaluation of ability to activate chimeric receptor-expressing cells having CD137 variant having an attenuated ability to bind to a ligand in extracellular domain

(10-1) Construction of lentivirus vector

Chimeric receptors (SEQ ID NOS: 27 to 32) were prepared in which among the CD137 variants having attenuated binding to CD137L, which had been constructed in Example 9, six variants BB0127, BB0128, BB0131, BB0133, BB0134 and BB0139 were extracellular domains, and a hinge region and a transmembrane region of a human CD8 alpha-chain (Genbank NM001768.7, Phe128 to Asn210), and cytoplasmic regions of a human CD28 molecule (GenbankNM006139.4, Arg180 to Ser220), a human CD137 molecule (Genbank NM001561.6, Arg209 to Leu255) and a human CD3 zeta molecule (Genbank NM000734.4, Arg52 to Arg164) were linked. These sequences were ligated into MCS of the pCDH-CMV-MCS-T2A-copGFP vector to construct a lentivirus vector coexpressing the chimeric receptor and copGFP.

(10-2) Introduction of gene into NFAT-RE-luc2 Jurkat cells

NFAT-RE-luc2 Jurkat cells (Promega Corporation) were transduced for analyzing the ability to activate the prepared chimeric receptor by an *in vitro* reporter assay.

The transduction was performed by a lentivirus method using the lentivirus vector constructed in Example 10-1. Specifically, first, 293Ta cell line (Genecopoeia) was transfected with the lentivirus vector and a packaging plasmid mix by Lenti-Pac™ HIV Expression Packaging Kit (Genecopoeia) to prepare lentivirus. 48 hours to 72 hours after the transfection, a supernatant containing the lentivirus was recovered.

Next, the recovered supernatant containing lentivirus and a polybrene solution (Nacalai Tesque, Inc.) were added to NFAT-RE-luc2 Jurkat cells, and the mixture was centrifuged at 3000 rpm for 60 minutes to obtain NFAT-RE-luc2 Jurkat cells.

(10-3) Confirmation of CR protein expression rate

The expression of the chimeric receptor on the surfaces of the Jurkat cells prepared in Example 10-2 was analyzed by BD FACSVerse™ (BD Biosciences) with CD137 antibody (BioLegend) used for staining. As a result, approximately 50% or more of the living cells were confirmed to express the chimeric receptor.

(10-4) Evaluation of activation ability by reporter assay

The chimeric receptor-expressing Jurkat cells prepared in Example 10-2 were co-cultured with tumor cells expressing a tumor antigen, and the ability to activate Jurkat cells in the presence of a bispecific antibody consisting of an antibody to a tumor antigen and an anti-CD137 antibody was analyzed. Here, human GPC3 was selected as a model tumor antigen, the bispecific antibody consisting of an anti-GPC3 antibody and an anti-CD137 antibody and prepared in Example 2-2 (H0000-F760nN17/GL4-k0a//hCD137VH-F760mnP17/hCD137VL-k0) was used in the experiment.

For the tumor cell line expressing human GPC3, SK-pca60 cells were used in which human GPC3 was stably expressed on SK-HEP1 (ATCC) that is a human liver cancer cell line. First, SK-pca60 cells were inoculated at 5 μL/well in a 384-well flat-bottom plate (Corning, Inc.) (1E + 04 ($1 \times 10^4$) cells/well). Subsequently, the chimeric receptor-expressing Jurkat cells prepared in Example 10-2, the CD137-CR1-copGFP Jurkat cells (the extracellular domain is BB0000) prepared in Example 3-2 or the CD137-CR2-copGFP Jurkat cells (the extracellular domain is BB0077) prepared in Example 7-2 as a positive control, and a parent strain of NFAT-R-luc2 Jurkat cells that were not subjected to gene introduction (Mock) as a negative control were mixed at 10 μL/well (1E + 04 ($1 \times 10^4$) cells/well) for each of the types of cells. Subsequently, the bispecific antibody was added at 5 μL/well with a final concentration of 0 or 10 μg/mL. After 24 hours, the luciferase activity was measured using Bio-Glo Luciferase (Promega Corporation). The luciferase activity was measured by EnVision™ Xcite (PerkinElmer, Inc.). The results are shown in Figure 17.

As a result, Jurkat cells expressing a chimeric receptor were activated only in the presence of the bispecific antibody. This indicates that T cells expressing a chimeric receptor having a CD137 variant in an extracellular domain are activated in a manner dependent on a bispecific antibody, and express cytotoxic activity on cells expressing a tumor antigen to induce an antitumor effect.

(10-5) Evaluation of ability to activate chimeric receptor-expressing cells in the presence of ligand-expressing cells.

Whether or not the chimeric receptor-expressing Jurkat cells prepared in Example 10-2 were activated by binding to a CD137 ligand, regardless of the presence or absence of a target antigen, was examined. Specifically, the chimeric receptor-expressing Jurkat cells prepared in Example 10-2 were co-cultured with cells expressing human CD 137L, and whether or not the Jurkat cells were activated was analyzed.

For the cell line expressing human CD137L, Raji cells (ATCC) derived from human B-cell lymphoma were used. First, a Raji cell suspension, or a medium free of Raji cells as a negative control was added at 5 μL/well in a 384-well flat-bottom plate (Corning, Inc.) (1E + 04 ($1 \times 10^4$) cells/well). Subsequently, the chimeric receptor-expressing Jurkat cells prepared in Example 10-2, the CD137-CR1-copGFP Jurkat cells (the extracellular domain is BB0000) prepared in Example 3-2 as a positive control, and the CD137-CR2-copGFP Jurkat cells (the extracellular domain is BB0077) prepared in Example 7-2 or a parent strain of NFAT-RE-luc2 Jurkat cells that were not subjected to gene introduction (Mock) as a negative control were mixed at 10 μL/well (1E + 04 ($1 \times 10^4$) cells/well) for each of the types of cells. After 24 hours, the luciferase activity was measured using Bio-Glo™ Luciferase (Promega Corporation). The luciferase activity was measured by EnVision™ Xcite (PerkinElmer, Inc.). The results are shown in Figure 18.

As a result, CD137-CR1-copGFP Jurkat cells as a positive control were activated by co-culture with Raji cells, and CD137-CR2-copGFP Jurkat cells as a negative control were not activated. This is consistent with the results of Example 8. It was confirmed that CD137-CR1-copGFP Jurkat cells bound to Raji cells via CD137L and were activated in a target antigen -independent manner, whereas CD137-CR2-copGFP Jurkat cells having attenuated binding to CD137L did not bind to Raji cells via CD137L and were not activated. Next, like CD137-CR2-copGFP Jurkat cells, the Jurkat cells prepared in Example 10-2 and expressing a chimeric receptor having BB0127, BB0128, BB0131, BB0133, BB0134 or BB0139 in an extracellular domain were not activated by co-culture with Raji cells. This indicated that the CD137 variant found in Example 9 enabled suppression of target antigen-independent activation by binding to a ligand.

The results of Examples 9 and 10 show that both non-specific activation caused by interaction with ECM and cytotoxic activity against CD137L-expressing cells can be avoided to selectively exhibit cytotoxic activity against target cells.

[Example 11] Evaluation of agonist action by bispecific antibody

(11-1) Construction of lentivirus vector expressing CD137-CR3 having no CD137 intracellular signal domain

In the system, administration of an antigen-binding molecule having an agonist action may enable agonist activity to be induced via an endogenous immunoreceptor to immunocytes of a host or CR-expressing cells administered in parallel. Thus, whether the antigen-binding molecule having a CD137 agonist action induced CD137 agonist to cells expressing an endogenous immunoreceptor was examined.

For examining whether agonist activity was induced via an endogenous immunoreceptor, cells expressing CR having no CD137 intracellular signal domain was prepared. Specifically, a lentivirus vector expressing chimeric receptor CD137-CD8-CD3 zeta consisting of a human CD137 extracellular domain, a human CD8 hinge, a transmembrane domain, and CD3 zeta (Figure 19, referred to as CD137-CR3). pCDH-CMV-MCS-T2A-copGFP (System Biosciences) was used as a lentivirus vector skeleton. A hinge region and a transmembrane region of a human CD8 alpha-chain (Genbank NM001768.7, Phe128 to Asn210), and a cytoplasmic region of a human CD3 zeta molecule (Genbank NM000734.4, Arg52 to Arg164) were linked to an extracellular domain (Genbank NM001561.6, Leu24 to Gln186) of codon-optimized human CD137. A gene encoding CD137-CR3 (SEQ ID NO: 33) was synthesized by a method known to those skilled in the art. This sequence was ligated into MCS of the pCDH-CMV-MCS-T2A-copGFP vector to construct a lentivirus vector coexpressing CD137-CR3 and copGFP.

(11-2) Introduction of gene into NF-kB-Luc2/4-1BB Jurkat cells

NF-kB-Luc2/4-1BB Jurkat cells (Promega Corporation) were transduced with a CD137-CR3-copGFP vector for analyzing the ability to activate T cells expressing CD137-CR3 by an *in vitro* reporter assay. The cells stably express CD137, have luciferase gene inserted downstream of an element responsive to the transcriptional factor: nuclear factor-kappa B (NF-kB), and enable the activation of the NF-kB pathway to be quantified by detecting the luminescence of luciferase.

The transduction was performed by a lentivirus method using the lentivirus vector constructed in Example 11-1. Specifically, first, 293Ta cell line (Genecopoeia) was transfected with the CD137-CR3-copGFP vector mentioned above and a packaging plasmid mix by Lenti-Pac™ HIV Expression Packaging Kit (Genecopoeia) to prepare lentivirus harboring the CD137-CR3-copGFP. 48 hours to 72 hours after the transfection, a supernatant containing the lentivirus was recovered, and concentrated using a Lenti-X™ concentrator (Takara Bio Inc.).

Next, the recovered supernatant containing lentivirus and a polybrene solution (Nacalai Tesque, Inc.) were added to NF-kB-Luc2/4-1BB Jurkat cells, and the mixture was centrifuged at 3000 rpm for 60 minutes to obtain NF-kB-Luc2/4-1BB Jurkat cells transduced with the CD137-CR3-copGFP vector (CD137-CR3-copGFP Jurkat cells). As comparative controls for the experiment, NF-kB-Luc2/4-1BB Jurkat cells transduced with a CD137-CR1 vector (CD137-CR1 Jurkat cells) and NF-kB-Luc2/4-1BB Jurkat cells expressing only copGFP without expressing a chimeric receptor were prepared in parallel.

(11-3) Confirmation of CR protein expression rate

The expression of a chimeric receptor on the surfaces of the CD137-CR3-copGFP Jurkat cells and the CD137-CR1 Jurkat cells prepared in Example 11-2 was analyzed by FACSAria™ III Cell Sorter (BD Biosciences) with CD137 antibody (BioLegend) used for staining. As a result, approximately 95% or more of the living cells were confirmed to express CD137-CR3 or CD137-CR1.

(11-4) Evaluation of ability to activate CD137-CR Jurkat cells with CD137 by reporter assay

The CD137-CR3-copGFP Jurkat cells or the CD137-CR1 Jurkat cells prepared in Example 11-2 were co-cultured with tumor cells expressing a tumor antigen, and the ability to induce CD137 agonist activity to Jurkat cells in the presence of a bispecific antibody consisting of an antibody to a tumor antigen and an anti-CD137 antibody was analyzed. Here, human GPC3 was selected as a model tumor antigen, the bispecific antibody consisting of an anti-GPC3 antibody and an anti-CD137 antibody and prepared in Example 2-2 (H0000-F760nN17/GL4-k0a//hCD137VH-F760mnP17/hCD137VL-k0) was used in the experiment.

For the tumor cell line expressing human GPC3, SK-pca60 cells were used in which human GPC3 was stably expressed on SK-HEP1 (ATCC) that is a human liver cancer cell line. First, SK-pca60 cells were inoculated at 10 µL/well in a 384-well flat-bottom plate (Corning, Inc.) (1E + 04 ($1 \times 10^4$) cells/well). Subsequently, CD137-CR3-copGFP Jurkat cells, CD137-CR1 Jurkat cells, and NF-kB-Luc2/4-1BB Jurkat cells expressing only copGFP were mixed at 5 µL/well (1E + 04 ($1 \times 10^4$) cells/well) for each of the types of cells. Subsequently, the bispecific antibody was added at 5 µL/well with a final concentration of 0 or 16 µg/mL. After 24 hours, the luciferase activity was measured using Bio-Glo™ Luciferase (Promega Corporation). The luciferase activity was measured by EnVision™ Xcite (PerkinElmer, Inc.). The results are shown in Figure 20.

As a result, agonist activity was detected in the presence of the bispecific antibody under any of the conditions. For the CD137-CR1, in particular, CD137 is fused as an intracellular domain, and agonist activity is considered to be induced not only via CD137 on the cell membrane, but also via CR subjected to gene introduction. On the other hand, in the NF-kB-Luc2/4-1BB Jurkat cells expressing CD137-CR3 in which CD137 is not fused as an intracellular domain and the NF-kB-Luc2/4-1BB Jurkat cells expressing only copGFP, agonist activity was considered to be induced via CD137 on the cell membrane. Thus, it was shown that in the system, an antigen-binding molecule having an agonist action enables agonist activity to be induced to cells expressing an endogenous immunoreceptor, regardless of whether or not CR was expressed.

[Example 12] Evaluation of ability to activate chimeric receptor-expressing cells having CD28 in extracellular region

(12-1) Construction of lentivirus vector

A lentivirus vector expressing a chimera receptor to which the extracellular domain of human CD28 (Genbank NM006139.4, Ash19 to Pro152) is fused was prepared. Two constructs were prepared, where one is a construct using human CD8 for a transmembrane domain (CD28-CR1) and the other is a construct using human CD28 (CD28-CR2) (Figure 21). pCDH-CMV-MCS-T2A-copGFP (System Biosciences) was used as a lentivirus vector skeleton. A gene encoding CD28-CR1 (SEQ ID NO: 34) and CD28-CR2 (SEQ ID NO: 35) were synthesized by a method known to those skilled in the art. This sequence was ligated into MCS of the pCDH-CMV-MCS-T2A-copGFP vector to construct a lentivirus vector.

(12-2) Introduction of gene into NFAT-RE-luc2 Jurkat cells

For analyzing the ability to activate T cells expressing CD28-CR1 or CD28-CR2 by an *in vitro reporter* assay, NFAT-RE-luc2 Jurkat cells (Promega Corporation) were transduced with the lentivirus vector constructed in Example 12-1.

Specifically, first, 293Ta cell line (Genecopoeia) was transfected with the CD28-CR1-copGFP or CD28-CR2-copGFP vector mentioned above and a packaging plasmid mix by Lenti-Pac™ HIV Expression Packaging Kit (Genecopoeia) to prepare lentivirus harboring the CD28-CR1-copGFP or CD28-CR2-copGFP vector. 48 hours to 72 hours after the transfection, a supernatant containing the lentivirus was recovered, and concentrated using a Lenti-X™ concentrator (Takara Bio Inc.).

Next, the recovered supernatant containing lentivirus and a polybrene solution (Nacalai Tesque, Inc.) were added to NFAT-RE-luc2 Jurkat cells, and the mixture was centrifuged at 3000 rpm for 60 minutes to obtain NFAT-RE-luc2 Jurkat cells transduced with the CD28-CR1-copGFP or CD28-CR2-copGFP vector. As a negative control for the experiment, NFAT-RE-luc2 Jurkat cells transduced with a pCDH-CMV-MCS-T2A-copGFP vector expressing only copGFP were also prepared.

(12-3) Confirmation of gene introduction efficiency

The gene introduction efficiency for the CD28-CR1-copGFP-expressing Jurkat cells and CD28-CR2-copGFP-expressing Jurkat cells prepared in Example 12-2 was analyzed by FACSAria™ III Cell Sorter (BD Biosciences) using the expression rate of coexpressed copGFP as an index. As a result, it was confirmed that the gene was introduced into approximately 40% of the cells for CD28-CR1, and the gene was introduced into approximately 20% of the cells for CD28-CR2.

(12-4) Evaluation of activation ability by reporter assay

The CD28-CR1-copGFP-expressing Jurkat cells and the CD28-CR2-copGFP-expressing Jurkat cells prepared in Example 12-2 were co-cultured with tumor cells expressing a tumor antigen, and the ability to activate Jurkat cells in the presence of a bispecific antibody consisting of an antibody to a tumor antigen and an anti-CD28 antibody was analyzed. Here, human GPC3 was selected as a model tumor antigen, and the bispecific antibody prepared in Example 2-2 and consisting of an anti-GPC3 antibody and an anti-CD28 antibody (H0000-F760mmN17/GL4-k0a//TGN1412VH-F760mnP17.v1/TGN1412VL-KT0.v1), and a bispecific antibody with the first or second arm substituted by Keyhole limpet hemocyanin (KLH) antibody (IC17HdK-F760mnN17/IC17L-k0//TGN1412VH-F760mnP17.v1/TGN1412VL-KT0.v1 or IC17HdK-F760mnN17/IC17L-k0//H0000-F760mnP17/GL4-k0a) as a negative control were used for the experiment.

For the tumor cell line expressing human GPC3, SK-pca60 cells were used in which human GPC3 was stably expressed on SK-HEP1 (ATCC) that is a human liver cancer cell line. First, SK-pca60 cells were inoculated at 10 μL/well in a 384-well flat-bottom plate (Corning, Inc.) (1E + 04 ($1 \times 10^4$) cells/well). Subsequently, CD28-CR1-copGFP Jurkat cells, CD28-CR2-copGFP Jurkat cells, or NFAT-RE-luc2 Jurkat cells expressing only copGFP without expressing a chimeric receptor as a negative control were mixed at 5 μL/well (1E + 04 ($1 \times 10^4$) cells/well). Subsequently, the various bispecific antibodies were each added at 5 μL/well with a final concentration of 0 or 10 μg/mL. After 24 hours, the luciferase activity was measured using Bio-Glo™ Luciferase (Promega Corporation). The luciferase activity was measured by EnVision™ Xcite (PerkinElmer, Inc.). The results are shown in Figure 22.

As is apparent from the results, both the CD28-CR1-copGFP Jurkat cell and the CD28-CR2-copGFP Jurkat cell were insignificantly activated in the case of not adding an antibody or in the case of adding the bispecific antibody as a negative control, and significantly activated only in the presence of the bispecific antibody consisting of an anti-GPC3 antibody and an anti-CD28 antibody. On the other hand, the NFAT-RE-luc2 Jurkat cells expressing only copGFP without expressing a chimeric receptor were not activated even in the presence of the bispecific antibody consisting of an anti-GPC3 antibody and an anti-CD28 antibody. This indicates that T cells expressing a chimeric receptor having CD28 in an extracellular region are activated in a manner dependent on a bispecific antibody, and express cytotoxic activity on cells expressing a tumor antigen to induce an antitumor effect.

[Table 5-1]

| SEQ ID NO: | Name | Full-length sequence |
|---|---|---|
| 1 | H0000 | QVQLVQSGAEVKKPGASVKVSCKASGYTFTDYEMHWIRQPPGQGLEWIGAIDPKTGDTAYSQKFKGRVTLTADKSTSTAYMELSSLTSEDTAVYYCTRFYSYTYWGQGTLVTVSS |
| 2 | GL4 | DIVMTQSPLSLPVTPGEPASISCRSSQSLVHSNRNTYLHWYQQKPGQAPRLLIYKVSNRFSGVPDRFSGSGSGTDFTLKISRVEAEDVGVYYCSQNTHVPPTFGQGTKLEIK |

(continued)

| SEQ ID NO: | Name | Full-length sequence |
|---|---|---|
| 3 | F760nN17 | ASTKGPSVFPLAPSSKSTSGGTAALGCLVKDYFPEPVTVSWNSGA LTSGVHTFPAVLQSSGLYSLSSVVTVPSSSLGTQTYICNVNHKPSN TKVDKKVEPKSCDKTHTCPPCPAPELRGGPKVFLFPPKPKDTLMIS RTPEVTCVVVDVSHEDPEVKFNWYVDGVEVHNAKTKPREEQYA STYRVVSVLTVLHQDWLNGKEYKCKVSNKALPAPIEKTISKAKG QPREPQVYTLPPSRDELTKNQVSLTCLVKGFYPSDIAVEWESNGQ PENNYKTTPPYLDSDGSFFLYSKLTVDKSRWQQGNVFSCSVMHE ALHNHYTQESLSLSP |
| 4 | k0a | RTVAAPSVFIFPPSDEQLKSGTASVVCLLNNFYPREAKVQWKVDN ALQSGNSQESVTEQDSKDSTYSLSSTLTLSKADYEKHKVYACEVT HQGLSSPVTKSFNRGEC |
| 5 | hCD137VH | QVQLQQWGAGLLKPSETLSLTCAVYGGSFSGYYWSWIRQSPEKG LEWIGEINHGGYVTYNPSLESRVTISVDTSKNQFSLKLSSVTAADT AVYYCARDYGPGNYDWYFDLWGRGTLVTVSS |
| 6 | hCD137VL | EIVLTQSPATLSLSPGERATLSCRASQSVSSYLAWYQQKPGQAPRL LIYDASNRATGIPARFSGSGSGTDFTLTISSLEPEDFAVYYCQQRSN WPPALTFGGGTKVEIK |
| 7 | F760mnP17 | ASTKGPSVFPLAPSSKSTSGGTAALGCLVKDYFPEPVTVSWNSGA LTSGVHTFPAVLQSSGLYSLSSVVTVPSSSLGTQTYICNVNHKPSN TKVDKKVEPKSCDKTHTCPPCPAPELRGGPKVFLFPPKPKDTLMIS RTPEVTCVVVDVSHEDPEVKFNWYVDGVEVHNAKTKPREEQYA STYRVVSVLTVLHQDWLNGKEYKCKVSNKALPAPIEKTISKAKG QPREPQVYTLPPSRKEMTKNQVSLTCLVKGFYPSDIAVEWESNGQ PENNYKTTPPYLDSDGSFFLYSKLTVDKSRWQQGNVFSCSVMHE ALHNHYTQKSLSLSP |
| 8 | k0 | RTVAAPSVFIFPPSDEQLKSGTASVVCLLNNFYPREAKVQWKVDN ALQSGNSQESVTEQDSKDSTYSLSSTLTLSKADYEKHKVYACEVT HQGLSSPVTKSFNRGEC |
| 9 | GPA0018H | QSVEESGGRLVTPGTPLTLTCTVSGFSLSSYAMSWVRQAPGKGLE WIGIINIRGSTYYASWAKGRFTISKTSTTVDLKITSPTTADTATYFC ARGYTAYSGAVSIWGPGTLVTVSS |
| 10 | GPA0018L | DVVMTQTPASVSEPVGGTVTIKCQASQNIYSNLAWYQQKPGQPP KLLIYAASNLASGVSSRFKGSGSGTQFTLTISDLECADSATYYCQC SDYGSSYVGAFGGGTEVVVK |

[Table 5-2]

| 11 | F760mnN17 | ASTKGPSVFPLAPSSKSTSGGTAALGCLVKDYFPEPVTVSWNSGA LTSGVHTFPAVLQSSGLYSLSSVVTVPSSSLGTQTYICNVNHKPSN TKVDKKVEPKSCDKTHTCPPCPAPELRGGPKVFLFPPKPKDTLMIS RTPEVTCVVVDVSHEDPEVKFNWYVDGVEVHNAKTKPREEQYA STYRVVSVLTVLHQDWLNGKEYKCKVSNKALPAPIEKTISKAKG QPREPQVYTLPPSREEMTKNQVSLTCLVKGFYPSDIAVEWESNGQ PENNYKTTPPYLDSDGSFFLYSKLTVDKSRWQQGNVFSCSVMHE ALHNHYTQESLSLSP |

(continued)

| 12 | k0C | RTVAAPSVFIFPPSDEQLKSGTASVVCLLNNFYPREAKVQWKVDN ALQSGNSQESVTEQDSKDCTYSLSSTLTLSKADYEKHKVYACEVT HQGLSSPVTKSFNRGEC |
|---|---|---|
| 13 | GPA0039H | QSVEESGGRLVTPGGTLTFTCTVSGFSLSSYAMGWVRQAPGEGLE YIGTIDTGGSAYYATWAKGRFTISKTSSTTVGLIMTSLTAADTATY FCARVADYNIGLRRLDLWGQGTLVTVSS |
| 14 | GPA0039L | ADVVMTQTPASVSEPVGGTVTIKCQASQYIYSNLAWYQQKPGQR PKLLIYTTSNLESGVPSRFRGSGSGTEFTLTISDLECADAATYYCQS AYYSSSYVFPFGGGTEVVVK |
| 15 | MRAH.v1 | QVQLQESGPGLVRPSQTLSLTCTVSGYSITSDHAWSWVRQPPGRG LEWIGYISYSGITTYNPSLKSRVTMLRDTSKNQFSLRLSSVTAADT AVYYCARSLARTTAMDYWGQGSLVTVSS |
| 16 | MRAL.v1 | DIQMTQSPSSLSASVGDRVTITCRASQDISSYLNWYQQKPGKAPK LLIYYTSRLHSGVPSRFSGSGSGTDFTFTISSLQPEDIATYYCQQGN TLPYTFGQGTKVEIK |
| 17 | k0.v1 | RTVAAPSVFIFPPSDEQLKSGTASVVCLLNNFYPREAKVQWKVDN ALQSGNSQESVTEQDSKDSTYSLSSTLTLSKADYEKHKVYACEVT HQGLSSPVTKSFNRGEC |
| 18 | IC17HdK | QVQLQQSGPQLVRPGASVKISCKASGYSFTSYWMHWVNQRPGQ GLEWIGMIDPSYSETRLNQKFKDKATLTVDKSSSTAYMQLSSPTS EDSAVYYCALYGNYFDYWGQGTTLTVSS |
| 19 | IC17L | DIQMTQSSSSFSVSLGDRVTITCKASEDIYNRLAWYQQKPGNAPR LLISGATSLETGVPSRFSGSGSGKDYTLSITSLQTEDVATYYCQQY WSTPYTFGGGTKLEVK |
| 20 | TGN1412VH | QVQLVQSGAEVKKPGASVKVSCKASGYTFTSYYIHWVRQAPGQG LEWIGCIYPGNVNTNYNEKFKDRATLTVDTSISTAYMELSRLRSD DTAVYFCTRSHYGLDWNFDVWGQGTTVTVSS |
| 21 | TGN1412VL | DIQMTQSPSSLSASVGDRVTITCHASQNTYVWLNWYQQKPGKAP KLLIYKASNLHTGVPSRFSGSGSGTDFTLTISSLQPEDFATYYCQQ GQTYPYTFGGGTKVEIK |
| 22 | F760mnP17.v1 | ASTKGPSVFPLAPSSKSTSGGTAALGCLVKDYFPEPVTVSWNSGA LTSGVHTFPAVLQSSGLYSLSSVVTVPSSSLGTQTYICNVNHKPSN TKVDKKVEPKSCDKTHTCPPCPAPELRGGPKVFLFPPKPKDTLMIS RTPEVTCVVVDVSHEDPEVKFNWYVDGVEVHNAKTKPREEQYA STYRVVSVLTVLHQDWLNGKEYKCKVSNKALPAPIEKTISKAKG QPREPQVYTLPPSRKEMTKNQVSLTCLVKGFYPSDIAVEWESNGQ PENNYKTTPPYLDSDGSFFLYSKLTVDKSRWQQGNVFSCSVMHE ALHNHYTQKSLSLSP |
| 23 | KT0.v1 | RTVAAPSVFIFPPSDEQLKSGTASVVCLLNNFYPREAKVQWKVDN ALQSGNSQESVTEQDSKDSTYSLSSTLTLSKADYEKHKVYACEVT HQGLSSPVTKSFNRGEC |

[Table 5-3]

| | | |
|---|---|---|
| 24 | CD137-CR1 | LQDPCSNCPAGTFCDNNRNQICSPCPPNSFSSAGGQRTCDICRQCK GVFRTRKECSSTSNAECDCTPGFHCLGAGCSMCEQDCKQGQELT KKGCKDCCFGTFNDQKRGICRPWTNCSLDGKSVLVNGTKERDVV CGPSPADLSPGASSVTPPAPAREPGHSPQAAAFVPVFLPAKPTTTP APRPPTPAPTIASQPLSLRPEACRPAAGGAVHTRGLDFACDIYIWA PLAGTCGVLLLSLVITLYCNHRNRSKRSRLLHSDYMNMTPRRPGP TRKHYQPYAPPRDFAAYRSRFSVVKRGRKKLLYIFKQPFMRPVQT TQEEDGCSCRFPEEEEGGCELRVKFSRSADAPAYQQGQNQLYNEL NLGRREEYDVLDKRRGRDPEMGGKPRRKNPQEGLYNELQKDKM AEAYSEIGMKGERRRGKGHDGLYQGLSTATKDTYDALHMQALP PR |
| 25 | trCD137-CR | LQDPCSNCPAGTFCDNNRNQICSPCPPNSFSSAGGQRTCDICRQCK GVFRTRKECSSTSNAECDAAAFVPVFLPAKPTTTPAPRPPTPAPTIA SQPLSLRPEACRPAAGGAVHTRGLDFACDIYIWAPLAGTCGVLLL SLVITLYCNHRNRSKRSRLLHSDYMNMTPRRPGPTRKHYQPYAPP RDFAAYRSRFSVVKRGRKKLLYIFKQPFMRPVQTTQEEDGCSCRF PEEEEGGCELRVKFSRSADAPAYQQGQNQLYNELNLGRREEYDV LDKRRGRDPEMGGKPRRKNPQEGLYNELQKDKMAEAYSEIGMK GERRRGKGHDGLYQGLSTATKDTYDALHMQALPPR |
| 26 | CD137-CR2 | LQDPCSNCPAGTFCDNNRNQICSPCPPNSFSSAGGQRTCDRCRQC KGRFRTRKECSSTSNAECDCTPGFHCLGAGCSMCEQDCKQGQEL TKKGCKDCCFGTFNDQKRGICRPWTNCSLDGKSVLVNGTKERDV VCGPSPADLSPGASSVTPPAPAREPGHSPQAAAFVPVFLPAKPTTT PAPRPPTPAPTIASQPLSLRPEACRPAAGGAVHTRGLDFACDIYIW APLAGTCGVLLLSLVITLYCNHRNRSKRSRLLHSDYMNMTPRRPG PTRKHYQPYAPPRDFAAYRSRFSVVKRGRKKLLYIFKQPFMRPVQ TTQEEDGCSCRFPEEEEGGCELRVKFSRSADAPAYQQGQNQLYNE LNLGRREEYDVLDKRRGRDPEMGGKPRRKNPQEGLYNELQKDK MAEAYSEIGMKGERRRGKGHDGLYQGLSTATKDTYDALHMQAL PPR |
| 33 | CD137-CR3 | LQDPCSNCPAGTFCDNNRNQICSPCPPNSFSSAGGQRTCDICRQCK GVFRTRKECSSTSNAECDCTPGFHCLGAGCSMCEQDCKQGQELT KKGCKDCCFGTFNDQKRGICRPWTNCSLDGKSVLVNGTKERDVV CGPSPADLSPGASSVTPPAPAREPGHSPQAAAFVPVFLPAKPTTTP APRPPTPAPTIASQPLSLRPEACRPAAGGAVHTRGLDFACDIYIWA PLAGTCGVLLLSLVITLYCNHRNRRVKFSRSADAPAYQQGQNQL YNELNLGRREEYDVLDKRRGRDPEMGGKPRRKNPQEGLYNELQ KDKMAEAYSEIGMKGERRRGKGHDGLYQGLSTATKDTYDALHM QALPPR |
| 34 | CD28-CR1 | NKILVKQSPMLVAYDNAVNLSCKYSYNLFSREFRASLHKGLDSA VEVCVVYGNYSQQLQVYSKTGFNCDGKLGNESVTFYLQNLYVN QTDIYFCKIEVMYPPPYLDNEKSNGTIIHVKGKHLCPSPLFPGPSKP AAAFVPVFLPAKPTTTPAPRPPTPAPTIASQPLSLRPEACRPAAGGA VHTRGLDFACDIYIWAPLAGTCGVLLLSLVITLYCNHRNRSKRSR LLHSDYMNMTPRRPGPTRKHYQPYAPPRDFAAYRSRFSVVKRGR KKLLYIFKQPFMRPVQTTQEEDGCSCRFPEEEEGGCELRVKFSRSA DAPAYQQGQNQLYNELNLGRREEYDVLDKRRGRDPEMGGKPRR KNPQEGLYNELQKDKMAEAYSEIGMKGERRRGKGHDGLYQGLS TATKDTYDALHMQALPPR |

[Table 5-4]

| 35 | CD28-CR2 | NKILVKQSPMLVAYDNAVNLSCKYSYNLFSREFRASLHKGLDSA VEVCVVYGNYSQQLQVYSKTGFNCDGKLGNESVTFYLQNLYVN QTDIYFCKIEVMYPPPYLDNEKSNGTIIHVKGKHLCPSPLFPGPSKP FWVLVVVGGVLACYSLLVTVAFIIFWVRSKRSRLLHSDYMNMTP RRPGPTRKHYQPYAPPRDFAAYRSRFSVVKRGRKKLLYIFKQPFM RPVQTTQEEDGCSCRFPEEEEGGCELRVKFSRSADAPAYQQGQNQ LYNELNLGRREEYDVLDKRRGRDPEMGGKPRRKNPQEGLYNELQ KDKMAEAYSEIGMKGERRRGKGHDGLYQGLSTATKDTYDALHM QALPPR |
|---|---|---|
| 137 | CD137 extracellular domain 1 | LQDPCSNCPAGTFCDNNRNQICSPCPPNSFSSAGGQRTCDICRQCK GVFRTRKECSSTSNAECDCTPGFHCLGAGCSMCEQDCKQGQELT KKGCKDCCFGTFNDQKRGICRPWTNCSLDGKSVLVNGTKERDVV CGPSPADLSPGASSVTPPAPAREPGHSPQ |
| 138 | CD 137 extracellular domain 2 | LQDPCSNCPAGTFCDNNRNQICSPCPPNSFSSAGGQRTCDICRQCK GVFRTRKECSSTSNAECDCTPGFHCLGAGCSMCEQDCKQGQELT KKGCKDCCFGTFNDQKRGICRPWTNCSLDGKSVLVNGTKERDVV CGPSPADLSPGASSVTPPAPARE |
| 139 | CD 137 extracellular domain 3 | SLQDPCSNCPAGTFCDNNRNQICSPCPPNSFSSAGGQRTCDICRQC KGVFRTRKECSSTSNAECDCTPGFHCLGAGCSMCEQDCKQGQEL TKKGCKDCCFGTFNDQKRGICRPWTNCSLDGKSVLVNGTKERDV VCGP |
| 140 | CD137 extracellular domain 4 | QDPCSNCPAGTFCDNNRNQICSPCPPNSFSSAGGQRTCDICRQCKG VFRTRKECSSTSNAECDCTPGFHCLGAGCSMCEQDCKQGQELTK KGCKDCCFGTFNDQKRGICRPWTNCSLDGKSVLVNGTKERDVVC GPSP |

**Claims**

1. A pharmaceutical composition comprising a cell expressing a chimeric receptor, for use in combination with the administration of an antigen-binding molecule, wherein

   the chimeric receptor comprises an extracellular domain, and the extracellular domain comprises an extracellular domain of an immunoreceptor, an extracellular domain variant of an immunoreceptor, or a fraction thereof, and the antigen-binding molecule is a multispecific antigen-binding molecule having a target antigen recognition site and an immunoreceptor recognition site which recognizes the immunoreceptor.

2. A pharmaceutical composition comprising an antigen-binding molecule, for use in combination with the administration of a cell expressing a chimeric receptor, wherein

   the chimeric receptor comprises an extracellular domain, and the extracellular domain comprises an extracellular domain of an immunoreceptor, an extracellular domain variant of an immunoreceptor, or a portion thereof, and the antigen-binding molecule is a multispecific antigen-binding molecule having a target antigen recognition site and an immunoreceptor recognition site which recognizes the immunoreceptor.

3. The pharmaceutical composition according to claim 1 or 2, wherein the immunoreceptor recognition site recognizes the extracellular domain of the chimeric receptor and an endogenous immunoreceptor.

4. The pharmaceutical composition according to any one of claims 1 to 3, wherein the extracellular domain of the chimeric receptor comprises an extracellular domain variant of an immunoreceptor, or a portion thereof, having attenuated binding to a ligand for the endogenous immunoreceptor.

5. The pharmaceutical composition according to any one of claims 1 to 4, wherein the immunoreceptor is a costimulatory molecule.

6. The pharmaceutical composition according to any one of claims 1 to 5, wherein the immunoreceptor is human CD137.

7. The pharmaceutical composition according to any one of claims 1 to 6, wherein the extracellular domain of the chimeric receptor comprises a human CD 137 extracellular domain variant, or a portion thereof, in which at least a part or all of a cysteine-rich domain 3 and a cysteine-rich domain 4 of a human CD137 extracellular domain are deleted.

8. The pharmaceutical composition according to any one of claims 1 to 7, wherein an intracellular signaling domain of the chimeric receptor comprises an intracellular signaling domain of CD3 zeta.

9. The pharmaceutical composition according to any one of claims 1 to 8, wherein the target antigen is a receptor, a tumor antigen, an MHC antigen, or a differentiation antigen.

10. The pharmaceutical composition according to any one of claims 1 to 8, wherein the target antibody is a tumor antigen.

11. The pharmaceutical composition according to any one of claims 1 to 10, wherein the antigen-binding molecule is a bispecific antibody.

12. The pharmaceutical composition according to any one of claims 1 to 11 for use in the treatment or prevention of a cancer.

13. A chimeric receptor comprising an extracellular domain comprising an extracellular domain of an immunoreceptor, an extracellular domain variant of an immunoreceptor, or a portion thereof.

14. The chimeric receptor according to claim 13, wherein the extracellular domain of the chimeric receptor comprises an extracellular domain variant of an immunoreceptor, or a portion thereof, having attenuated binding to a ligand for an endogenous immunoreceptor.

15. The chimeric receptor according to claim 13 or 14, wherein the immunoreceptor is a costimulatory molecule.

16. The chimeric receptor according to any one of claims 13 to 15, wherein the immunoreceptor is human CD137.

17. The chimeric receptor according to any one of claims 13 to 16, wherein the extracellular domain of the chimeric receptor is a human CD137 extracellular domain variant, or a portion thereof, in which at least a part or all of a cysteine-rich domain 3 and a cysteine-rich domain 4 of a human CD137 extracellular domain are deleted.

18. The chimeric receptor according to any one of claims 13 to 17, wherein an intracellular signaling domain of the chimeric receptor comprises an intracellular signaling domain of CD3 zeta.

19. A cell expressing the chimeric receptor according to any one of claims 13 to 18.

20. A composition comprising the cell according to claim 19.

21. A nucleic acid encoding the chimeric receptor according to any one of claims 13 to 18.

22. A vector harboring the nucleic acid according to claim 21.

23. A method for producing the cell according to claim 19, comprising transfecting or transducing a cell with the nucleic acid according to claim 21 or the vector according to claim 22.

# Fig. 1

ADMINISTRATION OF CHIMERIC RECEPTOR IN
COMBINATION WITH BISPECIFIC ANTIBODY

DAMAGE TO TARGET CELL

BISPECIFIC ANTIBODY

CHIMERIC
RECEPTOR-
EXPRESSING CELL

+

CHIMERIC RECEPTOR

ENDOGENOUS
IMMUNORECEPTOR

TARGET
CELL

TARGET
CELL

CHIMERIC
RECEPTOR-
EXPRESSING CELL

ENDOGENOUS
IMMUNOCYTE

# Fig. 2

CD137 EXTRACELLULAR DOMAIN

CD137-CR1 | CRD1 | CRD2 | CRD3 | CRD4 --- CD8 HINGE | CD8 TM | CD28 | CD137 | CD3zeta

trCD137-CR | CRD1 | CRD2 ......... CD8 HINGE | CD8 TM | CD28 | CD137 | CD3zeta

CD137-CR2 | CRD1 | CRD2** | CRD3 | CRD4 --- CD8 HINGE | CD8 TM | CD28 | CD137 | CD3zeta
        └── I64R/V71R mutations

# Fig. 3

pCDH CD137-CR1-
EXPRESSING
LENTIVIRUS VECTOR

5' LTR

3' LTR

copGFP

T2A

SIGNAL SEQUENCE

CD137 EXTRACELLULAR DOMAIN

CD8a

CD28 INTRACELLULAR DOMAIN

CD137 INTRACELLULAR DOMAIN

CD3zeta INTRACELLULAR DOMAIN

# Fig. 4

CO-CULTURE WITH SK-pca60 CELL

EMISSION INTENSITY (a.u.)

BISPECIFIC ANTIBODY CONCENTRATION (microg/mL)

····■···· copGFP

——●—— CD137-CR

# Fig. 5

# Fig. 6

## Fig. 7

pMSGV1 CD137-CR1-EXPRESSING RETROVIRUS VECTOR

SIGNAL SEQUENCE
CD137 EXTRACELLULAR DOMAIN
CD8a
CD28 INTRACELLULAR DOMAIN
CD137 INTRACELLULAR DOMAIN
CD3zeta INTRACELLULAR DOMAIN
F2A
eGFP

## Fig. 8A

# Fig. 8B

# Fig. 9

LIGAND-EXPRESSING CELL

CHIMERIC RECEPTOR-
EXPRESSING CELL

Fig. 10

CO-CULTURE WITH SK-pca60 CELL

Fig. 11

CO-CULTURE WITH SK-pca60 CELL

## Fig. 12A

## Fig. 12B

Fig. 12C

Fig. 13

## Fig. 14

## Fig. 15

Fig. 16A

Fig. 16B

# Fig. 17

# Fig. 18

# Fig. 19

# Fig. 20

# Fig. 21

# Fig. 22

## INTERNATIONAL SEARCH REPORT

| International application No. |
|---|
| **PCT/JP2021/028230** |

**A.    CLASSIFICATION OF SUBJECT MATTER**

*A61K 35/12*(2015.01)i; *A61K 39/395*(2006.01)i; *A61P 35/00*(2006.01)i; *A61P 43/00*(2006.01)i; *C07K 14/705*(2006.01)i; *C07K 19/00*(2006.01)i; *C12N 1/15*(2006.01)i; *C12N 1/19*(2006.01)i; *C12N 1/21*(2006.01)i; *C12N 5/10*(2006.01)i; *C12N 15/12*(2006.01)i; *C12N 15/62*(2006.01)i; *C12N 15/63*(2006.01)i

FI:    A61K35/12; C12N15/62 Z; C12N15/12; C12N15/63 Z; C12N1/15; C12N1/19; C12N1/21; A61P35/00; A61P43/00 105; C07K19/00; C07K14/705; A61K39/395 T; C12N5/10 ZNA

According to International Patent Classification (IPC) or to both national classification and IPC

**B.    FIELDS SEARCHED**

Minimum documentation searched (classification system followed by classification symbols)

A61K35/12; A61K39/395; A61P35/00; A61P43/00; C07K14/705; C07K19/00; C12N1/15; C12N1/19; C12N1/21; C12N5/10; C12N15/12; C12N15/62; C12N15/63

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/MEDLINE/EMBASE/BIOSIS (STN), JSTPlus/JMEDPlus/JST7580 (JDreamIII)

**C.    DOCUMENTS CONSIDERED TO BE RELEVANT**

| Category* | Citation of document, with indication, where appropriate, of the relevant passages | Relevant to claim No. |
|---|---|---|
| X | WO 2019/116046 A1 (AUTOLUS LIMITED) 20 June 2019 (2019-06-20) | 13, 15-16, 19-23 |
| | claims 1, 25, page 9, lines 27, 28 | |
| A | | 1-12, 14, 17-18 |
| X | WO 2019/129220 A1 (NANJING LEGEND BIOTECH CO., LTD.) 04 July 2019 (2019-07-04) | 13, 15, 18-23 |
| | claims 1, 28, paragraph [0031], fig. 1 | |
| A | | 1-12, 14, 16-17 |
| A | JP 2019-537429 A (MERUS N.V.) 26 December 2019 (2019-12-26) | 1-23 |
| | claims 1, 21, 34, 35 | |
| A | WO 2015/156268 A1 (CHUGAI PHARMACEUTICAL CO., LTD.) 15 October 2015 (2015-10-15) | 1-23 |
| | claims 1, 4, 40 | |

☑ Further documents are listed in the continuation of Box C.       ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **02 September 2021** | **28 September 2021** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

**EP 4 197 545 A1**

## INTERNATIONAL SEARCH REPORT

<table>
<tr><td colspan="3">International application No.<br>**PCT/JP2021/028230**</td></tr>
</table>

**C. DOCUMENTS CONSIDERED TO BE RELEVANT**

| Category* | Citation of document, with indication, where appropriate, of the relevant passages | Relevant to claim No. |
|---|---|---|
| A | JP 2020-504132 A (THE GENERAL HOSPITAL CORP.) 06 February 2020 (2020-02-06) claims | 1-23 |

Form PCT/ISA/210 (second sheet) (January 2015)

# EP 4 197 545 A1

## INTERNATIONAL SEARCH REPORT

International application No.

**PCT/JP2021/028230**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

INTERNATIONAL SEARCH REPORT
Information on patent family members

International application No.

**PCT/JP2021/028230**

| Patent document cited in search report | | | Publication date (day/month/year) | Patent family member(s) | | | Publication date (day/month/year) |
|---|---|---|---|---|---|---|---|
| WO | 2019/116046 | A1 | 20 June 2019 | CN | 111479918 | A | |
| WO | 2019/129220 | A1 | 04 July 2019 | CA | 3086932 | A | |
| | | | | TW | 201930342 | A | |
| JP | 2019-537429 | A | 26 December 2019 | US | 2020/0017595 | A1 | |
| | | | | claims 1, 21, 34, 35 | | | |
| | | | | WO | 2018/056821 | A1 | |
| | | | | EP | 3515951 | A1 | |
| | | | | KR | 10-2019-0065318 | A | |
| | | | | CN | 109983033 | A | |
| WO | 2015/156268 | A1 | 15 October 2015 | US | 2017/0022287 | A1 | |
| | | | | claims 1, 40 | | | |
| | | | | EP | 3130606 | A1 | |
| | | | | KR | 10-2016-0142332 | A | |
| | | | | CN | 106459206 | A | |
| JP | 2020-504132 | A | 06 February 2020 | US | 2019/0328787 | A1 | |
| | | | | claims | | | |
| | | | | WO | 2018/132513 | A1 | |
| | | | | EP | 3568406 | A1 | |
| | | | | CN | 110267973 | A | |

Form PCT/ISA/210 (patent family annex) (January 2015)

## REFERENCES CITED IN THE DESCRIPTION

### Patent documents cited in the description

- WO 2012082841 A
- WO 2016040441 A
- WO 2017161333 A
- WO 2018002358 A
- WO 2018177966 A
- WO 2019140425 A
- WO 2004044011 A
- WO 2005040229 A
- WO 2002032925 A
- WO 1995001937 A
- WO 2002020565 A
- WO 2003029462 A
- WO 2008016854 A
- WO 2015156268 A
- WO 2009125825 A
- WO 2003000883 A
- WO 2004022754 A
- WO 2006006693 A
- WO 2003104453 A
- WO 9411523 A
- EP 239400 A
- WO 1996002576 A
- WO 1993012227 A
- WO 1992003918 A
- WO 1994002602 A
- WO 1994025585 A
- WO 1996034096 A
- WO 1996033735 A
- WO 1992001047 A
- WO 1992020791 A
- WO 1993006213 A
- WO 1993011236 A
- WO 1993019172 A
- WO 1995001438 A
- WO 1995015388 A
- WO 1988001649 A
- US 4946778 A
- US 5260203 A
- WO 2006132352 A
- WO 2009011941 A
- WO 2013047752 A
- WO 2000042072 A
- WO 2006019447 A
- WO 9308829 A
- US 5731168 A
- WO 2009089004 A1
- US 4676980 A
- US 20060025576 A1
- WO 2006106905 A
- WO 1996027011 A
- US 20130336973 A
- WO 2011028952 A
- WO 2014018572 A
- WO 2008119353 A
- WO 2011131746 A
- WO 2012058768 A
- WO 2013063702 A
- WO 2012023053 A
- WO 2007114325 A
- WO 98050431 A
- WO 95033844 A
- WO 2004065611 A
- WO 2016047722 A1
- WO 9317706 A
- EP 2101823 B
- WO 2013120629 A
- US 4945050 A
- US 3773719 A
- EP 58481 A
- EP 133988 A
- WO 9610038 A
- WO 9718185 A
- WO 9725329 A
- WO 9730170 A
- WO 9731934 A
- US 5278056 A
- US 5399346 A, Anderson
- US 4650764 A, Temin
- US 5124263 A, Temin
- WO 9507358 A, Dougherty
- US 7435596 B
- US 8026097 B
- US 20140080153 A1

### Non-patent literature cited in the description

- **JUNE CH ; SADELAIN M.** *N Engl J Med.,* 2018, vol. 379, 64
- **TAMADA et al.** *Clin Cancer Res.,* 2012, vol. 18, 6436

- URBANSKA K et al. *J Transl Med.,* 2014, vol. 12, 347
- KUDO K et al. *Cancer Res.,* 2014, vol. 74, 93
- KARCHES CH et al. *Clin Cancer Res.,* 2019, vol. 25, 5890
- HODI FS et al. *N Engl J Med.,* 2010, vol. 363, 711
- ROBERT C et al. *N Engl J Med.,* 2015, vol. 372, 320
- HOUOT R et al. *Blood,* 2009, vol. 114, 3431
- MARDIANA S et al. *Cancer Res.,* 2017, vol. 77, 1296
- KUNKEL et al. *Proc. Natl. Acad. Sci. USA,* 1985, vol. 82, 488-492
- *Annu. Rev. Biophys. Biomol. Struct.,* 2006, vol. 35, 225-249
- *Proc. Natl. Acad. Sci. U.S.A.,* 2003, vol. 100 (11), 6353-6357
- KINDT et al. Kuby Immunology. W.H. Freeman and Co, 2007, 91
- CHOTHIA ; LESK. *J. Mol. Biol.,* 1987, vol. 196, 901-917
- KABAT et al. Sequences of Proteins of Immunological Interest. Public Health Service, National Institutes of Health, 1991
- MACCALLUM et al. *J. Mol. Biol.,* 1996, vol. 262, 732-745
- MULLBERG et al. *J. Immunol.,* 1994, vol. 152 (10), 4958-4968
- Hormones and their Actions Part II. New Comprehensive Biochemistry. Elsevier Science Publishers, 1988, vol. 18B, 1-46
- Adhesion Factor Handbook. Cell Technology suppl. Handbook series. Gakken Medical Shujunsha Co., Ltd, 1994
- PATTHY. *Cell,* 1990, vol. 61 (1), 13-14
- ULLRICH et al. *Cell,* 1990, vol. 61 (2), 203-212
- *Cell,* 1992, vol. 69 (6), 1067-1070
- MIYAJIMA et al. *Annu. Rev. Immunol.,* 1992, vol. 10, 295-331
- TAGA et al. *FASEB J.,* 1992, vol. 6, 3387-3396
- FANTL et al. *Annu. Rev. Biochem.,* 1993, vol. 62, 453-481
- SMITH et al. *Cell,* 1994, vol. 76 (6), 959-962
- FLOWER DR. *Biochim. Biophys. Acta,* 1999, vol. 1422 (3), 207-234
- *Blood,* 1990, vol. 76 (1), 31-35
- *Cell,* 1989, vol. 57 (2), 277-285
- *Proc. Natl. Acad. Sci. USA.,* 1990, vol. 87 (22), 8702-8706
- *Cell,* 1990, vol. 61 (2), 341-350
- *Proc Natl Acad Sci USA.,* 1992, vol. 89 (12), 5640-5644
- *EMBO J.,* 1993, vol. 12 (7), 2645-53
- *Nature,* 1985, vol. 313 (6005), 756-761
- *Proc. Natl. Acad. Sci. USA.,* 1994, vol. 91 (2), 459-463
- *Proc. Natl. Acad. Sci. USA.,* 1988, vol. 85 (10), 3435-3439
- *Cell,* 1990, vol. 60 (2), 225-234
- *Cell,* 1994, vol. 77 (3), 391-400
- *Int J Cancer,* 2003, vol. 103 (4), 455-65
- *Proc Natl Acad Sci USA.,* 1989, vol. 86 (1), 27-31
- SASTRY et al. *J Virol,* 2011, vol. 85 (5), 1935-1942
- SERGEEVA et al. *Bood,* 2011, vol. 117 (16), 4262-4272
- VERMA et al. *J Immunol,* 2010, vol. 184 (4), 2156-2165
- WILLEMSEN et al. *Gene Ther,* 2001, vol. 8 (21), 1601-1608
- DAO et al. *Sci Transl Med,* 2013, vol. 5 (176), 176ra33
- TASSEV et al. *Cancer Gene Ther,* 2012, vol. 19 (2), 84-100
- *CHEMICAL ABSTRACTS,* 934823-49-1
- Epitope Mapping Protocols in Methods. Molecular Biology. 1996, vol. 66
- CHEN et al. *J. Mol. Biol.,* 1999, vol. 293, 865-881
- *Methods Enzymol.,* 2000, vol. 323, 325-40
- Antibodies: A Laboratory Manual. Cold Spring Harbor Laboratory, 1988, 359-420
- *J Clin Invest.,* August 1953, vol. 32 (8), 746-768
- *Antibodies,* 2015, vol. 4 (3), 141-156
- *J Pharm Sci.,* October 2008, vol. 97 (10), 4167-83
- *Mol Cell Biol.,* 1988, vol. 8, 466-472
- Current protocols. Molecular Biology. Publish. John Wiley & amp; Sons, 1987
- SAMBROOK, J. et al. Molecular Cloning. Cold Spring Harbor Lab. Press, 1989, 9.47-9.58
- *J. Immunol.,* 1979, vol. 123 (4), 1548-1550
- Current Topics. *Microbiology and Immunology,* 1978, vol. 81, 1-7
- *C. Eur. J. Immunol.,* 1976, vol. 6 (7), 511-519
- *Cell,* 1976, vol. 8 (3), 405-415
- *Nature,* 1978, vol. 276 (5685), 269-270
- *J. Immunol. Methods,* 1980, vol. 35 (1-2), 1-21
- *J. Exp. Med.,* 1978, vol. 148 (1), 313-323
- *Nature,* 1979, vol. 277 (5692), 131-133
- KOHLER ; MILSTEIN et al. *Methods Enzymol.,* 1981, vol. 73, 3-46
- VANDAMME et al. *Eur. J. Biochem.,* 1990, vol. 192 (3), 767-775
- *Biochemistry,* 1979, vol. 18 (24), 5294-5299
- *Anal. Biochem.,* 1987, vol. 162 (1), 156-159
- *Proc. Natl. Acad. Sci. USA,* 1988, vol. 85 (23), 8998-9002

- *Nucleic Acids Res.,* 1989, vol. 17 (8), 2919-2932
- *Bio/Technology,* 1994, vol. 12 (7), 699-702
- **SATO, K. et al.** *Cancer Res.,* 1993, vol. 53, 851-856
- *Science,* 1988, vol. 240 (4855), 1038-1041
- **PLUCKTHUN.** The Pharmacology of Monoclonal Antibodies. Springer-Verlag, 1994, vol. 113, 269-315
- *Proc. Natl. Acad. Sci. U.S.A.,* 1988, vol. 85 (16), 5879-5883
- *J Immunol. Methods,* 1999, vol. 231 (1-2), 177-189
- *Journal of Immunology,* 1994, vol. 152 (11), 5368-5374
- *Protein Engineering,* 1996, vol. 9 (3), 299-305
- *Proc. Natl. Acad. Sci. USA,* 2006, vol. 103 (11), 4005-4010
- **DAVIS et al.** *Immunological Reviews,* 2002, vol. 190, 123-136
- *J. Rheumatol,* 2007, vol. 34, 11
- *Hum. Antibod. Hybridomas,* 1990, vol. 1 (1), 47-54
- *Blood,* 2007, vol. 109, 1185-1192
- **CLARK, M.** *Chemical Immunology,* 1997, vol. 65, 88-110
- **GREENWOOD J ; CLARK M ; WALDMANN H.** *Eur. J. Immunol.,* 1993, vol. 23, 1098-1104
- **MORGAN A ; JONES ND ; NESBITT AM ; CHAPLIN L ; BODMER MW ; EMTAGE JS.** *Immunology,* 1995, vol. 86, 319-324
- **MILSTEIN C et al.** *Nature,* 1983, vol. 305, 537-540
- **MILSTEIN ; CUELLO.** *Nature,* 1983, vol. 305, 537
- **TRAUNECKER et al.** *EMBO J.,* 1991, vol. 10, 3655
- **BRENNAN et al.** *Science,* 1985, vol. 229, 81
- **KOSTELNY et al.** *J. Immunol.,* 1992, vol. 148 (5), 1547-1553
- **HOLLINGER et al.** *Proc. Natl. Acad. Sci. USA,* 1993, vol. 90, 6444-6448
- **GRUBER et al.** *J. Immunol.,* 1994, vol. 152, 5368
- **TUTT et al.** *J. Immunol.,* 1991, vol. 147, 60
- **RIDGWAY JB et al.** *Protein Engineering,* 1996, vol. 9, 617-621
- **MERCHANT AM et al.** *Nature Biotechnology,* 1998, vol. 16, 677-681
- *Protein Engineering Design & Selection,* 2010, vol. 23, 195-202
- *Nat Biotechnol.,* February 2014, vol. 32 (2), 191-8
- **CHRISTOPH et al.** *Nature Biotechnology,* 2013, vol. 31, 753-758
- *Nature Biotechnology,* 1998, vol. 16, 677-681
- **HASHIMOTO-GOTOH ; T, MIZUNO ; T, OGASA-HARA, Y ; NAKAGAWA, M.** An oligodeoxyribonucleotide-directed dual amber method for site-directed mutagenesis. *Gene,* 1995, vol. 152, 271-275
- **ZOLLER, MJ ; SMITH, M.** Oligonucleotide-directed mutagenesis of DNA fragments cloned into M13 vectors. *Methods Enzymol.,* 1983, vol. 100, 468-500
- **KRAMER, W ; DRUTSA, V ; JANSEN, HW ; KRAMER, B ; PFLUGFELDER, M ; FRITZ, HJ.** The gapped duplex DNA approach to oligonucleotide-directed mutation construction. *Nucleic Acids Res.,* 1984, vol. 12, 9441-9456
- **KRAMER W ; FRITZ HJ.** Oligonucleotide-directed construction of mutations via gapped duplex DNA Methods. *Enzymol,* 1987, vol. 154, 350-367
- **KUNKEL, TA.** Rapid and efficient site-specific mutagenesis without phenotypic selection. *Proc Natl Acad Sci USA.,* 1985, vol. 82, 488-492
- **MARK, D. F. et al.** *Proc. Natl. Acad. Sci. USA,* 1984, vol. 81, 5662-6
- **ZOLLER, M. J. ; SMITH, M.** *Nucleic Acids Res.,* 1982, vol. 10, 6487-500
- **WANG, A. et al.** *Science,* 1984, vol. 224, 1431-3
- **DALBADIE-MCFARLAND, G. et al.** *Proc. Natl. Acad. Sci. USA,* 1982, vol. 79, 6409-13
- **KARLIN ; ALTSCHUL.** *Proc. Natl. Acad. Sci. USA,* 1993, vol. 90, 5873-7
- **ALTSCHUL et al.** *J. Mol. Biol.,* 1990, vol. 215, 403-10
- **ADOLPH.** Viral Genome Method. CRC Press, 1996
- Remington's Pharmaceutical Science. 1980
- *J. Biomed. Mater. Res.,* 1981, vol. 15, 267-277
- *Chemtech.,* 1982, vol. 12, 98-105
- *Biopolymers,* 1983, vol. 22, 547-556
- **CHIN SM et al.** *Nat Commun.,* 2018, vol. 9, 4679
- **BATZER et al.** *Nucleic Acid Res.,* 1991, vol. 19, 5081
- **OHTSUKA et al.** *J. Biol. Chem.,* 1985, vol. 260, 2605-2608
- **ROSSOLINI et al.** *Mol. Cell. Probes,* 1994, vol. 8, 91-98
- **MILONE et al.** *Mol. Ther.,* 2009, vol. 17 (8), 1453-1464
- *Nature,* 1989, vol. 338, 383-384
- *J Immunol.,* 1999, vol. 162 (2), 897-902
- **SCHMITZ J et al.** *J Immunol.,* 2000, vol. 164, 848-54
- **TOMIDA M et al.** *Blood,* 1999, vol. 93, 1934-41
- **LOZZIO et al.** *Blood,* 1975, vol. 45 (3), 321-334
- **KLEIN et al.** *Int. J. Cancer,* 1976, vol. 18, 421-431

- **FEHNIGER T A ; CALIGIURI M A.** *Int Rev Immunol,* 2001, vol. 20 (3-4), 503-534
- **HARADA H et al.** *Exp Hematol,* 2004, vol. 32 (7), 614-621
- **HARADA H. et al.** *Jpn. J. Cancer Res,* 2002, vol. 93, 313-319
- Suicide Gene Therapy: Methods and Reviews, Springer, Caroline J. Humana Press, 2004
- *Proceedings of the National Academy of Sciences of the United States of America,* 1988, vol. 85, 6460-6464
- **MANN et al.** *Cell,* 1983, vol. 33, 153
- **KUO et al.** *Blood,* 1993, vol. 82, 845
- **TORIKAI et al.** *Blood,* 2012, vol. 119, 5697-5705
- Remington's Pharmaceutical Sciences. Mack Pub. Co, 1991
- **GRUPP et al.** *N. Engl. J. Med.,* 2013, vol. 368 (16), 1509-1518
- Physician's Desk Reference. Thomson P D R, 2005
- Remington's The Science and Practice of Pharmacy. Lippincott Williams and Wilkins, 2000
- Harrison's Principles of Internal Medicine. McGraw Hill, 2001
- The Merck Manual of Diagnosis and Therapy. Merck Research Laboratories, 1992
- *Protein Science,* 1995, vol. 4, 2411-2423
- **LABRIJN et al.** *Proc. Natl. Acad. Sci.,* 2013, vol. 110, 5145
- **TAMADA K et al.** *Clin Cancer Res,* 2012, vol. 18, 6436-6445